# EUROPEAN PATENT APPLICATION

(11) **EP 4 129 064 A1**
(43) Date of publication of application: **08.02.2023**
(21) Application number: 21782374.9
(22) Date of filing: 30.03.2021
(51) Int. Cl.: A01N 37/46, C07K 7/06, A61K 38/08, A61P 31/00, A61K 8/64, A23K 20/147, A23L 33/18

(54) **NOVEL ANTIBACTERIAL PEPTIDE AND USE THEREOF**

(30) Priority: 30.03.2020 KR 20200038642
(71) Applicant: Anygen Co., Ltd., Gwangju 61008 (KR)
(72) Inventor: KIM, Jae Il, Gwangju 62254 (KR); RYU, Jae Ha, Gwangju 61008 (KR); KIM, Shang Hyeon, Daejeon 35216 (KR); LEE, Sol, Suwon-si, Gyeonggi-do 16517 (KR)
(74) Representative: HGF
(86) International application number: PCT/KR2021/003939
(87) International publication number: WO 2021/201570

(57) **Abstract**

The present invention relates to a novel antibacterial peptide and a composition comprising the same as an active ingredient. The novel antibacterial peptide of the present invention exhibits excellent antibacterial activity against antibiotic-resistant bacteria as well as Gram-positive bacteria and Gram-negative bacteria and is low in cytotoxicity and, as such, can be advantageously utilized as an active ingredient in an antibiotic, a cosmetic composition, a food additive, a feed additive, a biotic pesticide, a quasi-drug product, and the like.

## Description

### Technical Field

The present invention relates to a novel antibacterial peptide and a composition comprising the same as an active ingredient.

### Background Art

Bacterial infection is one of the most common and fatal causes of human disease. Unfortunately, however, due to the abuse of antibiotics, bacteria resistant to antibiotics have emerged. In fact, the rate at which bacteria develop resistance to new antibiotics is much faster than the rate at which new antibiotics are developed. For example, life-threatening bacterial species such as *Enterococcus faecalis, Pseudomonas aeruginosa* and *Klebsiella pneumoniae* are resistant to all known antibiotics to date.

On the other hand, antibiotic tolerance was first discovered in *Pneumococcus sp.* in the 1970s and provided important clues about the mechanism of action of penicillin. Species that are resistant to antibiotics stop growing in the presence of normal concentrations of antibiotics, but do not die as a result. Tolerance occurs because the activity of bacterial autolytic enzymes such as autolysin does not occur when antibiotics inhibit cell wall synthesis enzyme. This fact is that penicillin kills bacteria by activating endogenous hydrolase, and the bacteria can also survive even when treated with antibiotic by inhibiting their activity (KR 10-2039400 B1).

It is clinically very important for bacteria to have tolerance to various antibiotics, because if it becomes impossible to eliminate the bacteria that have tolerance to antibiotics, the effectiveness of antibiotic treatment in case of bacterial infection is reduced. In addition, the development of tolerance is considered to be a prerequisite for the development of resistance to antibiotics, since this results in the generation of strains that survive despite antibiotic treatment. These strains acquire new genetic elements that are resistant to antibiotics and continue to grow in the presence of antibiotics. In fact, all bacteria that are resistant to antibiotics are known to have tolerance as well, so it is necessary to develop novel antibiotics that can kill these antibiotic-resistant bacteria.

On the other hand, bacteria can kill neighboring bacteria by synthesizing peptides or small organic molecules. Animals, including insects, produce naturally occurring peptide antibiotics, which are structurally divided into three groups. The first is a cysteine-rich β-sheet peptide, the second is an α-helical amphiphilic molecule, and the third is a proline-rich peptide. These antibacterial peptides are known to play important roles in host defense and innate immune system. These antibacterial peptides have various structures depending on the amino acid sequence. Among these structures, the antibacterial peptide mBjAMPI found in an amphioxus forms an amphiphilic alpha helical structure.

### Prior Art Document

### Patent Document

(Patent Document 1) KR 10-2039400 B1

### Detailed Description of Invention

### Technical Problem

The present inventors have completed the present invention by confirming that a peptide consisting of a specific amino acid sequence exhibits antibacterial activity, and in particular, exhibits antibacterial activity against bacteria that are resistant to antibiotics. Specifically, it is an object of the present invention to provide an antibacterial peptide containing three tryptophans (Trp).

### Solution to Problem

In order to achieve the above object, in one aspect of the present invention, there is provided an antibacterial peptide containing three tryptophans (Trp) or a salt thereof.

In another aspect of the present invention, there is provided an antibacterial peptide represented by Structural Formula I or Structural Formula II below or a salt thereof:

[Structural Formula I] N'-A-B-C-C'

[Structural Formula II] N'-C-B-A-C'

in which,
N' is the N-terminus of the antibacterial peptide,
C' is the C-terminus of the antibacterial peptide,
the C-terminus is one in which a carboxy group or hydroxy (-OH) of the carboxy group is substituted with an amine group (-NH₂),
A consists of the amino acid sequence of (X₁)ₐ-(X₂)_{b}-(X₃)_{c}-(X₄)_{d},
wherein a, b, c and d are each independently 0 or 1, and
X₁ to X₄ are each independently any one amino acid selected from the group consisting of Arg, Lys, Asn, Gln, Asp, Val, Leu, Ser, His, Gly and Tyr, and
B consists of the amino acid sequence of Trp-(Z₁)ₑ-(Z₂)_{f}-Trp-(Z₃)_{g}-(Z₄)ₕ-Trp,
wherein e, f, g and h are each independently 0 or 1, and
Z₁ to Z₄ are each independently any one amino acid selected from the group consisting of Val, Leu, Ile, Gly, Ala, Ser, Phe, Tyr, Trp, Lys and His, and
C consists of the amino acid sequence of (X₅)ᵢ-(X₆)ⱼ-(X₇)ₖ-(X₈)ₗ,
wherein i, j, k and 1 are each independently 0 or 1, and
X₅ to X₈ are each independently any one amino acid selected from the group consisting of Arg, Lys, Asn, Gln, Asp, Val, Leu, Ser, His, Gly and Tyr, and
wherein Trp may be substituted with C₁₋₆ alkoxy or halogen, or nitrogen (N) in the indole ring of Trp may be modified with sulfur (S), and
when an amino acid at the N-terminus is Lys, a C₃ to C₁₀ fatty acid may be further bound to an amine group,
Tyr may be substituted with halogen, and
Asn may be glycosylated.

In another aspect of the present invention, there is provided an antibacterial peptide or a salt thereof consisting of any one amino acid sequence selected from the group consisting of: Trp-Leu-Leu-Trp-Ile-Ala-Leu-Arg-Lys-Lys-Arg, Trp-Leu-Leu-Trp-Ile-Gly-Leu-Arg-Lys-Lys-Arg,
Trp-Leu-Leu-Trp-Ile-Ala-Leu-Arg-Lys-Lys,
Lys-Trp-Leu-Leu-Trp-Ile-Gly-Leu-Arg-Lys-Lys-Arg,
Trp-Ala-Ala-Trp-Ile-Gly-Leu-Arg-Lys-Lys-Arg,
Trp-Leu-Val-Trp-Ile-Gly-Leu-Arg-Lys-Lys-Arg,
Trp-Leu-Ile-Trp-Ile-Gly-Leu-Arg-Lys-Lys-Arg,
Trp-Leu-Phe-Trp-Ile-Gly-Leu-Arg-Lys-Lys-Arg,
Trp-Leu-Tyr-Trp-Ile-Leu-Leu-Arg-Lys-Lys-Arg,
Trp-Leu-Val-Trp-Ile-Tyr-Leu-Arg-Lys-Lys-Arg,
Trp-Leu-Val-Trp-Val-Tyr-Leu-Arg-Lys-Lys-Arg,
Trp-Leu-Val-Trp-Ile-Tyr-Val-Arg-Lys-Lys-Arg, Trp-Leu-Val-Trp-Ile-Tyr-Arg-Lys-Lys-Arg, Trp-Leu-Ile-Trp-Val-Tyr-Arg-Lys-Lys-Arg, Trp-Leu-Val-Trp-Ile-Tyr-Arg-Arg-Arg, Trp-Val-Val-Val-Val-Trp-Arg-Arg-Arg, Trp-Val-Val-His-Val-Val-Trp-Arg-Arg-Arg, Arg-Arg-Arg-His-Val-His-Val-Val-Trp-Lys,
Trp-Leu-Leu-Trp-Ile-Gly-Leu-Arg-Lys-Lys-Arg-Tyr,
Trp-Leu-Val-Tyr-Val-Trp-Leu-Arg-Lys-Lys-Arg,
Ser-Trp-Leu-Leu-Trp-Ile-Gly-Leu-Arg-Lys-Lys-Arg,
Trp-Val-Val-His-Val-Val-Trp-Arg-Arg-Arg-Asn (glycosylated), Arg-Arg-Arg-Trp-Val-Val-Val-Val-Val-Trp,
Trp-Leu-Leu-Trp-Ile-Ala-Leu-Arg-Lys-Lys-Arg,
Trp-Leu-Leu-Trp-Ile-Gly-Leu-Arg-Lys-Lys, Leu-Leu-Trp-Ile-Ala-Leu-Arg-Lys-Lys-Arg, Leu-Leu-Trp-Ile-Ala-Leu-Lys-Lys-Lys-Lys, Leu-Leu-Trp-Ile-Gly-Leu-Arg-Lys-Lys-Arg,
Tyr-Trp-Leu-Leu-Trp-Ile-Gly-Leu-Arg-Lys-Lys-Arg,
Trp-Leu-Leu-Trp-Val-Tyr-Val-Arg-Lys-Lys-Arg,
Trp-Leu-Trp-Val-Trp-Val-Arg-Lys-Lys-Arg,
Val-Trp-Val-Trp-Val-Trp-Val-Arg-Lys-Lys-Arg,
Trp-Val-Val-Trp-Val-Tyr-Val-Arg-Lys-Lys-Arg,
Trp-Val-Val-Trp-Val-Val-Tyr-Arg-Lys-Lys-Arg,
Trp-Leu-Ala-Trp-Leu-Tyr-Leu-Arg-Lys-Lys-Arg,
Trp-Leu-Leu-Trp-Leu-Tyr-Ala-Arg-Lys-Lys-Arg,
Trp-Ala-Ala-Trp-Ile-Gly-Leu-Arg-Arg-Arg, Trp-Ala-Ala-Trp-Ile-Gly-Leu-Arg-Lys-Lys-Lys, Trp-Ala-Ala-Trp-Ile-Gly-Leu-Arg-Arg-Lys-Lys,
Trp-Ala-Ala-Trp-Ile-Gly-Leu-Arg-Lys-Arg-Lys, Trp-Ala-Ala-Trp-Ile-Gly-Leu-Arg-Arg, Arg-Arg-Arg-Trp-Val-Trp-Val-Val-Val-Trp and Arg-Arg-Arg-Trp-Val-Val-Val-Val-Trp-Trp.

In another aspect of the present invention, there is provided an antibiotic comprising the antibacterial peptide or salt thereof as an active ingredient.

In another aspect of the present invention, there is provided a cosmetic composition comprising the antibacterial peptide or salt thereof as an active ingredient.

In another aspect of the present invention, there is provided a food additive comprising the antibacterial peptide or salt thereof as an active ingredient.

In another aspect of the present invention, there is provided a feed additive comprising the antibacterial peptide or salt thereof as an active ingredient.

In another aspect of the present invention, there is provided a non-human antibacterial method comprising administering a pharmaceutically effective amount of the antibacterial peptide or salt thereof to a subject other than a human.

### Effects of Invention

The novel antibacterial peptide of the present invention exhibits excellent antibacterial activity against antibiotic-resistant bacteria as well as Gram-positive bacteria and Gram-negative bacteria and is low in cytotoxicity and, as such, can be advantageously utilized as an active ingredient in an antibiotic, a cosmetic composition, a food additive, a feed additive, a biotic pesticide, a quasi-drug product, and the like.

### Brief Description of Drawings

FIG. 1 illustrates the process by which the antibacterial peptide of the present invention is derived.
FIG. 2 illustrates the results obtained by analyzing the stability of some of the antibacterial peptides of the present invention (KSH42 and KSH43) for pronase (pronase cocktail).
FIG. 3 illustrates the results obtained by comparing the degree of artificial cell membrane leakage according to treatment with KSH29, KSH42 and KSH43.
FIG. 4 is a photograph of the morphology of the multi-drug resistant strains (*Acinetobacter baumannii* and *Staphylococcus aureus*) according to treatment with KSH29 under an electron microscope.
FIG. 5 illustrates the results obtained by analyzing the degree of liposome leakage according to treatment with KSH29, KSH42 and KSH43.
FIG. 6 illustrates the results obtained by analyzing the degree of dynamic light scattering according to treatment with KSH29, KSH42 and KSH43.
FIG. 7 illustrates the results obtained by analyzing the degree of inducing membrane potential difference disturbance of multi-drug resistant *Staphylococcus aureus* (MRSA) according to treatment with KSH29, KSH42 and KSH43.
FIG. 8 illustrates the results obtained by analyzing the degree of inducing membrane potential difference disturbance of multi-drug resistant *Enterococcus faecalis* (MREF) according to treatment with KSH29, KSH42 and KSH43.
FIG. 9 illustrates the results obtained by analyzing the degree of inducing membrane potential difference disturbance of multi-drug resistant *Acinetobacter baumannii* (MRAB) according to treatment with KSH29, KSH42 and KSH43.
FIG. 10 illustrates the results obtained by analyzing the cell membrane permeability of *E. coli* according to treatment with KSH29, KSH42 and KSH43 and nitrocefin or ONPG (O-nitrophenyl-β-D-galactopyranoside).
FIG. 11 illustrates the results obtained by analyzing the antibacterial effect of KSH29 for each treatment time against multi-drug resistant *Pseudomonas aeruginosa* (MRPA), multi-drug resistant *Enterococcus faecalis* (MREF), multi-drug resistant *Acinetobacter baumannii* (MRAB) and multi-drug resistant *Staphylococcus aureus* (MRSA).
FIG. 12 illustrates the results obtained by analyzing the antibacterial effect of KSH42 and KSH43 for each treatment time against multi-drug resistant *Acinetobacter baumannii* (MRAB) and multi-drug resistant *Staphylococcus aureus* (MRSA).
FIG. 13 illustrates the results obtained by analyzing the degree of resistance acquisition induction of microorganisms according to treatment with KSH29, KSH42 and KSH43.
FIG. 14 illustrates the results obtained by analyzing the antibacterial activity against bacteria (*A. baumannii, S. aureus* and *E. faecium*) according to treatment with KSH37 (negative control), KSH42 and KSH43 as a survival rate of *Galleria mellonella.*
FIGs. 15 to 23 illustrate the results obtained by analyzing the toxicity evaluation of the antibacterial peptides of the present invention.
FIGs. 24 and 25 illustrate the results obtained by measuring the purity and molecular weight of KSH 1, the antibacterial peptide of the present invention, respectively.
FIGs. 26 and 27 illustrate the results obtained by measuring the purity and molecular weight of KSH 3, the antibacterial peptide of the present invention, respectively.
FIGs. 28 and 29 illustrate the results obtained by measuring the purity and molecular weight of KSH 4, the antibacterial peptide of the present invention, respectively.
FIGs. 30 and 31 illustrate the results obtained by measuring the purity and molecular weight of KSH 5, the antibacterial peptide of the present invention, respectively.
FIGs. 32 and 33 illustrate the results obtained by measuring the purity and molecular weight of KSH 6, the antibacterial peptide of the present invention, respectively.
FIGs. 34 and 35 illustrate the results obtained by measuring the purity and molecular weight of KSH 7, the antibacterial peptide of the present invention, respectively.
FIGs. 36 and 37 illustrate the results obtained by measuring the purity and molecular weight of KSH 8, the antibacterial peptide of the present invention, respectively.
FIGs. 38 and 39 illustrate the results obtained by measuring the purity and molecular weight of KSH 9, the antibacterial peptide of the present invention, respectively.
FIGs. 40 and 41 illustrate the results obtained by measuring the purity and molecular weight of KSH 10, the antibacterial peptide of the present invention, respectively.
FIGs. 42 and 43 illustrate the results obtained by measuring the purity and molecular weight of KSH 11, the antibacterial peptide of the present invention, respectively.
FIGs. 44 and 45 illustrate the results obtained by measuring the purity and molecular weight of KSH 12, the antibacterial peptide of the present invention, respectively.
FIGs. 46 and 47 illustrate the results obtained by measuring the purity and molecular weight of KSH 13, the antibacterial peptide of the present invention, respectively.
FIGs. 48 and 49 illustrate the results obtained by measuring the purity and molecular weight of KSH 15, the antibacterial peptide of the present invention, respectively.
FIGs. 50 and 51 illustrate the results obtained by measuring the purity and molecular weight of KSH 16, the antibacterial peptide of the present invention, respectively.
FIGs. 52 and 53 illustrate the results obtained by measuring the purity and molecular weight of KSH 18, the antibacterial peptide of the present invention, respectively.
FIGs. 54 and 55 illustrate the results obtained by measuring the purity and molecular weight of KSH 20, the antibacterial peptide of the present invention, respectively.
FIGs. 56 and 57 illustrate the results obtained by measuring the purity and molecular weight of IKSH 5-1, the antibacterial peptide of the present invention, respectively.
FIGs. 58 and 59 illustrate the results obtained by measuring the purity and molecular weight of IKSH 5-2, the antibacterial peptide of the present invention, respectively.
FIGs. 60 and 61 illustrate the results obtained by measuring the purity and molecular weight of KSH YL, the antibacterial peptide of the present invention, respectively.
FIGs. 62 and 63 illustrate the results obtained by measuring the purity and molecular weight of KSH VY, the antibacterial peptide of the present invention, respectively.
FIGs. 64 and 65 illustrate the results obtained by measuring the purity and molecular weight of KSH VY2, the antibacterial peptide of the present invention, respectively.
FIGs. 66 and 67 illustrate the results obtained by measuring the purity and molecular weight of KSH VY3, the antibacterial peptide of the present invention, respectively.
FIGs. 68 and 69 illustrate the results obtained by measuring the purity and molecular weight of KSH VY4, the antibacterial peptide of the present invention, respectively.
FIGs. 70 and 71 illustrate the results obtained by measuring the purity and molecular weight of XSH 2, the antibacterial peptide of the present invention, respectively.
FIGs. 72 and 73 illustrate the results obtained by measuring the purity and molecular weight of LSH 6, the antibacterial peptide of the present invention, respectively.
FIGs. 74 and 75 illustrate the results obtained by measuring the purity and molecular weight of LSH 7, the antibacterial peptide of the present invention, respectively.
FIGs. 76 and 77 illustrate the results obtained by measuring the purity and molecular weight of LSH 8, the antibacterial peptide of the present invention, respectively.
FIGs. 78 and 79 illustrate the results obtained by measuring the purity and molecular weight of LSH 9, the antibacterial peptide of the present invention, respectively.
FIGs. 80 and 81 illustrate the results obtained by measuring the purity and molecular weight of KSH 29, the antibacterial peptide of the present invention, respectively.
FIGs. 82 and 83 illustrate the results obtained by measuring the purity and molecular weight of KSH 30, the antibacterial peptide of the present invention, respectively.
FIGs. 84 and 85 illustrate the results obtained by measuring the purity and molecular weight of KSH 31, the antibacterial peptide of the present invention, respectively.
FIGs. 86 and 87 illustrate the results obtained by measuring the purity and molecular weight of KSH 32, the antibacterial peptide of the present invention, respectively.
FIGs. 88 and 89 illustrate the results obtained by measuring the purity and molecular weight of KSH 33, the antibacterial peptide of the present invention, respectively.
FIGs. 90 and 91 illustrate the results obtained by measuring the purity and molecular weight of KSH 35, the antibacterial peptide of the present invention, respectively.
FIGs. 92 and 93 illustrate the results obtained by measuring the purity and molecular weight of KSH 36, the antibacterial peptide of the present invention, respectively.
FIGs. 94 and 95 illustrate the results obtained by measuring the purity and molecular weight of KSH 37, the antibacterial peptide of the present invention, respectively.
FIGs. 96 and 97 illustrate the results obtained by measuring the purity and molecular weight of KSH 39, the antibacterial peptide of the present invention, respectively.
FIGs. 98 and 99 illustrate the results obtained by measuring the purity and molecular weight of KSH 40, the antibacterial peptide of the present invention, respectively.
FIGs. 100 and 101 illustrate the results obtained by measuring the purity and molecular weight of KSH 41, the antibacterial peptide of the present invention, respectively.
FIGs. 102 and 103 illustrate the results obtained by measuring the purity and molecular weight of KSH 42, the antibacterial peptide of the present invention, respectively.
FIGs. 104 and 105 illustrate the results obtained by measuring the purity and molecular weight of KSH 43, the antibacterial peptide of the present invention, respectively.
FIGs. 106 and 107 illustrate the results obtained by measuring the purity and molecular weight of KSH 44, the antibacterial peptide of the present invention, respectively.
FIGs. 108 and 109 illustrate the results obtained by measuring the purity and molecular weight of KSH 45, the antibacterial peptide of the present invention, respectively.
FIGs. 110 and 111 illustrate the results obtained by measuring the purity and molecular weight of KSH 46, the antibacterial peptide of the present invention, respectively.
FIGs. 112 and 113 illustrate the results obtained by measuring the purity and molecular weight of KSH 47, the antibacterial peptide of the present invention, respectively.
FIGs. 114 and 115 illustrate the results obtained by measuring the purity and molecular weight of KSH 48, the antibacterial peptide of the present invention, respectively.
FIGs. 116 and 117 illustrate the results obtained by measuring the purity and molecular weight of KSH 49, the antibacterial peptide of the present invention, respectively.
FIGs. 118 and 119 illustrate the results obtained by measuring the purity and molecular weight of KSH 50, the antibacterial peptide of the present invention, respectively.
FIGs. 120 and 121 illustrate the results obtained by measuring the purity and molecular weight of KSH 51, the antibacterial peptide of the present invention, respectively.
FIGs. 122 and 123 illustrate the results obtained by measuring the purity and molecular weight of KSH 52, the antibacterial peptide of the present invention, respectively.
FIGs. 124 and 125 illustrate the results obtained by measuring the purity and molecular weight of KSH 54, the antibacterial peptide of the present invention, respectively.
FIGs. 126 and 127 illustrate the results obtained by measuring the purity and molecular weight of KSH 55, the antibacterial peptide of the present invention, respectively.
FIGs. 128 and 129 illustrate the results obtained by measuring the purity and molecular weight of KSH 56, the antibacterial peptide of the present invention, respectively.
FIGs. 130 and 131 illustrate the results obtained by measuring the purity and molecular weight of KSH 57, the antibacterial peptide of the present invention, respectively.
FIGs. 132 and 133 illustrate the results obtained by measuring the purity and molecular weight of KSH 58, the antibacterial peptide of the present invention, respectively.
FIGs. 134 and 135 illustrate the results obtained by measuring the purity and molecular weight of KSH 59, the antibacterial peptide of the present invention, respectively.
FIGs. 136 and 137 illustrate the results obtained by measuring the purity and molecular weight of KSH 60, the antibacterial peptide of the present invention, respectively.
FIGs. 138 and 139 illustrate the results obtained by measuring the purity and molecular weight of KSH 61, the antibacterial peptide of the present invention, respectively.
FIGs. 140 and 141 illustrate the results obtained by measuring the purity and molecular weight of KSH 62, the antibacterial peptide of the present invention, respectively.
FIGs. 142 and 143 illustrate the results obtained by measuring the purity and molecular weight of KSH 63, the antibacterial peptide of the present invention, respectively.
FIGs. 144 and 145 illustrate the results obtained by measuring the purity and molecular weight of KSH 64, the antibacterial peptide of the present invention, respectively.
FIGs. 146 and 147 illustrate the results obtained by measuring the purity and molecular weight of KSH 19, the antibacterial peptide of the present invention, respectively.
FIGs. 148 and 149 illustrate the results obtained by measuring the purity and molecular weight of LSH 28, the antibacterial peptide of the present invention, respectively.
FIGs. 150 and 151 illustrate the results obtained by measuring the purity and molecular weight of LSH 1, the antibacterial peptide of the present invention, respectively.
FIGs. 152 and 153 illustrate the results obtained by measuring the purity and molecular weight of LSH 2, the antibacterial peptide of the present invention, respectively.
FIGs. 154 and 155 illustrate the results obtained by measuring the purity and molecular weight of LSH 3, the antibacterial peptide of the present invention, respectively.
FIGs. 156 and 157 illustrate the results obtained by measuring the purity and molecular weight of LSH 5, the antibacterial peptide of the present invention, respectively.
FIGs. 158 and 159 illustrate the results obtained by measuring the purity and molecular weight of LSH 99, the antibacterial peptide of the present invention, respectively.
FIGs. 160 and 161 illustrate the results obtained by measuring the purity and molecular weight of KSH 66, the antibacterial peptide of the present invention, respectively.
FIGs. 162 and 163 illustrate the results obtained by measuring the purity and molecular weight of KSH 67, the antibacterial peptide of the present invention, respectively.
FIGs. 164 and 165 illustrate the results obtained by measuring the purity and molecular weight of KSH 68, the antibacterial peptide of the present invention, respectively.
FIGs. 166 and 167 illustrate the results obtained by measuring the purity and molecular weight of KSH 69, the antibacterial peptide of the present invention, respectively.
FIGs. 168 and 169 illustrate the results obtained by measuring the purity and molecular weight of KSH 70, the antibacterial peptide of the present invention, respectively.
FIGs. 170 and 171 illustrate the results obtained by measuring the purity and molecular weight of KSH 71, the antibacterial peptide of the present invention, respectively.
FIGs. 172 and 173 illustrate the results obtained by measuring the purity and molecular weight of KSH 72, the antibacterial peptide of the present invention, respectively.
FIGs. 174 and 175 illustrate the results obtained by measuring the purity and molecular weight of KSH 73, the antibacterial peptide of the present invention, respectively.
FIGs. 176 and 177 illustrate the results obtained by measuring the purity and molecular weight of KSH 74, the antibacterial peptide of the present invention, respectively.
FIGs. 178 and 179 illustrate the results obtained by measuring the purity and molecular weight of KSH 75, the antibacterial peptide of the present invention, respectively.
FIGs. 180 and 181 illustrate the results obtained by measuring the purity and molecular weight of KSH 76, the antibacterial peptide of the present invention, respectively.
FIGs. 182 and 183 illustrate the results obtained by measuring the purity and molecular weight of KSH 77, the antibacterial peptide of the present invention, respectively.
FIGs. 184 and 185 illustrate the results obtained by measuring the purity and molecular weight of KSH 78, the antibacterial peptide of the present invention, respectively.
FIGs. 186 and 187 illustrate the results obtained by measuring the purity and molecular weight of KSH 79, the antibacterial peptide of the present invention, respectively.
FIGs. 188 and 189 illustrate the results obtained by measuring the purity and molecular weight of KSH 80, the antibacterial peptide of the present invention, respectively.
FIGs. 190 and 191 illustrate the results obtained by measuring the purity and molecular weight of KSH 81, the antibacterial peptide of the present invention, respectively.
FIGs. 192 and 193 illustrate the results obtained by measuring the purity and molecular weight of KSH 82, the antibacterial peptide of the present invention, respectively.
FIGs. 194 and 195 illustrate the results obtained by measuring the purity and molecular weight of KSH 83, the antibacterial peptide of the present invention, respectively.
FIGs. 196 and 197 illustrate the results obtained by measuring the purity and molecular weight of KSH 84, the antibacterial peptide of the present invention, respectively.
FIGs. 198 and 199 illustrate the results obtained by measuring the purity and molecular weight of KSH 85, the antibacterial peptide of the present invention, respectively.
FIGs. 200 and 201 illustrate the results obtained by measuring the purity and molecular weight of KSH 86, the antibacterial peptide of the present invention, respectively.
FIGs. 202 and 203 illustrate the results obtained by measuring the purity and molecular weight of KSH 90, the antibacterial peptide of the present invention, respectively.
FIGs. 204 and 205 illustrate the results obtained by measuring the purity and molecular weight of KSH 91, the antibacterial peptide of the present invention, respectively.
FIGs. 206 and 207 illustrate the results obtained by measuring the purity and molecular weight of KSH 27, the antibacterial peptide of the present invention, respectively.
FIGs. 208 and 209 illustrate the results obtained by measuring the purity and molecular weight of KSH 28, the antibacterial peptide of the present invention, respectively.
FIGs. 210 and 211 illustrate the results obtained by measuring the purity and molecular weight of KSH V, the antibacterial peptide of the present invention, respectively.
FIGs. 212 and 213 illustrate the results obtained by measuring the purity and molecular weight of KSH I, the antibacterial peptide of the present invention, respectively.
FIGs. 214 and 215 illustrate the results obtained by measuring the purity and molecular weight of KSH F, the antibacterial peptide of the present invention, respectively.
FIG. 216 illustrates the results obtained by analyzing the antibacterial activity against bacteria (*E. coli*) according to treatment with KSH43 as a survival rate of mice (BALB/c, female, 7-weeks).

### Best Mode for Carrying out the Invention

Hereinafter, the present invention will be described in more detail.

In one aspect of the present invention, there is provided an antibacterial peptide containing three Trps or a salt thereof. In this case, the three Trps may be separated by one or two amino acids. In this case, the amino acid present between Trps may be any one amino acid selected from the group consisting of Val, Leu, Ile, Gly, Ala, Ser, Phe, Tyr, Trp, Lys and His. In addition, the antibacterial peptide may further comprise at least one or more amino acids at the N-terminus and/or C-terminus. In this case, the amino acid bound to the N-terminus and/or C-terminus may be any one amino acid selected from the group consisting of Arg, Lys, Asn, Gln, Asp, Val, Leu, Ser, His, Gly and Tyr. The antibacterial peptide may consist of 6 to 12 amino acids. In this case, it may be in a form in which -COOH at the C-terminus of the antibacterial peptide is modified with -CONH₂. In addition, a fatty acid may be bound to amino acids constituting the peptide.

In one aspect of the present invention, there is provided an antibacterial peptide represented by Structural Formula I or Structural Formula II below or a salt thereof:

[Structural Formula I] N'-A-B-C-C'

[Structural Formula II] N'-C-B-A-C'

in which,
N' is the N-terminus of the antibacterial peptide,
C' is the C-terminus of the antibacterial peptide,
the C-terminus is one in which a carboxy group or hydroxy (-OH) of the carboxy group is substituted with an amine group (-NH₂),
A consists of the amino acid sequence of (X₁)ₐ-(X₂)_{b}-(X₃)_{c}-(X₄)_{d},
wherein a, b, c and d are each independently 0 or 1, and
X₁ to X₄ are each independently any one amino acid selected from the group consisting of Arg, Lys, Asn, Gln, Asp, Val, Leu, Ser, His, Gly and Tyr, and
B consists of the amino acid sequence of Trp-(Z₁)ₑ-(Z₂)_{f}-Trp-(Z₃)_{g}-(Z₄)ₕ-Trp,
wherein e, f, g and h are each independently 0 or 1, and
Z₁ to Z₄ are each independently any one amino acid selected from the group consisting of Val, Leu, Ile, Gly, Ala, Ser, Phe, Tyr, Trp, Lys and His, and
C consists of the amino acid sequence of (X₅)ᵢ-(X₆)ⱼ-(X₇)ₖ-(X₈)ₗ,
wherein i, j, k and 1 are each independently 0 or 1, and
X₅ to X₈ are each independently any one amino acid selected from the group consisting of Arg, Lys, Asn, Gln, Asp, Val, Leu, Ser, His, Gly and Tyr, and
wherein Trp may be substituted with C₁₋₆ alkoxy or halogen, or nitrogen (N) in the indole ring of Trp may be modified with sulfur (S), and
when an amino acid at the N-terminus is Lys, a C₃ to C₁₀ fatty acid may be further bound to an amine group,
Tyr may be substituted with halogen, and
Asn may be glycosylated.

The above "alkoxy," unless otherwise indicated, refers to a group having the Formula -O-alkyl, in which an alkyl group as defined above is attached to a parent compound through an oxygen atom. The alkyl portion of the alkoxy group may have 1 to 20 carbon atoms (i.e., C₁-C₂₀ alkoxy), 1 to 12 carbon atoms (i.e., C₁-C₁₂ alkoxy), or 1 to 6 carbon atoms (i.e., C₁-C₆ alkoxy). Examples of suitable alkoxy groups include methoxy (-O-CH₃ or -OMe), ethoxy (-OCH₂CH₃ or -OEt), t-butoxy (-O-C(CH₃)₃ or -O-tBu), and the like.

As used herein, the term "halogen," unless otherwise stated, refers to F, Cl, Br or I.

In the present invention, any one of e, f, g and h may be 0, and the remainder may be 1.

In addition, X₁ to X₈, Z₁ to Z₄ and Trp may be each independently an L-form or D-form amino acid.

In the present invention, said C' of the antibacterial peptide may be one in which a hydroxy group (-OH) of a carboxy group is substituted with an amine group (-NH₂).

The antibacterial peptide according to the present invention may have excellent antibacterial activity against Gram-positive bacteria. In addition, the antibacterial peptide may have excellent antibacterial activity against Gram-negative bacteria.

In the present invention, the "Gram-positive bacteria" is a type of prokaryotes, and refers to bacteria whose cell walls are stained purple by the Gram staining method. Since the cell wall of Gram-positive bacteria is composed of several layers of peptidoglycan, it appears purple without discoloration even if it is treated with ethanol after staining with a basic dye such as crystal violet. In the present invention, the Gram-positive bacteria may be *Staphylococcus aureus, Streptococcus pneumoniae, Enterococcus faecium* or *Lactobacillus lactis,* but is not limited thereto. In the present invention, the Gram-positive bacteria may be bacteria resistant to antibiotics, and may be Gram-positive multi-drug resistant bacteria that are resistant to two or more antibiotics.

In the present invention, the "Gram-negative bacteria" is a type of prokaryotic cells, and has an outer membrane composed of lipopolysaccharide, lipoprotein, and other complex high molecular substances, instead of having a relatively small amount of peptidoglycan in the cell wall compared to Gram-positive bacteria. After staining with a basic dye such as crystal violet, treatment with ethanol causes discoloration, and counterstaining with a red dye such as safranin results in a red color. The cell wall of Gram-negative bacteria is composed of very thin peptidoglycan and outer membrane compared to Gram-positive bacteria. Peptidoglycan is bound to the lipoprotein connected to the outer membrane and does not contain teichoic acid. The periplasm, a space having a thickness of about 15 nm, is present between the outer and inner membranes of Gram-negative bacteria, and contains a high concentration of protein and maintains a cytoplasm-like state.

In the present invention, the Gram-negative bacteria may be *Acinetobacter baumannii, Escherichia coli, Klebsiella penumoniae, Salmonella spp., Pseudomonas aeruginosa, Haemophilus influenzae, Enterobacter spp.* or *Yersinia pestis,* but is not limited thereto. In the present invention, the Gram-negative bacteria may be bacteria resistant to antibiotics, and may be Gram-negative multi-drug resistant bacteria that are resistant to two or more antibiotics.

The bacteria resistant to antibiotics may be *Acinetobacter baumannii, Pseudomonas aeruginosa, Enterococcus faecalis* or *Staphylococcus aureus,* but is not limited thereto. The antibiotics may include antibiotics such as aminoglycoside class (aminoglycoside, gentamicin, neomycin, and the like), penicillin class (ampicillin and the like), sulfonamide class, beta-lactam class (beta-lactam, amoxicillin/clavulanic acid, and the like), chloramphenicol class, erythromycin class, florfenicol class, fosfomycin class, kanamycin class, lincomycin class, methicillin class, quinolone class, streptomycin class, tetracycline class, trimethoprim class, and vancomycin class, but are not limited thereto.

In one embodiment of the antibacterial peptide of the present invention, B may consist of the amino acid sequence of Trp-Leu-Val-Trp-Ile-Trp (SEQ ID NO: 1). In this case, the antibacterial peptide may be a peptide consisting of any one amino acid sequence selected from the group consisting of Trp-Leu-Val-Trp-Ile-Trp-Arg-Arg-Lys-Arg (SEQ ID NO: 2), Trp-Leu-Val-Trp-Ile-Trp-Arg-Arg-Arg (SEQ ID NO: 3), Trp-Leu-Val-Trp-Ile-Trp-Gln-Arg-Arg-Arg (SEQ ID NO: 4), Trp-Leu-Val-Trp-Ile-Trp-Arg-Arg-Gln-Arg (SEQ ID NO: 5), Trp-Leu-Val-Trp-Ile-Trp-Arg-Arg (SEQ ID NO: 6), Lys-Trp-Leu-Val-Trp-Ile-Trp-Arg-Arg-Arg (SEQ ID NO: 7) and Trp-Leu-Val-Trp-Ile-Trp-Arg-Arg-Lys-Arg (SEQ ID NO: 75).

In one embodiment of the antibacterial peptide of the present invention, the antibacterial peptide may consist of the amino acid sequence of Trp-Val-Val-Trp-Val-Val-Trp-Arg-Arg-Arg (SEQ ID NO: 8).

In one embodiment of the antibacterial peptide of the present invention, B may consist of the amino acid sequence of Trp-Ile-Trp-Val-Leu-Trp (SEQ ID NO: 9). In this case, the antibacterial peptide may consist of any one amino acid sequence selected from the group consisting of Arg-Arg-Arg-Trp-Ile-Trp-Val-Leu-Trp-Lys (SEQ ID NO: 10), Lys-Lys-Lys-Trp-Ile-Trp-Val-Leu-Trp-Lys (SEQ ID NO: 11), Arg-Arg-Trp-Ile-Trp-Val-Leu-Trp-Arg (D-form) (SEQ ID NO: 12), Arg-Arg-Trp-Ile-Trp-Val-Leu-Trp-Arg (L-form) (SEQ ID NO: 13), Asn (glycosylated)-Arg-Arg-Arg-Trp-Ile-Trp-Val-Leu-Trp-Lys (SEQ ID NO: 57), Arg-Arg-Arg-Trp-Ile-Trp-Val-Leu-Trp-Lys-Asn (SEQ ID NO: 79) and Arg-Arg-Arg-Trp-Ile-Trp-Val-Leu-Trp (SEQ ID NO: 84).

In one embodiment of the present invention, the antibacterial peptide may consist of any one amino acid sequence selected from the group consisting of Trp-Leu-Val-Trp-Lys-Trp-Arg-Arg-Arg (SEQ ID NO: 14), Trp-Leu-Val-Trp-Ser-Trp-Arg-Arg-Arg (SEQ ID NO: 15), Trp-Val-Val-Trp-Val-Trp-Arg-Arg-Arg (SEQ ID NO: 16), Trp-Leu-Trp-Ile-Trp-Arg-Arg-Arg (SEQ ID NO: 17), Arg-Arg-Trp-Val-Trp-Val-Val-Trp-Lys (SEQ ID NO: 18), Arg-Arg-Arg-Trp-Val-Trp-Val-Val-Trp-Lys (SEQ ID NO: 19), Arg-Arg-Arg-Trp-Ile-Trp-Ile-Leu-Trp (SEQ ID NO: 20), Arg-Arg-Arg-Trp-Ile-Trp-Ile-Ile-Trp (SEQ ID NO: 21), and Trp-Ile-Ile-Trp-Ile-Trp-Arg-Arg-Arg (SEQ ID NO: 22), Trp-Val-Leu-Trp-Val-Trp-Leu-Arg-Lys-Lys-Arg (SEQ ID NO: 67), Asp-Trp-Leu-Val-Trp-Ile-Trp-Arg-Arg-Arg (SEQ ID NO: 56), Trp-Val-Val-Trp-Val-Trp-Lys-Lys-Lys (SEQ ID NO: 77), Trp-Val-Val-Trp-Val-Val-Trp-Lys-Lys-Lys (SEQ ID NO: 78), Trp-Ala-Ala-Trp-Val-Val-Trp-Arg-Arg-Arg (SEQ ID NO: 81), Trp-Val-Val-Trp-Ala-Ala-Trp-Arg-Arg-Arg (SEQ ID NO: 83), Arg-Arg-Arg-Gly-Trp-Val-Trp-Val-Val-Trp-Lys (SEQ ID NO: 98) and Arg-Arg-Arg-Trp-Ile-Trp-Trp-Leu-Trp-Lys (SEQ ID NO: 40), Arg-Arg-Lys-Trp-Ile-Trp-Trp-Leu-Trp-Lys (SEQ ID NO: 41), Arg-Arg-Lys-Trp-Ile-Trp-Trp-Leu-Trp-Lys-Lys-Lys (SEQ ID NO: 42), Trp-Leu-Leu-Trp-Val-Leu-Trp-Arg-Lys-Lys-Arg (SEQ ID NO: 69), Trp-Val-Ala-Trp-Ala-Val-Trp-Arg-Arg-Arg (SEQ ID NO: 82), Arg-Arg-Arg-Trp-Ile-Trp-Ala-Ala-Trp-Lys (SEQ ID NO: 85), Arg-Arg-Arg-Trp-Val-Trp-Trp-Val-Trp-Trp (SEQ ID NO: 90), Arg-Arg-Arg-Trp-Val-Trp-Trp-Val-Val-Trp (SEQ ID NO: 91), Arg-Arg-Arg-Trp-Val-Trp-Val-Val-Trp-Trp (SEQ ID NO: 92), Arg-Arg-Arg-Trp-Val-Val-Trp-Val-Trp-Trp (SEQ ID NO: 94), Arg-Arg-Arg-Trp-Val-Val-Trp-Val-Val-Trp (SEQ ID NO: 95) and Arg-Arg-His-His-Trp-Val-Val-Trp-Val-Val-Trp-Lys (SEQ ID NO: 99).

In another aspect of the present invention, there is provided an antibacterial peptide consisting of any one amino acid sequence selected from the group consisting of Trp-Leu-Leu-Trp-Ile-Gly-Leu-Arg-Lys-Lys-Arg (SEQ ID NO: 24), Trp-Leu-Leu-Trp-Ile-Ala-Leu-Arg-Lys-Lys (SEQ ID NO: 25), Lys-Trp-Leu-Leu-Trp-Ile-Gly-Leu-Arg-Lys-Lys-Arg (SEQ ID NO: 48), Trp-Ala-Ala-Trp-Ile-Gly-Leu-Arg-Lys-Lys-Arg (SEQ ID NO: 27), Trp-Leu-Val-Trp-Ile-Gly-Leu-Arg-Lys-Lys-Arg (SEQ ID NO: 28), Trp-Leu-Ile-Trp-Ile-Gly-Leu-Arg-Lys-Lys-Arg (SEQ ID NO: 29), Trp-Leu-Phe-Trp-Ile-Gly-Leu-Arg-Lys-Lys-Arg (SEQ ID NO: 30), Trp-Leu-Tyr-Trp-Ile-Leu-Leu-Arg-Lys-Lys-Arg (SEQ ID NO: 31), Trp-Leu-Val-Trp-Ile-Tyr-Leu-Arg-Lys-Lys-Arg (SEQ ID NO: 32), Trp-Leu-Val-Trp-Val-Tyr-Leu-Arg-Lys-Lys-Arg (SEQ ID NO: 33), Trp-Leu-Val-Trp-Ile-Tyr-Val-Arg-Lys-Lys-Arg (SEQ ID NO: 34), Trp-Leu-Val-Trp-Ile-Tyr-Arg-Lys-Lys-Arg (SEQ ID NO: 35), Trp-Leu-Ile-Trp-Val-Tyr-Arg-Lys-Lys-Arg (SEQ ID NO: 36), Trp-Leu-Val-Trp-Ile-Tyr-Arg-Arg-Arg (SEQ ID NO: 37), Trp-Val-Val-Val-Val-Trp-Arg-Arg-Arg (SEQ ID NO: 38), Trp-Val-Val-His-Val-Val-Trp-Arg-Arg-Arg (SEQ ID NO: 39), Arg-Arg-Arg-His-Val-His-Val-Val-Trp-Lys (SEQ ID NO: 43), Trp-Leu-Leu-Trp-Ile-Gly-Leu-Arg-Lys-Lys-Arg-Tyr (SEQ ID NO: 65), Trp-Leu-Val-Tyr-Val-Trp-Leu-Arg-Lys-Lys-Arg (SEQ ID NO: 68), Ser-Trp-Leu-Leu-Trp-Ile-Gly-Leu-Arg-Lys-Lys-Arg (SEQ ID NO: 55), Trp-Val-Val-His-Val-Val-Trp-Arg-Arg-Arg-Asn (glycosylated) (SEQ ID NO: 59), Arg-Arg-Arg-Trp-Val-Val-Val-Val-Val-Trp (SEQ ID NO: 97), Trp-Leu-Leu-Trp-Ile-Ala-Leu-Arg-Lys-Lys-Arg (SEQ ID NO: 23), Lys(caproic acid (C₆) attached)-Trp-Leu-Leu-Trp-Ile-Gly-Leu-Arg-Lys-Lys-Arg (SEQ ID NO: 26), Trp-Leu-Leu-Trp-Ile-Gly-Leu-Arg-Lys-Lys (SEQ ID NO: 47), Leu-Leu-Trp-Ile-Ala-Leu-Arg-Lys-Lys-Arg (SEQ ID NO: 49), Leu-Leu-Trp-Ile-Ala-Leu-Lys-Lys-Lys-Lys (SEQ ID NO: 50), Leu-Leu-Trp-Ile-Gly-Leu-Arg-Lys-Lys-Arg (SEQ ID NO: 51), Tyr-Trp-Leu-Leu-Trp-Ile-Gly-Leu-Arg-Lys-Lys-Arg (SEQ ID NO: 64), Trp Leu Leu Trp Val Tyr Val Arg Lys Lys Arg (SEQ ID NO: 66), Trp-Leu-Trp-Val-Trp-Val-Arg-Lys-Lys-Arg (SEQ ID NO: 70), Val-Trp-Val-Trp-Val-Trp-Val-Arg-Lys-Lys-Arg (SEQ ID NO: 76), Trp-Val-Val-Trp-Val-Tyr-Val-Arg-Lys-Lys-Arg (SEQ ID NO: 71), Trp-Val-Val-Trp-Val-Val-Tyr-Arg-Lys-Lys-Arg (SEQ ID NO: 72), Trp-Leu-Ala-Trp-Leu-Tyr-Leu-Arg-Lys-Lys-Arg (SEQ ID NO: 73), Trp-Leu-Leu-Trp-Leu-Tyr-Ala-Arg-Lys-Lys-Arg (SEQ ID NO: 74), Trp-Ala-Ala-Trp-Ile-Gly-Leu-Arg-Arg-Arg (SEQ ID NO: 80), Trp-Ala-Ala-Trp-Ile-Gly-Leu-Arg-Lys-Lys-Lys (SEQ ID NO: 86), Trp-Ala-Ala-Trp-Ile-Gly-Leu-Arg-Arg-Lys-Lys (SEQ ID NO: 87), Trp-Ala-Ala-Trp-Ile-Gly-Leu-Arg-Lys-Arg-Lys (SEQ ID NO: 88), Trp-Ala-Ala-Trp-Ile-Gly-Leu-Arg-Arg (SEQ ID NO: 89), Arg-Arg-Arg-Trp-Val-Trp-Val-Val-Val-Trp (SEQ ID NO: 93) and Arg-Arg-Arg-Trp-Val-Val-Val-Val-Trp-Trp (SEQ ID NO: 96).

In one embodiment of the present invention, the amino acid of the antibacterial peptide may be an L-form or a D-form. Specifically, the amino acid that may have an L-form in the antibacterial peptide is represented by an L-form, but the form of the amino acid represented by an L-form may include a D-form depending on the processing environment. Thus, the form of the amino acid is not limited by the indication.

In one embodiment of the present invention, each amino acid of the antibacterial peptide may be a modified derivative. Specifically, the tryptophan (Trp) may be methoxy-tryptophan (Wm) represented by Formula 1 below, may be benzothienyl-alanine (Ws) represented by Formula 2 below, and may be fluoro-tryptophan (Wf) represented by Formula 3 below.

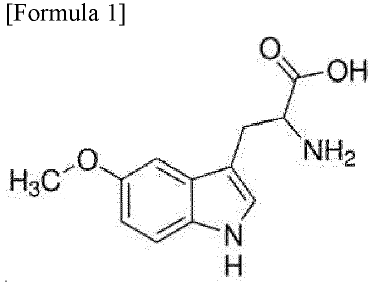

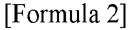

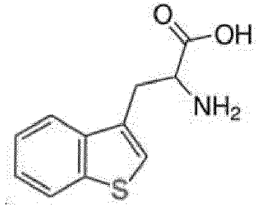

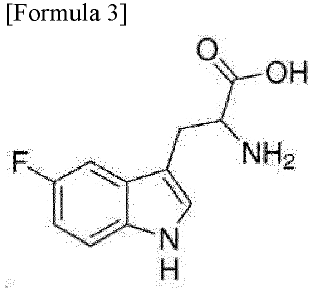

In addition, in one embodiment of the present invention, the tyrosine (Tyr) may be monoiodotyrosine represented by Formula 4 below.

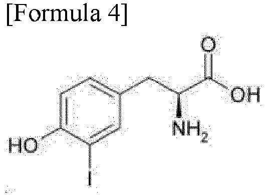

In addition, in one embodiment of the present invention, hydrogen, C₁₋₁₀ alkyl or C₁-C₂₀ fatty acid may be bound to the NH₃⁺ terminus of the lysine (Lys), and in one embodiment, it may be caproic acid (C₆ fatty acid) or capric acid (C₁₀ fatty acid), but is not limited thereto. On the other hand, in one embodiment of the present invention, the lysine of the antibacterial peptide may form multiple antigenic peptide conjugation (MAP conjugation). The multiple antigenic peptide (MAP) is an artificially branched peptide, and lysine moieties can be used as a scaffolding core to support the formation of 8 or less branches having variable or identical peptide sequences. In one embodiment of the present invention, the lysine of the antibacterial peptide formed a branch with the peptide containing tryptophan.

In addition, in one embodiment of the present invention, the asparagine (Asn) may be a glycosylated asparagine.

In one embodiment of the present invention, the antibacterial peptide consisting of the amino acid sequence of Lys-Trp-Leu-Leu-Trp-Ile-Gly-Leu-Arg-Lys-Lys-Arg may be one in which a C₁ to C₂₀ fatty acid is further bound to the amine group of Lys at the N-terminus. Specifically, it may be one in which a C₃ to C₁₀ fatty acid is further bound. The fatty acid may be caproic acid or capric acid. Specifically, it may be caproic acid, but is not limited thereto.

The antibacterial peptide consisting of the amino acid sequence of Trp-Leu-Leu-Trp-Ile-Ala-Leu-Arg-Lys-Lys-Arg, Trp-Leu-Leu-Trp-Ile-Ala-Leu-Arg-Lys-Lys may be one in which a C₃ to C₁₀ fatty acid is further bound to the amine group of Lys at the N-terminus. Specifically, it may be one in which a C₃ to C₁₀ fatty acid is further bound. The fatty acid may be caproic acid or capric acid. Specifically, it may be caproic acid, but is not limited thereto.

Arg at the C-terminus of the antibacterial peptide consisting of the amino acid sequence of Trp-Leu-Leu-Trp-Ile-Ala-Leu-Arg-Lys-Lys-Arg may be an L-form or a D-form.

Trp of the antibacterial peptide consisting of the amino acid sequence of Trp-Leu-Leu-Trp-Ile-Gly-Leu-Arg-Lys-Lys-Arg may be substituted with C₁₋₆ alkoxy or halogen, or nitrogen (N) in the indole ring of Trp may be modified with sulfur (S).

The salt should have low toxicity to humans and should not have any negative effect on the biological activity and physicochemical properties of the parent compound. For example, the salt may be an acid addition salt formed by a pharmaceutically acceptable free acid.

The free acid may be an inorganic acid or an organic acid, wherein the inorganic acid may be hydrochloric acid, sulfuric acid, nitric acid, phosphoric acid, perchloric acid, bromic acid, and the like, and the organic acid may be acetic acid, methanesulfonic acid, ethanesulfonic acid, p-toluenesulfonic acid, fumaric acid, maleic acid, malonic acid, phthalic acid, succinic acid, lactic acid, citric acid, gluconic acid, tartaric acid, salicylic acid, malic acid, oxalic acid, benzoic acid, embonic acid, aspartic acid, glutamic acid, and the like.

The acid addition salt can be prepared by a conventional method, for example, by dissolving the peptide in an excess aqueous acid solution, and precipitating the salt using a water-miscible organic solvent such as methanol, ethanol, acetone or acetonitrile..

In addition, the salt may be an alkali metal salt (sodium salt, etc.) or an alkaline earth metal salt (potassium salt, etc.). The alkali metal salt or alkaline earth metal salt can be obtained, for example, by dissolving the peptide in an excess alkali metal hydroxide or alkaline earth metal hydroxide solution, filtering the undissolved compound salt, and then evaporating and drying the filtrate.

The antibacterial peptide of the present invention may exhibit excellent antibacterial activity compared to commercially available antibiotics. Specifically, in one embodiment of the present invention, when treated with carbapenem, an antibiotic known as an indicator of whether multi-drug resistant bacteria is determined, the number of strains having KPC (Klebsiella pneumoniae carbapenemase) and NDM (New Delhi metallo-beta-lactamase), which are resistance enzymes, was increased, but it was confirmed that the antibacterial peptide of the present invention exhibits excellent antibacterial activity against carbapenem-resistant *Acinetobacter baumannii* and *Pseudomonas aeruginosa.* In addition, in one embodiment of the present invention, the antibiotic tetracycline has insufficient sterilization ability and the target protein tends to be mutated, but it was confirmed that the antibacterial peptide of the present invention exhibits antibacterial activity against tetracycline-resistant *Acinetobacter baumannii,* and has excellent sterilization ability, and the tendency to mutate the target protein is reduced. In addition, in one embodiment of the present invention, vancomycin, an antibiotic against Gram-positive bacteria, has a problem in that resistant strains occur, but it was confirmed that the antibacterial peptide of the present invention exhibits antibacterial activity against vancomycin-resistant *Enterococcus faecalis.* In one embodiment of the present invention, bezlotoxumab, known as a novel antibody-based antibiotic, is used exclusively for preventing reinfection of *Clostridium Difficile* (*C. Difficile*)*,* but the antibacterial peptide of the present invention has the advantage that the spectrum of pathogens, against which the antibacterial peptide of the present invention exhibits antibacterial activity, is wide.

In one embodiment of the present invention, it was confirmed that the antibacterial peptide of the present invention exhibits antibacterial activity against Gram-positive bacteria and Gram-negative bacteria, but daptomycin and gramicidin, which are commercially available antibiotics, exhibit antibacterial activity only against Gram-positive bacteria. In addition, protegrin, a peptide that partially satisfies the minimum growth inhibitory concentration for Gram-positive bacteria and Gram-negative bacteria, has a high cytotoxicity problem.

On the other hand, colistin, which is a multi-drug resistant Gram-negative bacteria antibiotic, causes fatal kidney damage, it is difficult to administer in combination with other drugs, and there are problems that resistant bacteria occur. Accordingly, antibiotics such as SPR206 and SPR741 have been developed. Specifically, the SPR206 increased the antibacterial activity of colistin by 3 to 4 times, and reduced renal toxicity by 1/3. However, there is a problem in that resistant bacteria are shared by colistin. The SPR741 eliminates the renal toxicity of colistin, but has no antibacterial activity by itself, so there is a problem that it must be administered in combination with antibiotics of Gram-positive bacteria. On the other hand, the antibacterial peptide of the present invention not only exhibits antibacterial activity against Gram-positive bacteria and Gram-negative bacteria, but also exhibits antibacterial activity against colistin-resistant bacteria, and also inhibits the generation of resistant strains.

The antibacterial peptide of the present invention may have low cytotoxicity to human-derived cells.

In another aspect of the present invention, there is provided an antibiotic comprising the antibacterial peptide as an active ingredient.

The antibacterial peptide of the present invention may be administered orally or parenterally during clinical administration, and may be used in the form of general pharmaceutical preparations. Formulations for oral administration may take various forms such as syrups, tablets, capsules, creams and lozenges. Parenteral administration may refer to administration via a route other than oral administration, such as rectal, intravenous, peritoneal, muscle, arterial, transdermal, nasal, inhalation, ocular, and subcutaneous administration. When the antibacterial peptide of the present invention is used as a pharmaceutical, it may further include one or more active ingredients exhibiting the same or similar function.

That is, the antibacterial peptide of the present invention may be administered in a variety of formulations for parenteral administration. When it is formulated, it is prepared using a commonly used diluent or excipient such as a filler, an extender, a binder, a wetting agent, a disintegrant, a surfactant, and the like. Formulations for parenteral administration include sterile aqueous solutions, non-aqueous solutions, suspensions, emulsions, lyophilized preparations, and suppositories. As the non-aqueous solvents and suspending agents, propylene glycol, polyethylene glycol, vegetable oils such as olive oil, and injectable esters such as ethyl oleate may be used. As the base of the suppositories, Witepsol, Macrogol, Tween 61, cacao butter, laurin, glycerogelatin, and the like may be used.

In addition, the antibacterial peptide of the present invention may be used by mixing with various pharmaceutically acceptable carriers such as physiological saline or organic solvents. In addition, carbohydrates such as glucose, sucrose or dextran, for increasing stability or absorbability, antioxidants such as ascorbic acid or glutathione, chelating agents, low molecular weight proteins or other stabilizers may be used as pharmaceuticals.

The effective dose of the antibacterial peptide of the present invention is 0.01 µg/kg to 2 mg/kg, specifically 0.5 mg/kg to 1 mg/kg, and may be administered once to 3 times a day.

In the antibiotic of the present invention, the total effective amount of the novel peptide of the present invention may be administered to a patient in a single dose in the form of a bolus or by infusion for a relatively short period of time, and multiple doses may be administered by a fractionated treatment protocol in which they are administered over a long period of time. The effective dose of the patient is determined by considering various factors such as the age and health status of the patient as well as the route of administration and the frequency of treatments. Considering this point, one of ordinary skill in the art will be able to determine an appropriate effective dose according to the specific use of the novel peptide of the present invention as an antibiotic.

In another aspect of the present invention, there is provided an antibacterial cosmetic composition comprising the antibacterial peptide as an active ingredient.

The cosmetic composition of the present invention includes components commonly used in cosmetic compositions in addition to the antibacterial peptide, for example, conventional adjuvants such as antioxidants, stabilizers, solubilizers, vitamins, pigments and fragrances, and carriers.

In the cosmetic composition of the present invention, the antibacterial peptide of the present invention may be added in an amount of 0.1 to 50 % by weight, preferably 1 to 10 % by weight, in a cosmetic composition usually contained therein.

The cosmetic composition of the present invention may be prepared in any formulation conventionally prepared in the art. It may be formulated as, for example, a solution, a suspension, an emulsion, a paste, a gel, a cream, a lotion, a powder, a soap, a surfactant-containing cleansing agent, an oil, a powder foundation, an emulsion foundation, a wax foundation and a spray, etc., but is not limited thereto. More specifically, it may be prepared in the form of a skin sotfner (skin toner), a nourishing lotion (milk lotion), a nourishing cream, a massage cream, an essence, an eye cream, a cleansing cream, a cleansing foam, a cleansing water, a pack, a spray, or a powder..

When the formulation of the present invention is a paste, a cream or a gel, animal oil, vegetable oil, wax, paraffin, starch, tragacanth, cellulose derivative, polyethylene glycol, silicone, bentonite, silica, talc, zinc oxide, and the like may be used as a carrier component. When the formulation of the present invention is a powder or a spray, lactose, talc, silica, aluminum hydroxide, calcium silicate or polyamide powder may be used as a carrier component, and in particular, in the case of a spray, a propellant such as chlorofluorohydrocarbon, propane/butane or dimethyl ether may be further included. When the formulation of the present invention is a solution or an emulsion, a solvent, a solubilizer or an emulsifier is used as a carrier component, for example, water, ethanol, isopropanol, ethyl carbonate, ethyl acetate, benzyl alcohol, benzyl benzoate, propylene glycol, 1,3-butylglycol oil, glycerol fatty esters, fatty acid esters of polyethylene glycol or sorbitan. When the formulation of the present invention is a suspension, a liquid diluent such as water, ethanol or propylene glycol, a suspending agent such as ethoxylated isostearyl alcohol, polyoxyethylene sorbitol ester and polyoxyethylene sorbitan ester, and microcrystalline cellulose, aluminum metahydroxide, bentonite, agar or tragacanth may be used as a carrier component. When the formulation of the present invention is a surfactant-containing cleansing agent, aliphatic alcohol sulfate, aliphatic alcohol ether sulfate, sulfosuccinic acid monoester, isethionate, imidazolinium derivative, methyltaurate, sarcosinate, fatty acid amide ether sulfate, alkylamidobetaine, aliphatic alcohol, fatty acid glyceride, fatty acid diethanolamide, vegetable oil, lanolin derivative or ethoxylated glycerol fatty acid ester and the like may be used as a carrier component.

In another aspect of the present invention, there is provided an antibacterial food additive comprising the antibacterial peptide as an active ingredient.

When the antibacterial peptide of the present invention is used as a food additive, the antibacterial peptide may be added as it is or used together with other food ingredients, and may be appropriately used according to a conventional method. The mixing amount of the active ingredient may be appropriately determined according to the purpose of its use. In general, the peptide of the present invention is added in an amount of 15 parts by weight or less, preferably 10 parts by weight or less, based on the raw material. However, in the case of long-term administration, the amount may be below the above range, and since there is no problem in terms of stability, the active ingredient may be used in an amount above the above range.

The type of the food is not particularly limited. Examples of foods to which the above substances can be added include meat, sausage, bread, chocolate, candies, snacks, confectionery, pizza, ramen, other noodles, gums, dairy products including ice cream, various soups, beverages, tea, drinks, alcoholic beverages and vitamin complexes, and the like, and include all foods in a conventional sense.

In another aspect of the present invention, there is provided an antibacterial feed additive comprising the antibacterial peptide as an active ingredient.

The feed composition of the present invention replaces the existing antibiotics and inhibits the growth of harmful food pathogens, thereby improving the health status of the animal body, improving the weight gain and meat quality of livestock, and increasing milk production and immunity. The feed composition of the present invention may be prepared in the form of a fermented feed, a compound feed, a pellet form, a silage, and the like.

The fermented feed may be prepared by fermenting organic matter by adding various microbial populations or enzymes other than the peptide of the present invention, and the compounded feed may be prepared by mixing several types of general feed with the peptide of the present invention. The feed in the form of pellets may be prepared by applying heat and pressure to the compounded feed, etc. in a pellet machine, and the silage may be prepared by fermenting green fodder with microorganisms. The wet fermented feed may be prepared by collecting and transporting organic matter such as food waste, mixing excipients for sterilization process and moisture control in a certain ratio, and then fermenting it at a temperature suitable for fermentation for more than 24 hours so that the moisture content is about 70%. The dry fermented feed may be prepared by applying an additional drying process to the wet fermented feed so that the moisture content is about 30% to 40%.

In another aspect of the present invention, there is provided an antibacterial biotic pesticide comprising the antibacterial peptide as an active ingredient.

In another aspect of the present invention, there is provided an preservative composition comprising the antibacterial peptide as an active ingredient.

The preservative composition includes a cosmetic preservative or a pharmaceutical preservative. The food preservatives, cosmetic preservatives and pharmaceutical preservatives are additives used to prevent deterioration, decay, discoloration and chemical changes of pharmaceuticals, and include a sterilizing agent and an antioxidant, and also include functional antibiotics that inhibit the proliferation of microorganisms such as bacteria, mold, yeast, and the like, thereby inhibiting the growth or sterilization of spoilage microorganisms in food and pharmaceuticals. Under ideal conditions for such a preservative composition, it should be non-toxic and should be effective even in a small amount.

In another aspect of the present invention, there is provided an antibacterial quasi-drug product composition comprising the antibacterial peptide as an active ingredient.

When the antibacterial peptide is used as a quasi-drug product additive, the antibacterial peptide may be added as it is or used together with other quasi-drug products or quasi-drug product ingredients, and may be appropriately used according to a conventional method. The mixing amount of the active ingredient may be appropriately determined according to the purpose of its use. The quasi-drug product composition of the present invention may be a disinfectant cleaner, a shower foam, a gargrin, a wet tissue, a detergent soap, a handwash, a humidifier filler, a mask, an ointment, a patch or a filter filler, but is not limited thereto.

In another aspect of the present invention, there is provided an antibacterial method comprising administering a pharmaceutically effective amount of the antibacterial peptide to a subject. The subject may be a mammal other than a human, but is not limited thereto.

### Mode for Carrying out the Invention

Hereinafter, the present invention will be described in detail by way of Examples. However, the following examples are only for illustrating the present invention, and the present invention is not limited to the following examples.

### Example 1. Preparation of antibacterial peptides

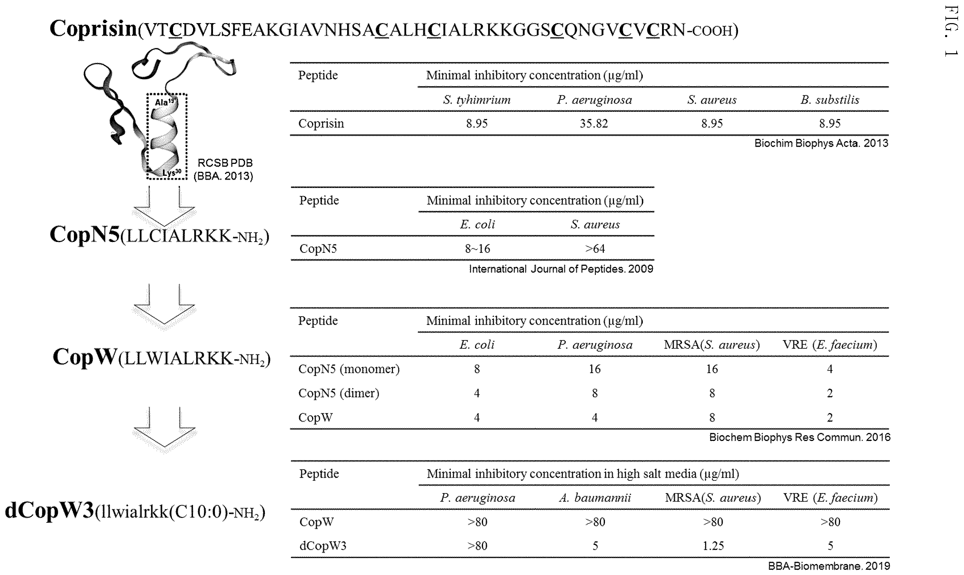

The antibacterial peptides of the present invention were prepared by the preparation method shown in FIG. 1.

Specifically, Rink-amide-MBHA resin was swelled with dichloromethane and then deprotected with piperidine. D,D-dimethylformaide, diisopropylcarbodiimide and 1-hydroxybenzotriazole solutions were used to connect Fmoc-protected amino acids while changing, and then cleaved with thioanisole and TFA to prepare the antibacterial peptide of the present invention.

The prepared antibacterial peptides were purified with acetonitrile using HPLC C18 colume.

### Experimental Example 1. Analysis of antibacterial peptides

### Experimental Example 1.1. Analysis of solubility of antibacterial peptides

The solubility of the antibacterial peptides of the present invention was measured, and the results are shown in Table 1 below.

**[Table 1]**

| Component | Solubility in solution (µM) | | |
|---|---|---|---|
| | H₂O | PBS | CAMHB |
| KSH29 | >2000 | 100 | 50 |
| KSH30 | >2000 | 25 | 50 |
| KSH31 | >2000 | 100 | 25 |
| KSH32 | >2000 | 25 | 50 |
| KSH35 | >2000 | >200 | 25 |
| KSH36 | >2000 | >200 | 100 |
| KSH40 | >2000 | 100 | 100 |
| KSH42 | >2000 | 100 | 100 |
| KSH43 | >2000 | >200 | >25 |
| KSH44 | >2000 | 100 | 100 |
| KSH45 | >2000 | >200 | 50 |
| KSH46 | >2000 | >200 | 200 |
| Colistin | >2000 | >200 | >200 |
| Vancomycin | >2000 | >200 | >200 |

The concentration of the peptide was 25 to 200 µM, and was observed for 12 hours at room temperature.

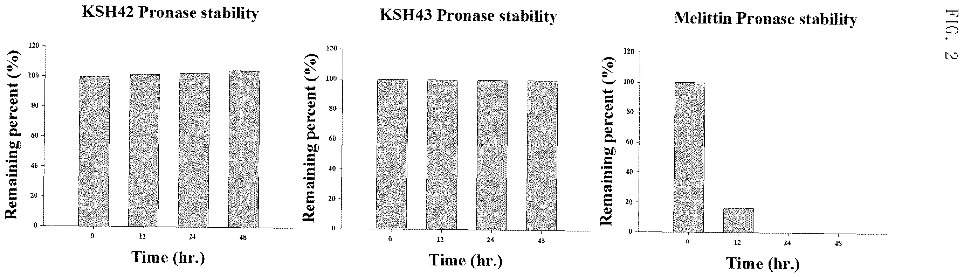

In addition, the results obtained by analyzing the stability of KSH42 and KSH43 of the antibacterial peptides of the present invention for pronase are shown in FIG. 2.

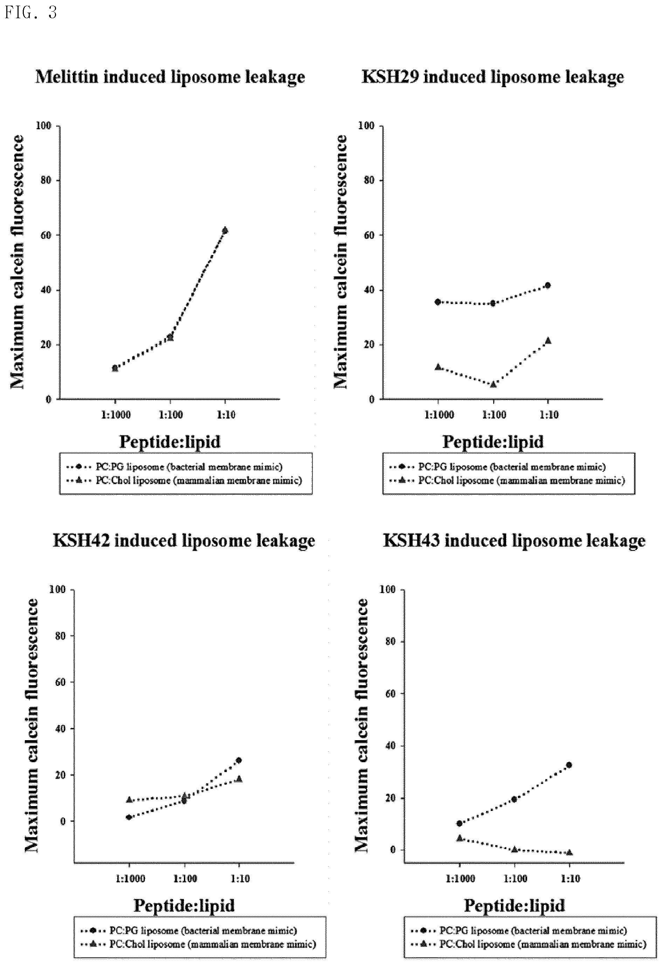

In addition, the results obtained by comparing the degree of artificial cell membrane leakage according to treatment with KSH29, KSH42 and KSH43 are shown in FIG. 3.

### Experimental Example 1.2. Measurement of molecular weight and purity of antibacterial peptides

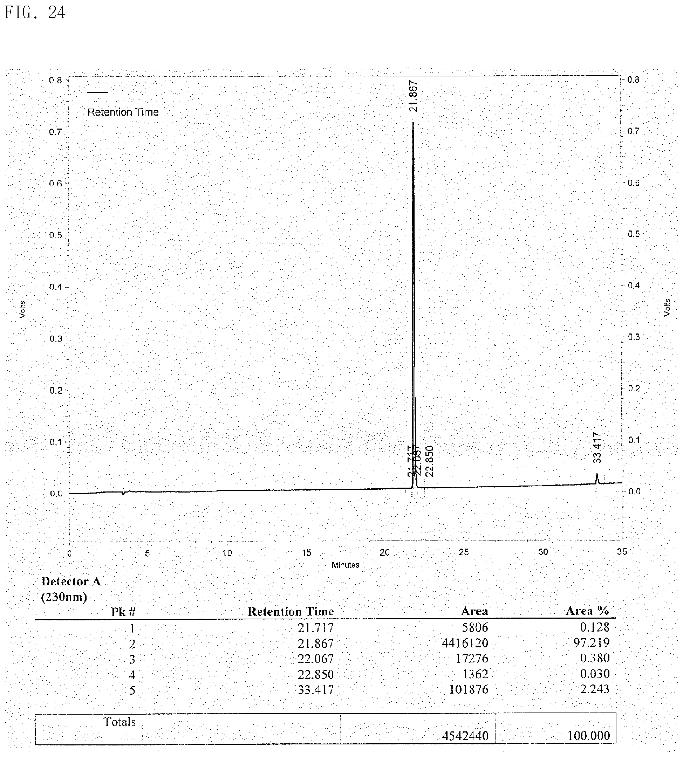

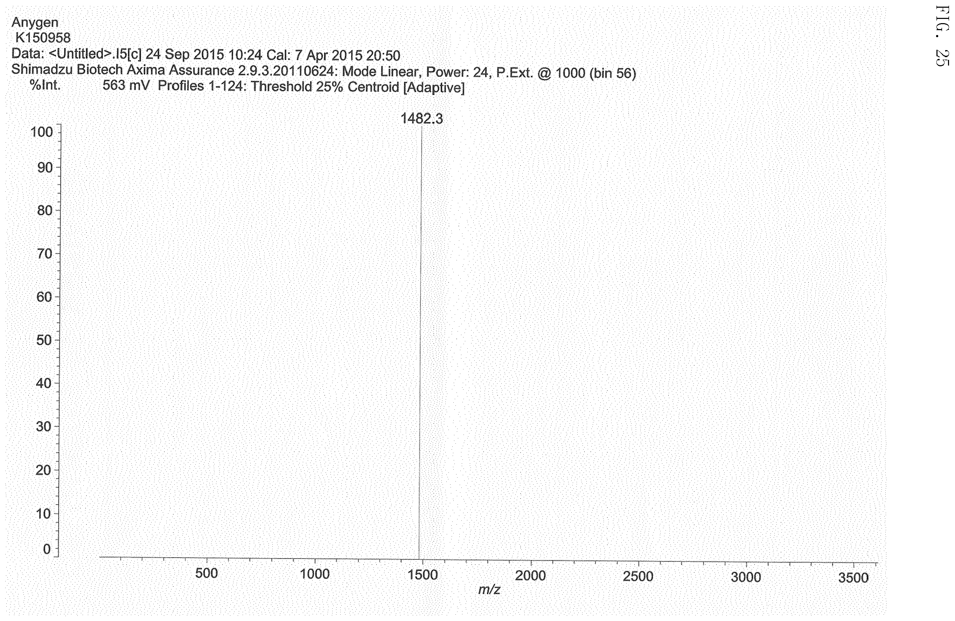

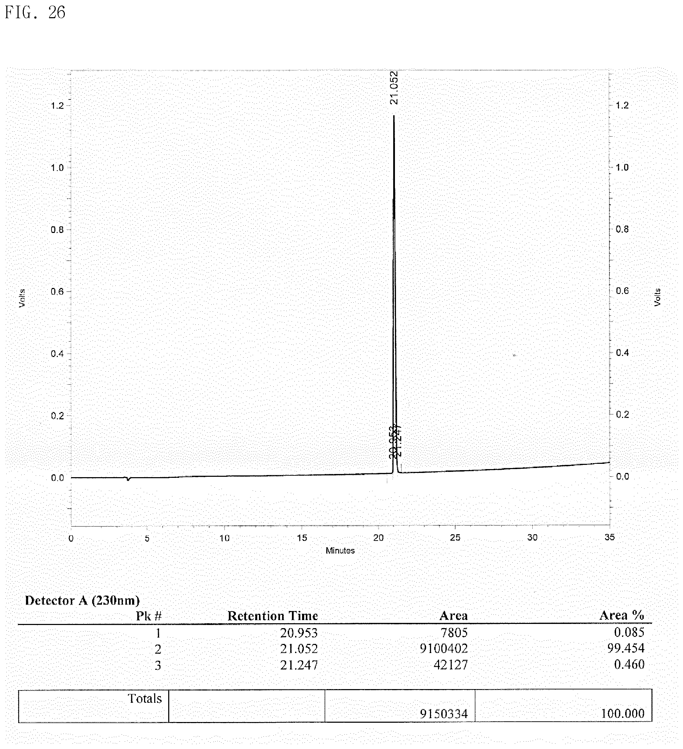

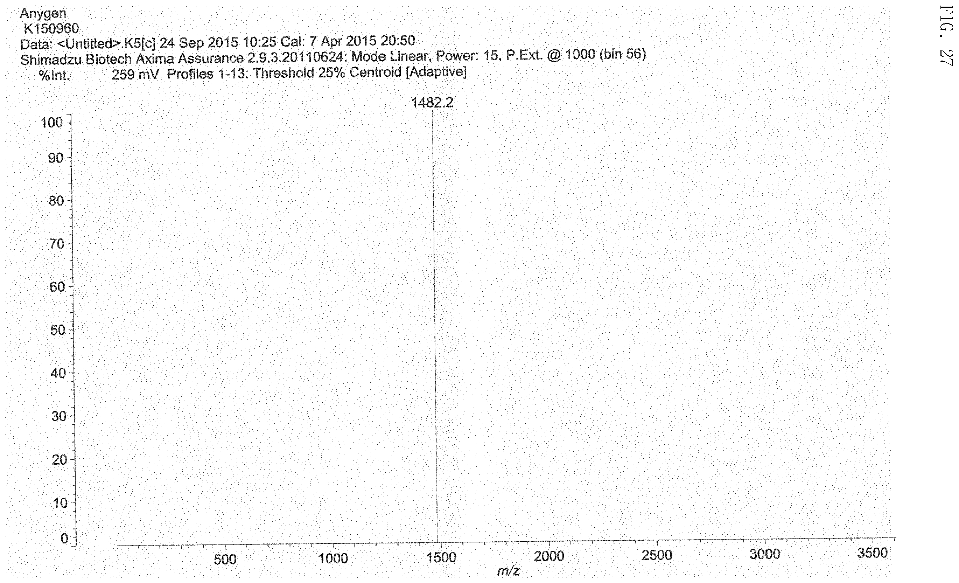

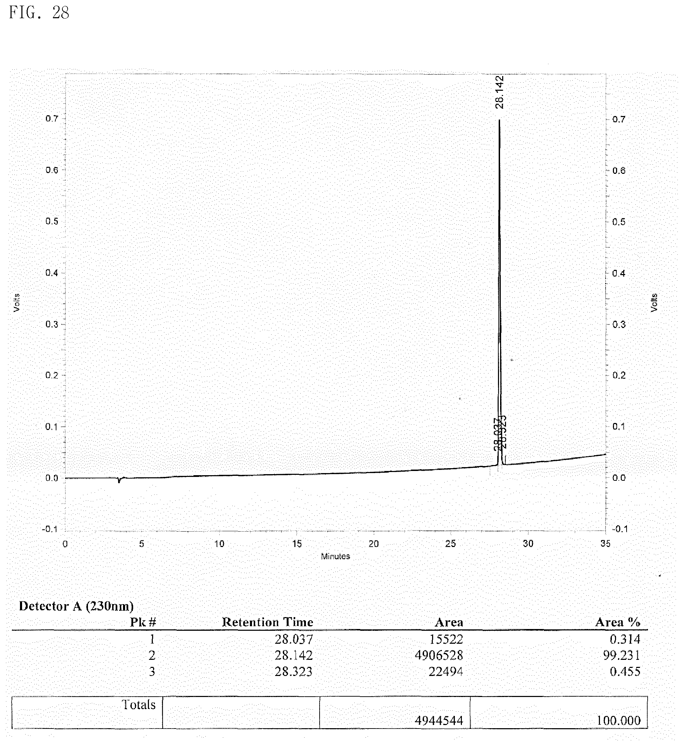

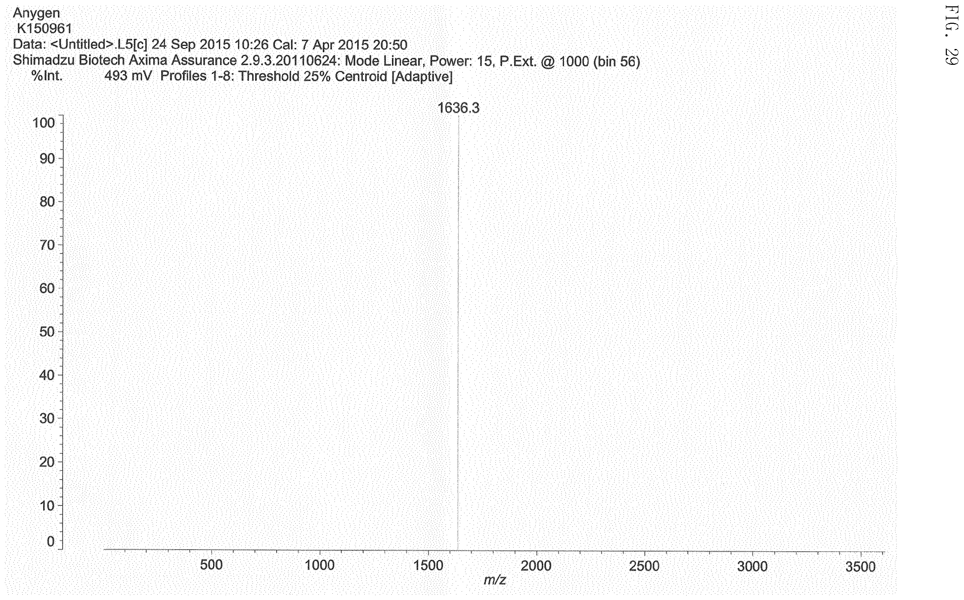

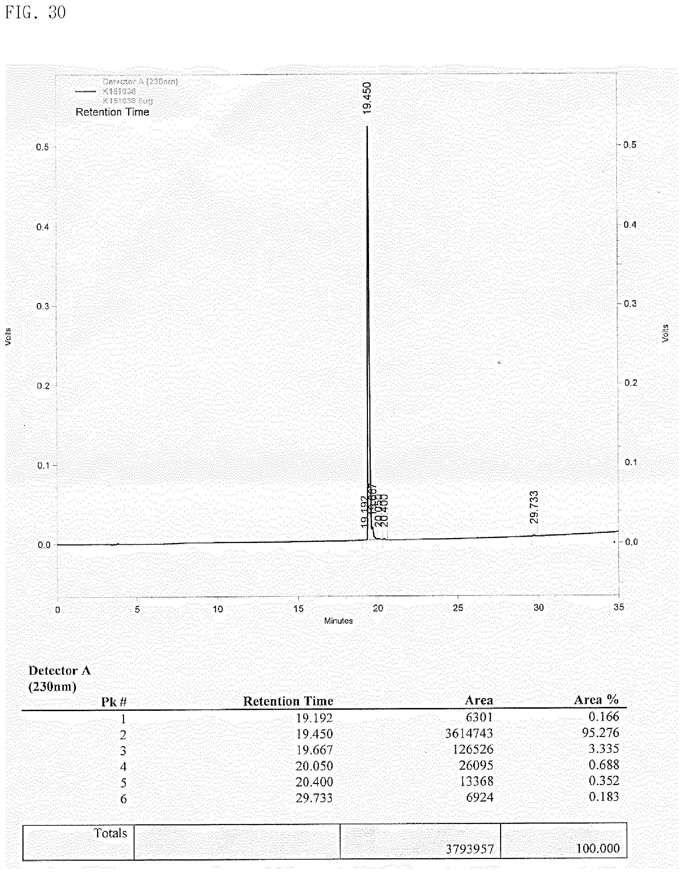

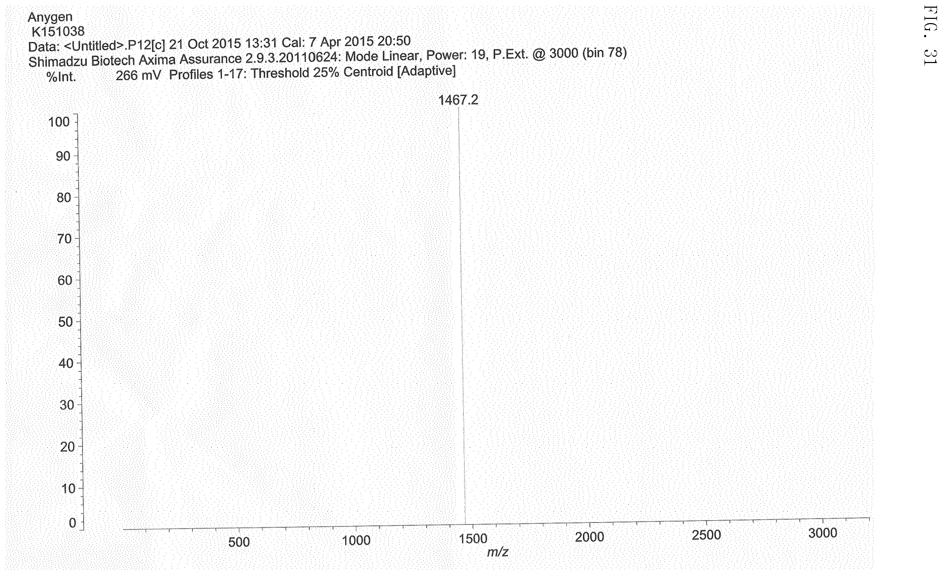

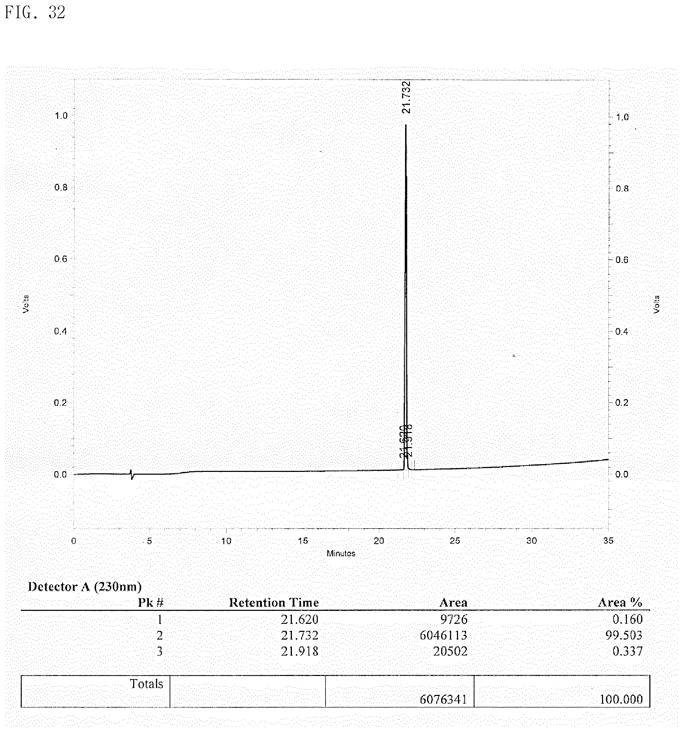

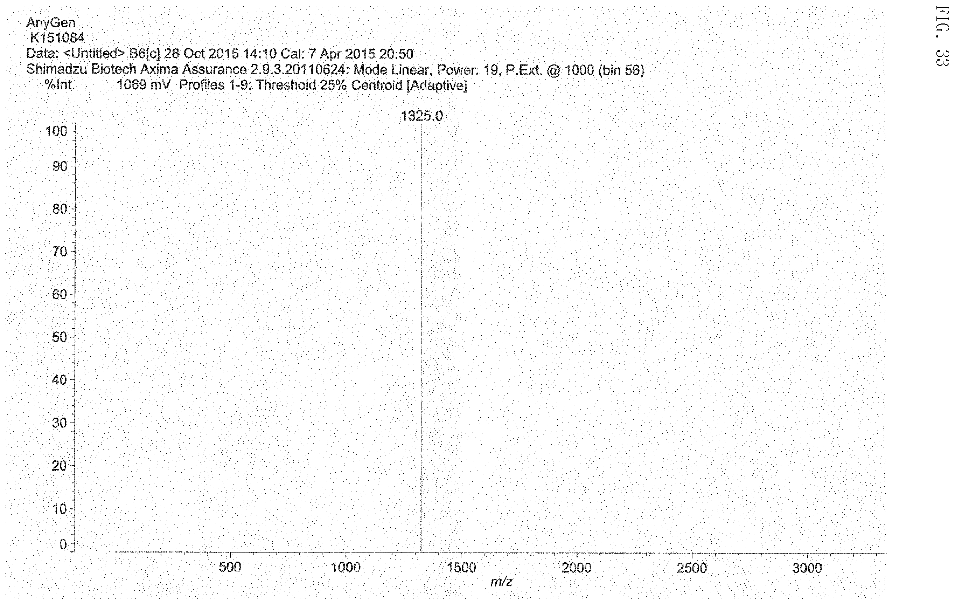

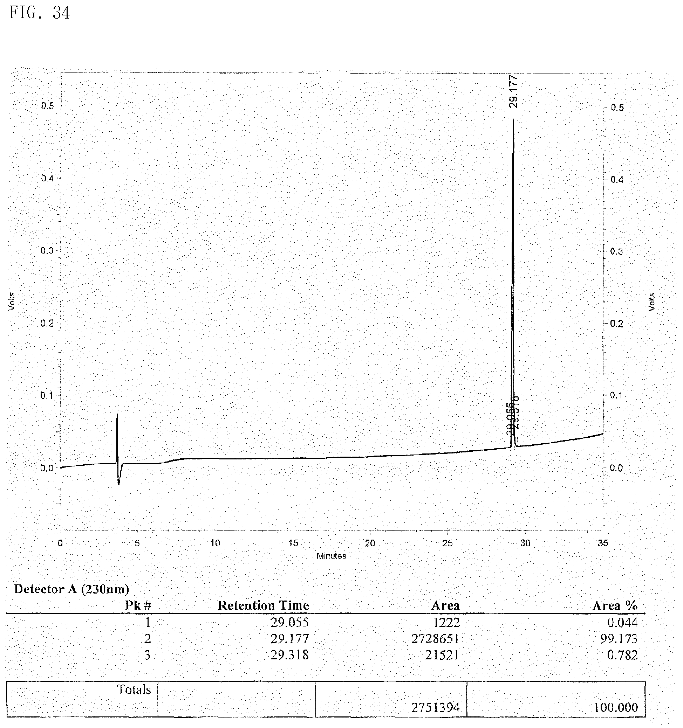

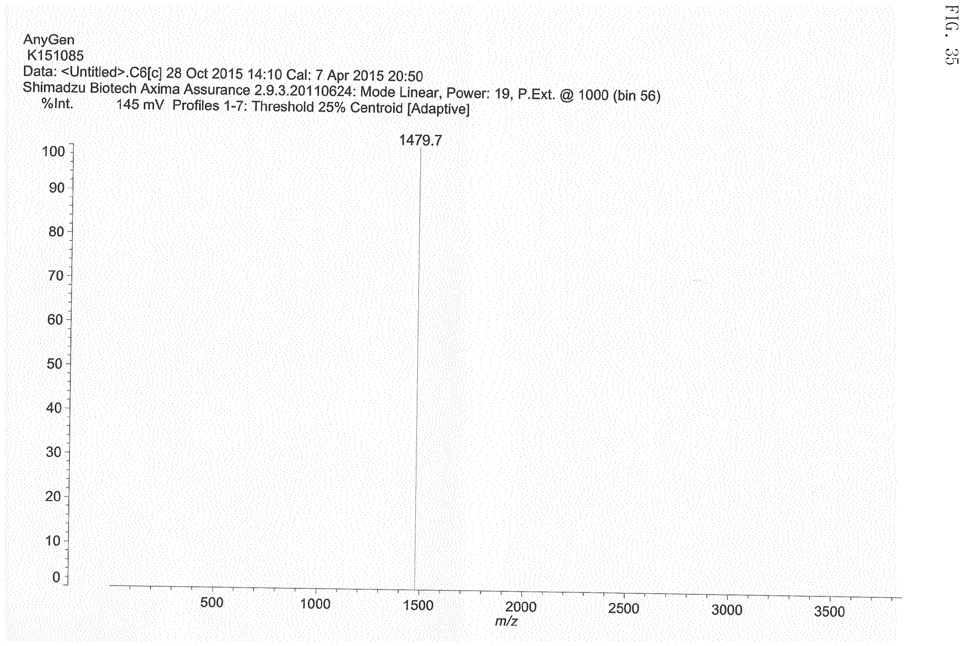

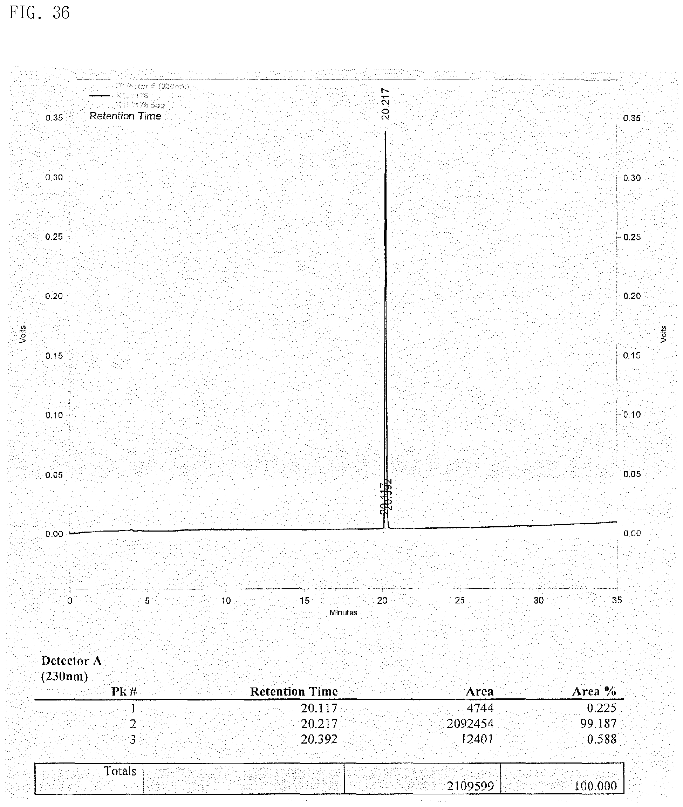

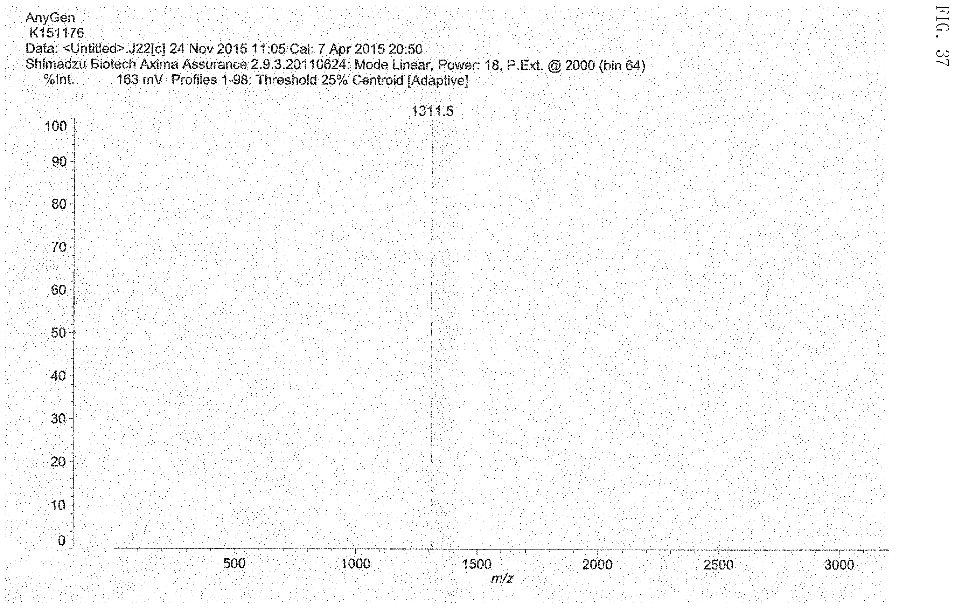

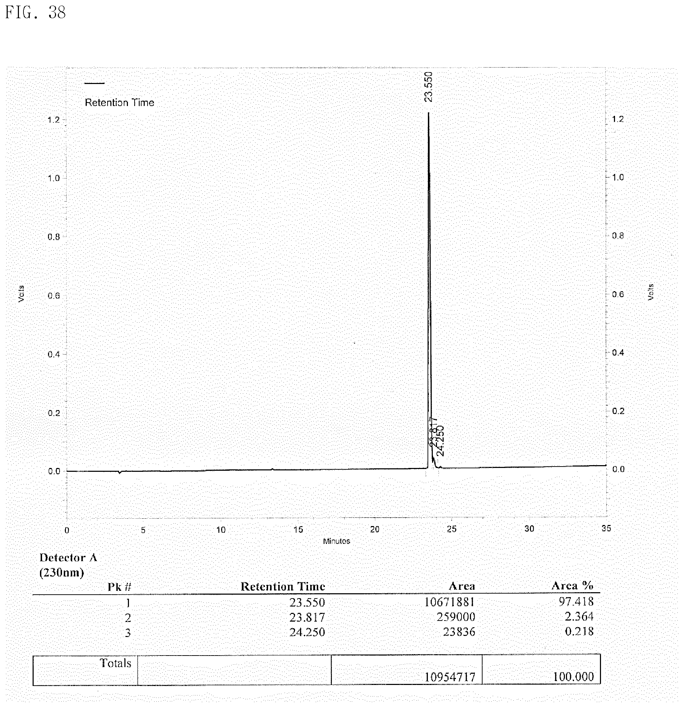

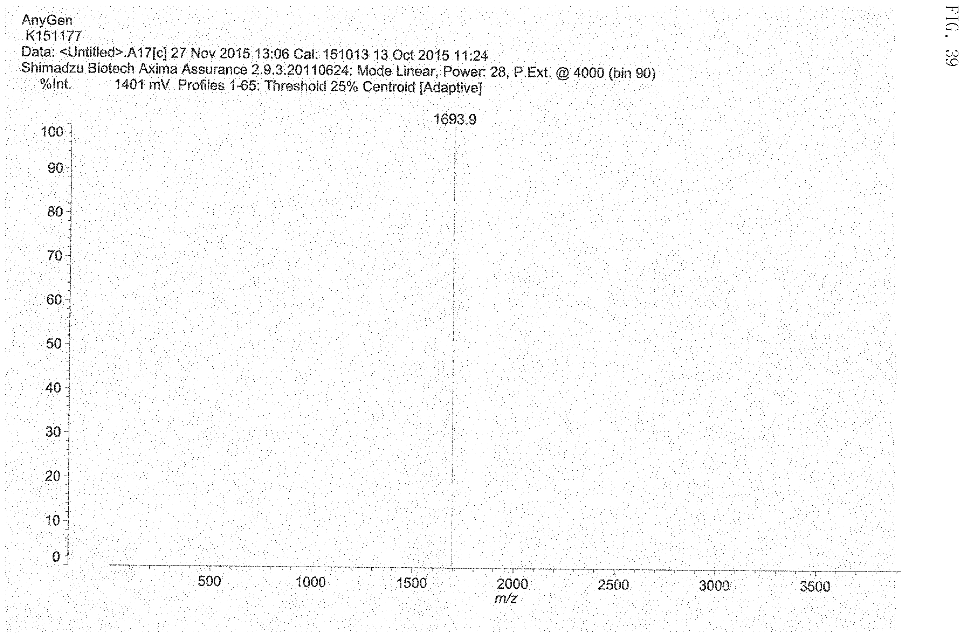

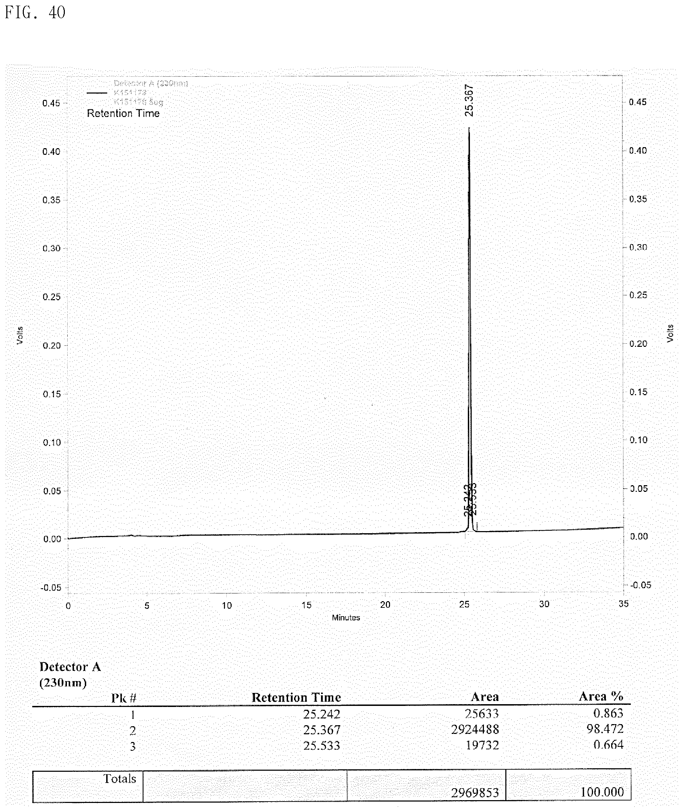

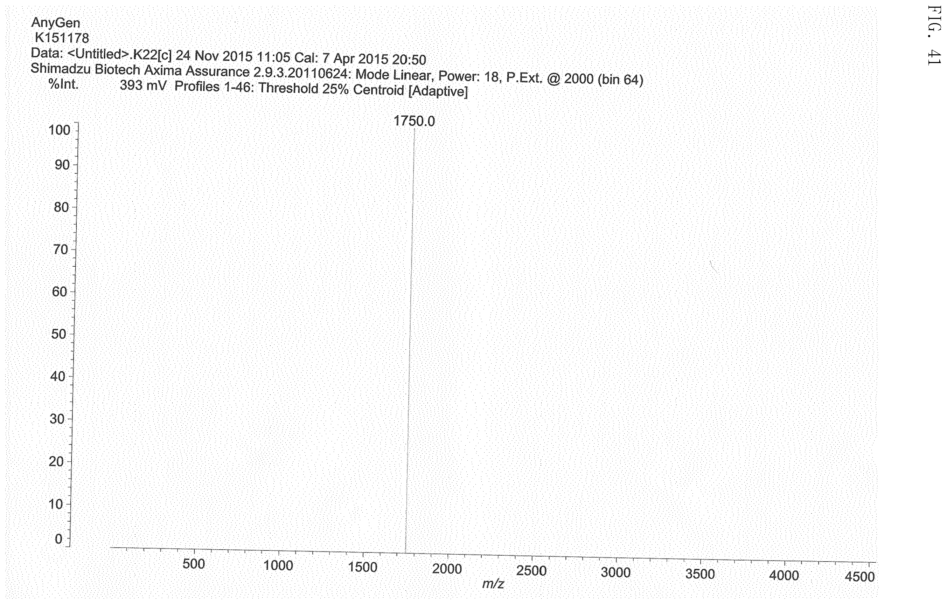

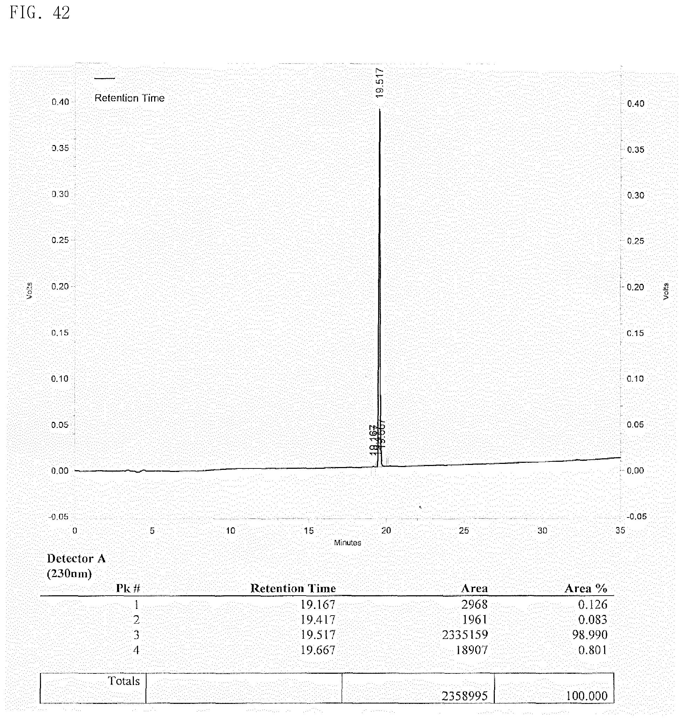

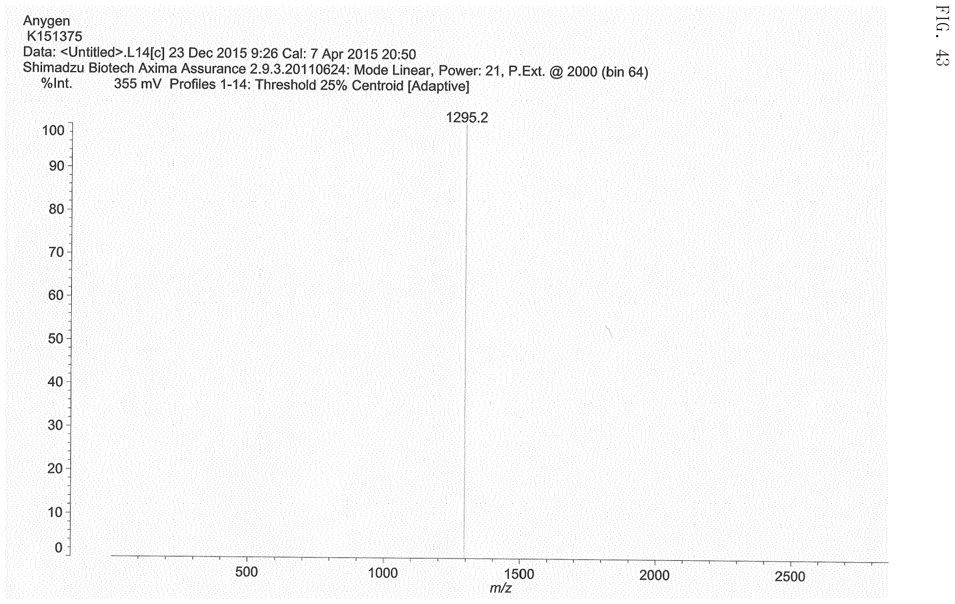

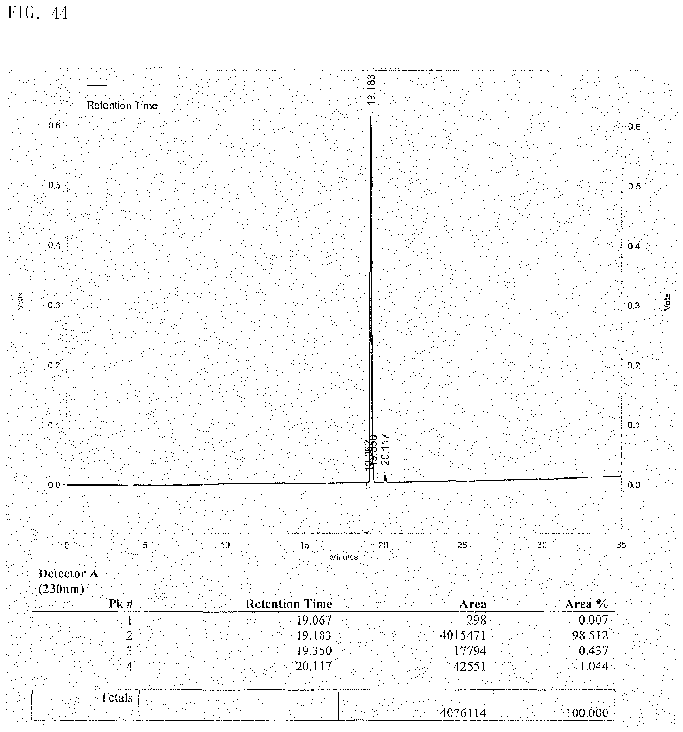

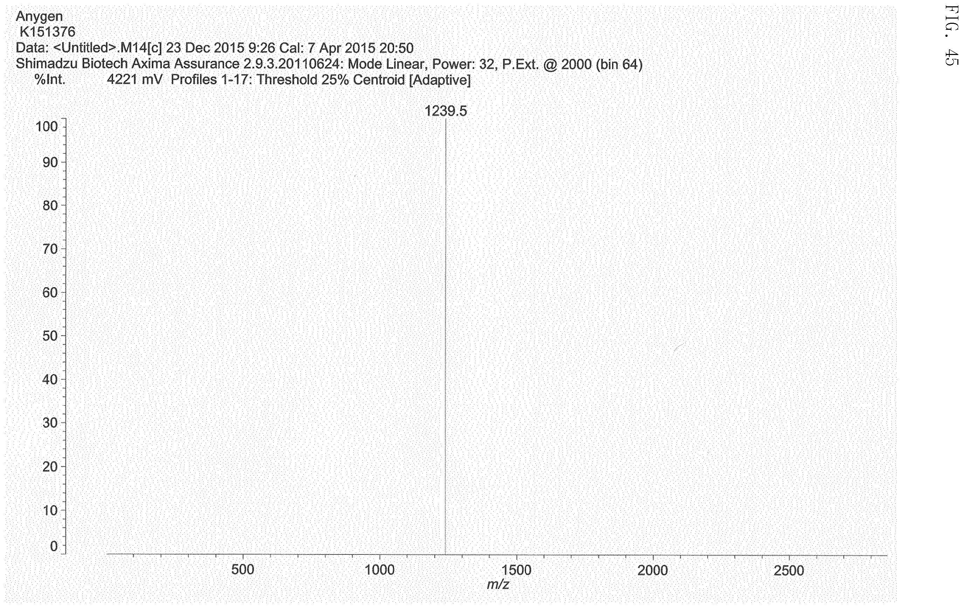

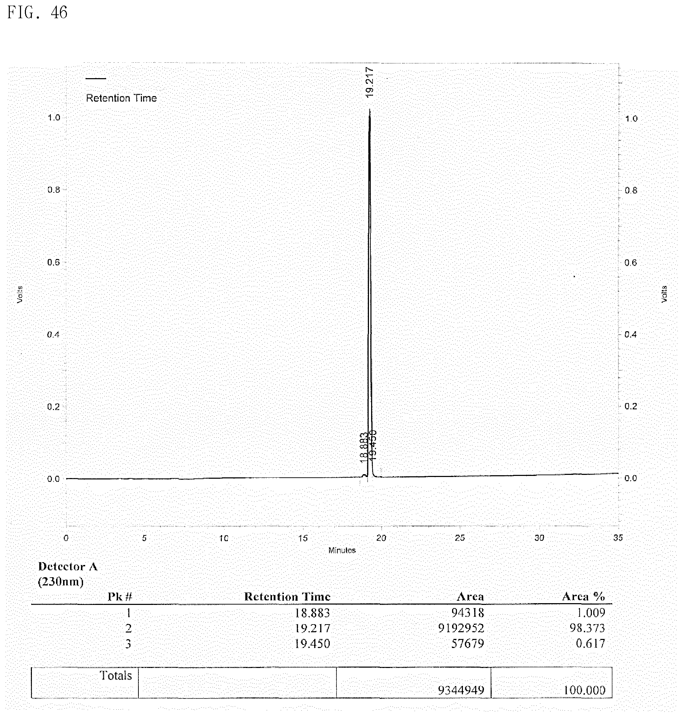

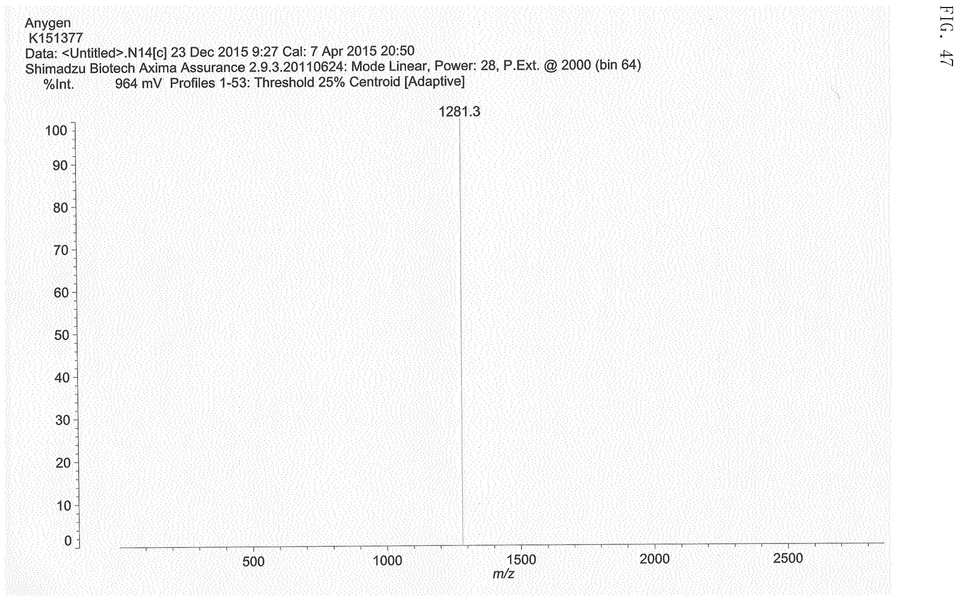

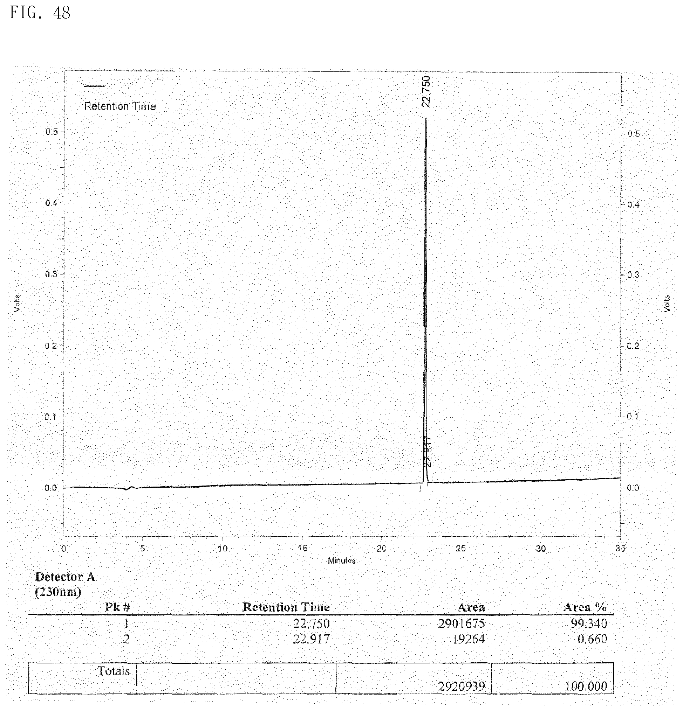

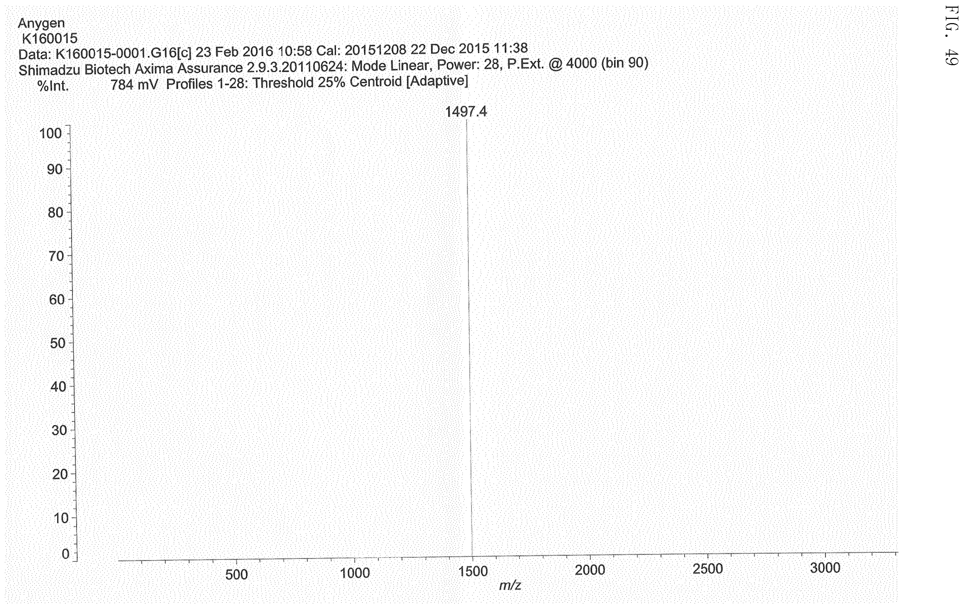

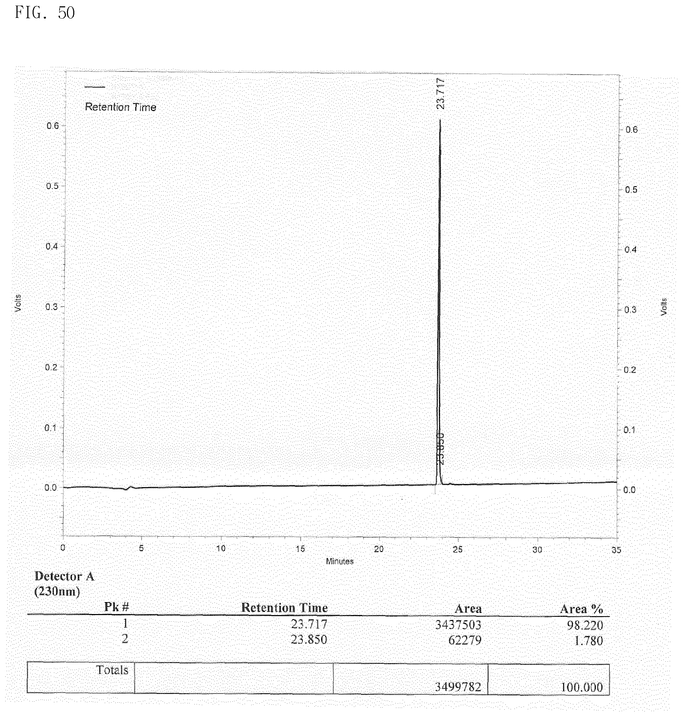

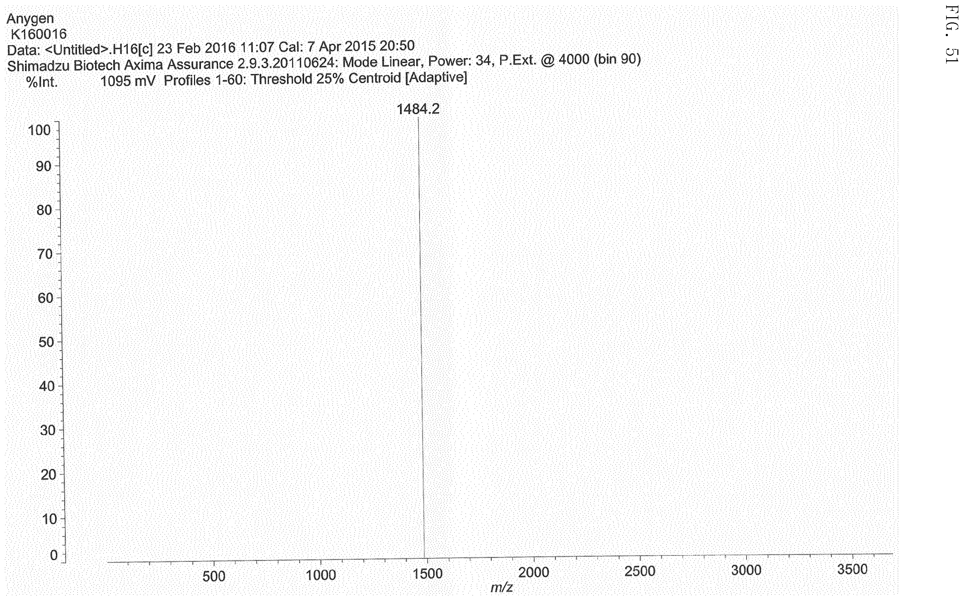

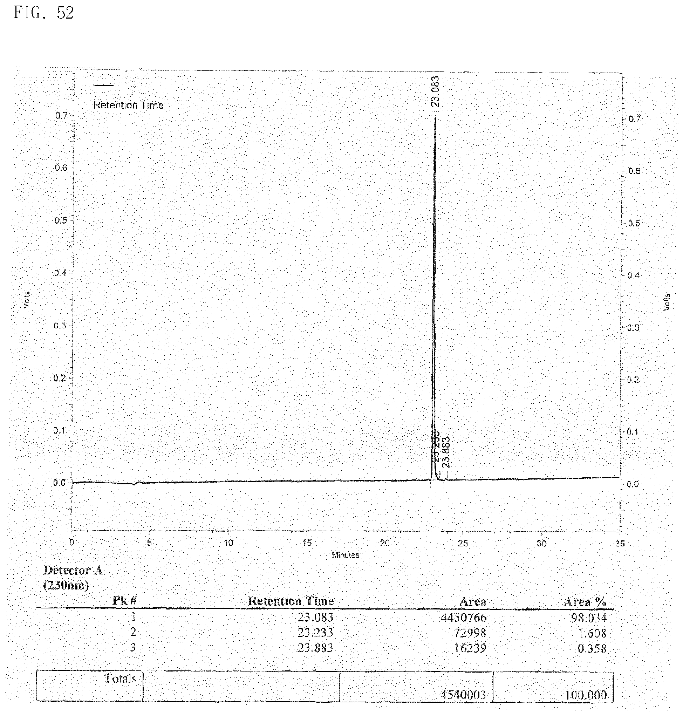

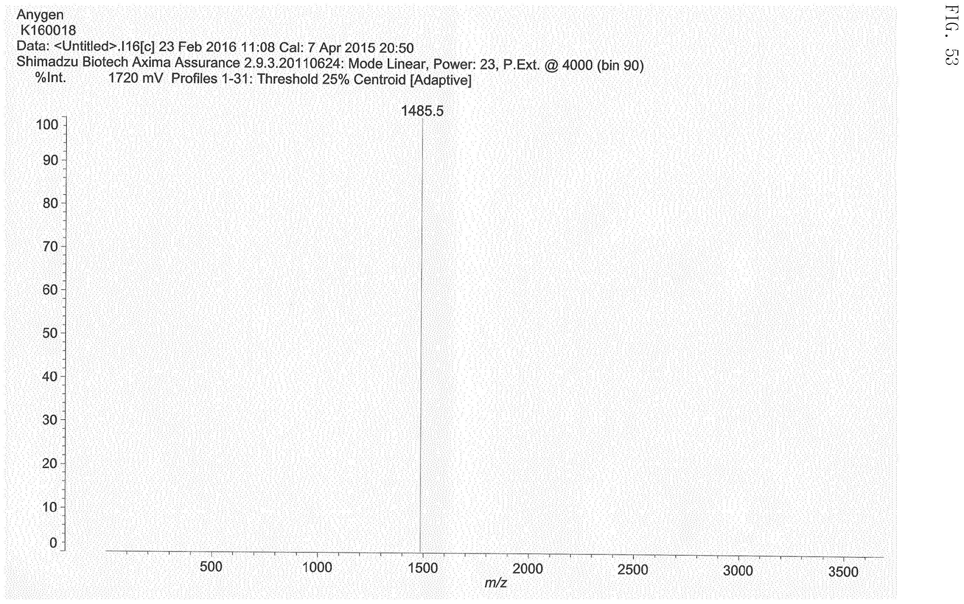

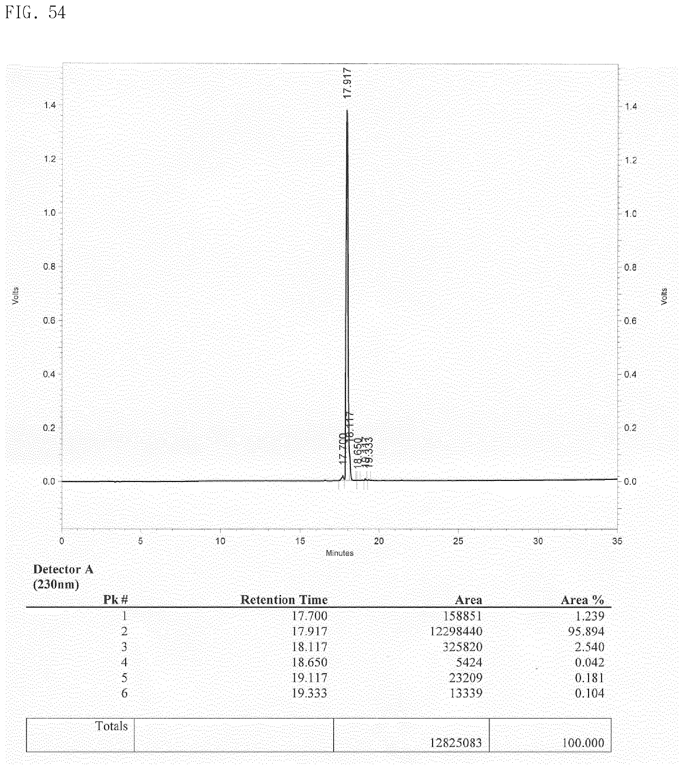

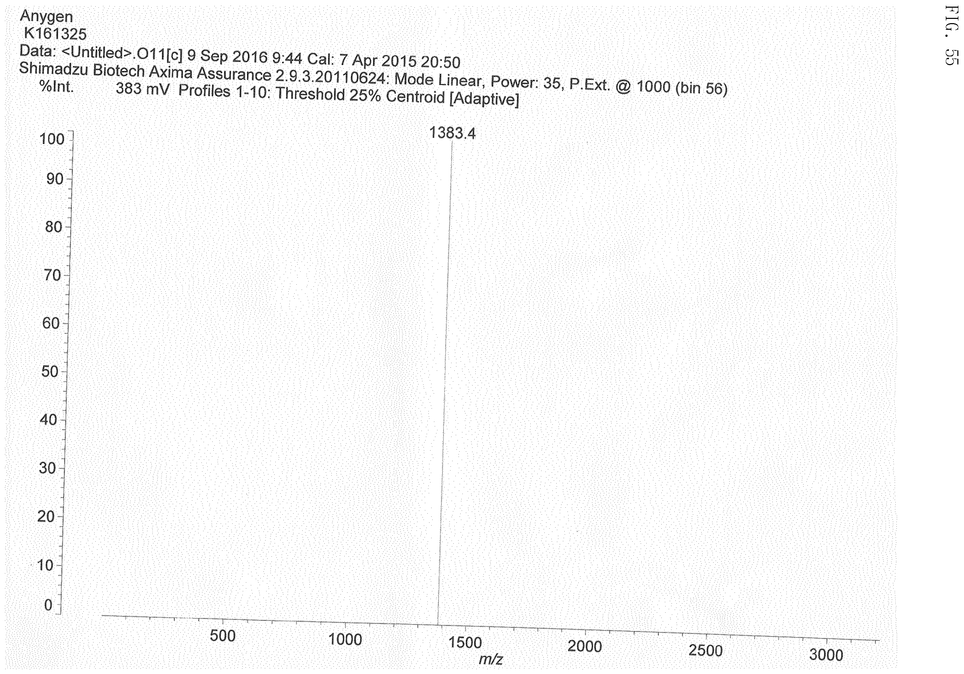

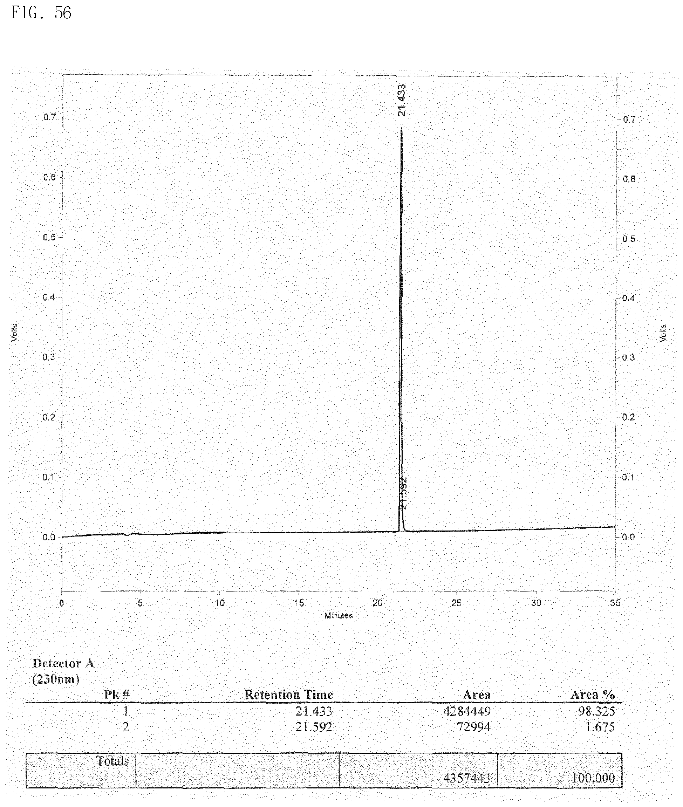

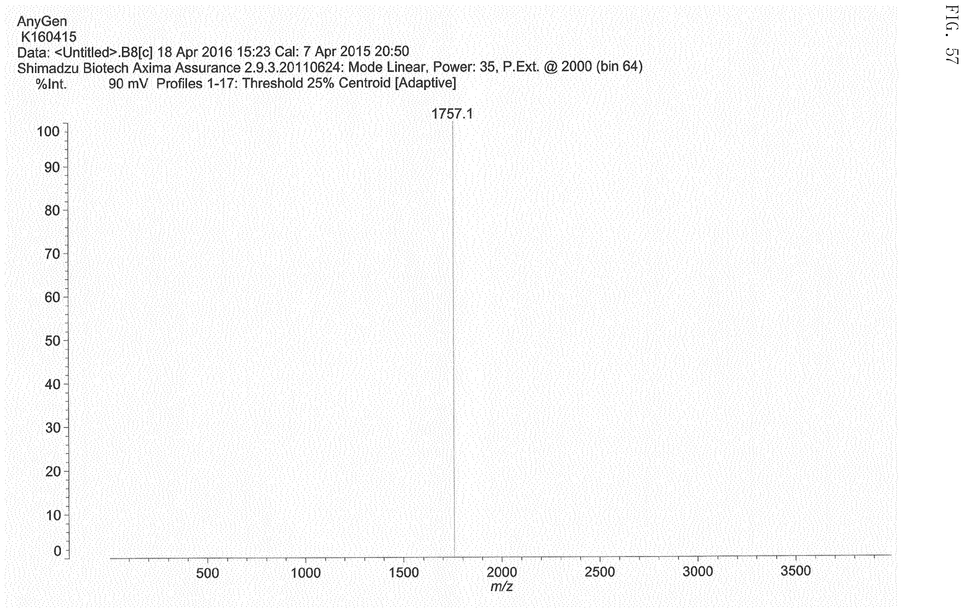

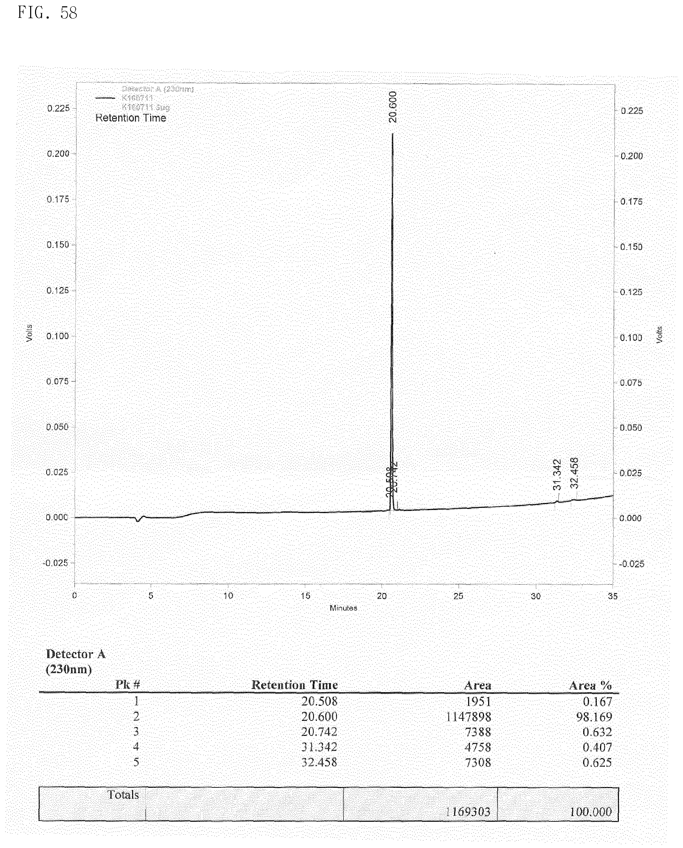

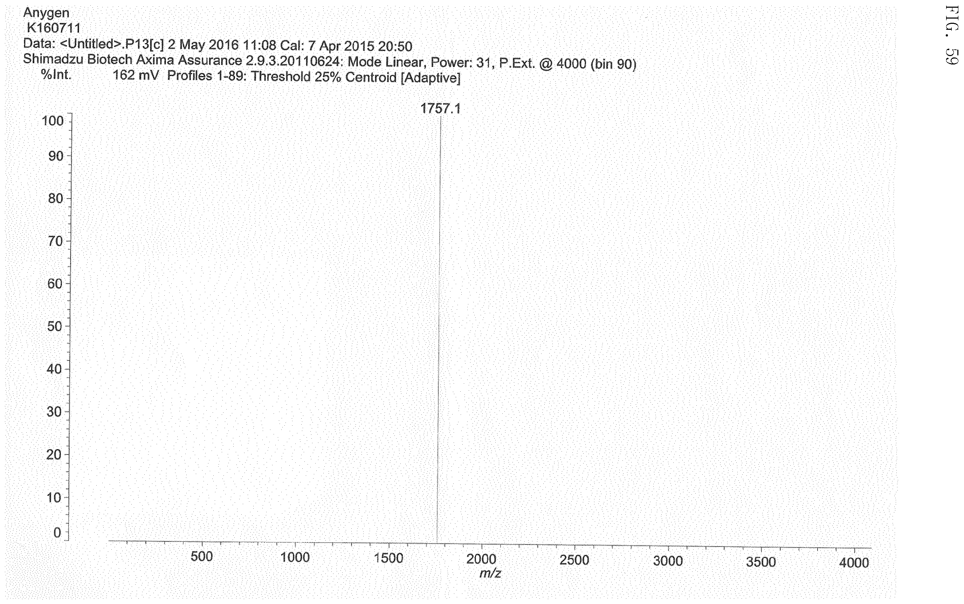

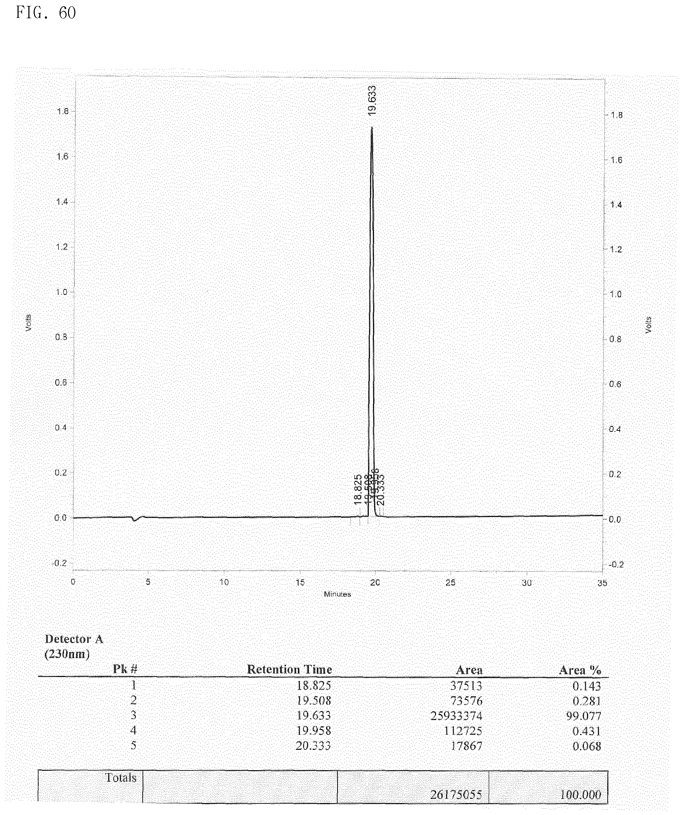

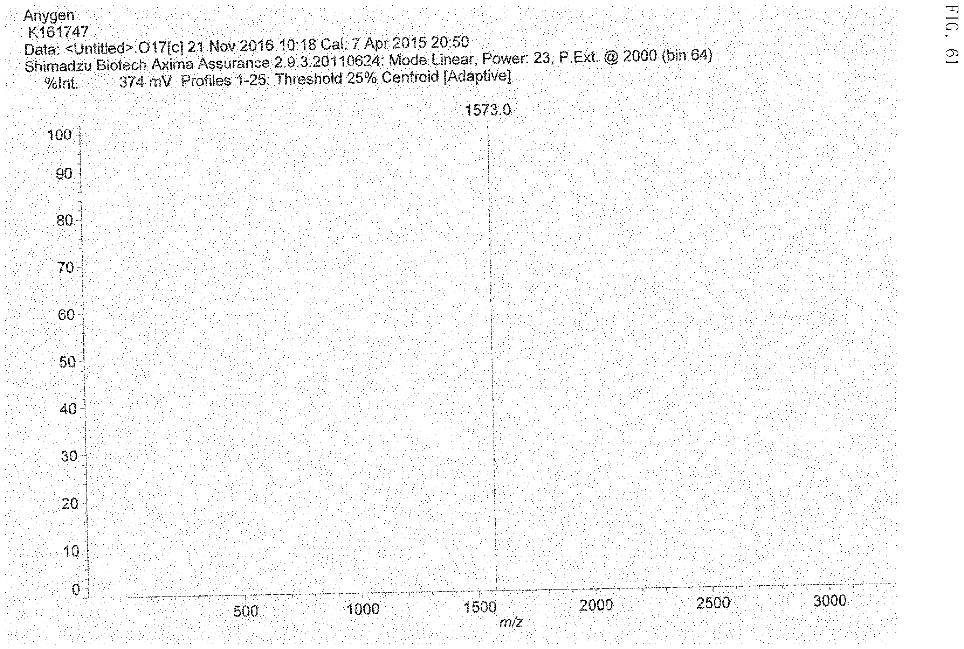

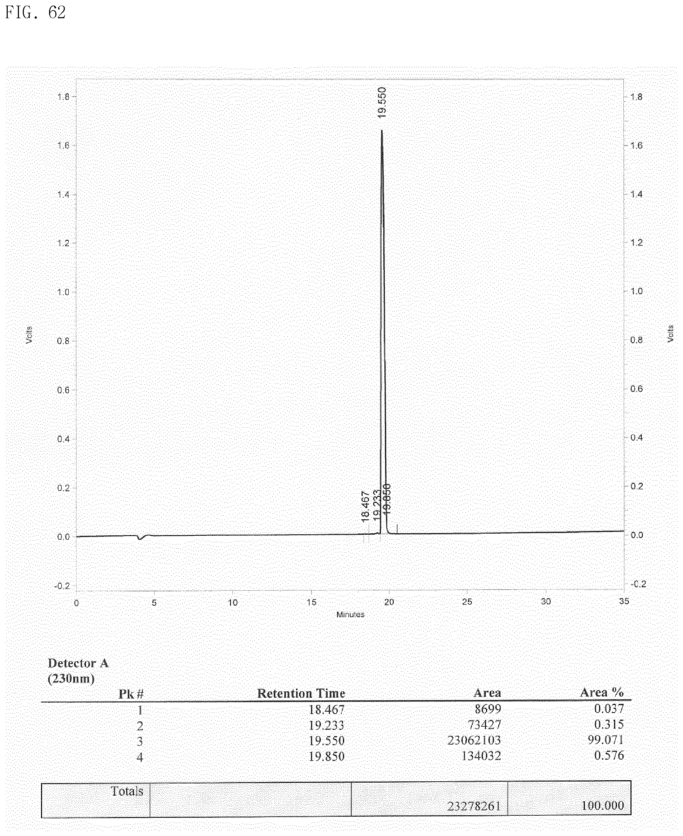

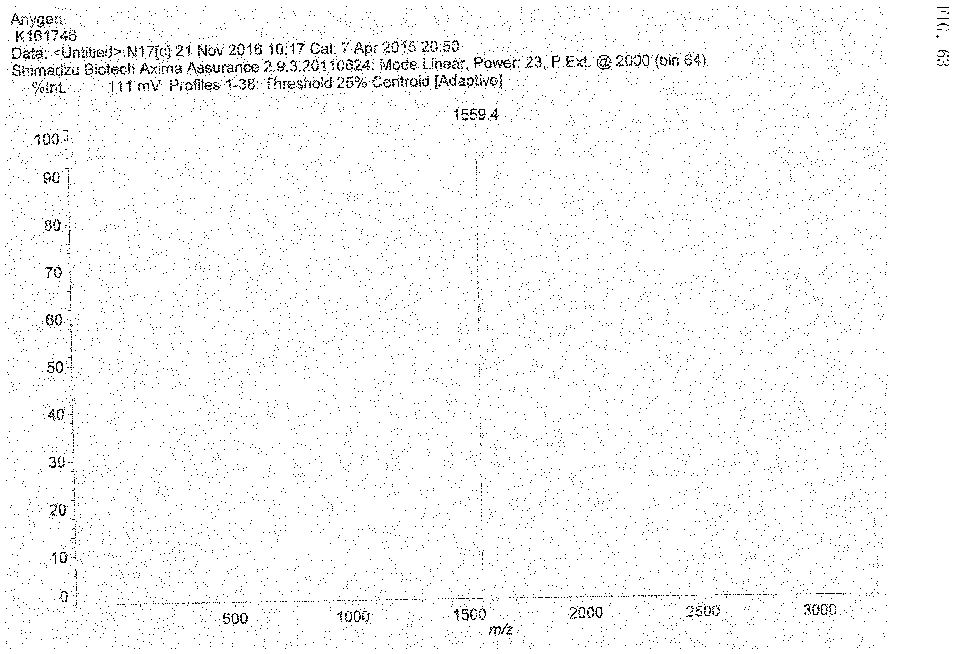

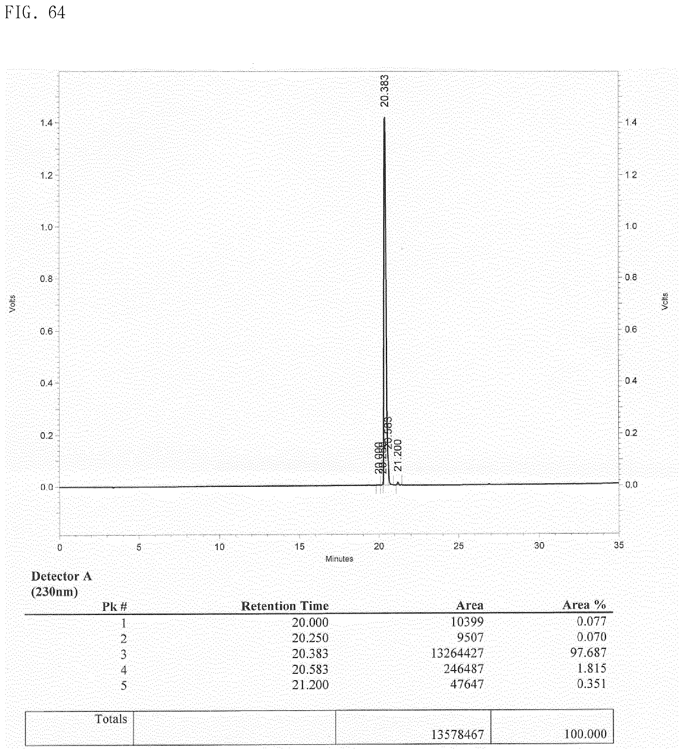

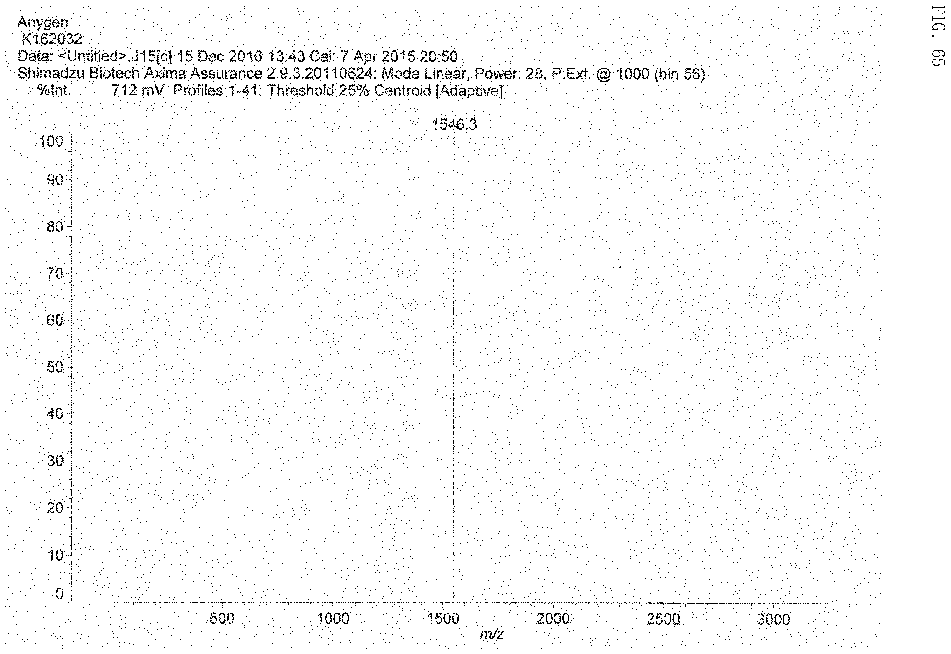

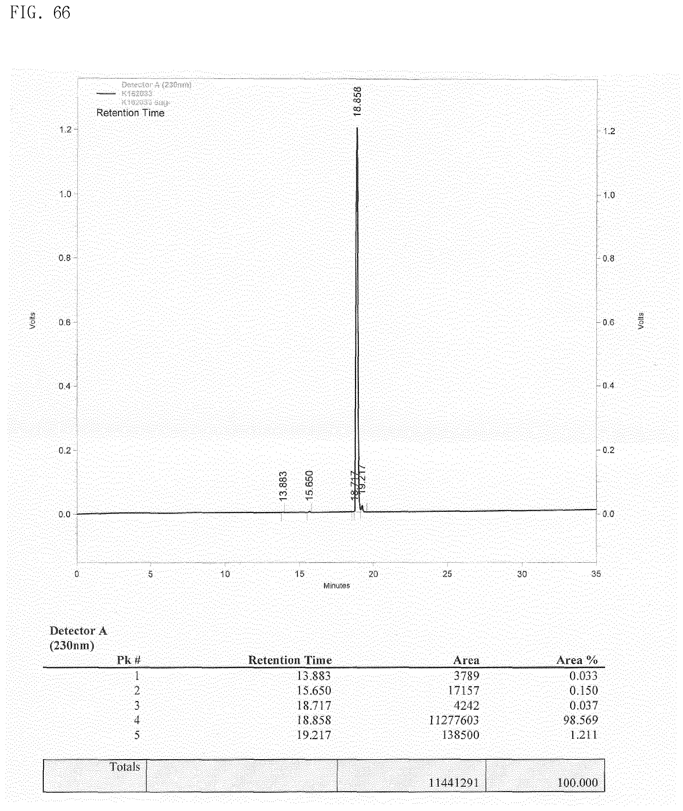

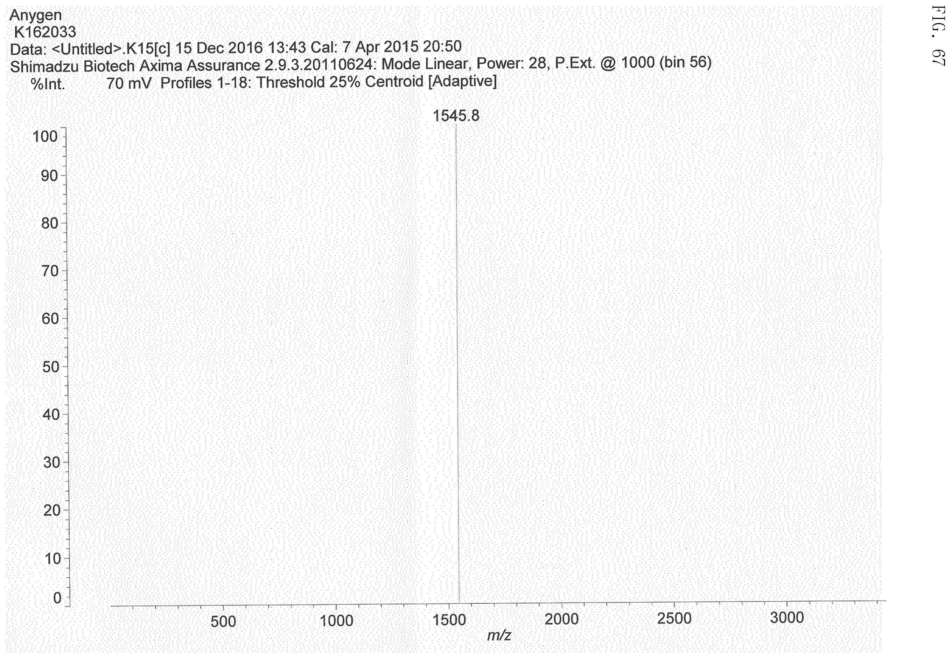

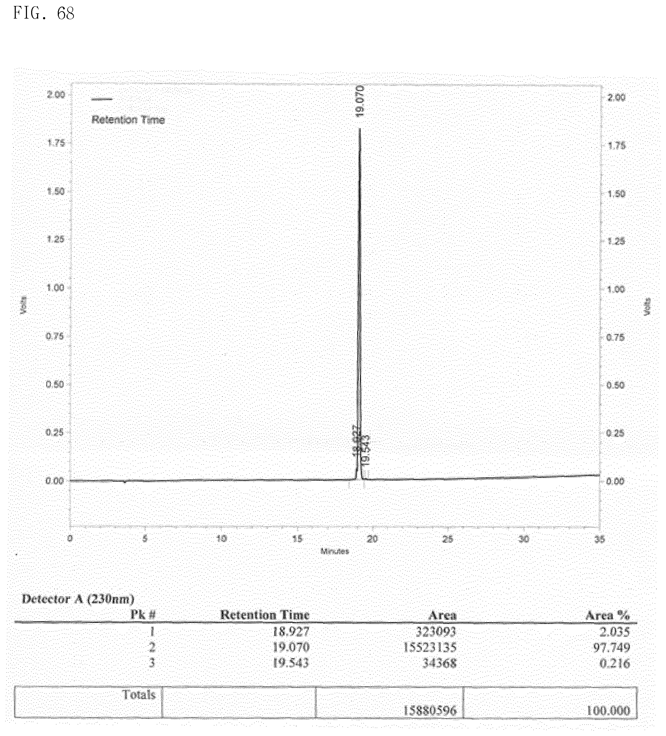

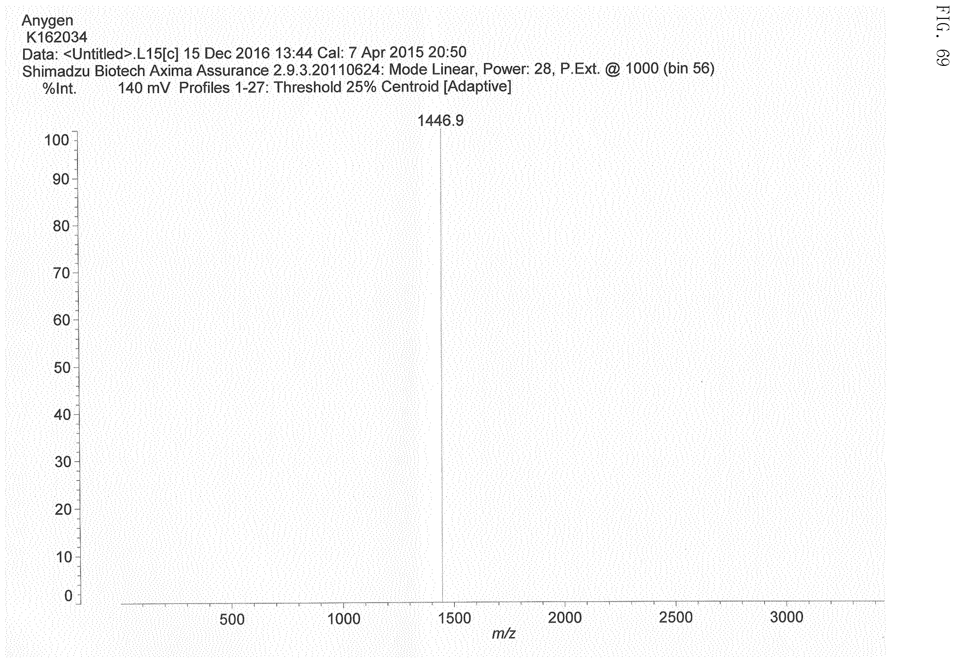

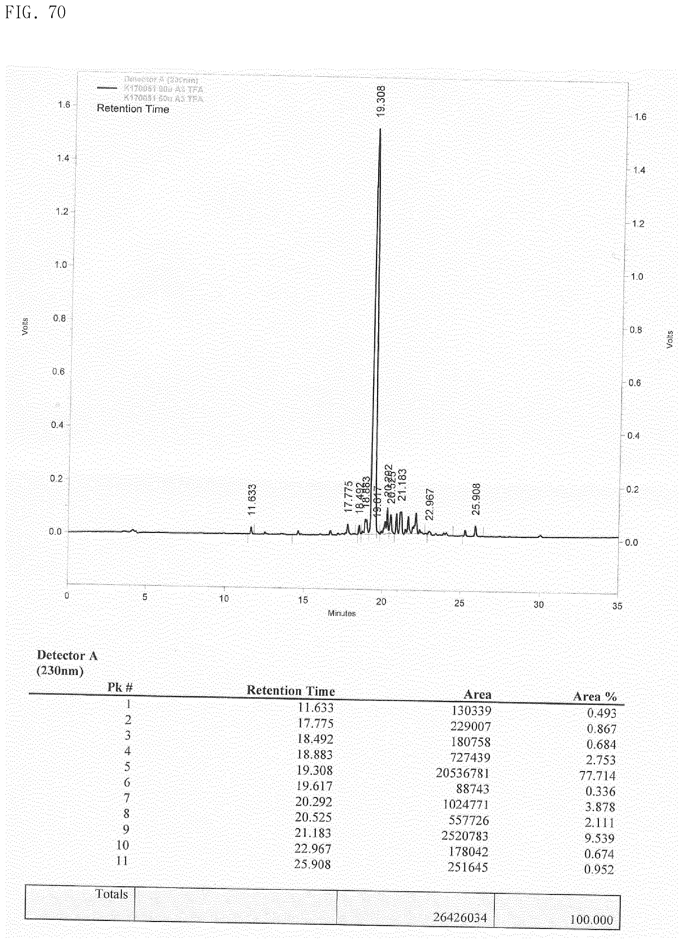

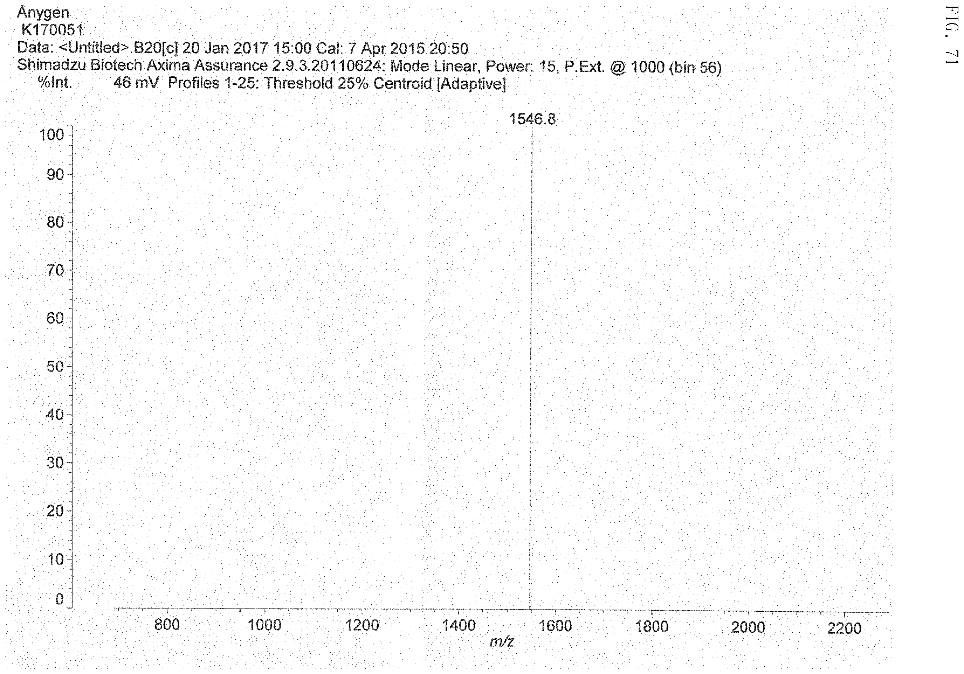

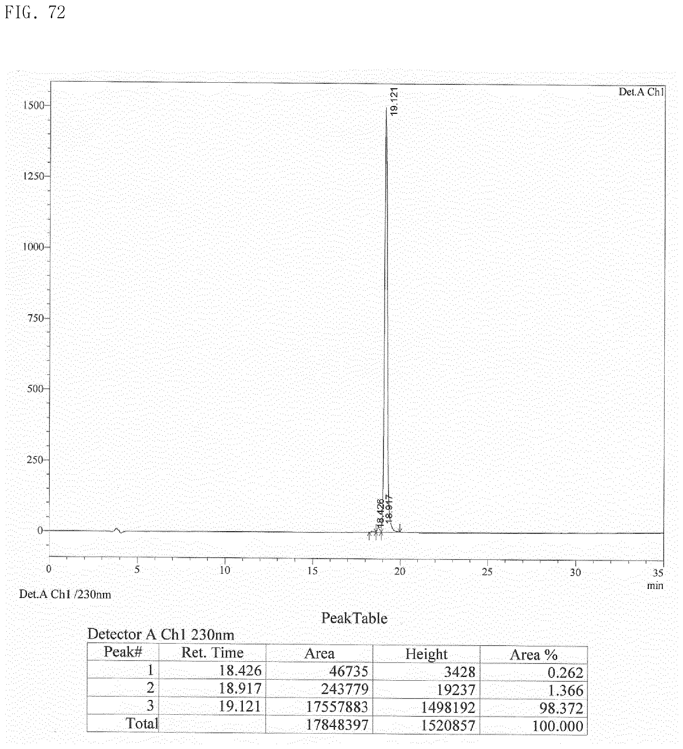

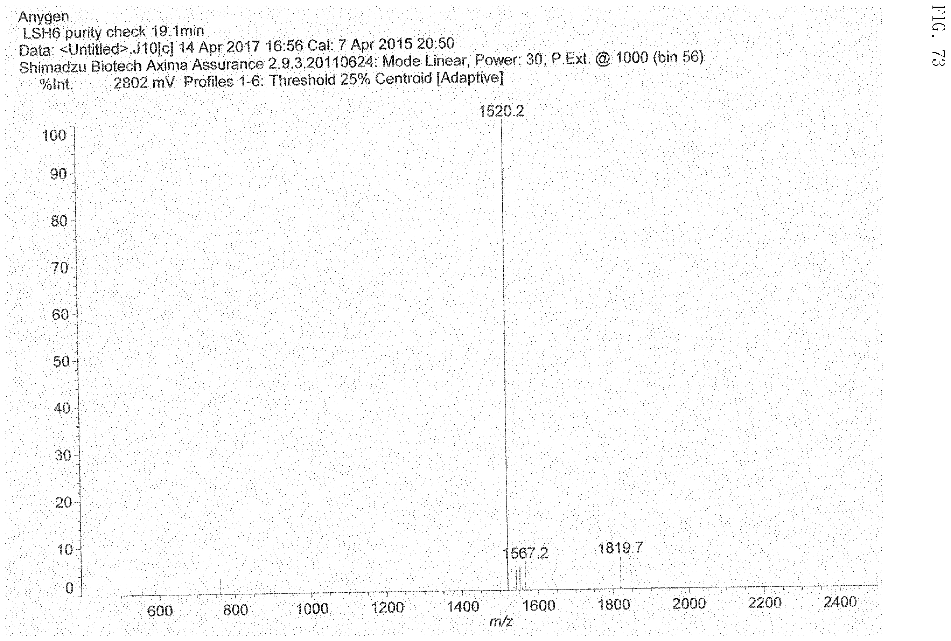

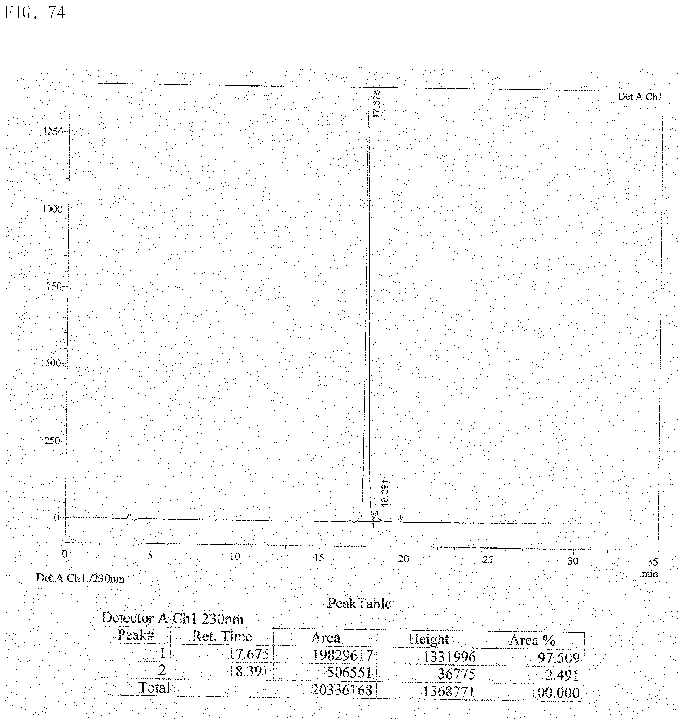

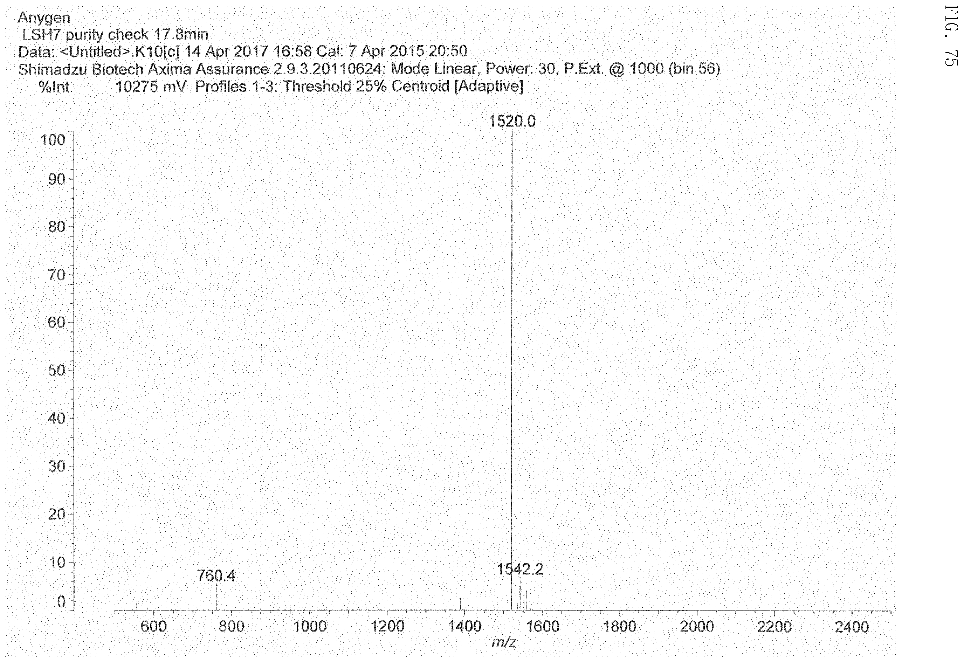

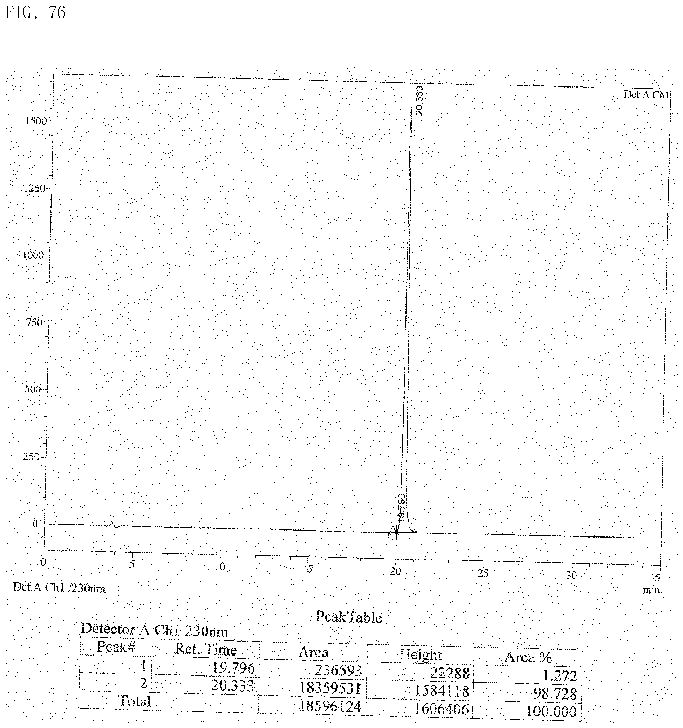

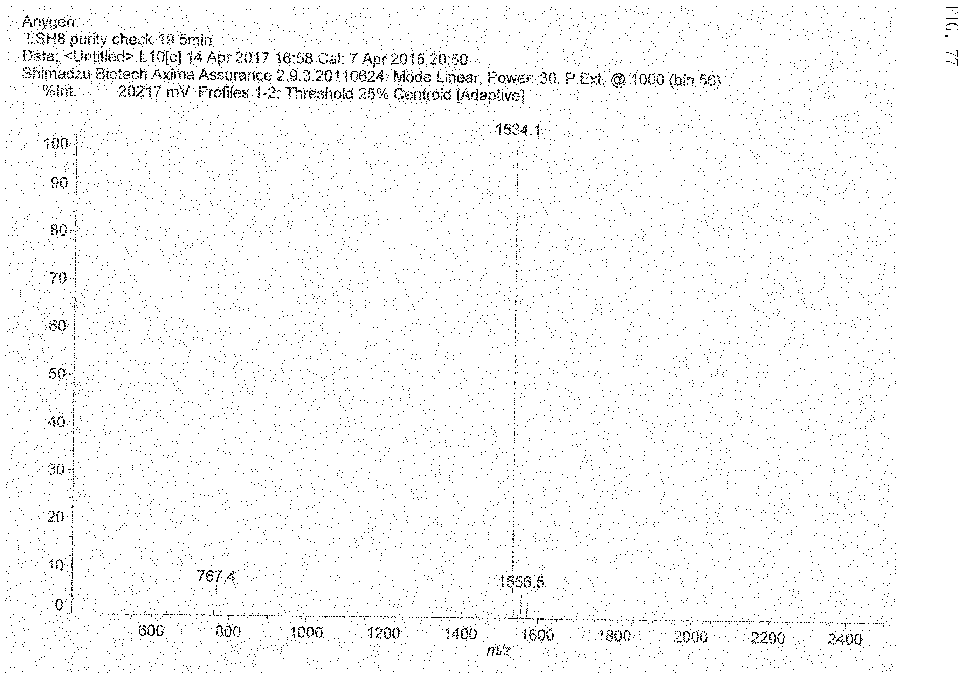

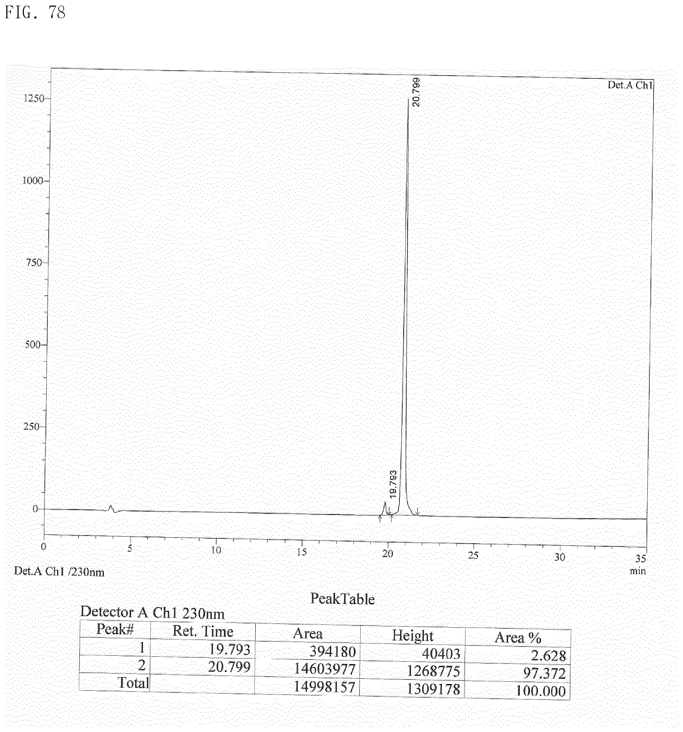

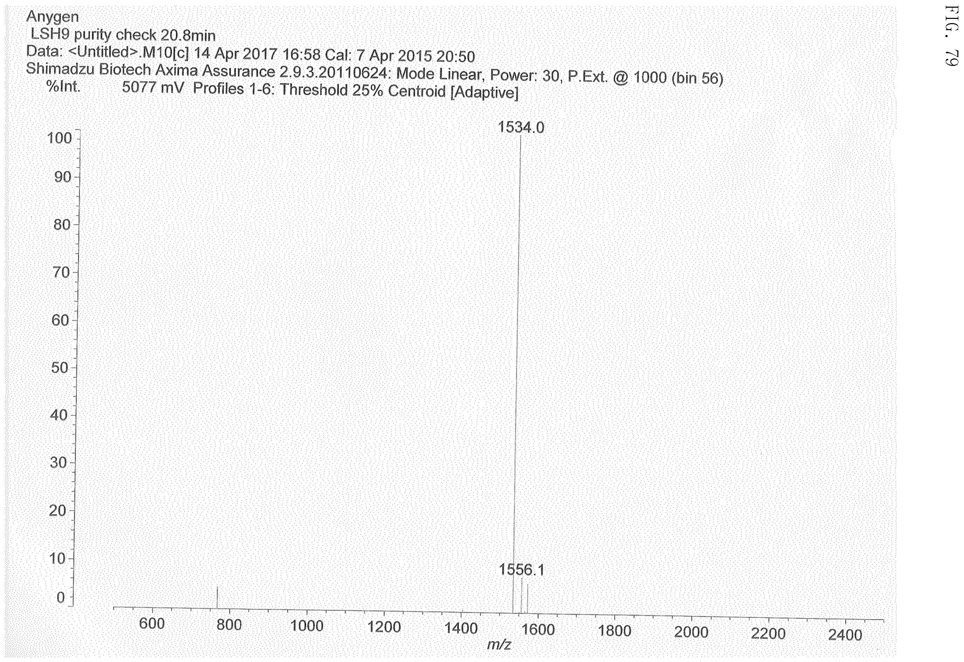

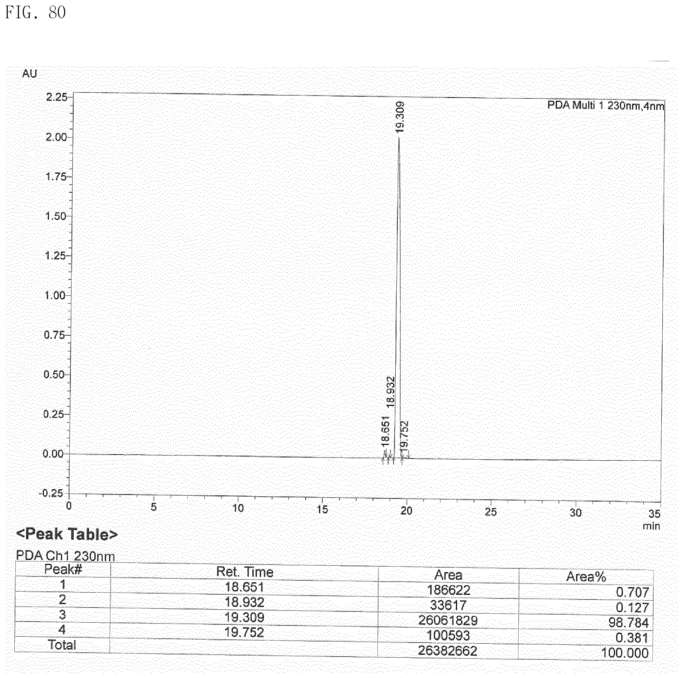

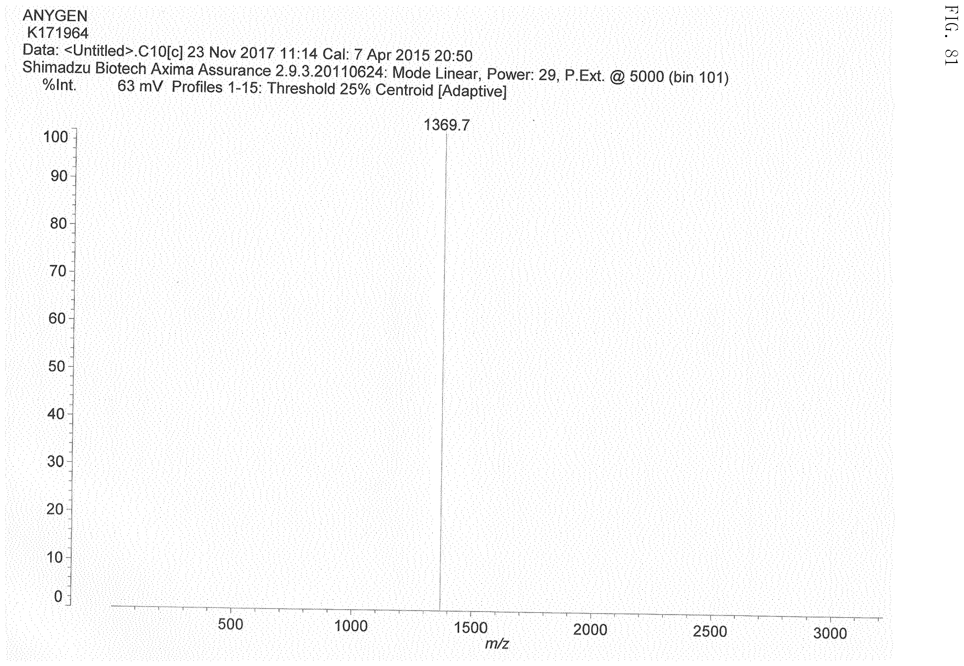

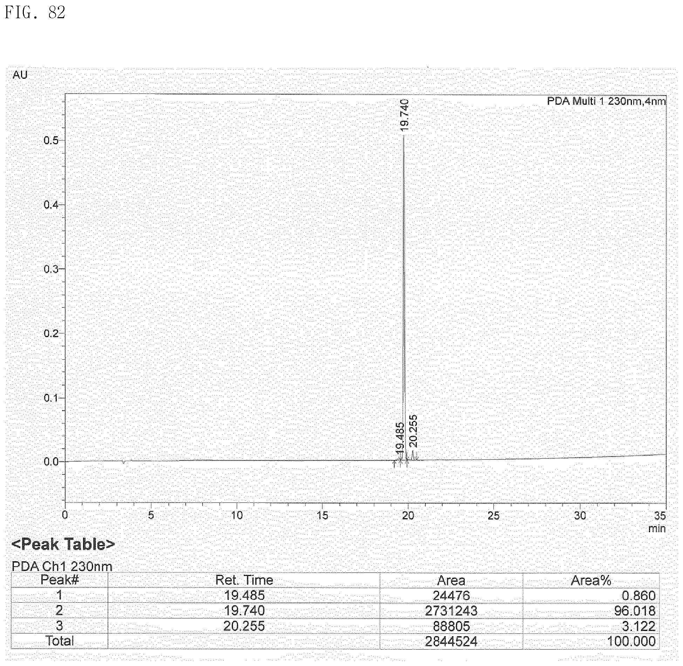

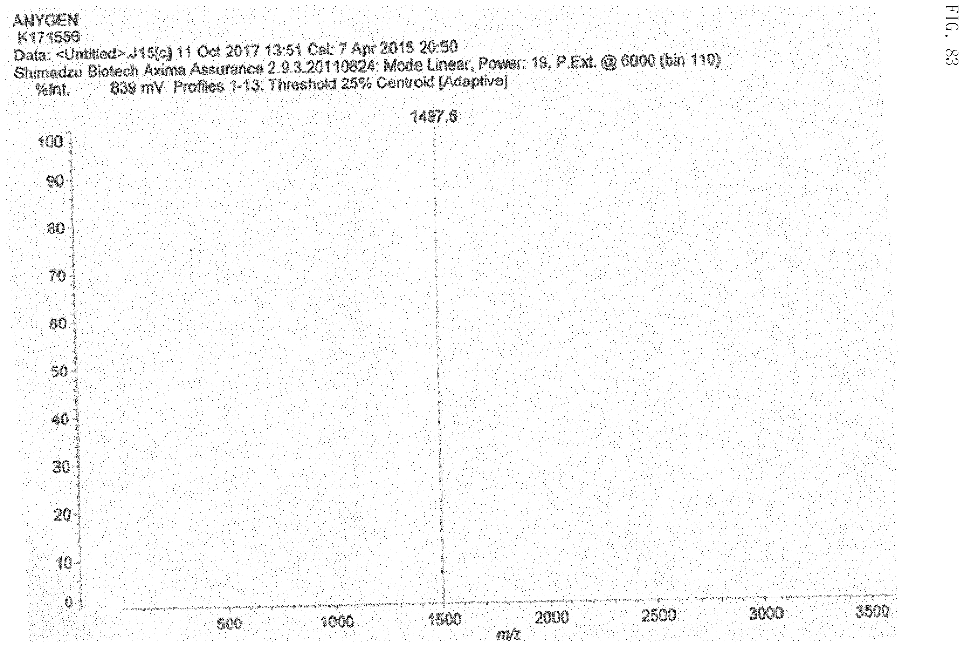

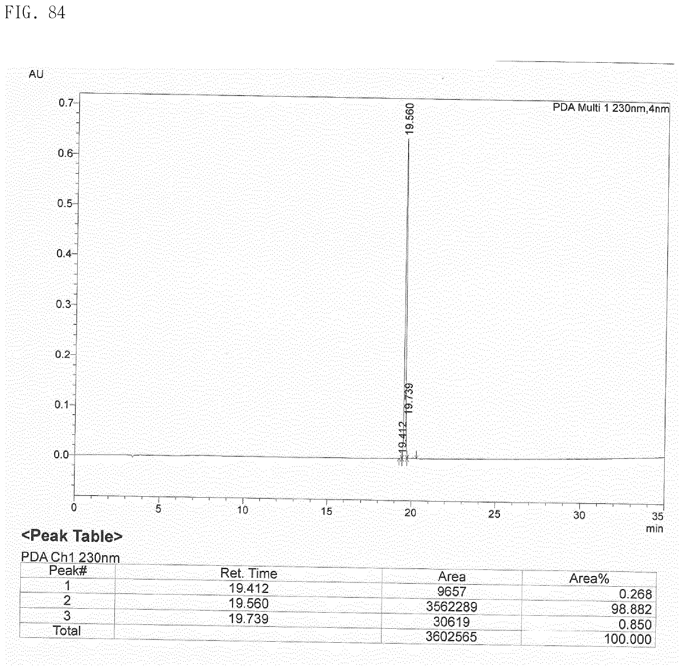

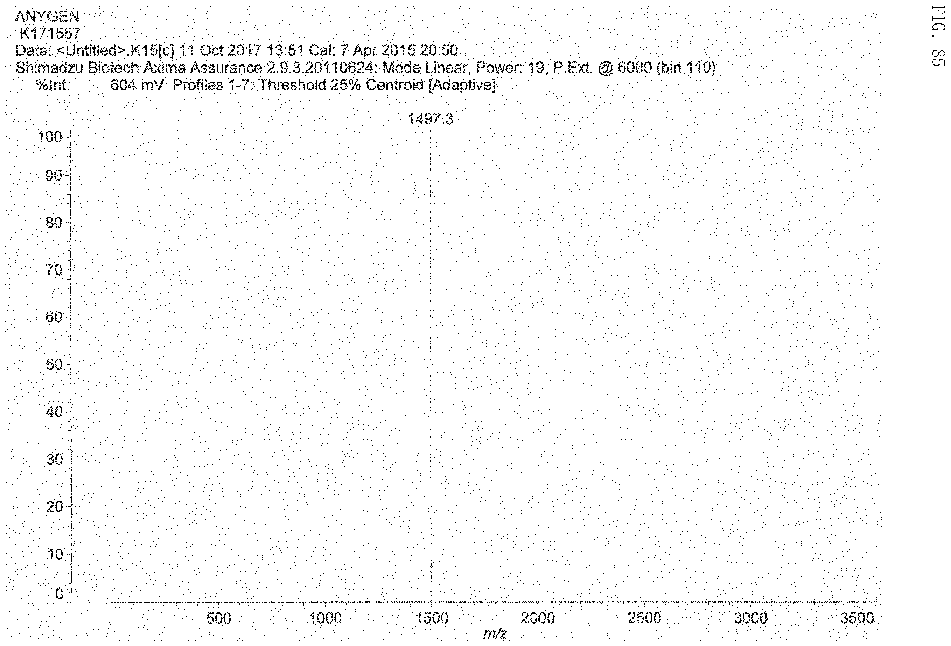

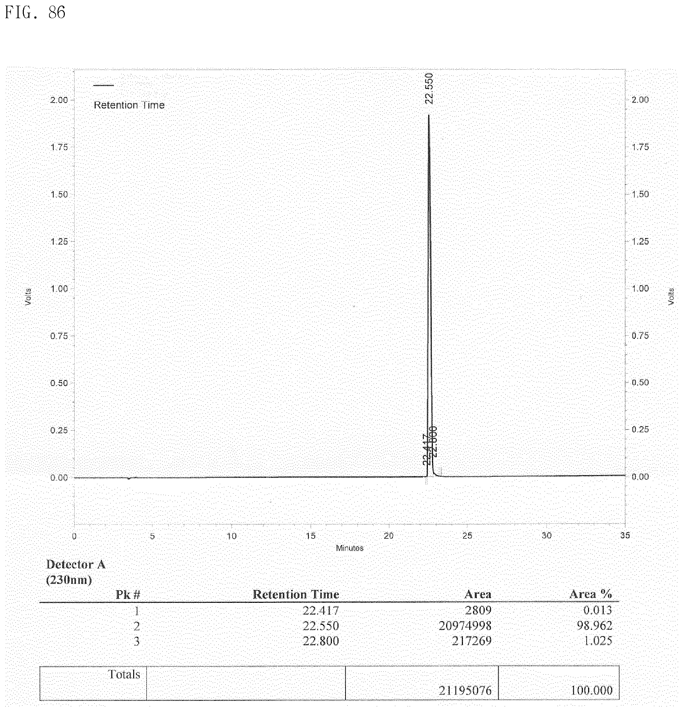

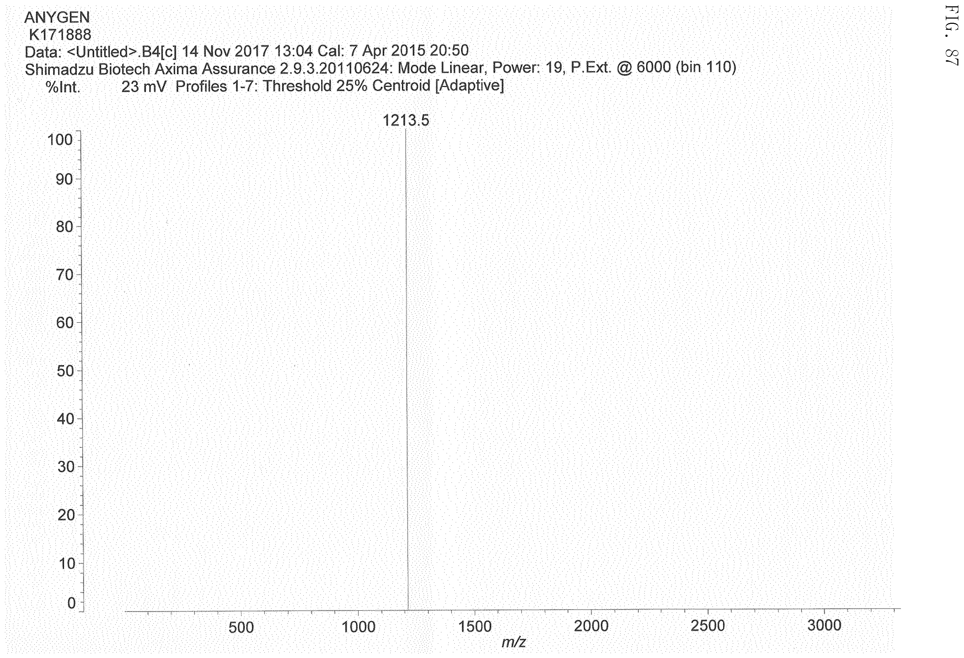

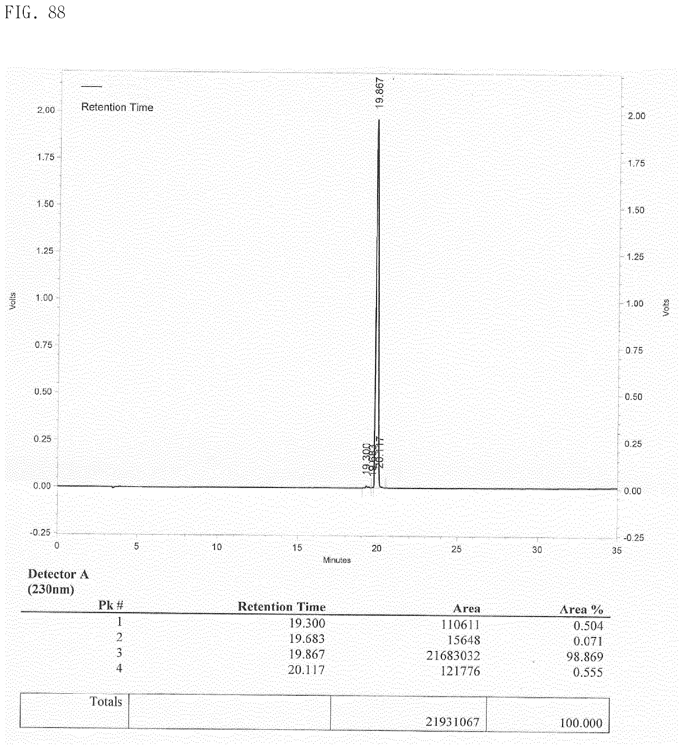

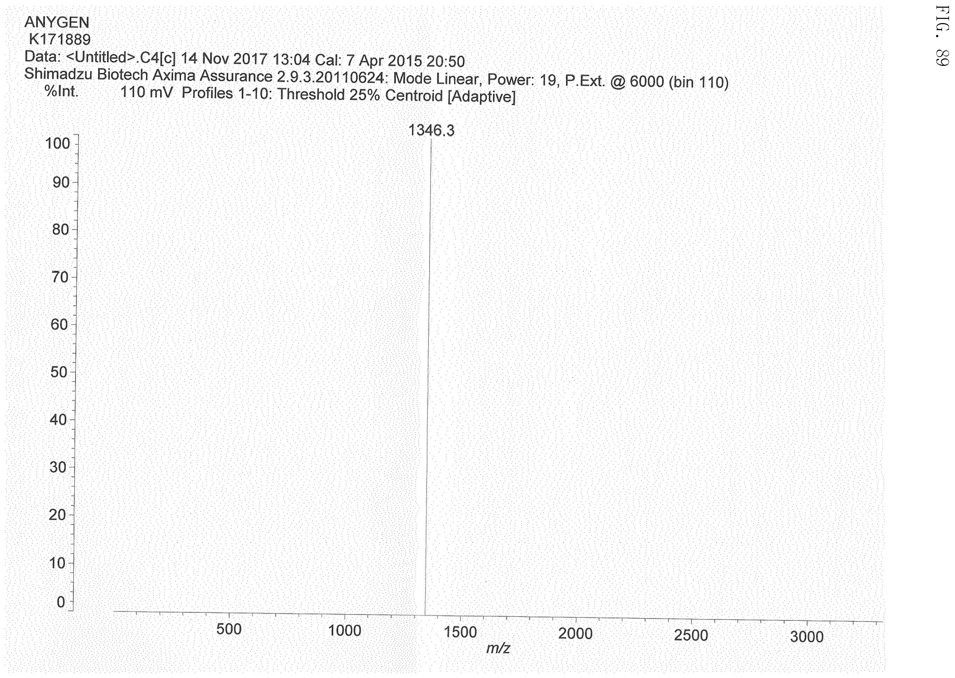

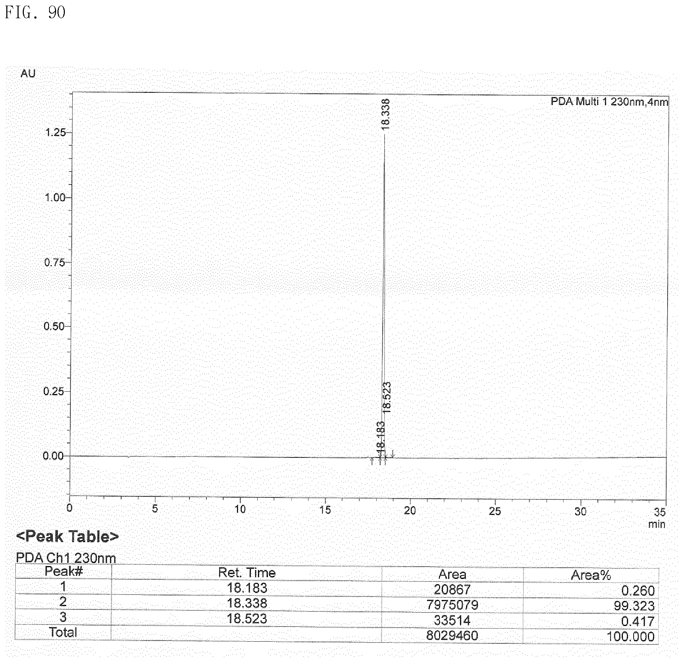

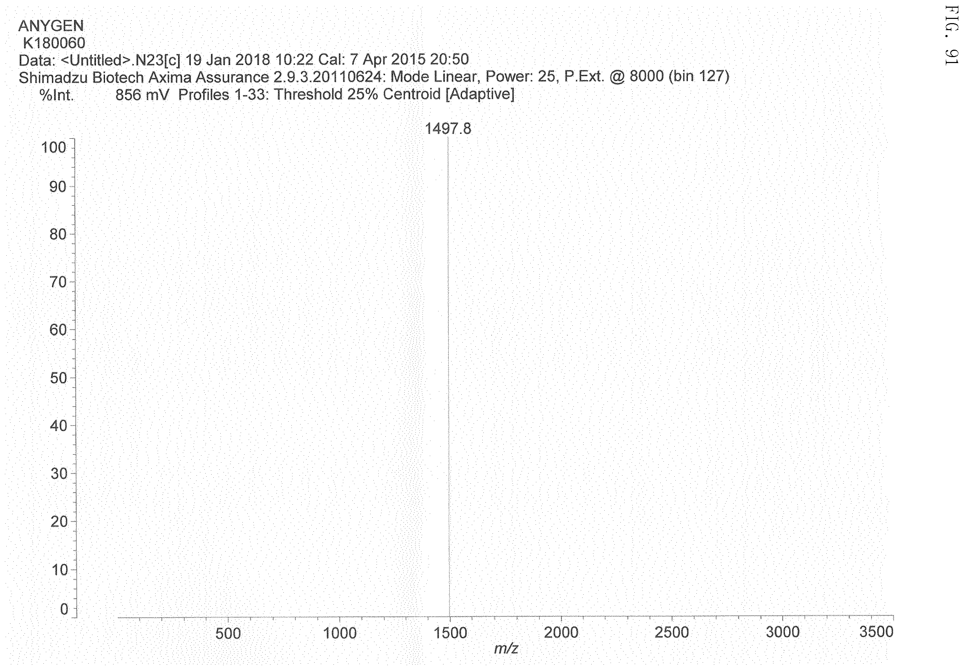

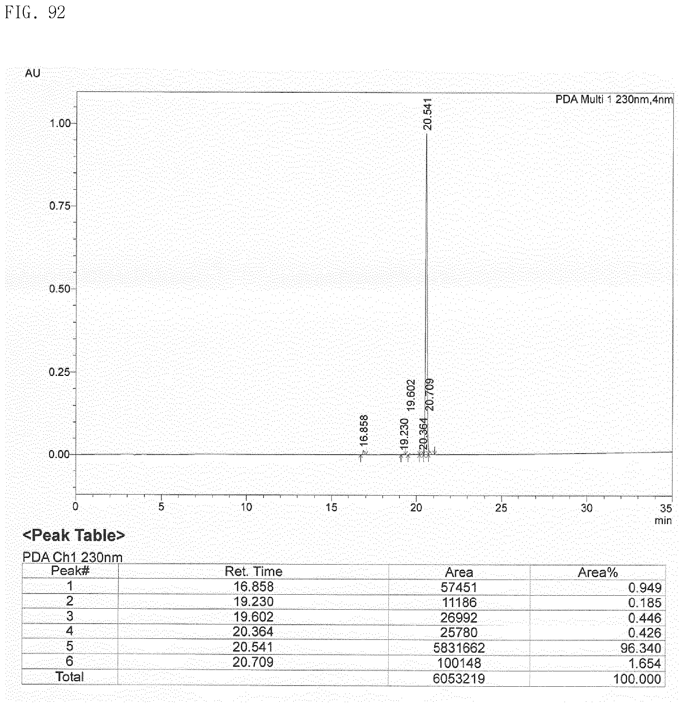

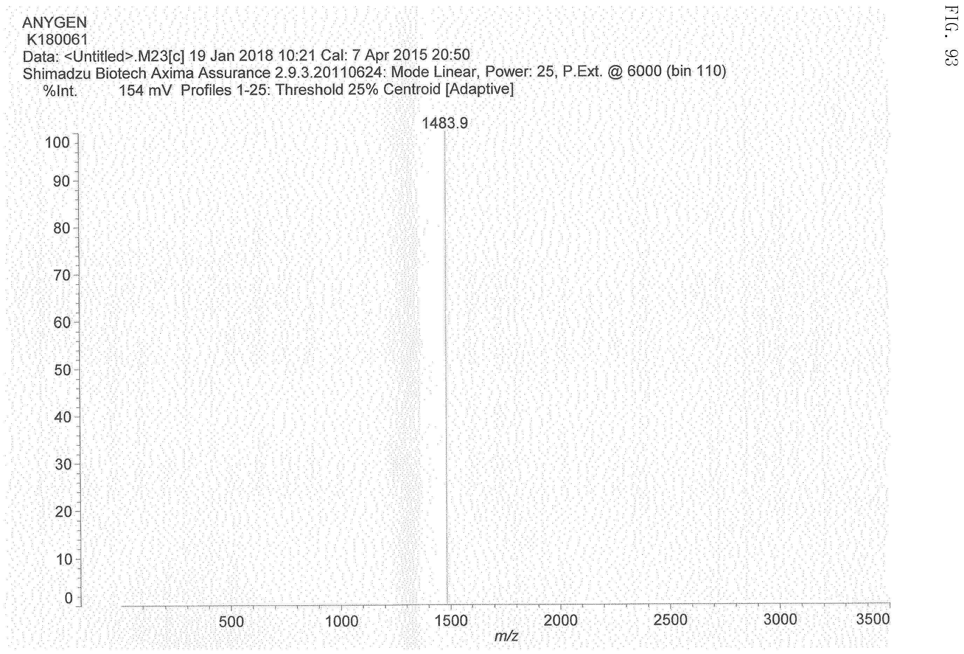

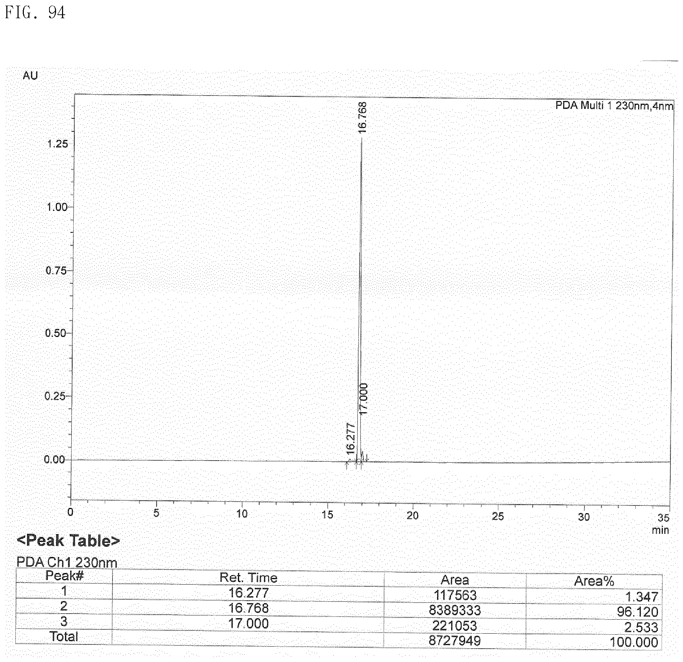

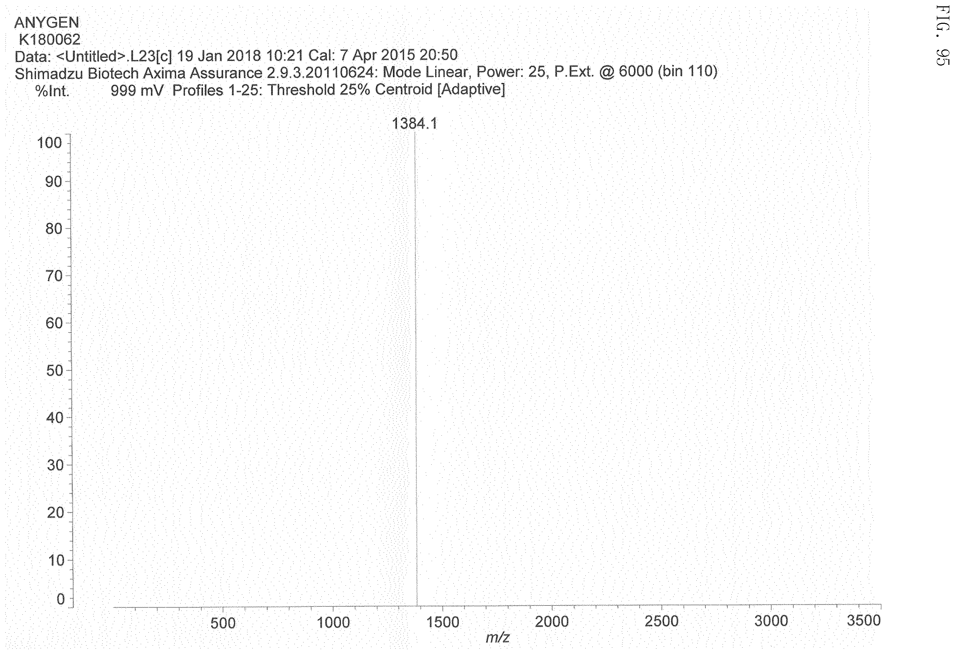

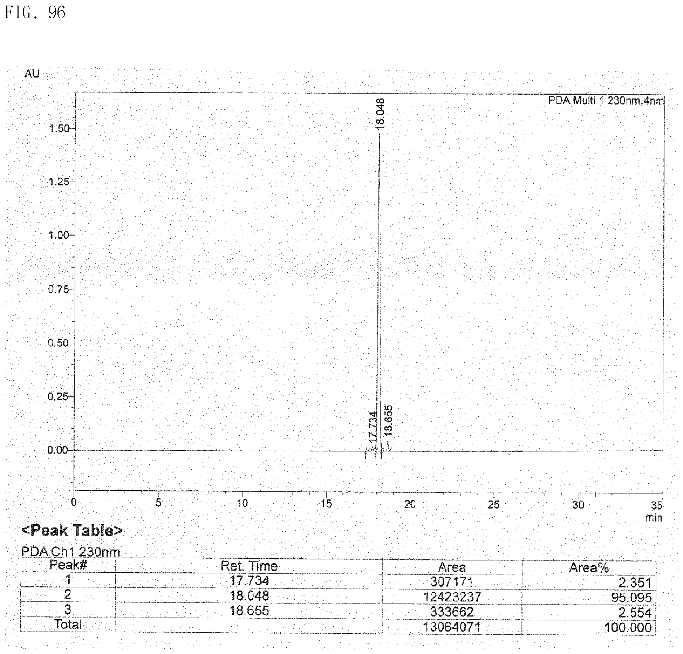

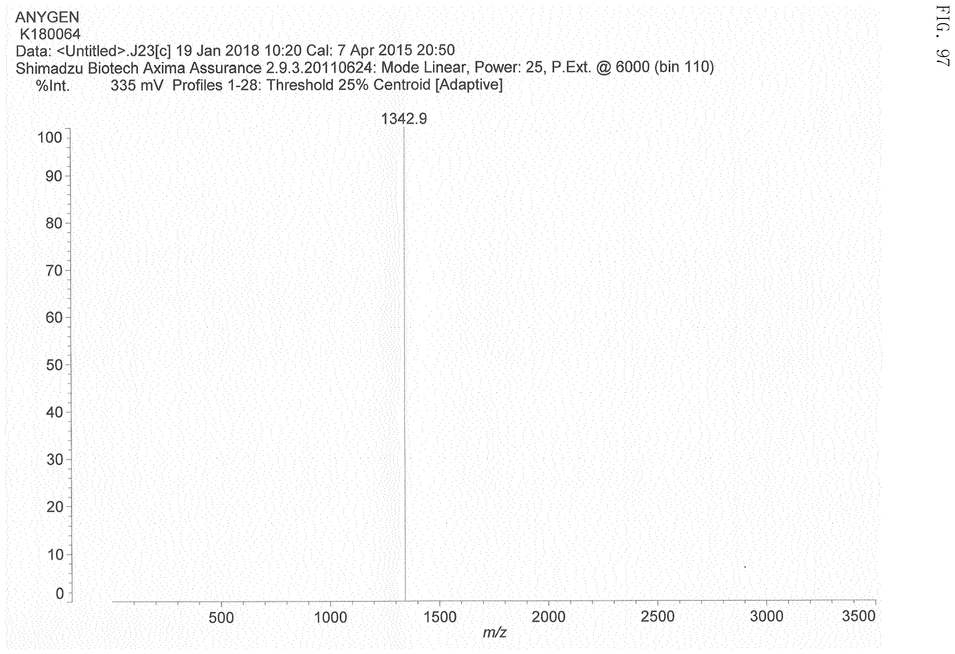

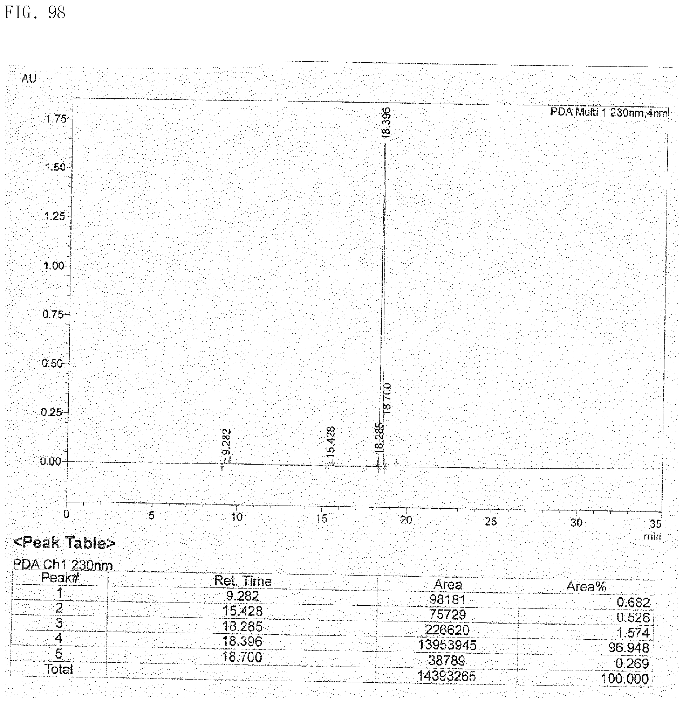

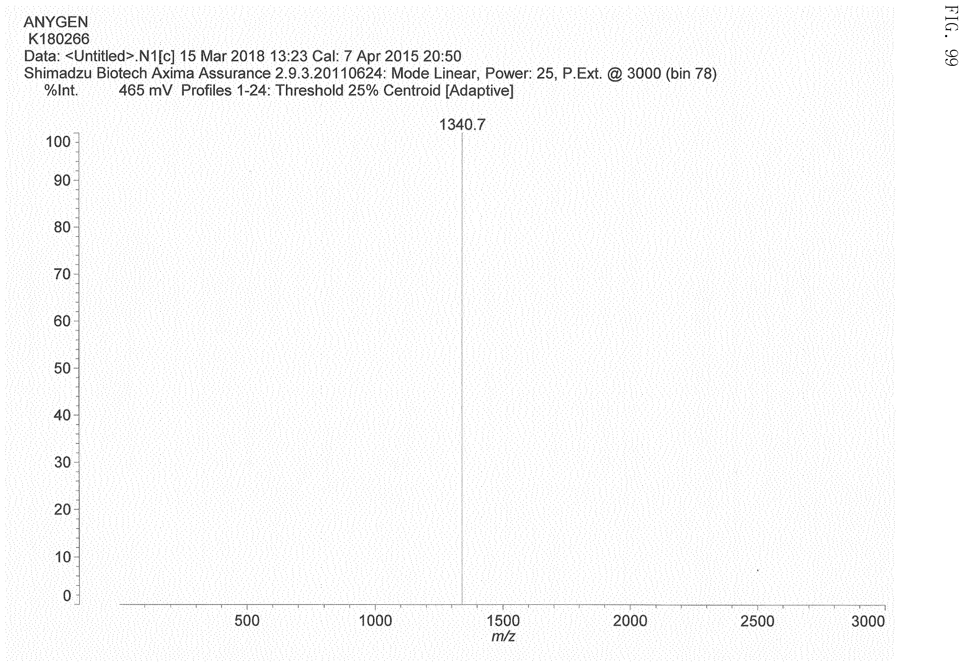

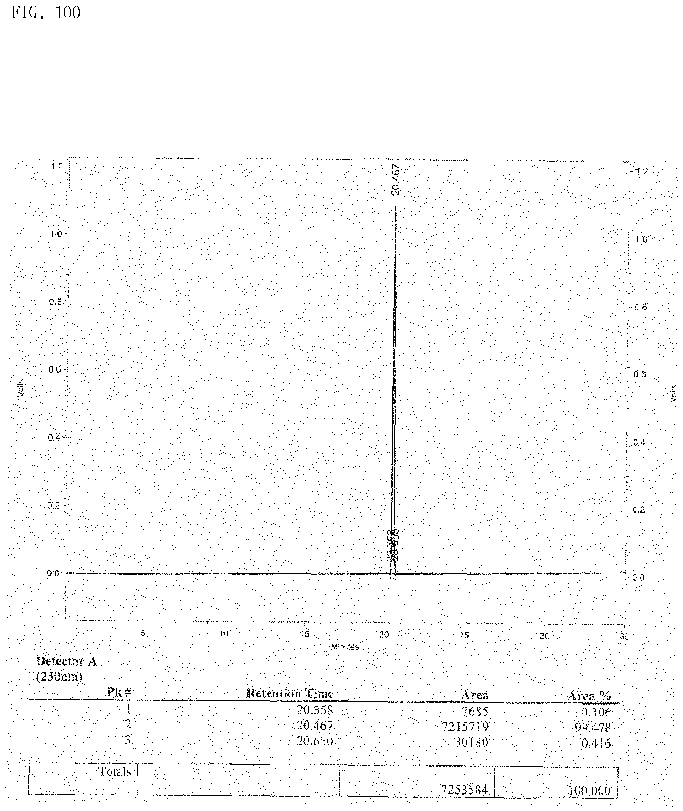

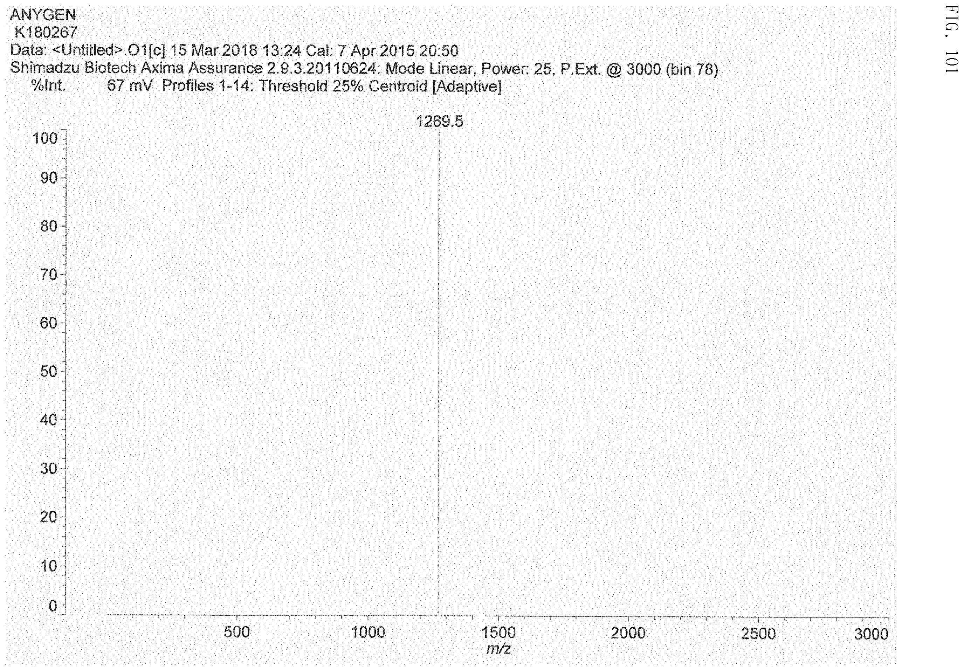

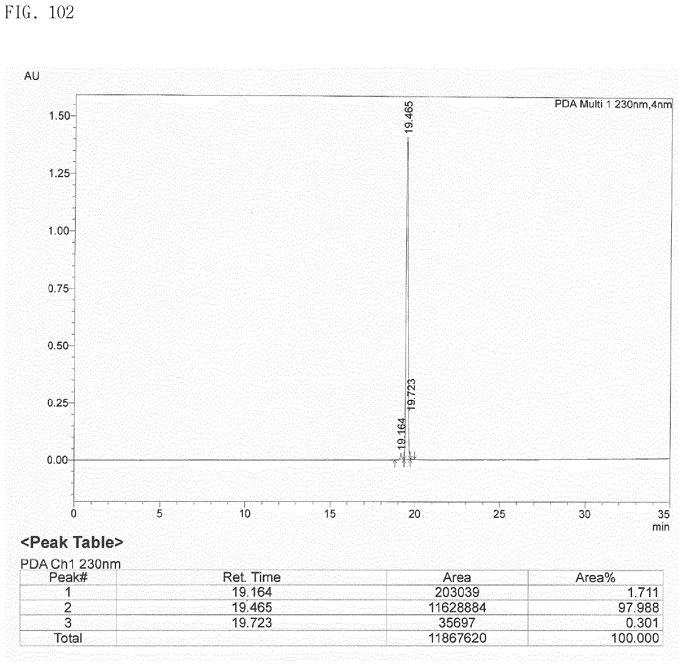

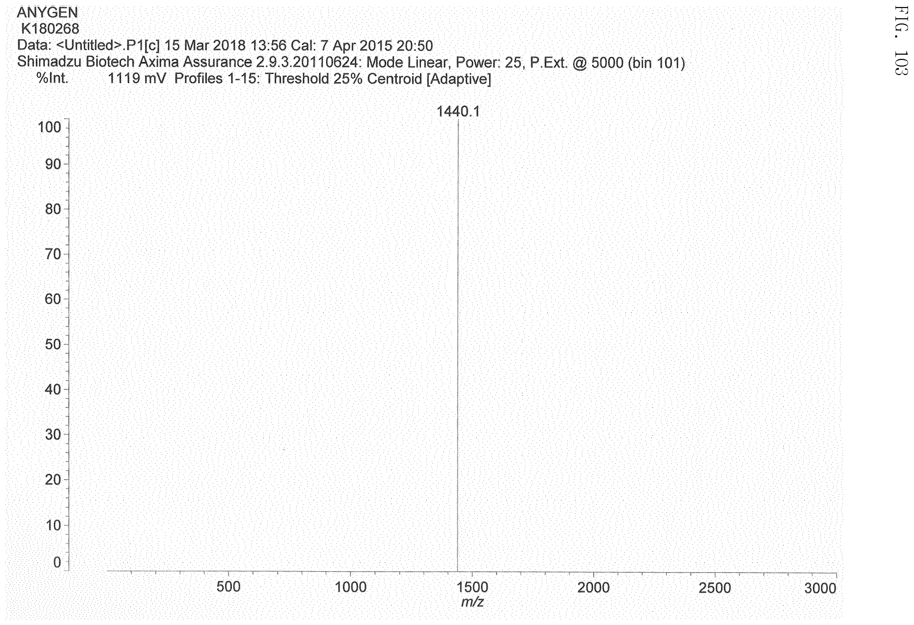

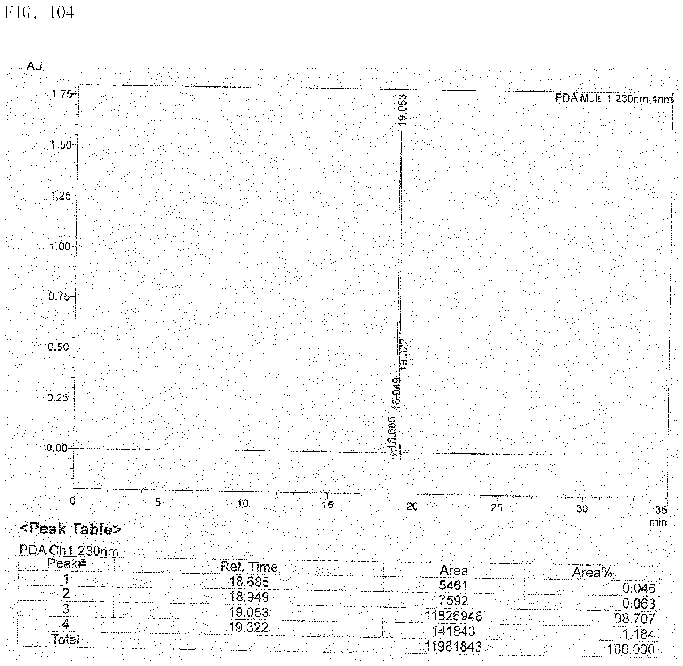

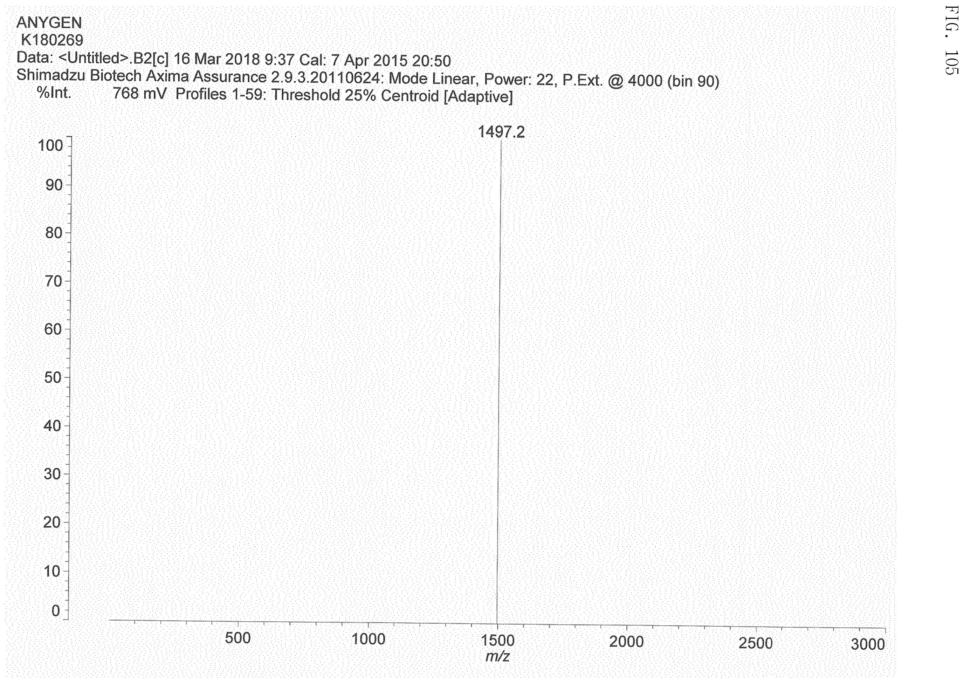

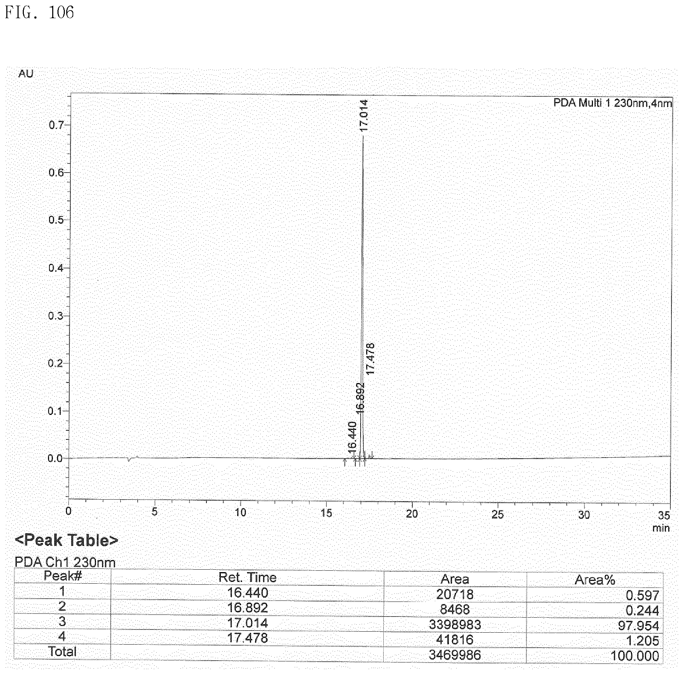

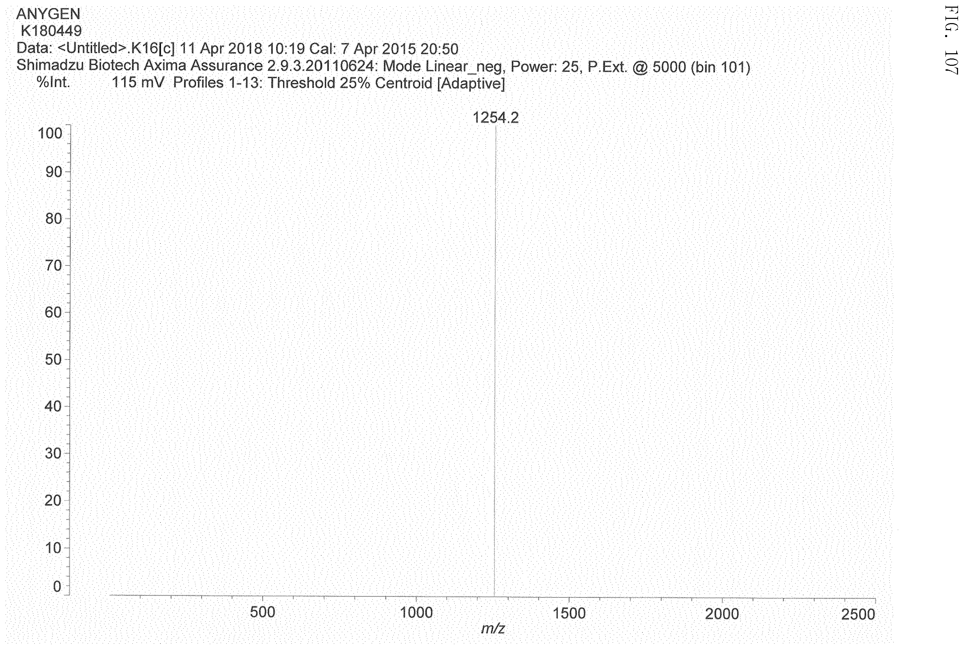

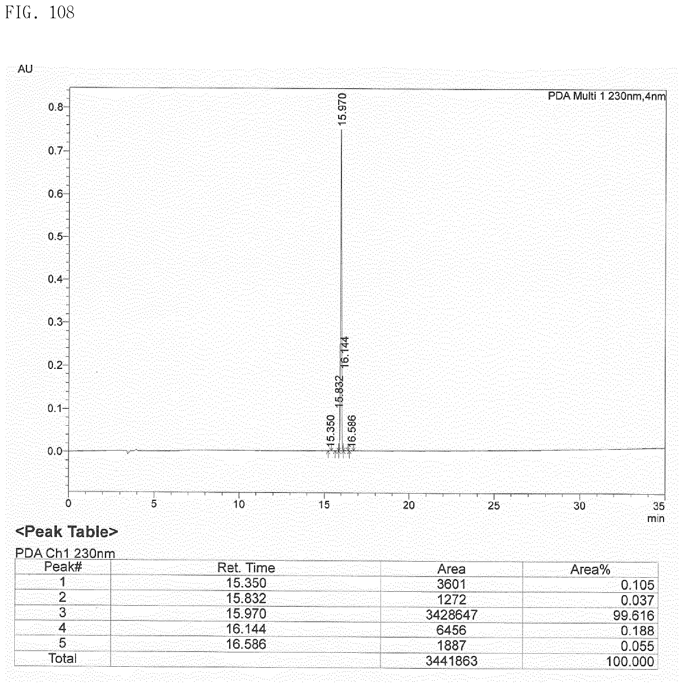

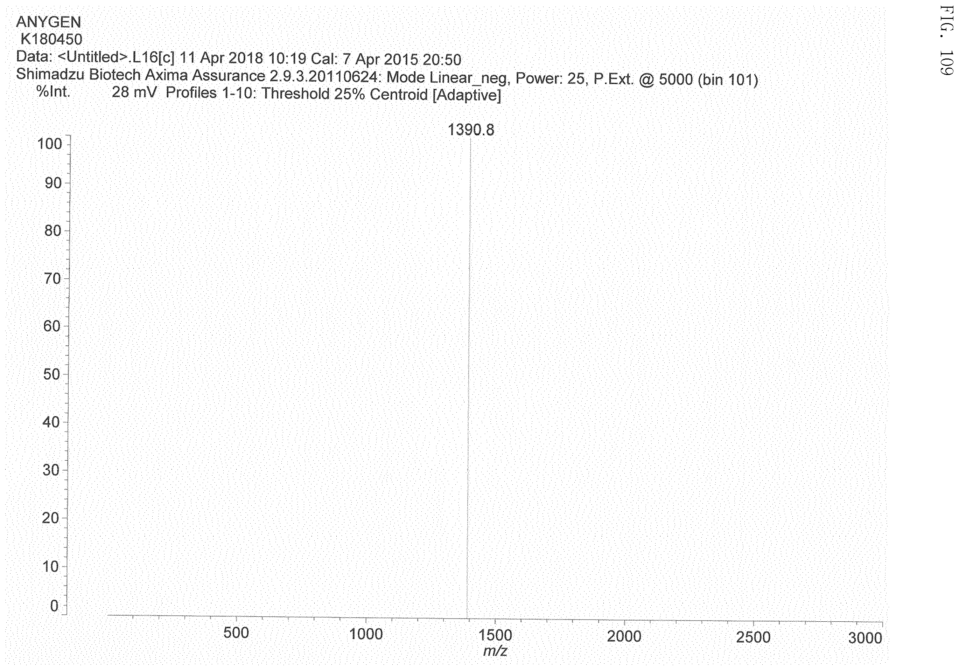

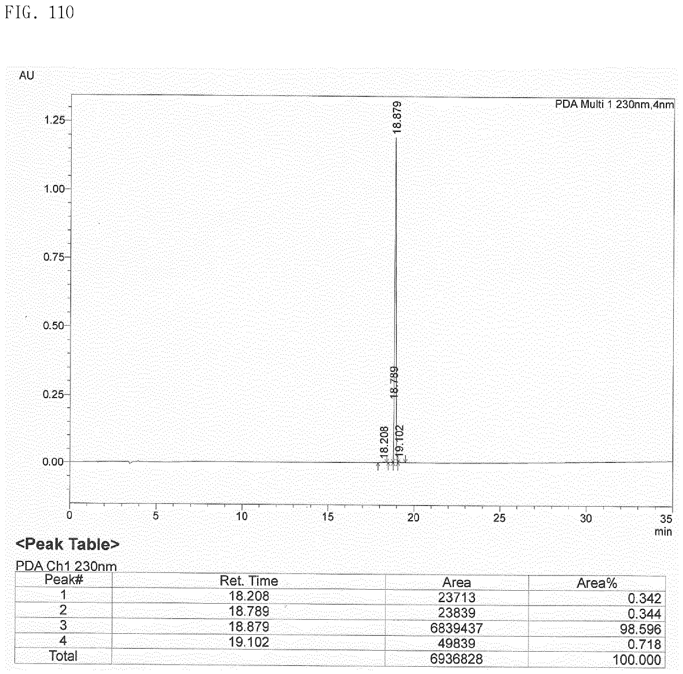

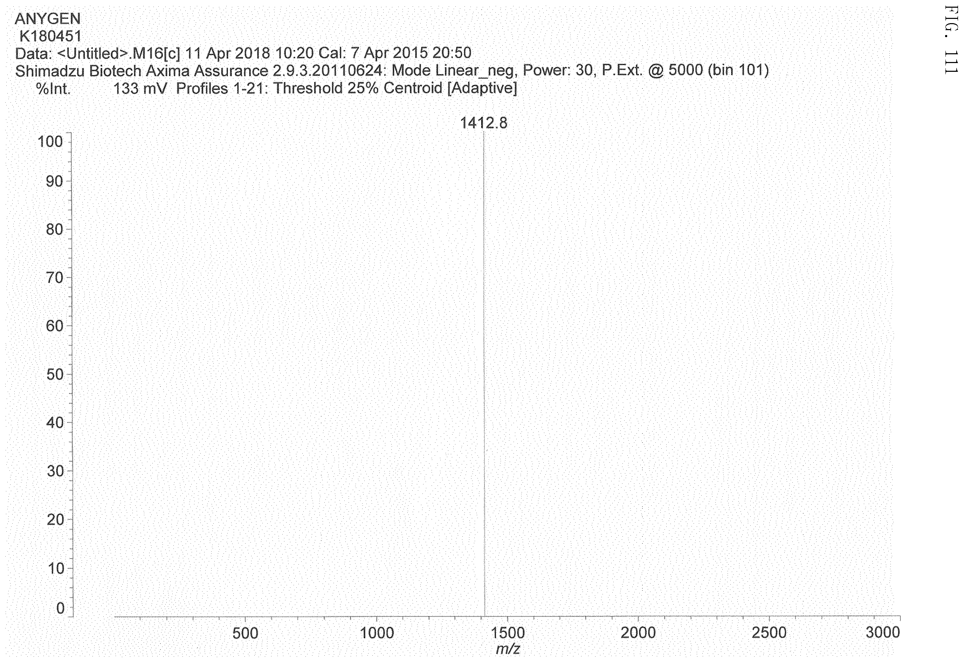

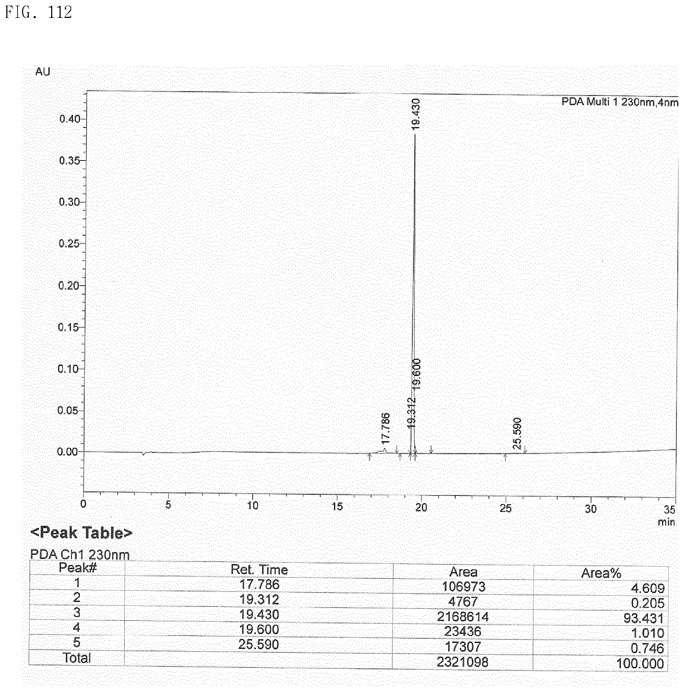

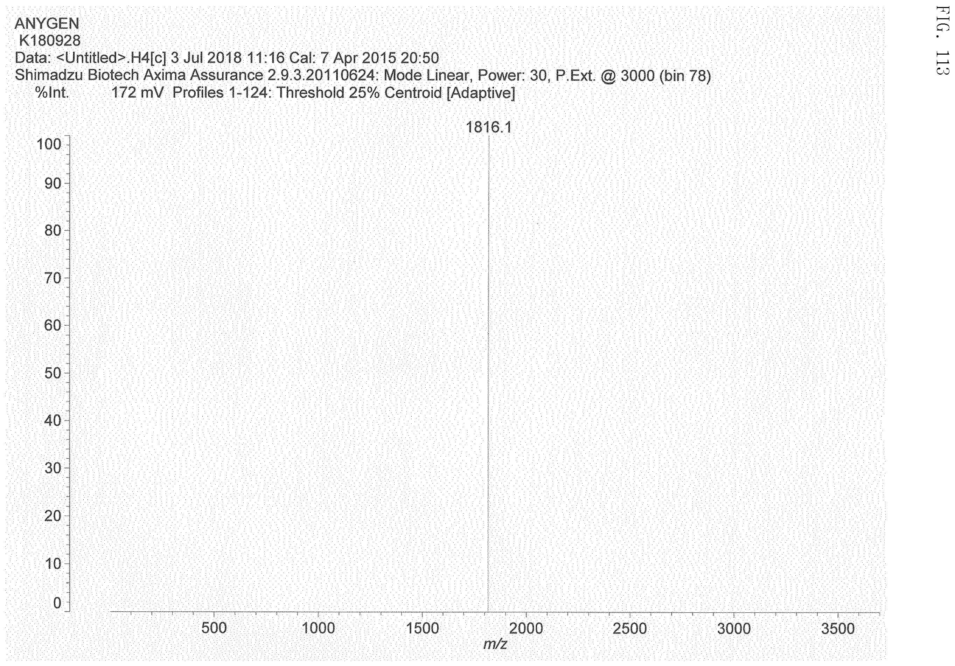

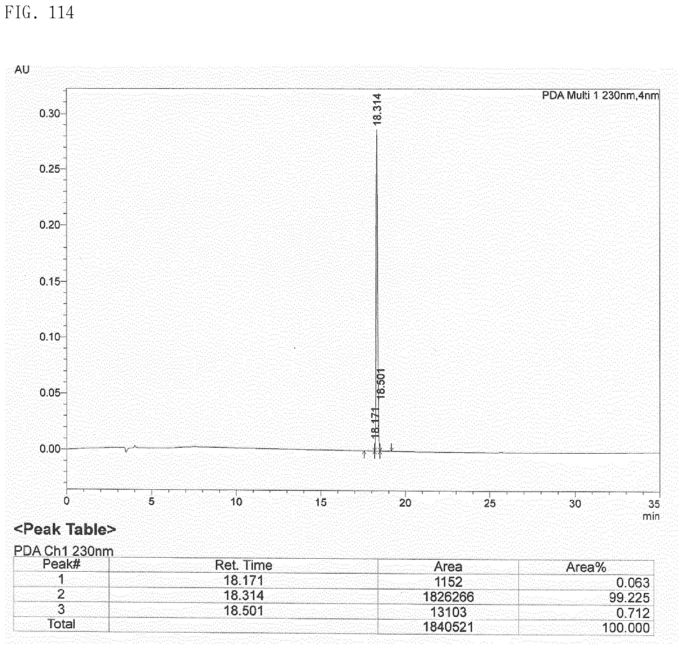

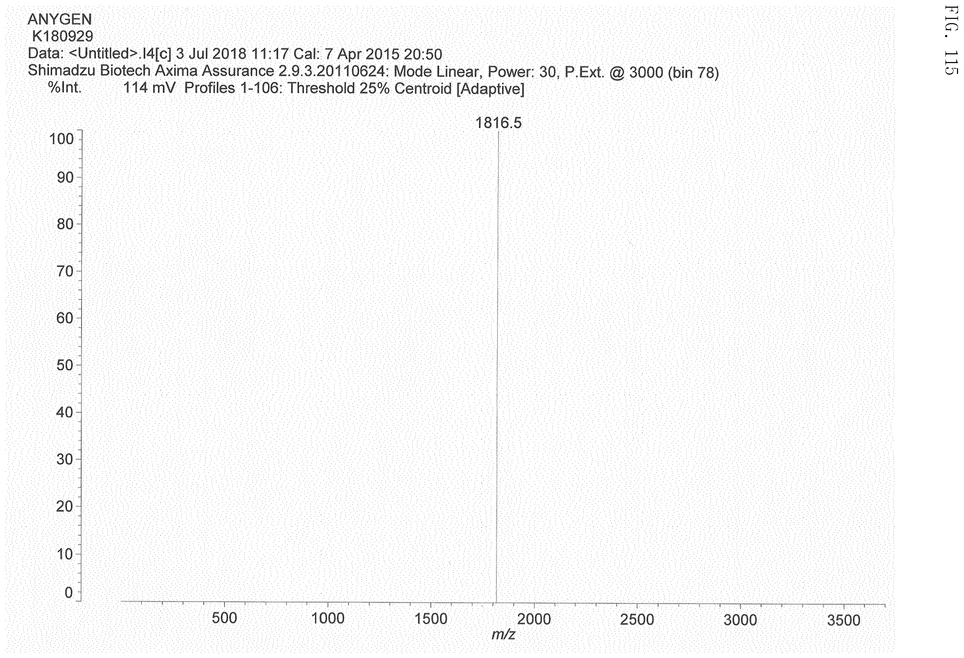

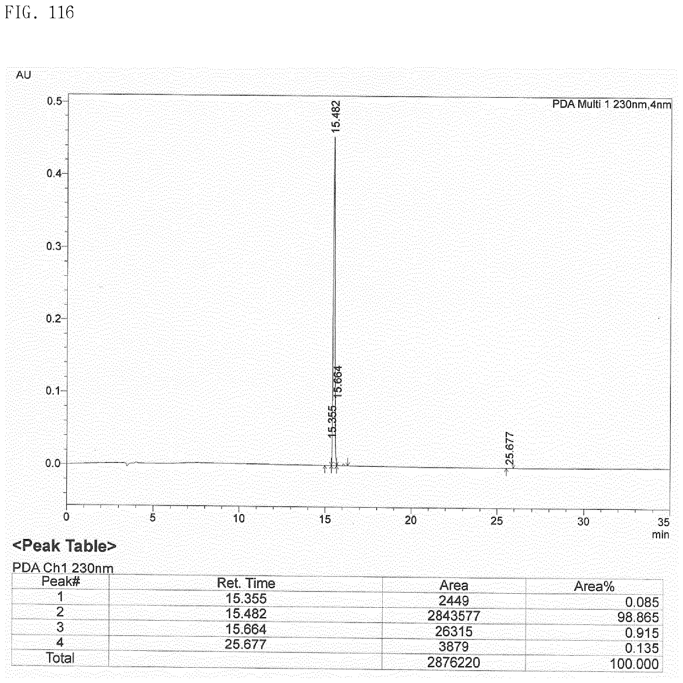

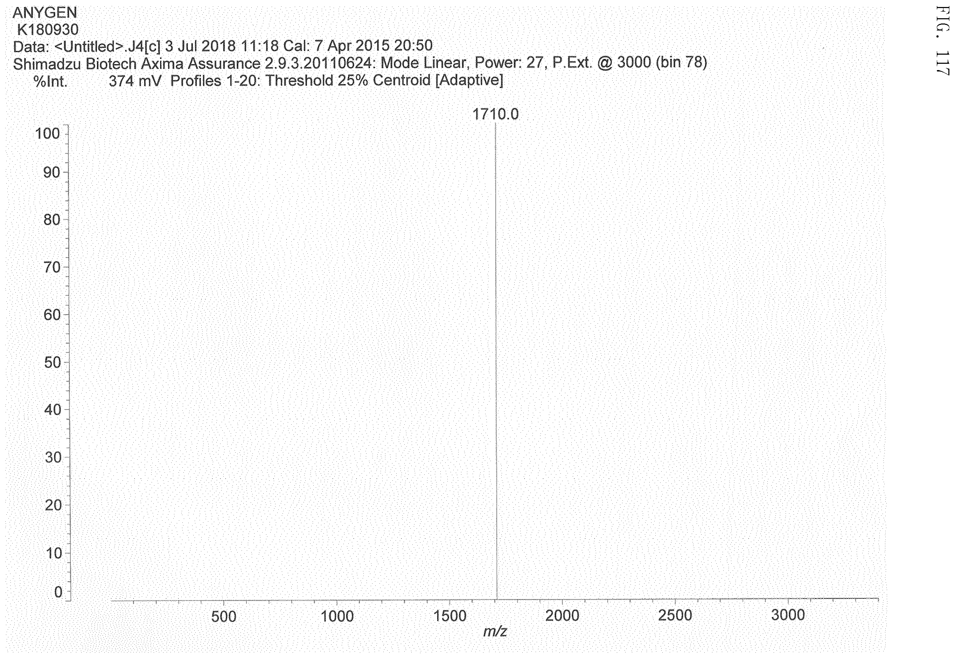

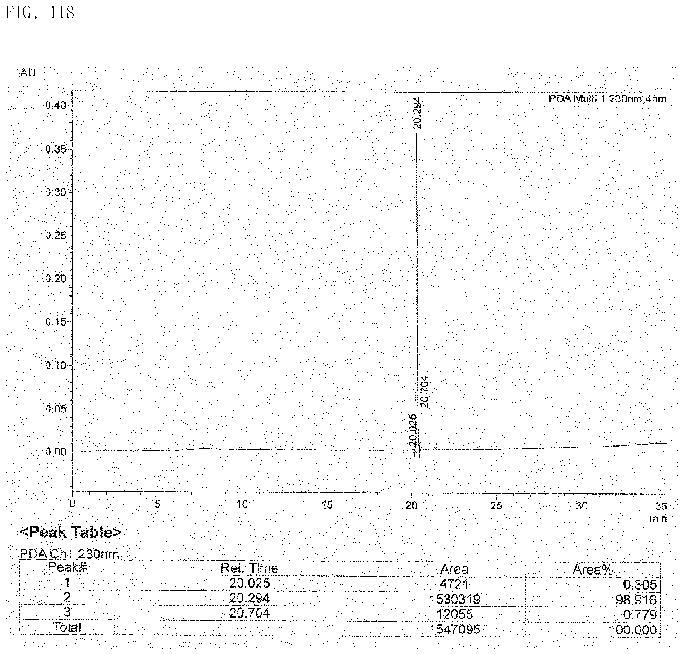

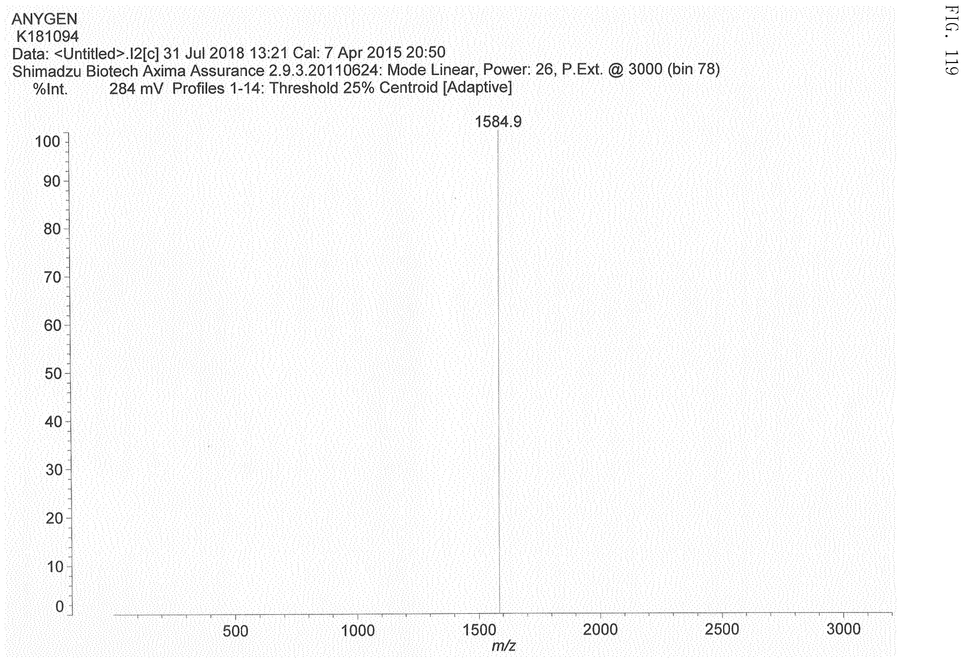

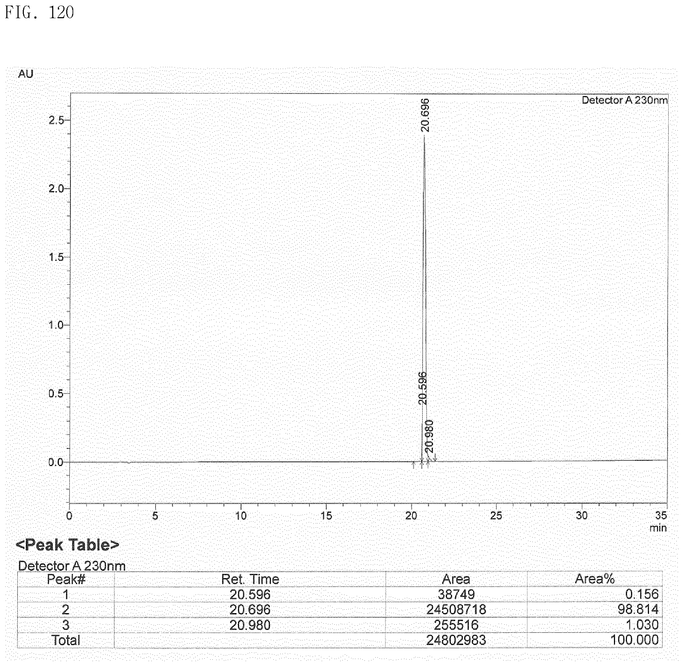

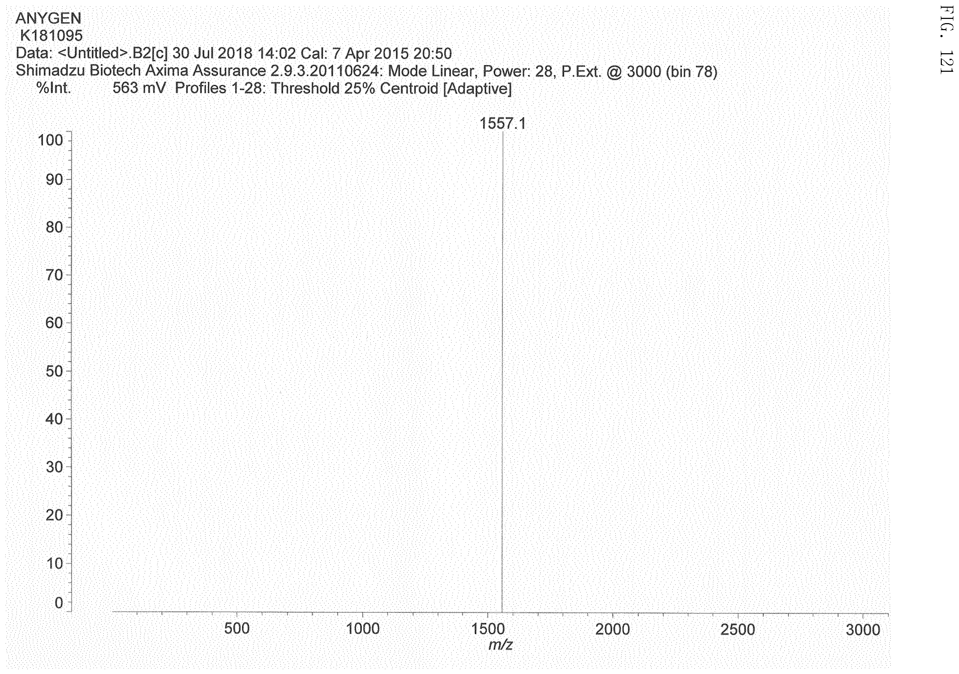

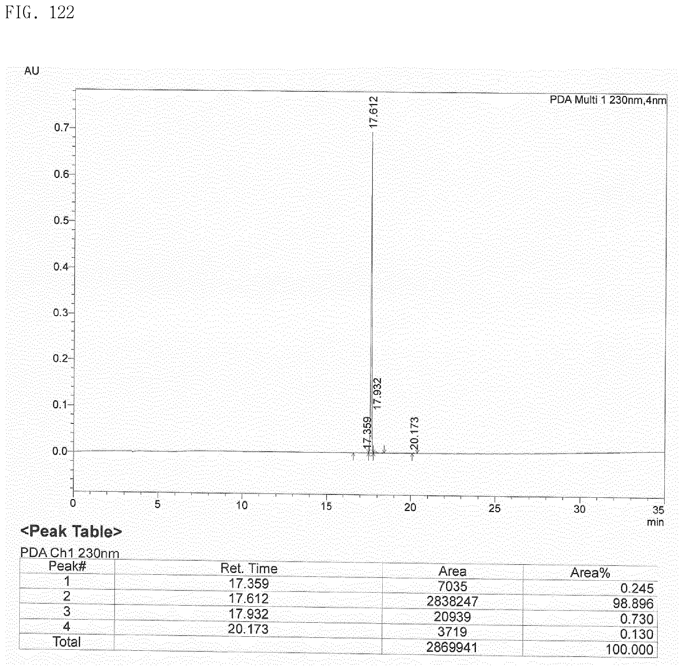

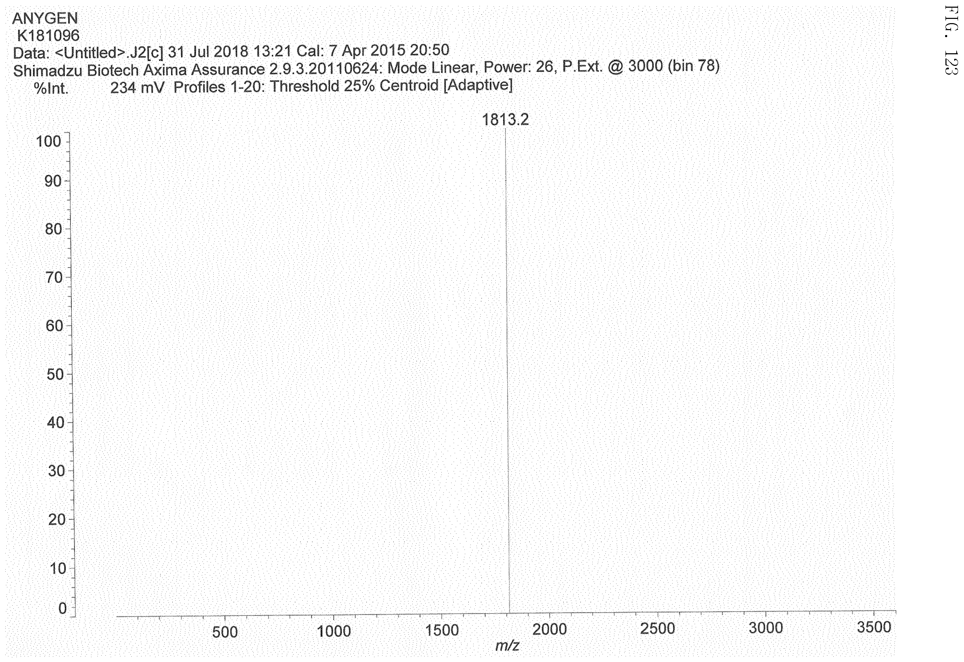

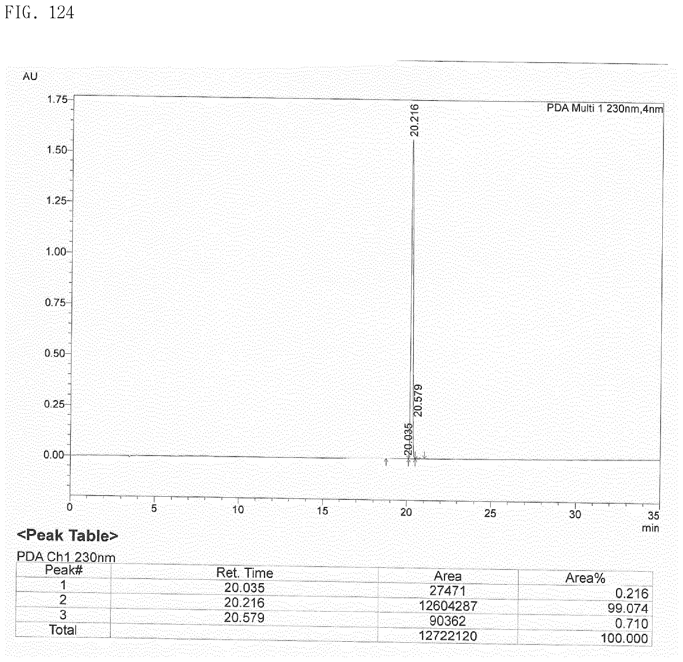

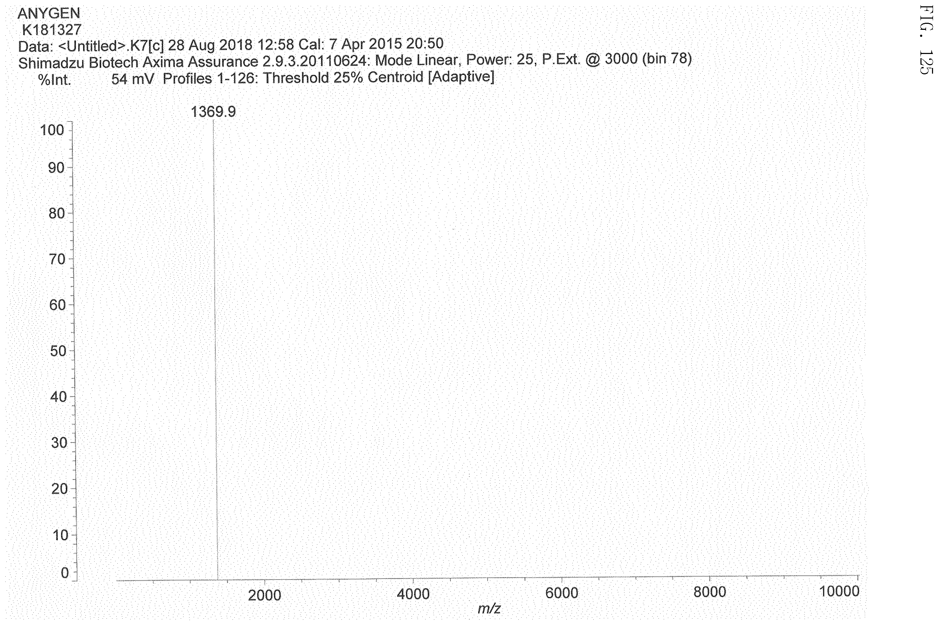

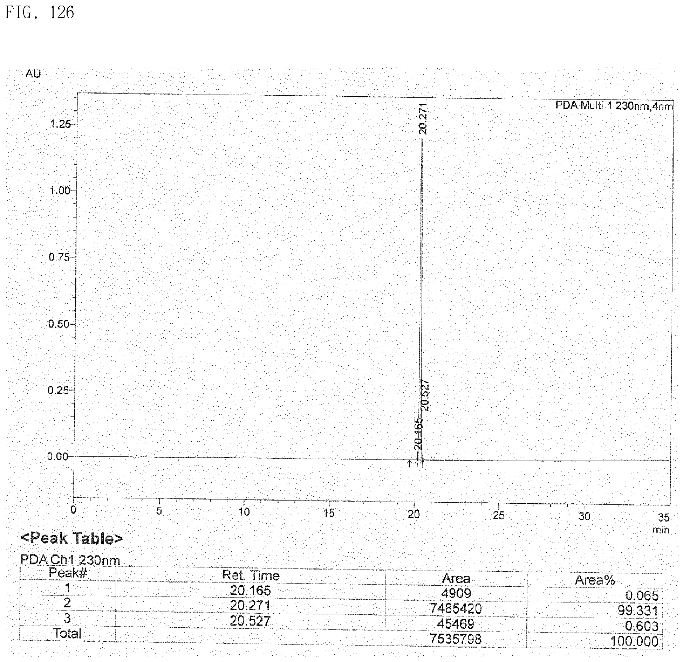

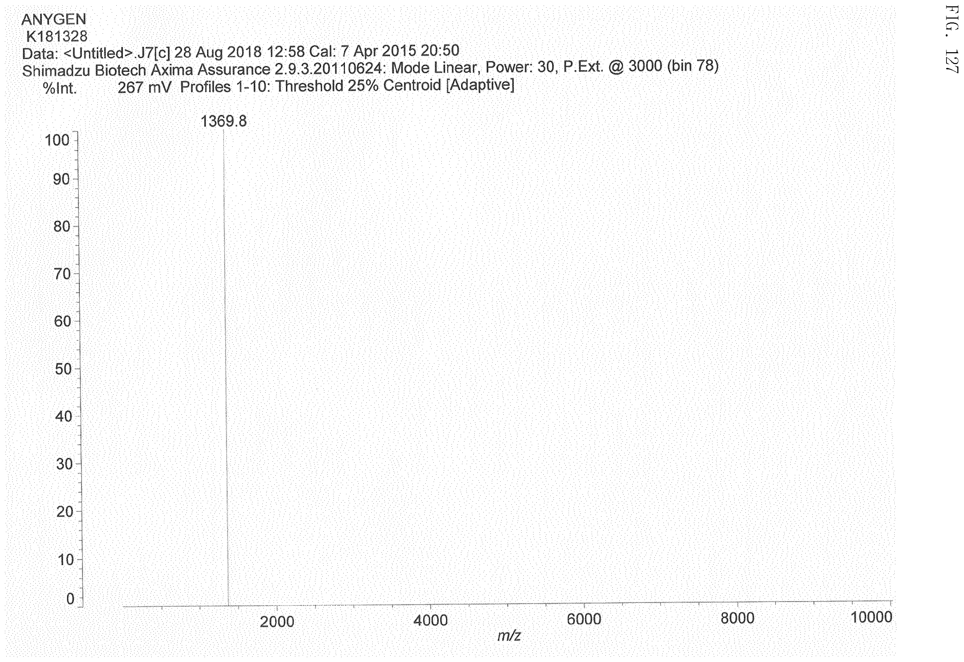

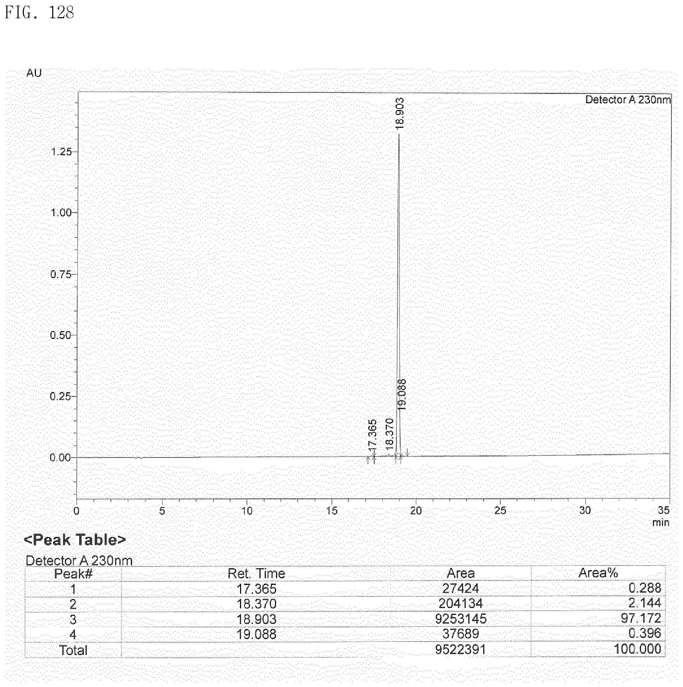

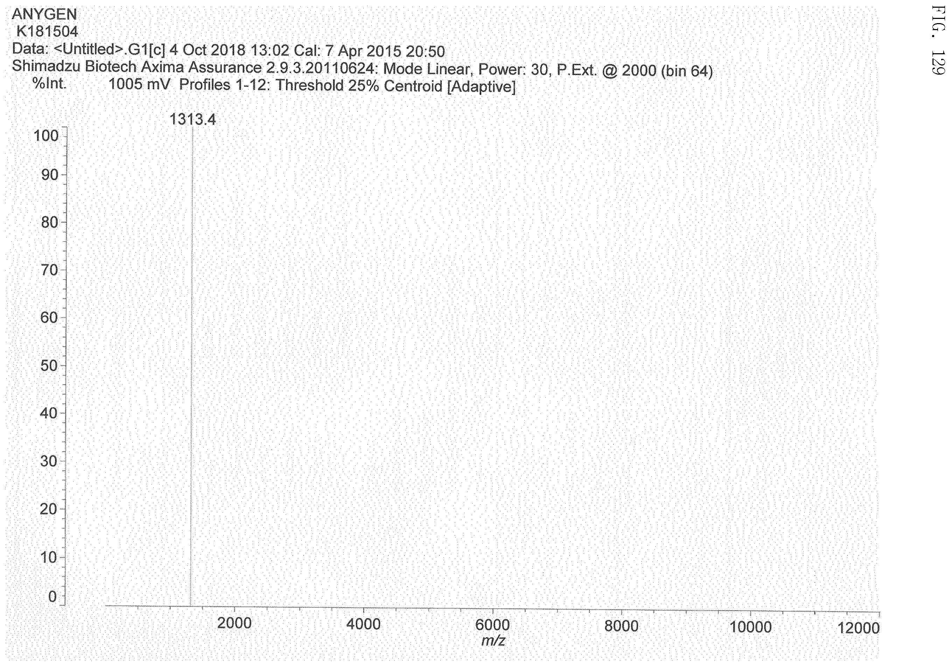

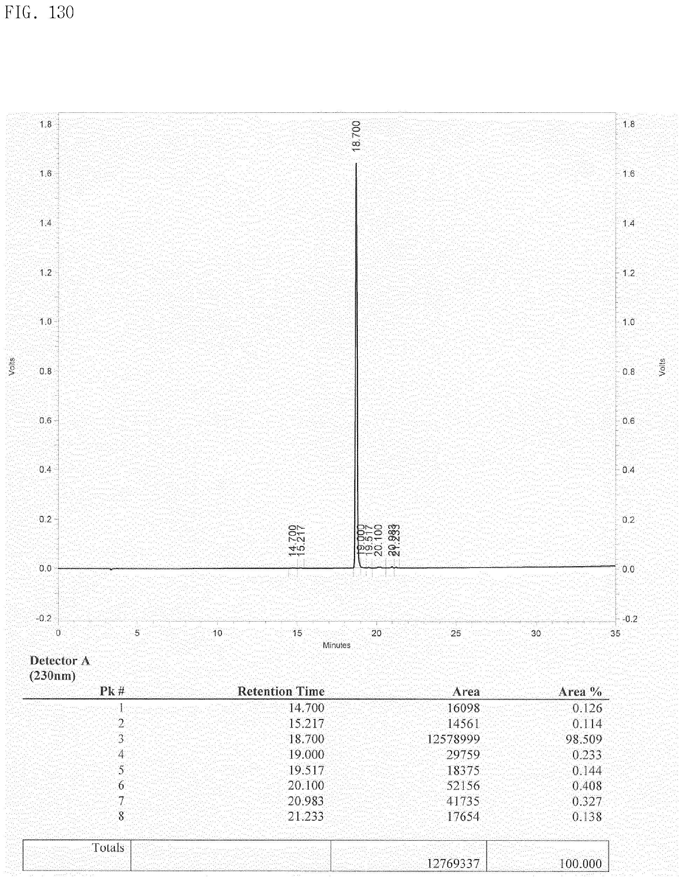

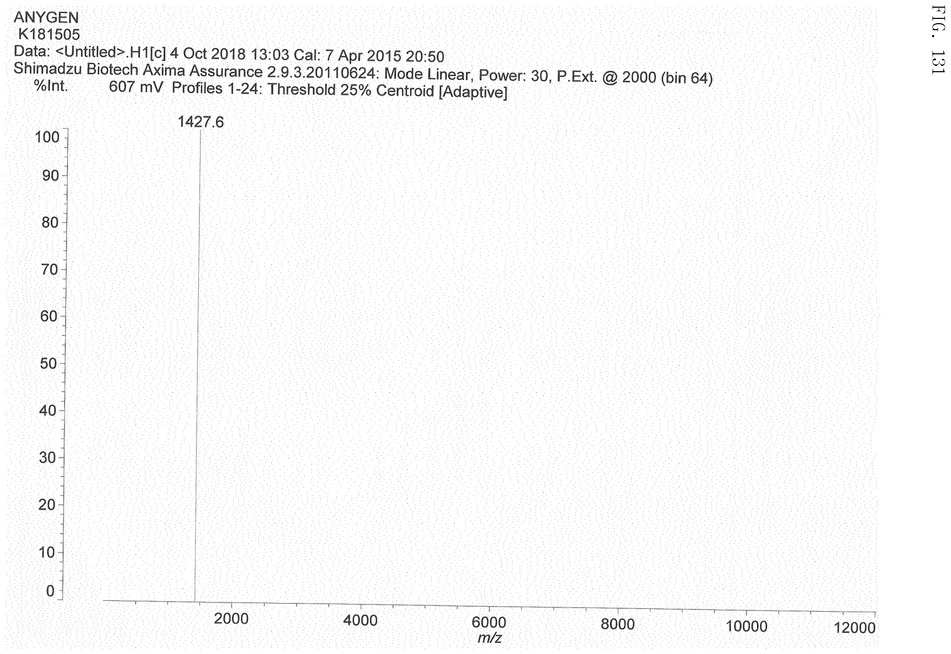

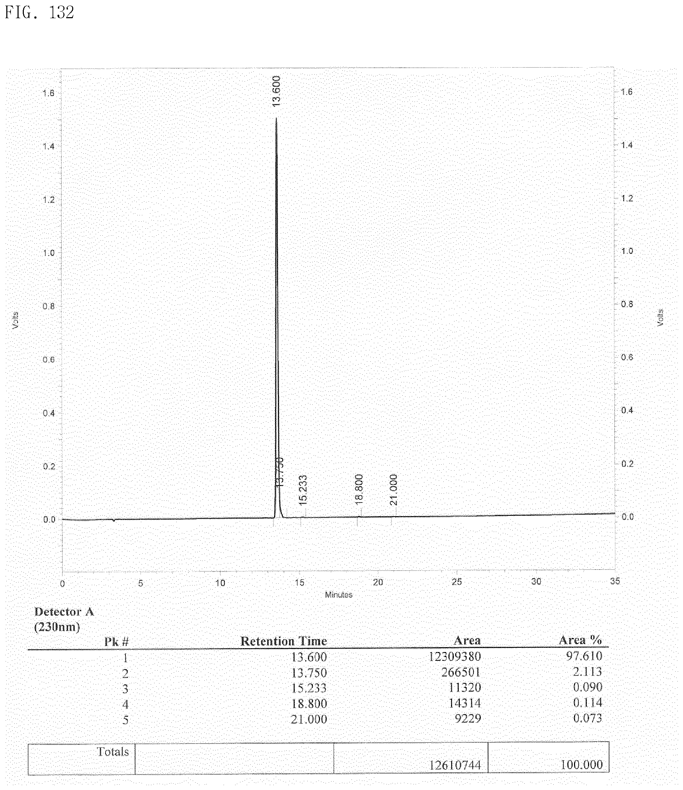

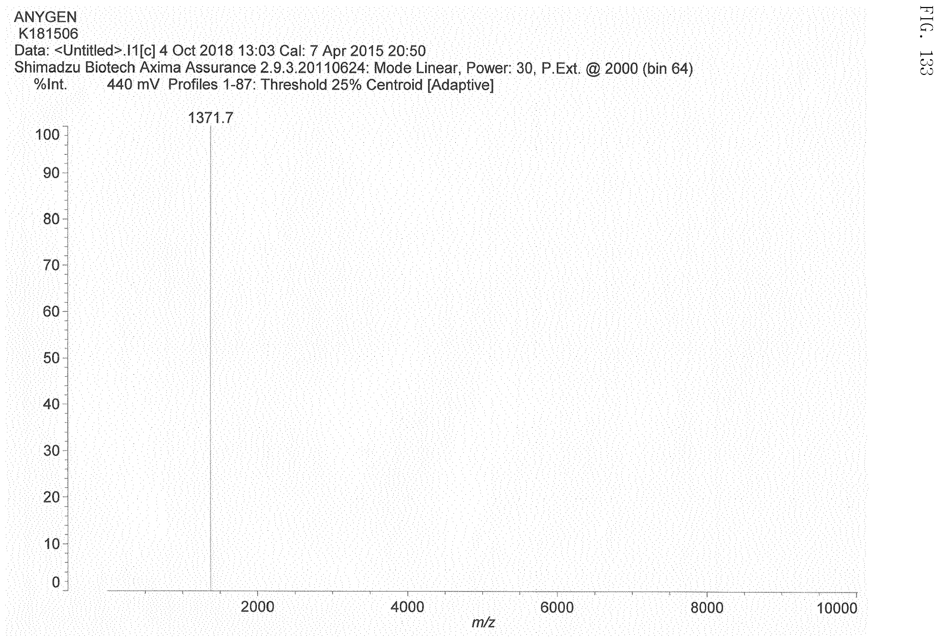

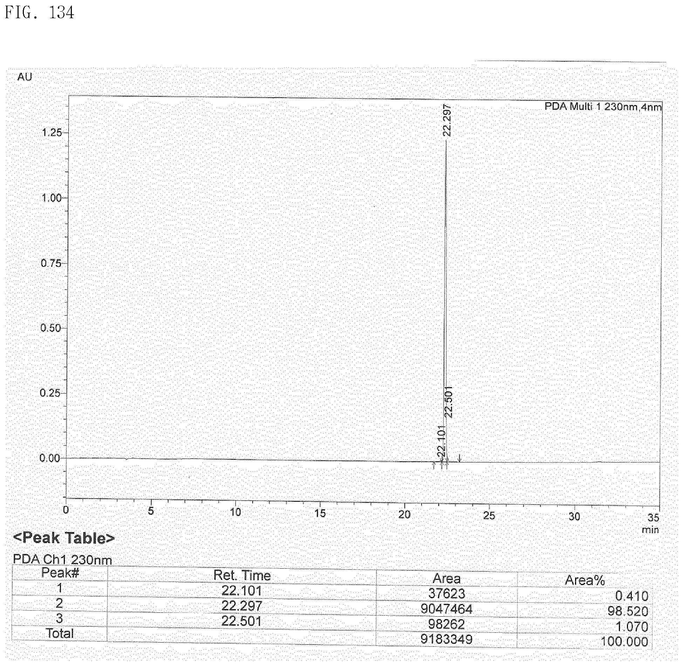

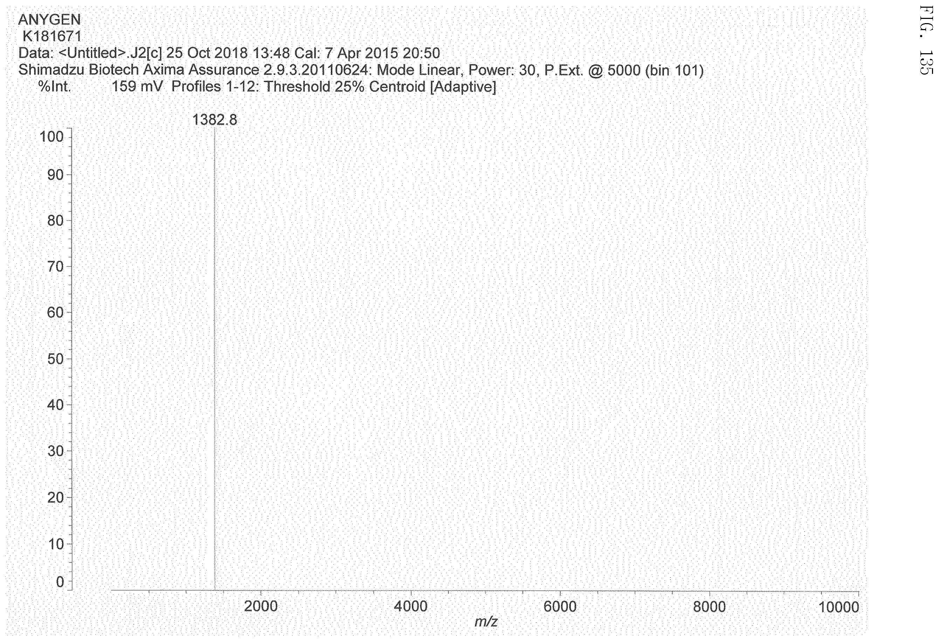

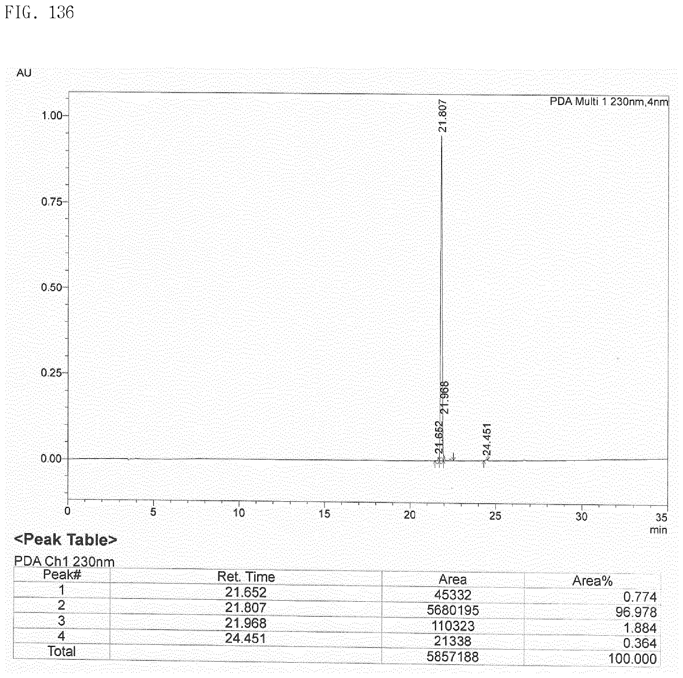

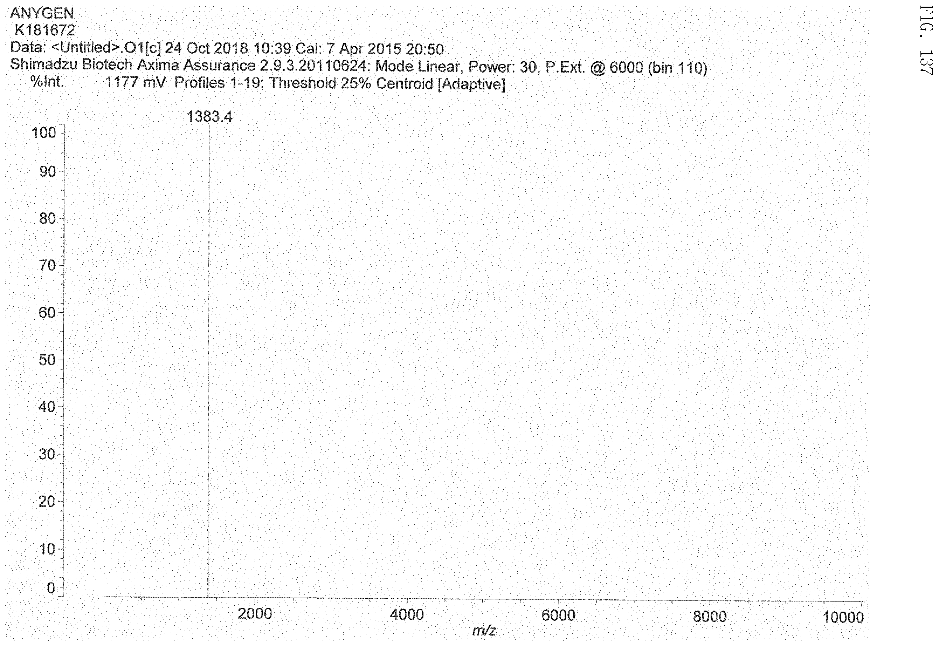

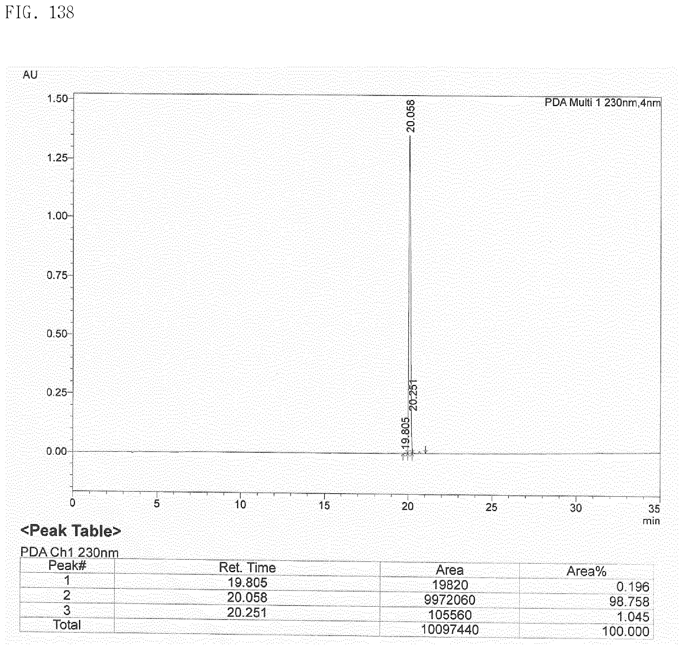

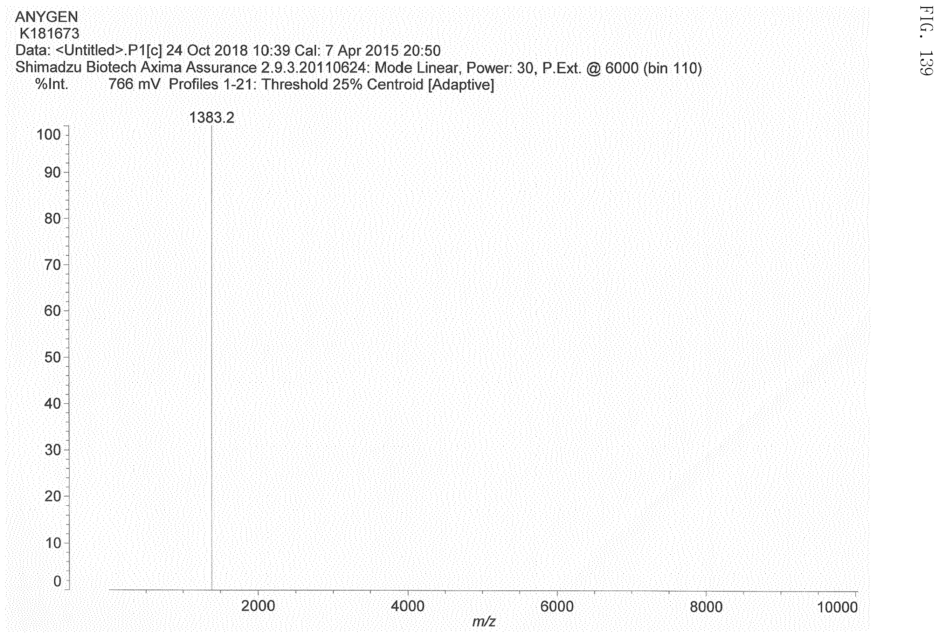

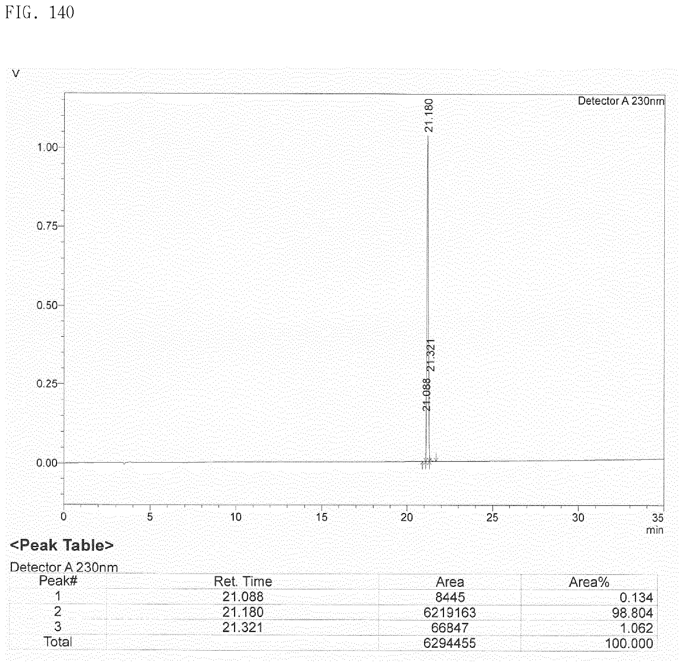

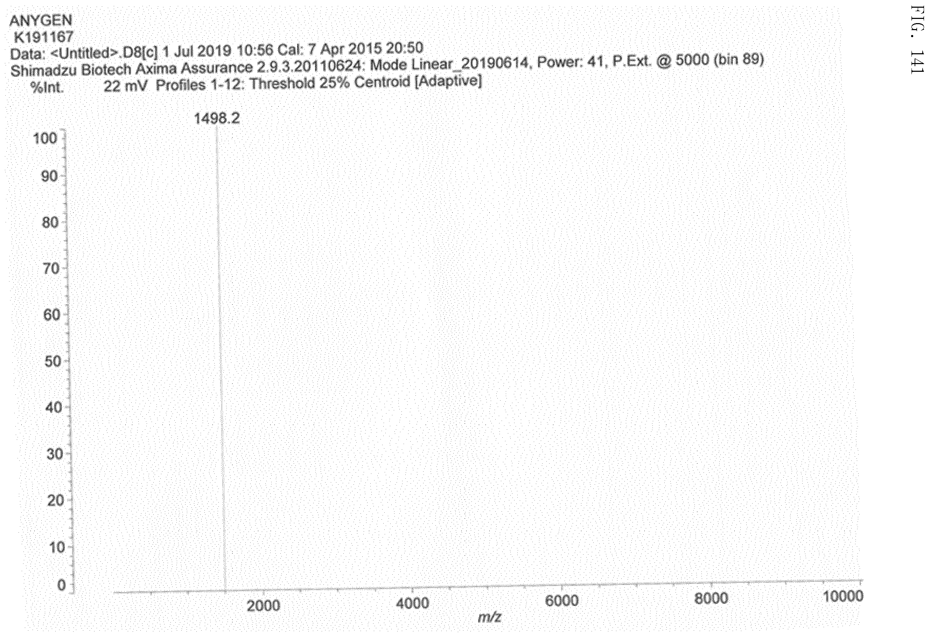

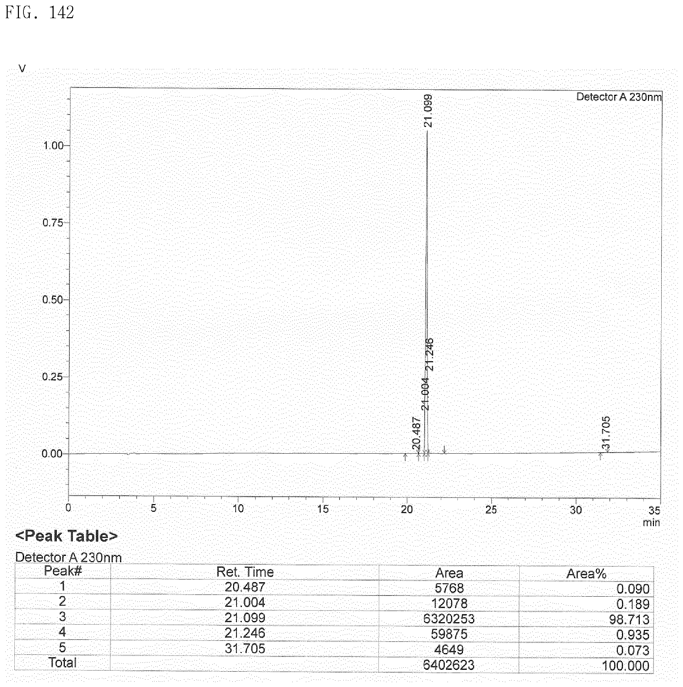

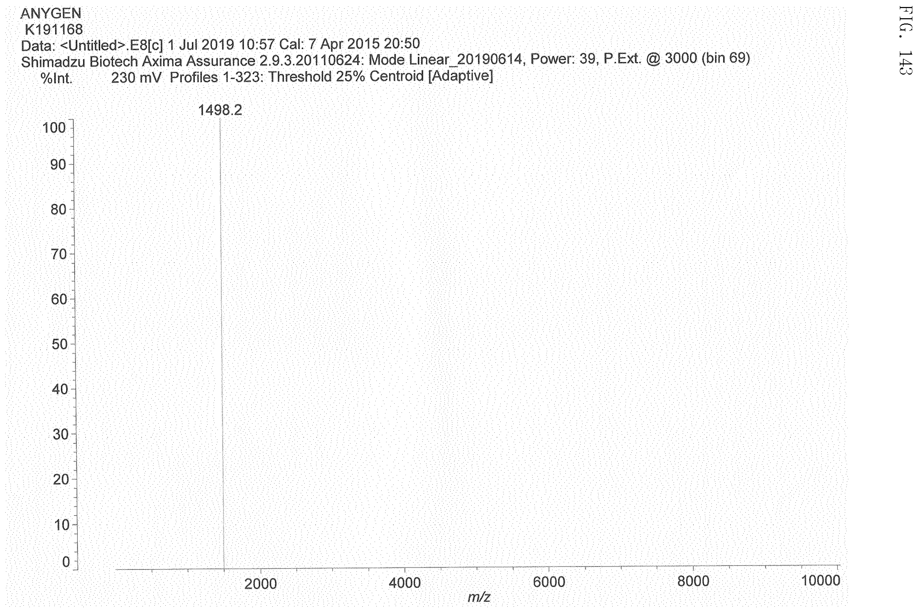

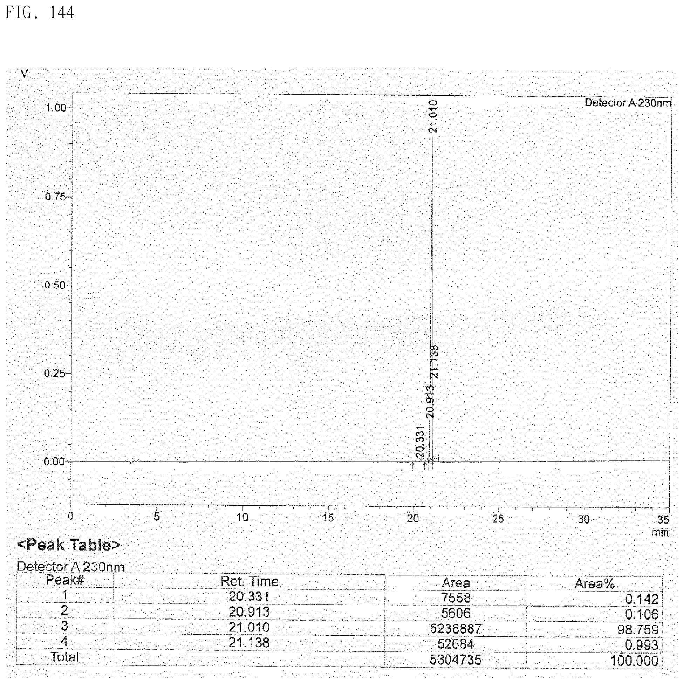

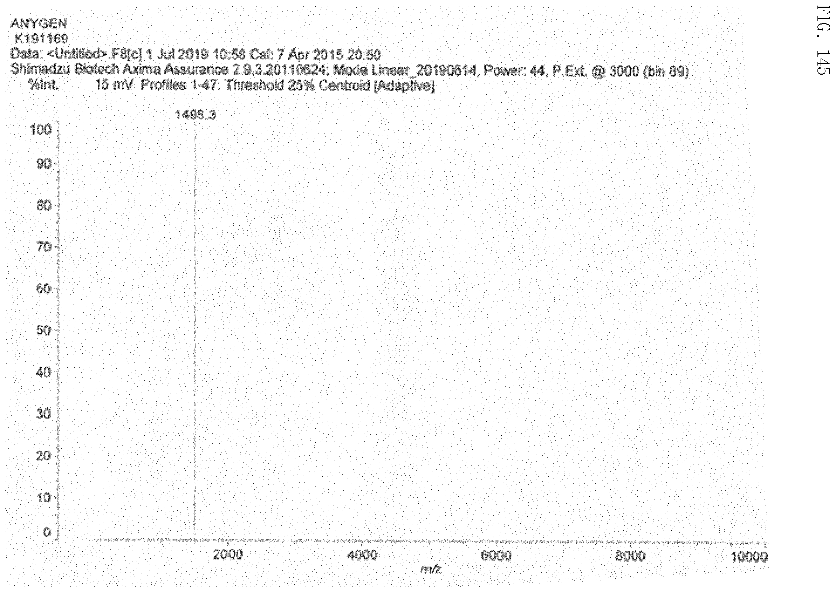

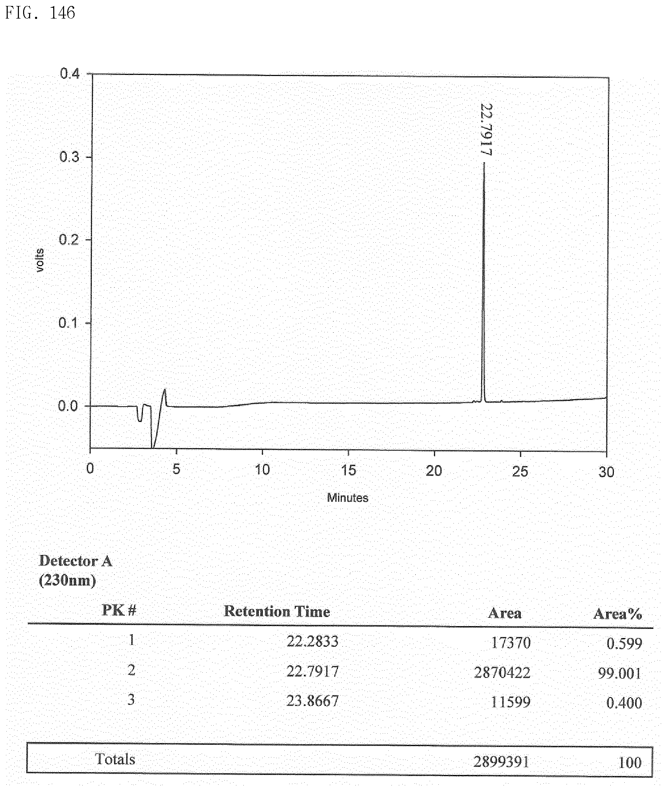

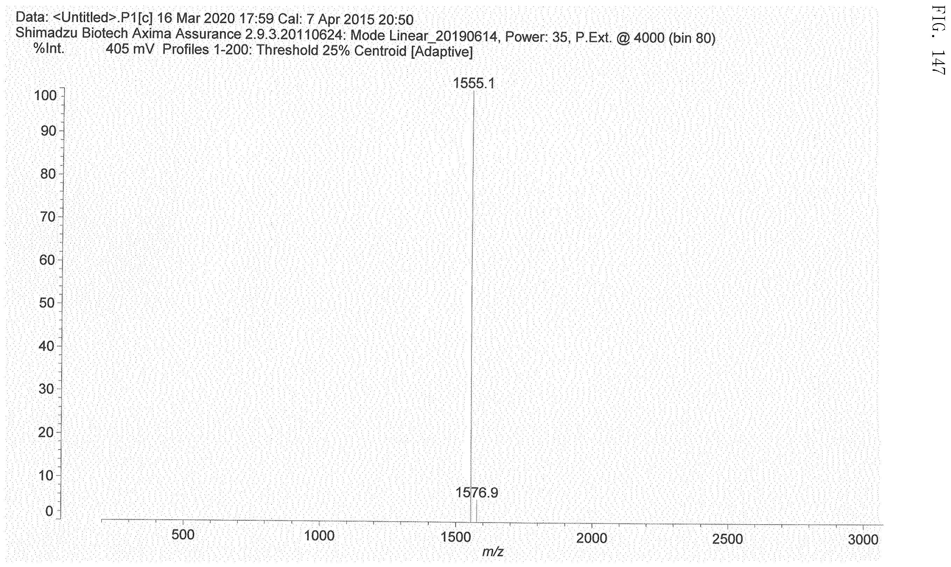

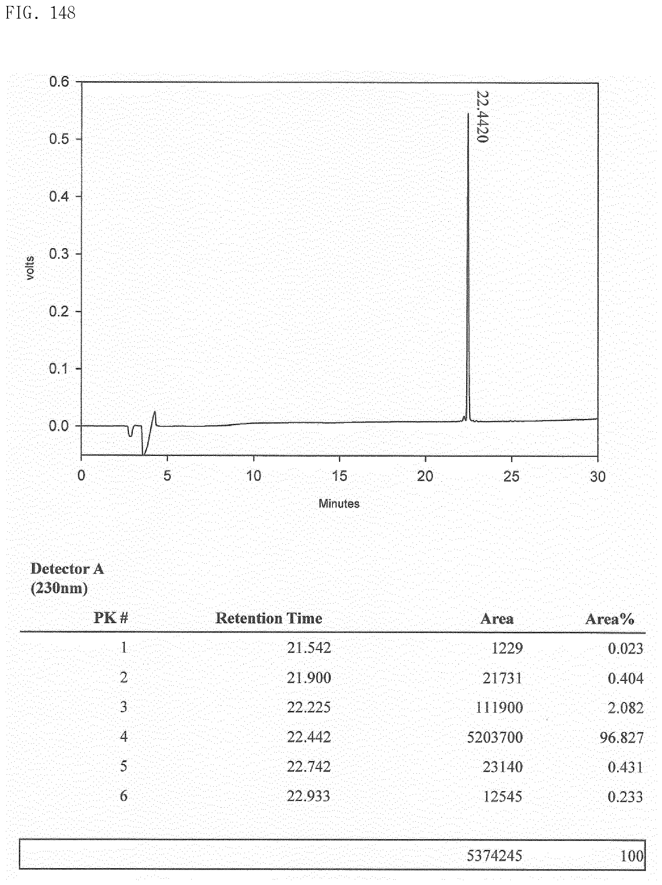

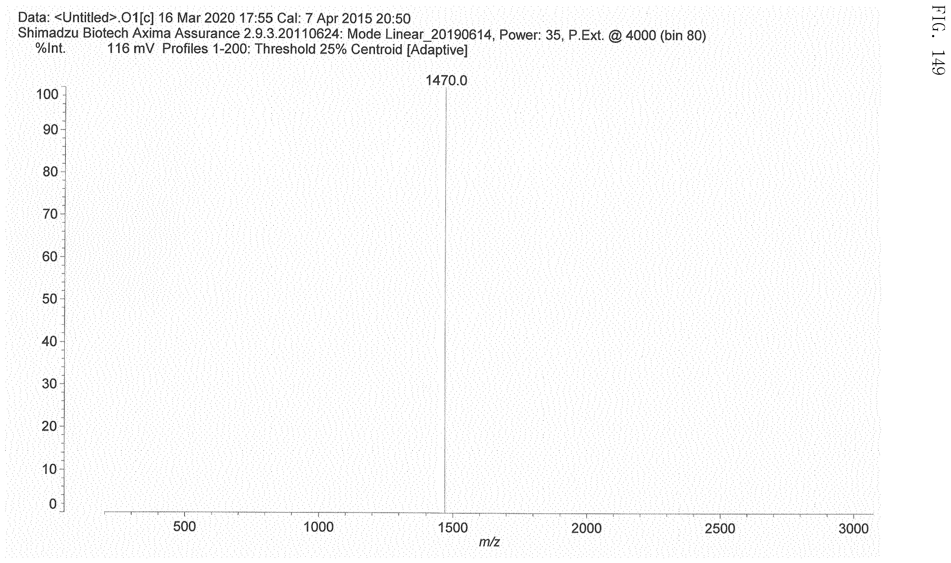

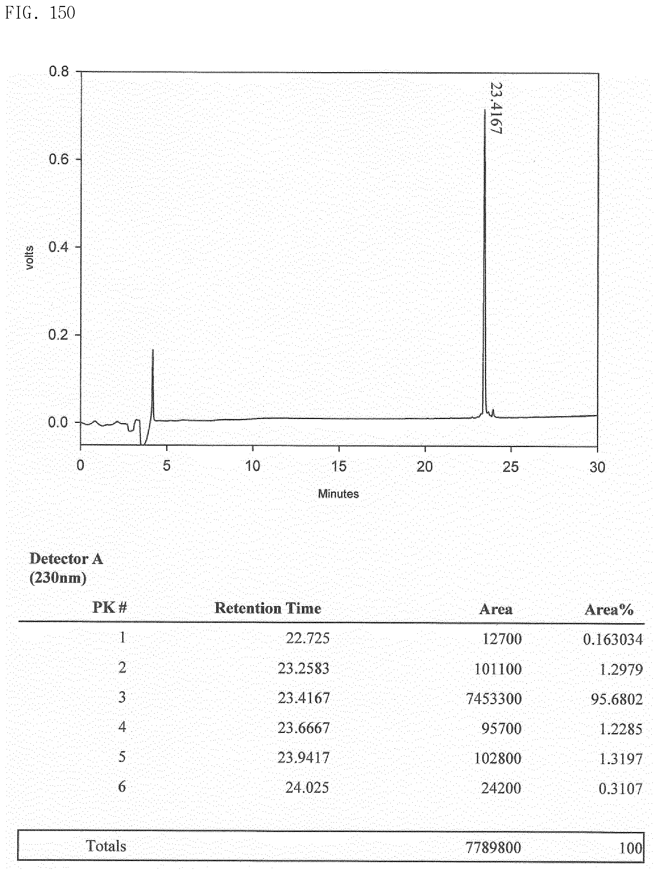

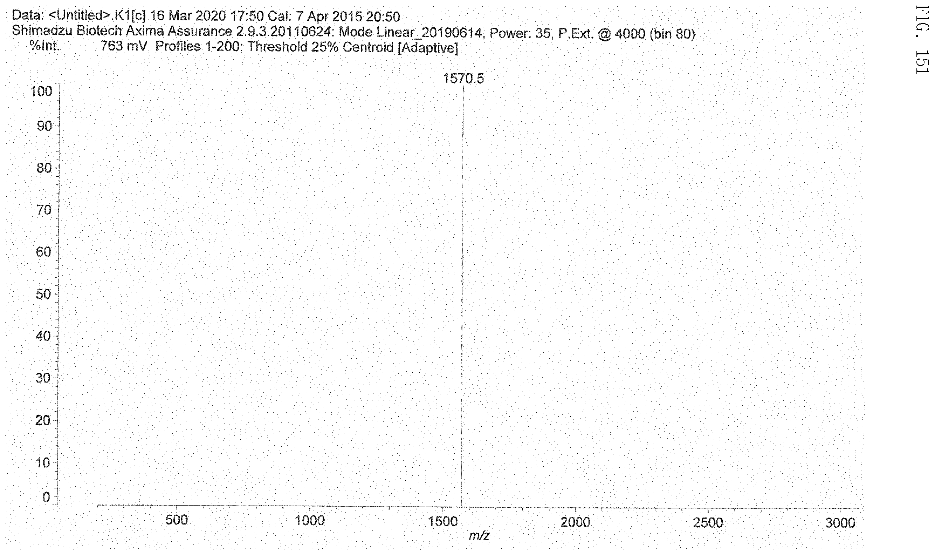

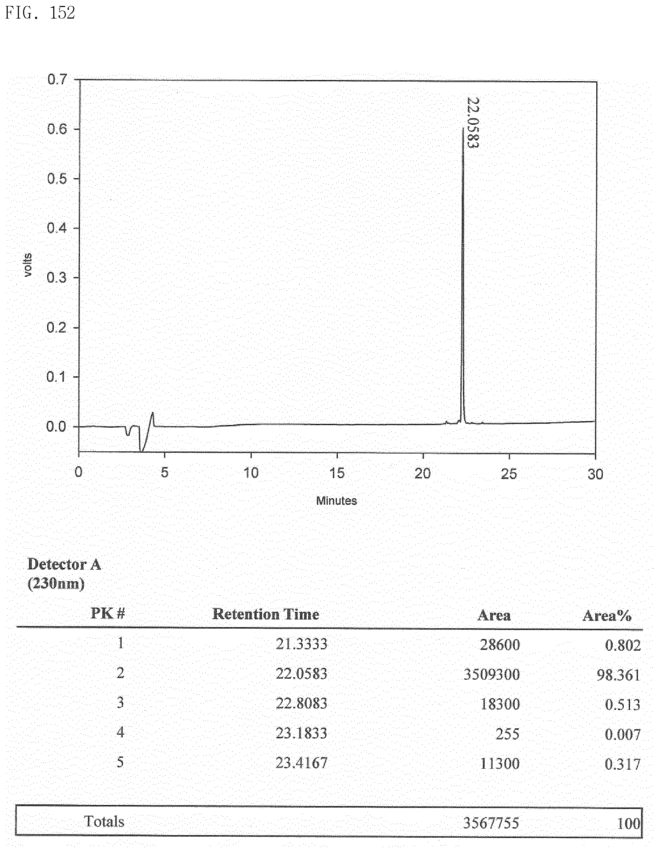

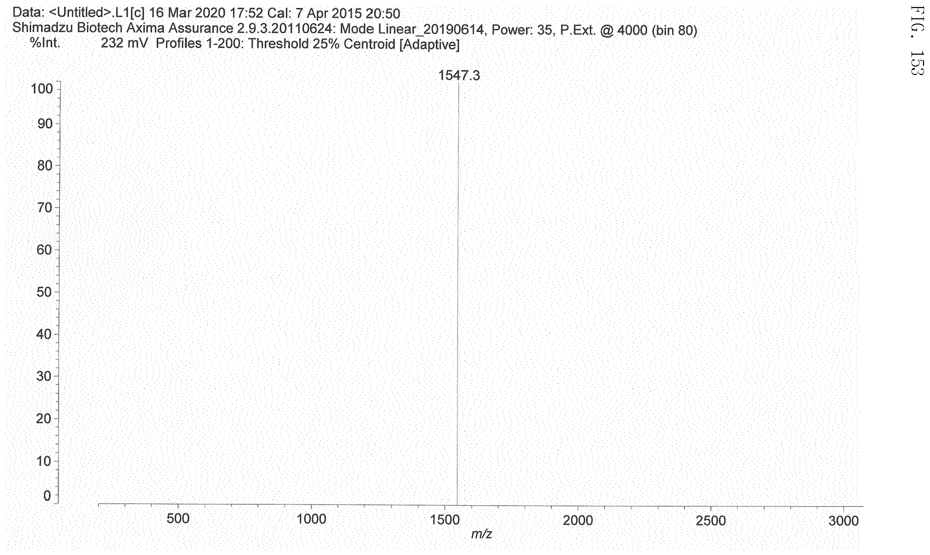

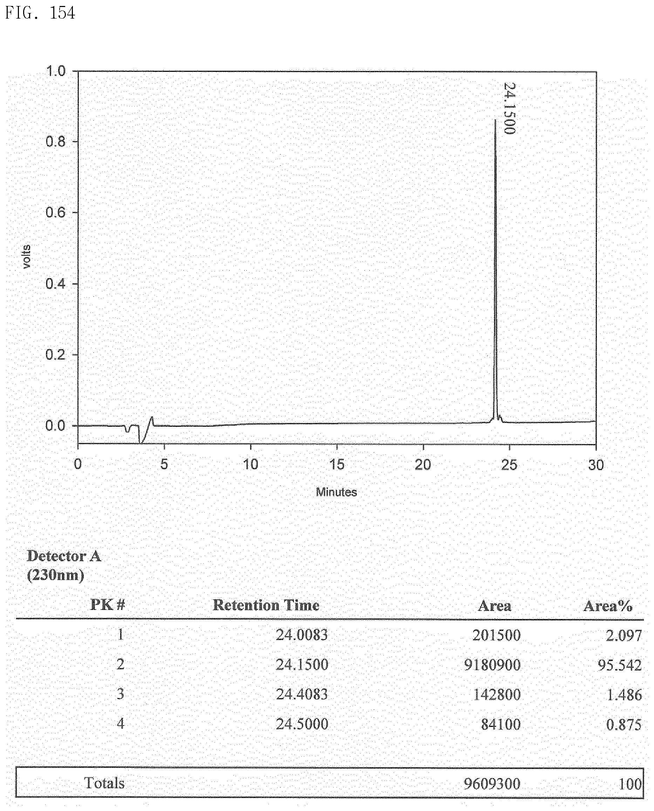

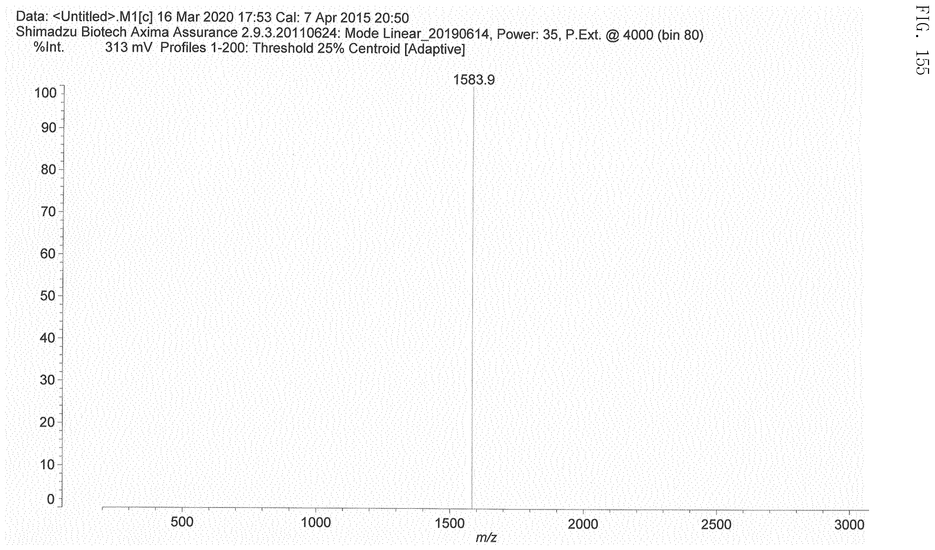

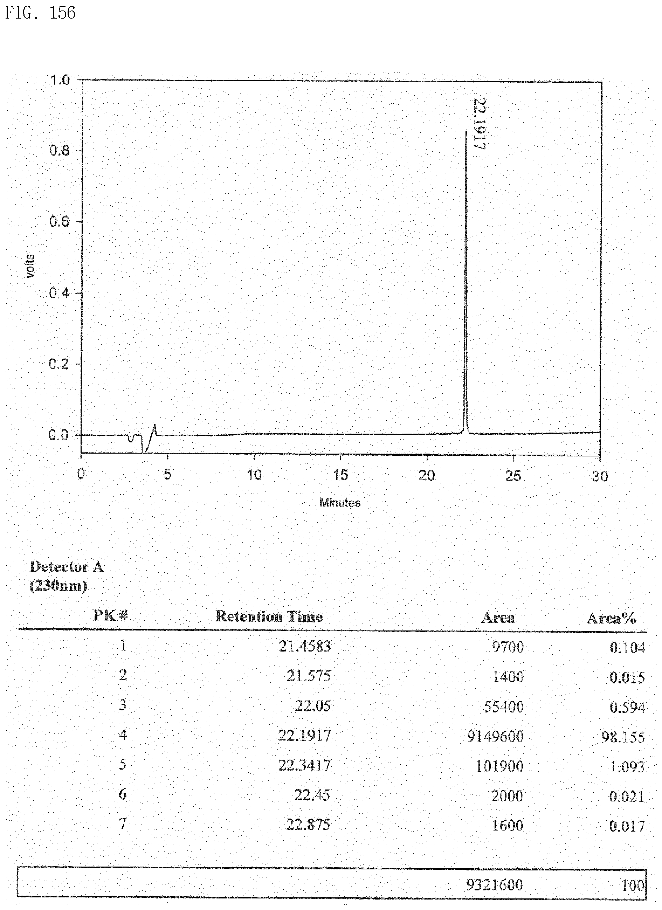

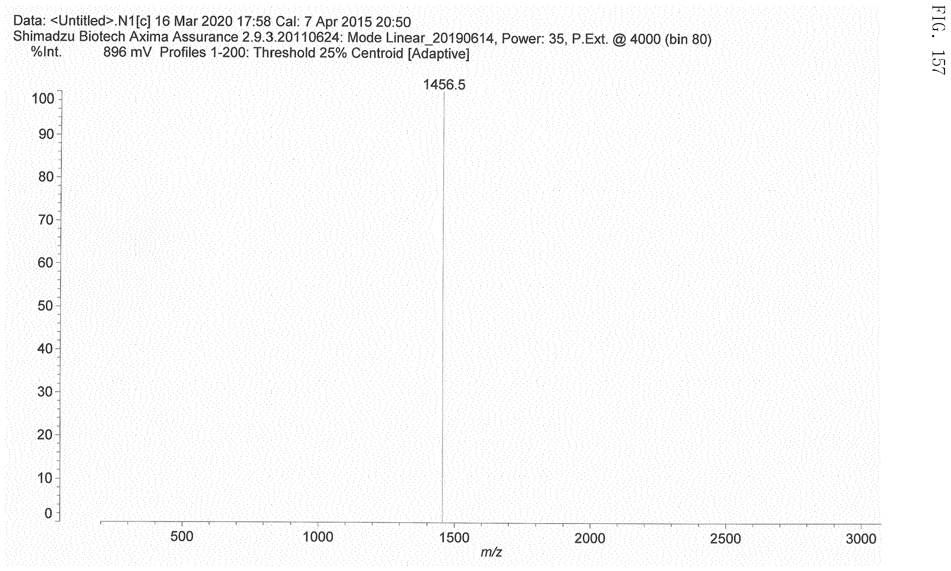

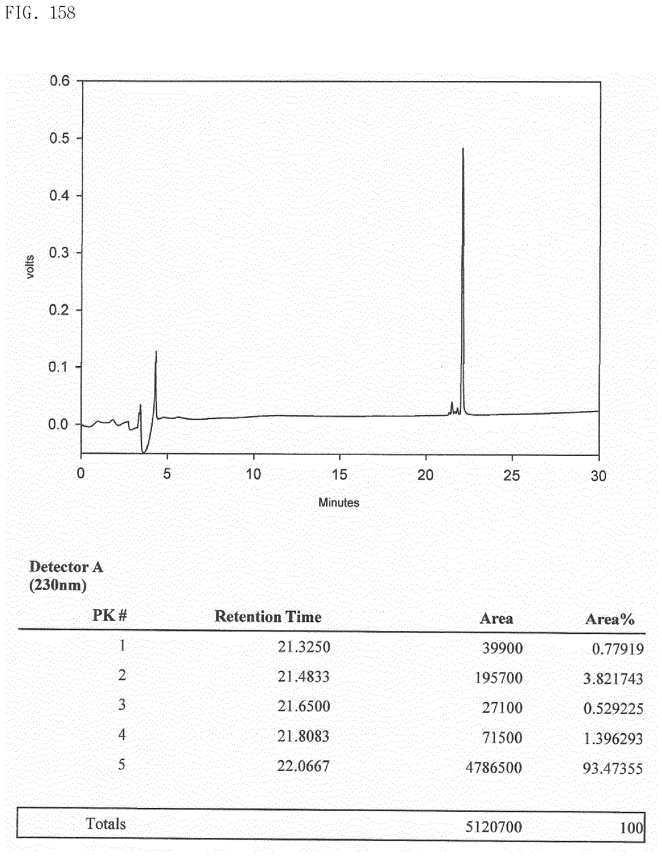

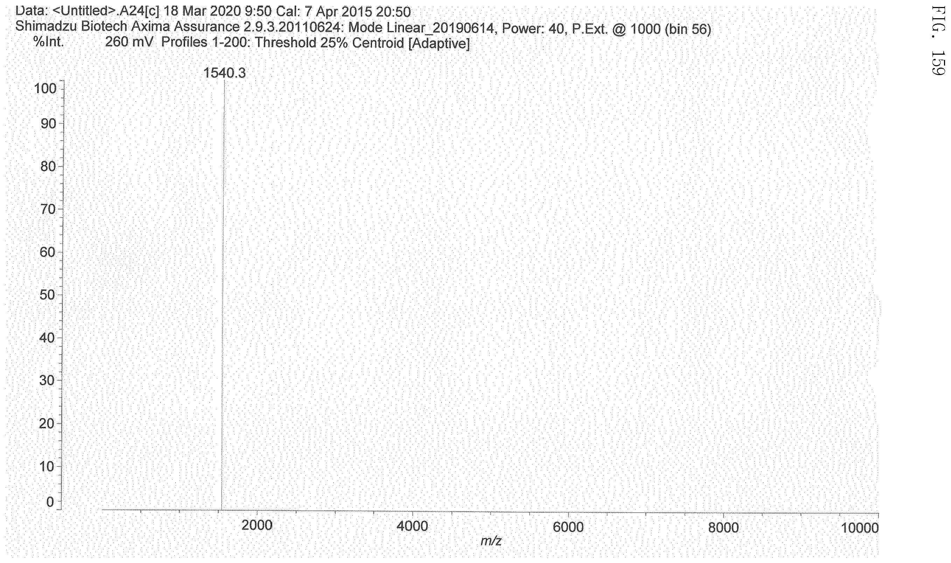

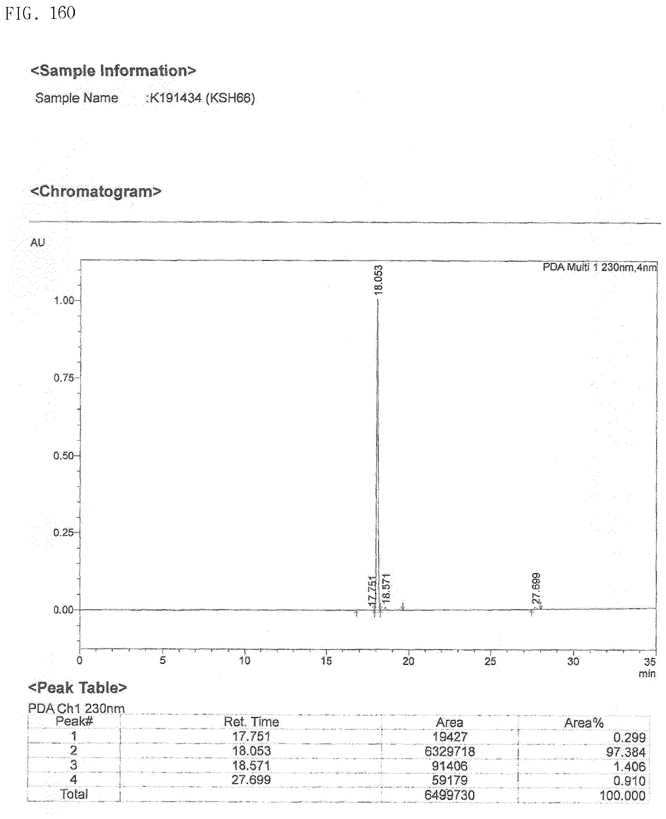

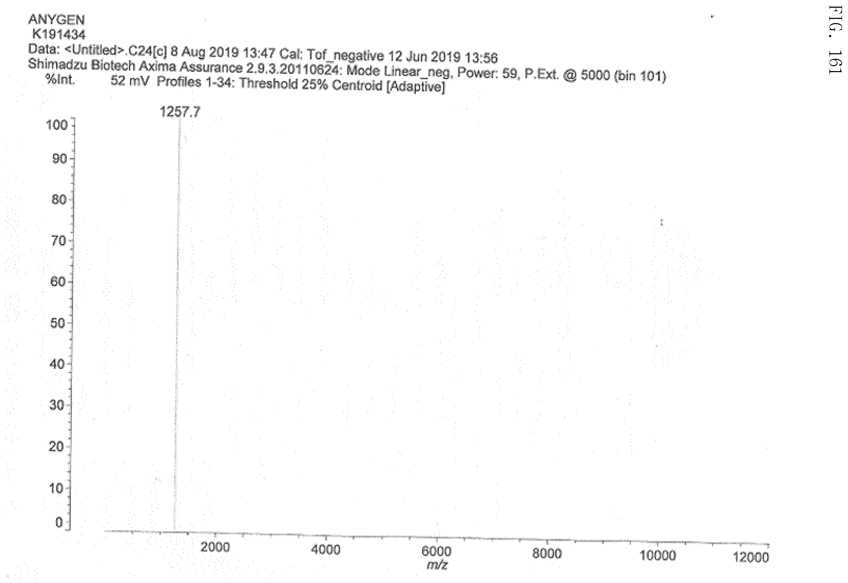

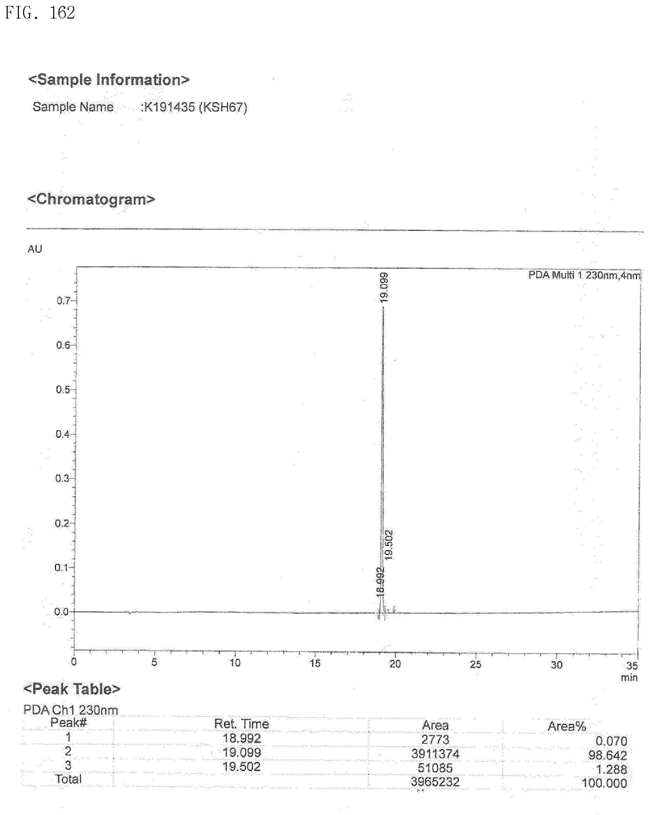

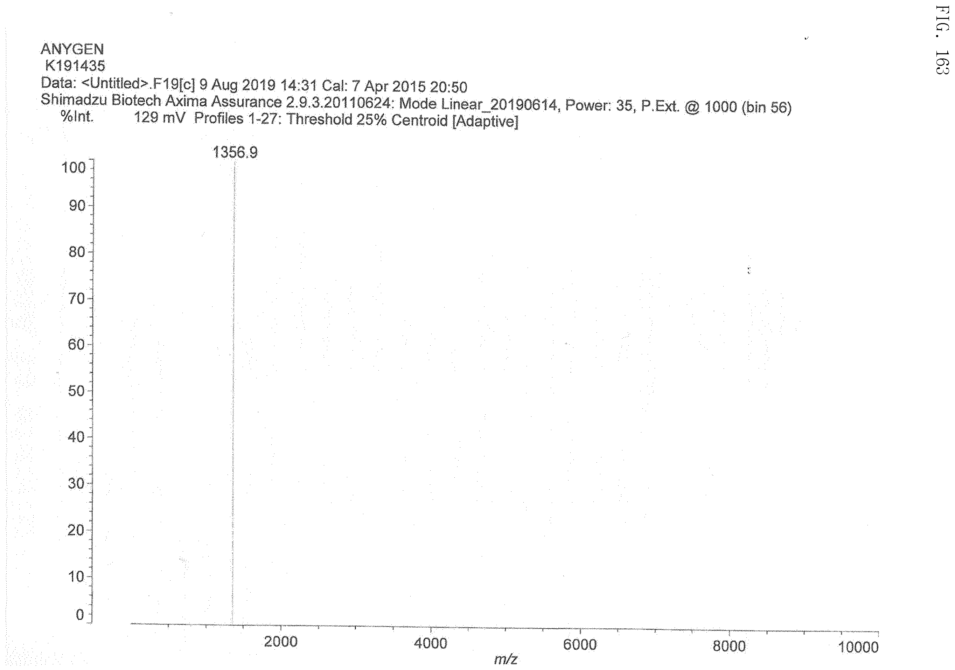

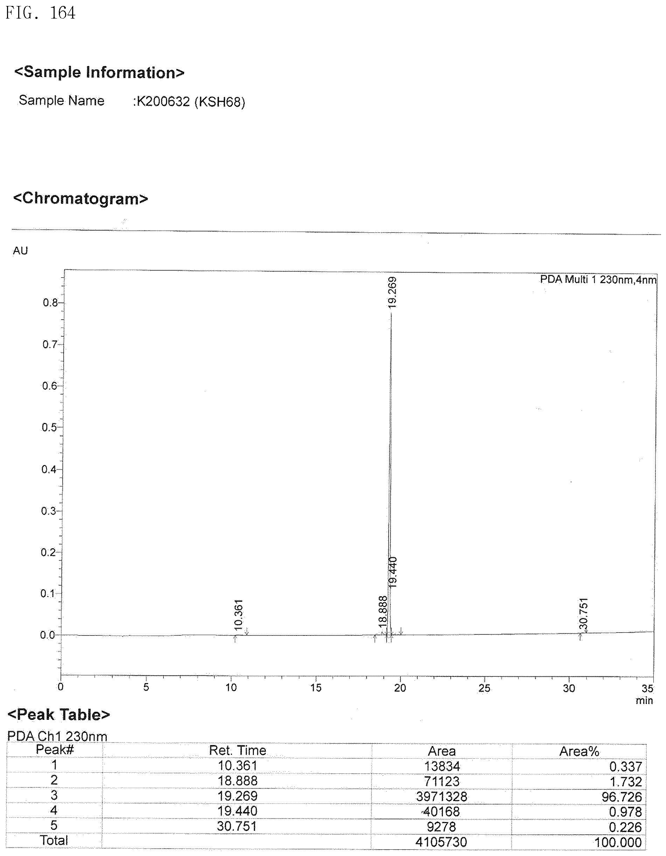

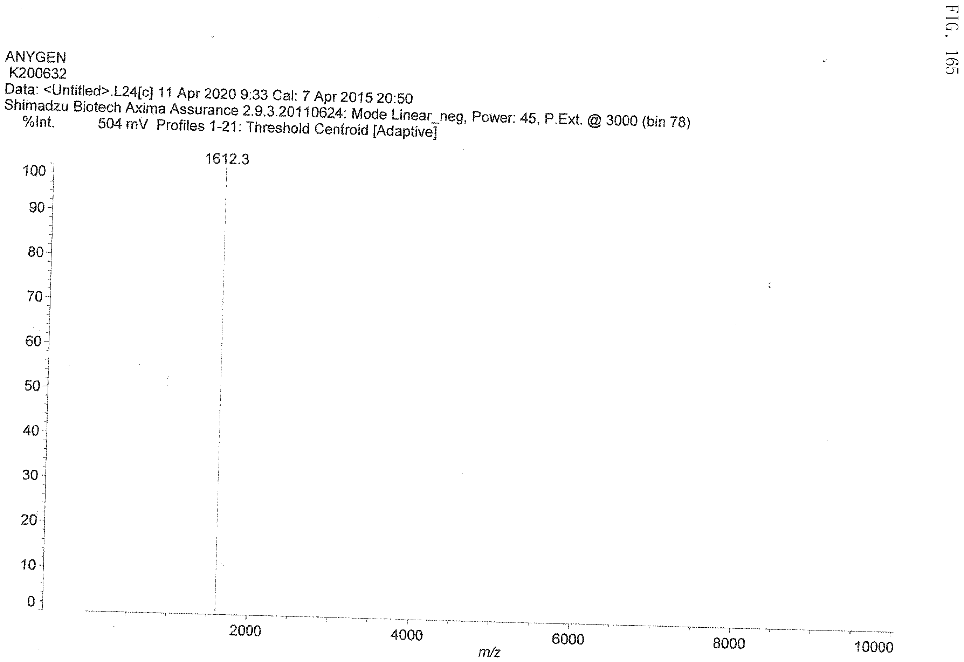

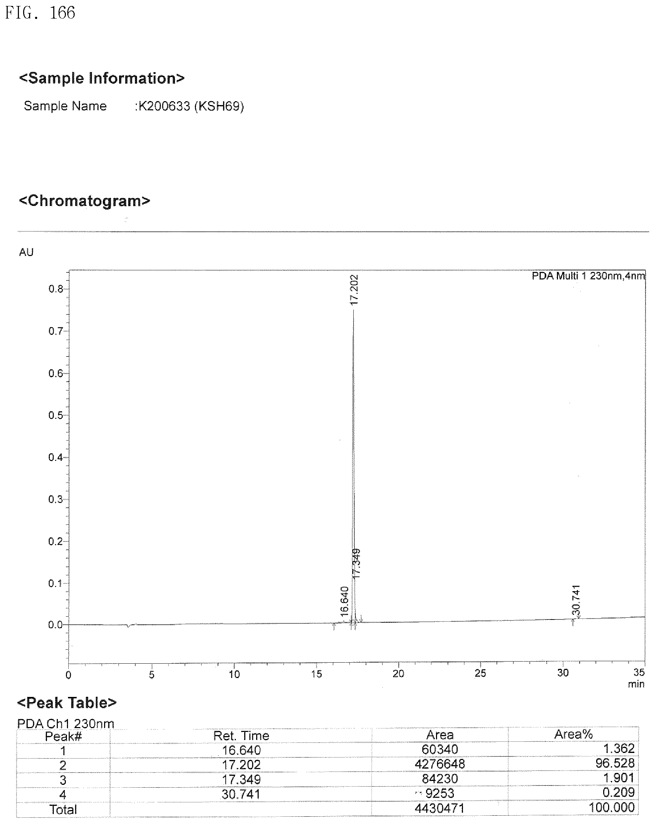

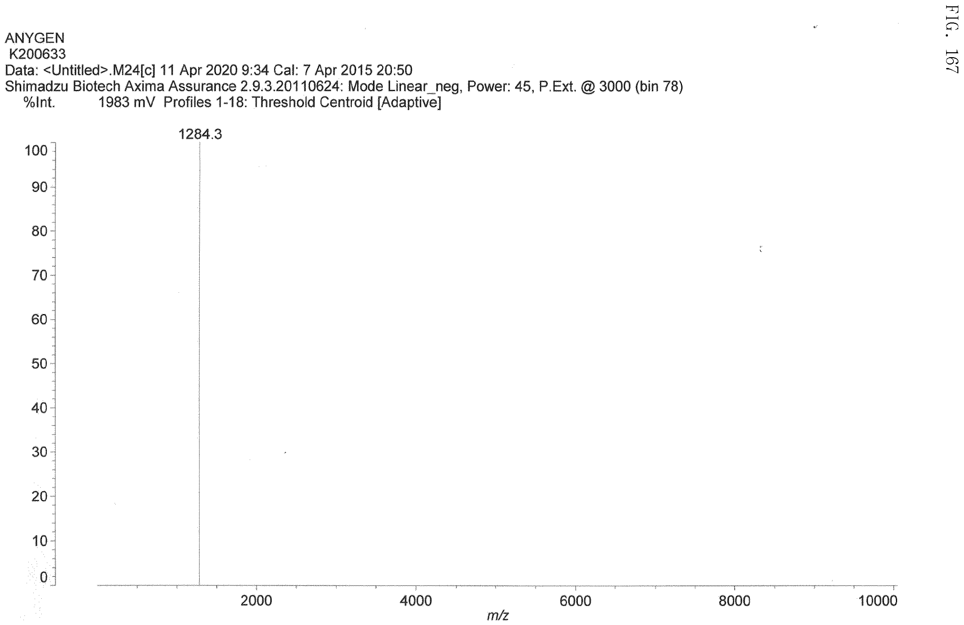

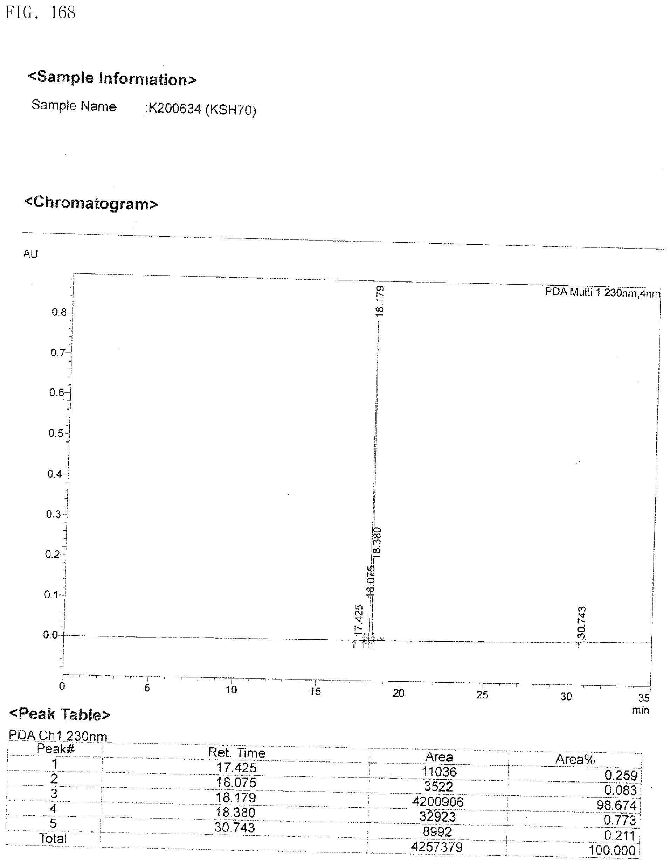

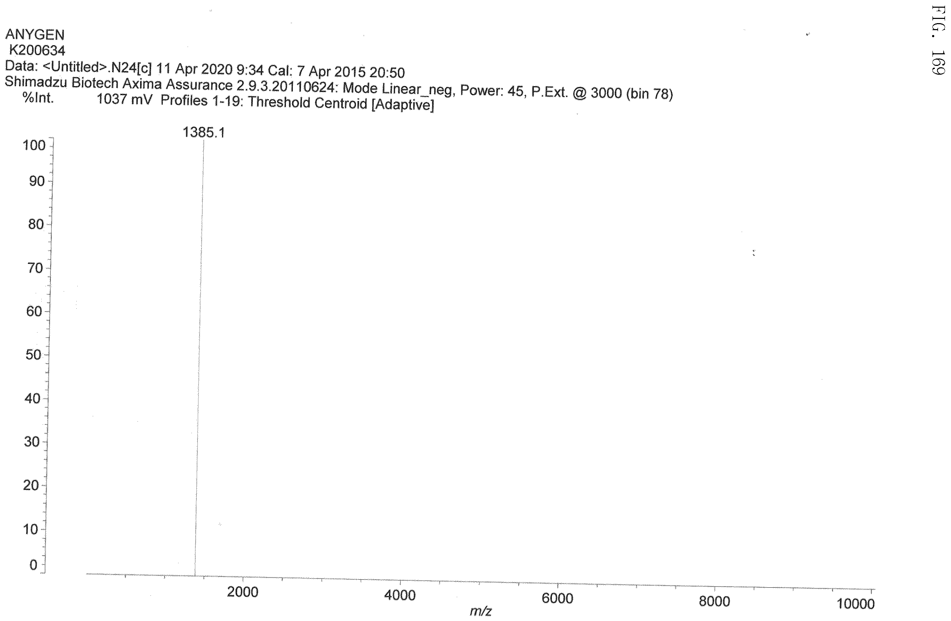

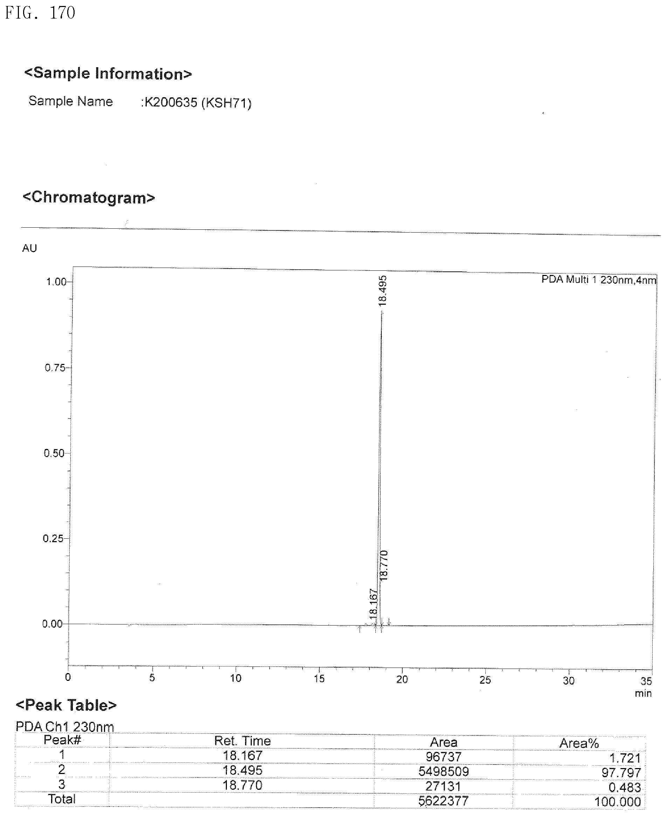

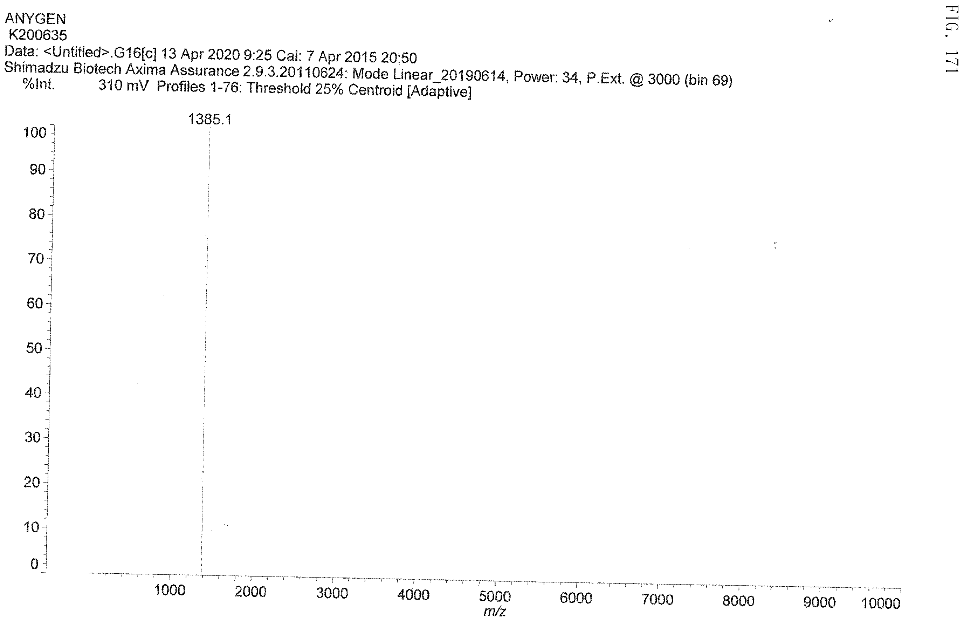

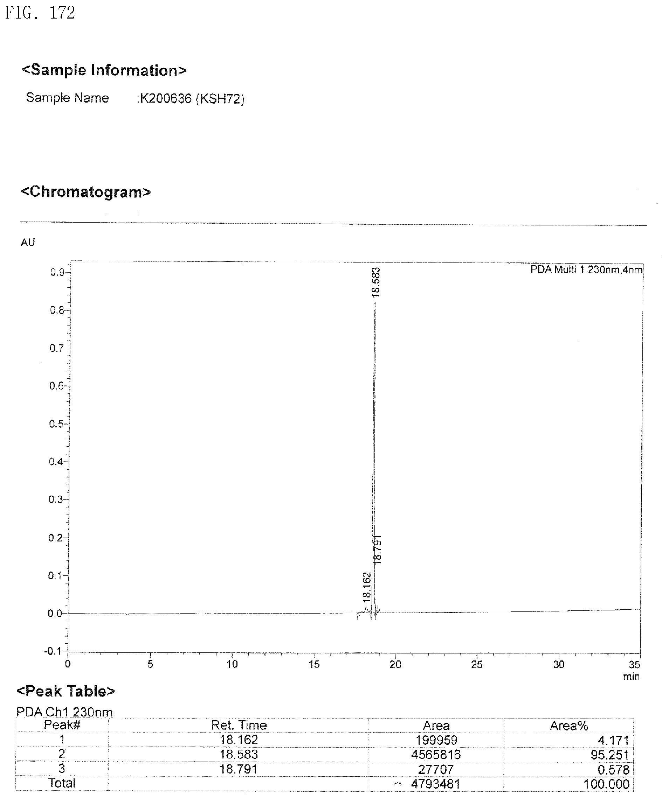

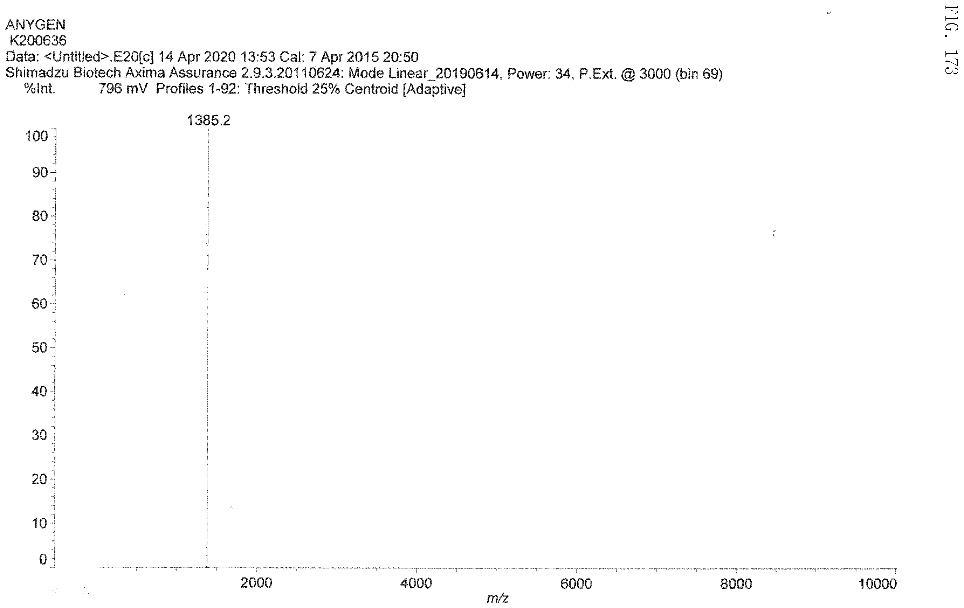

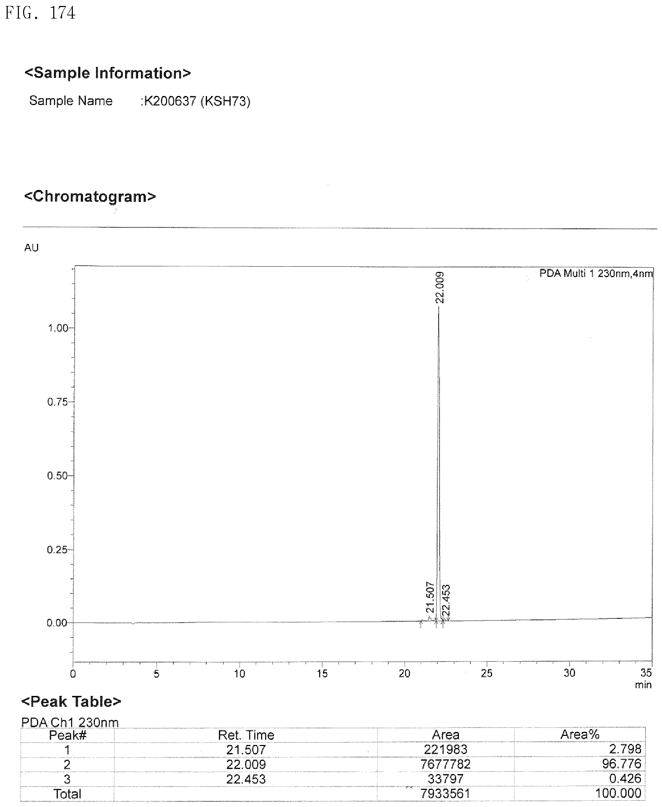

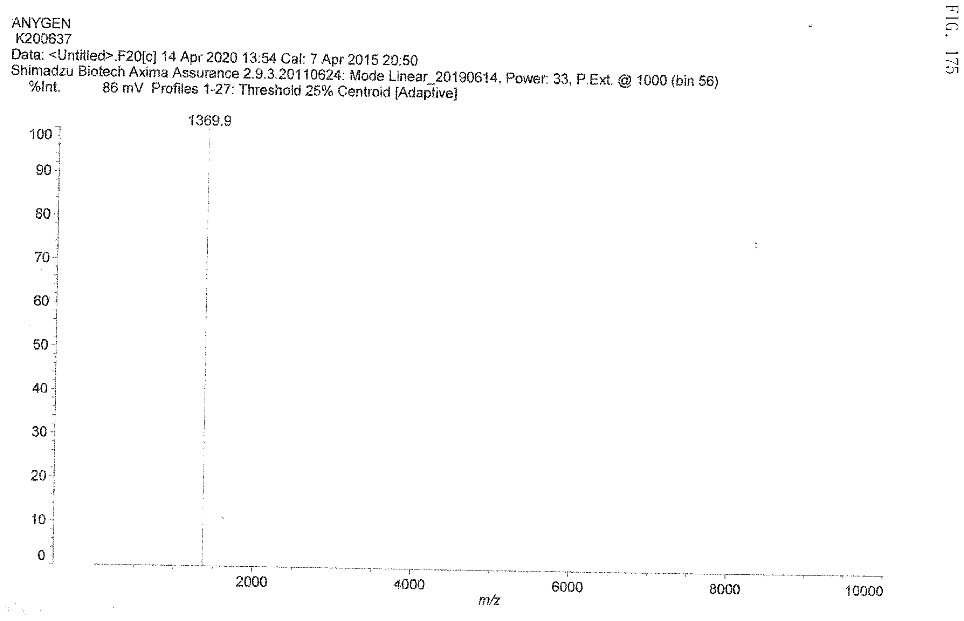

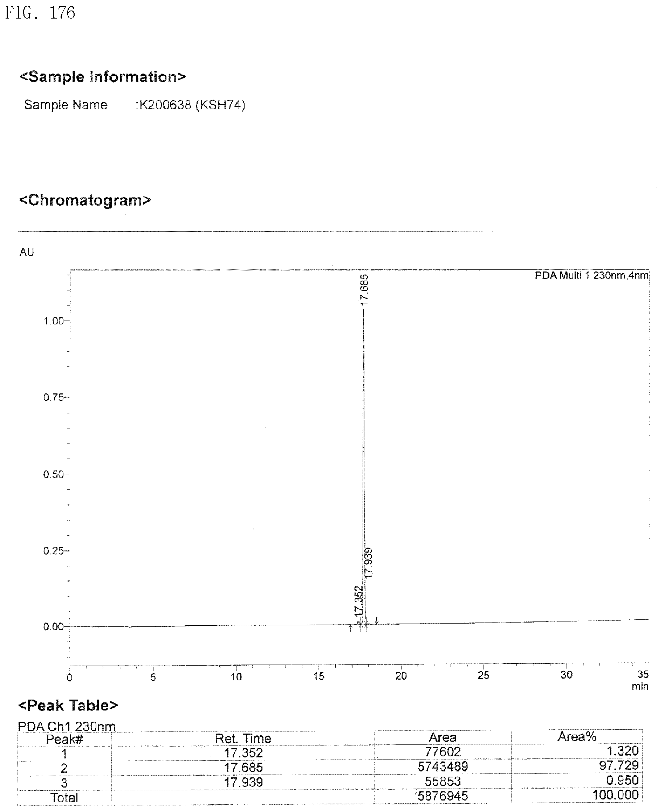

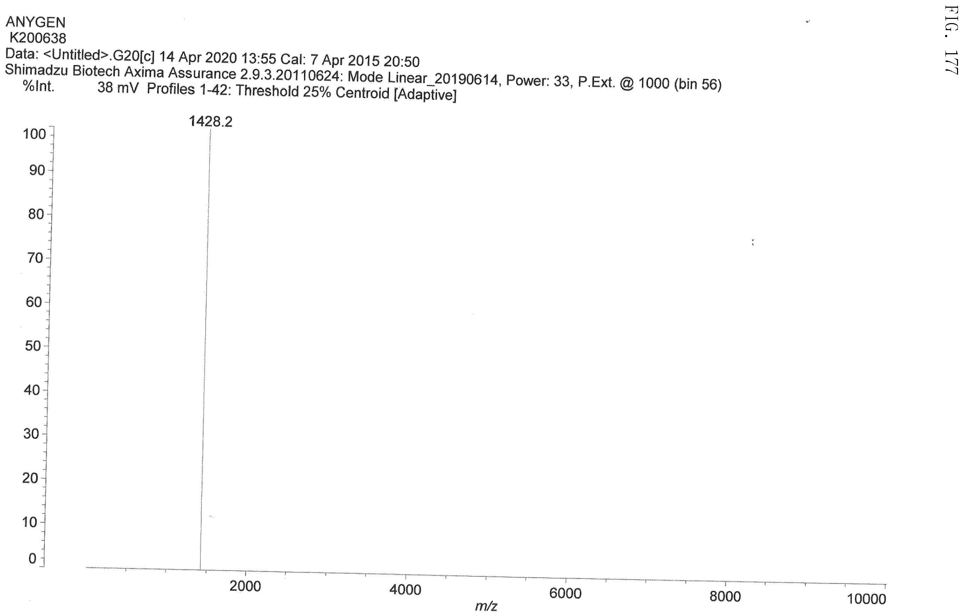

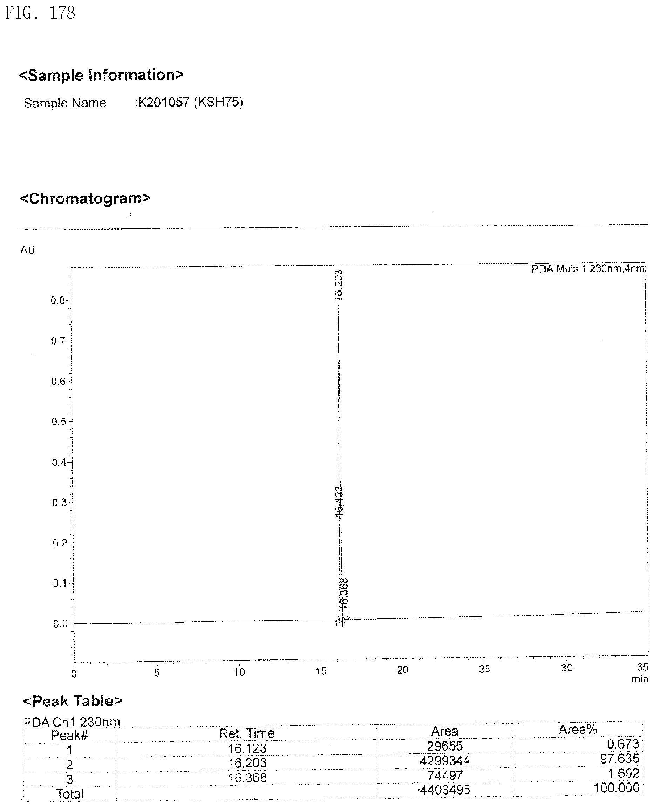

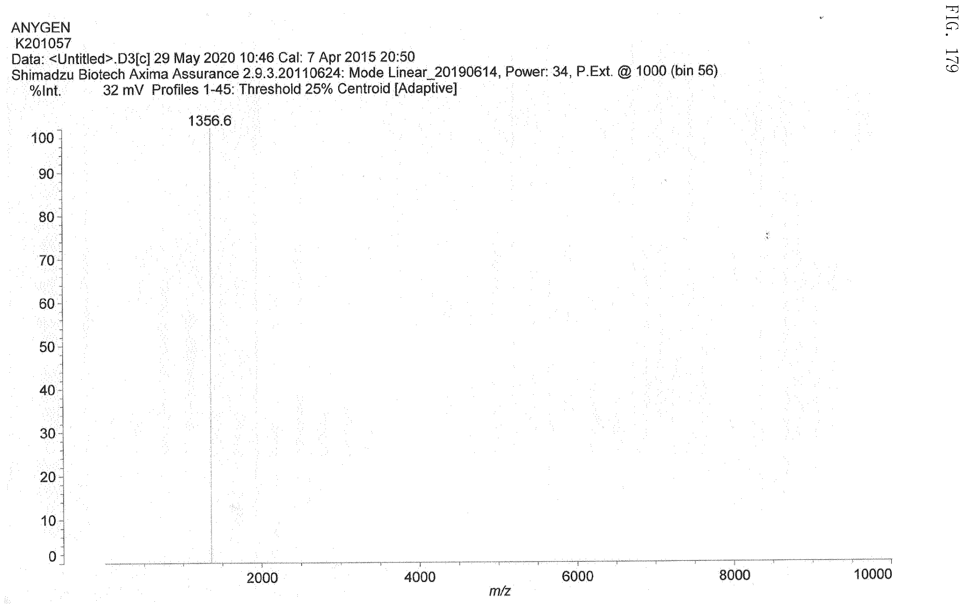

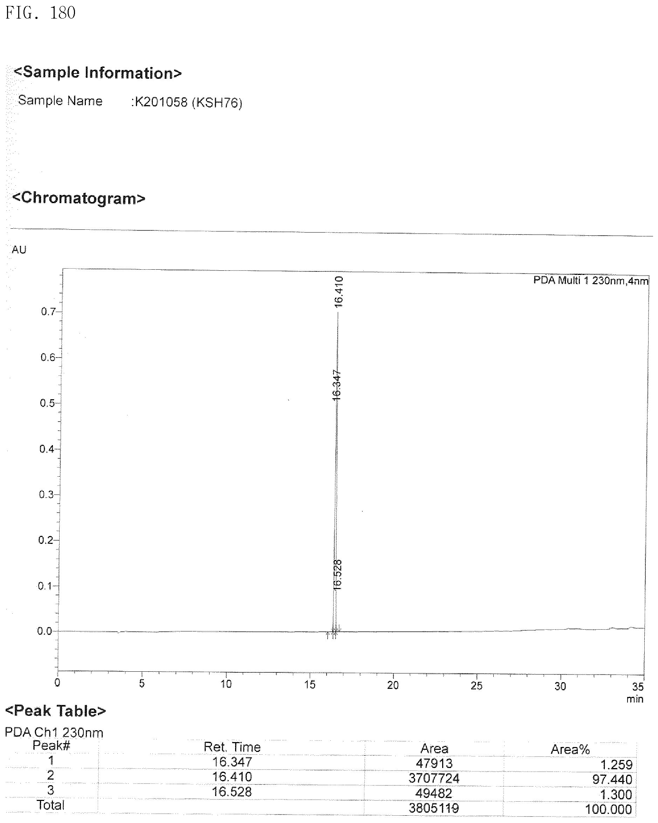

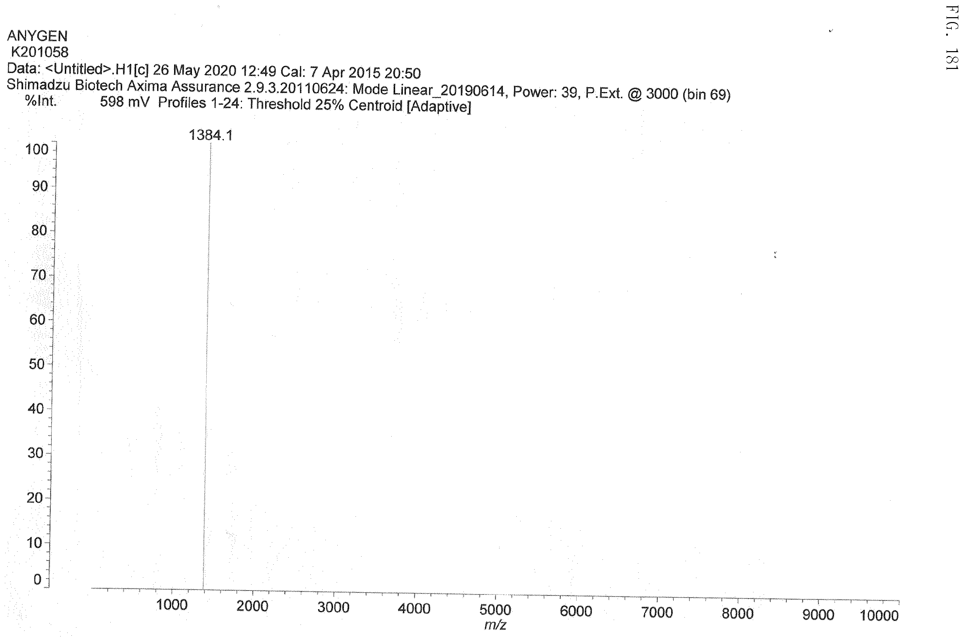

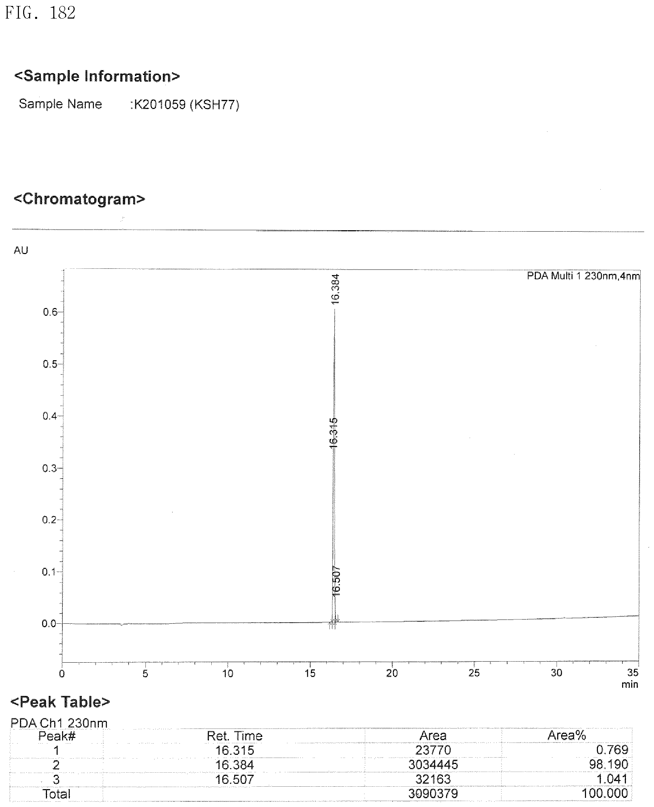

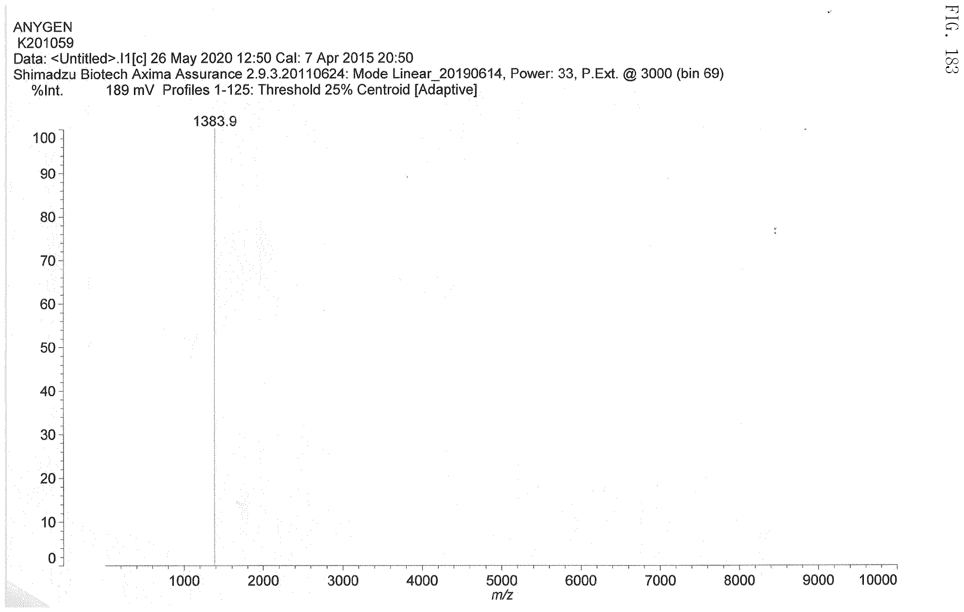

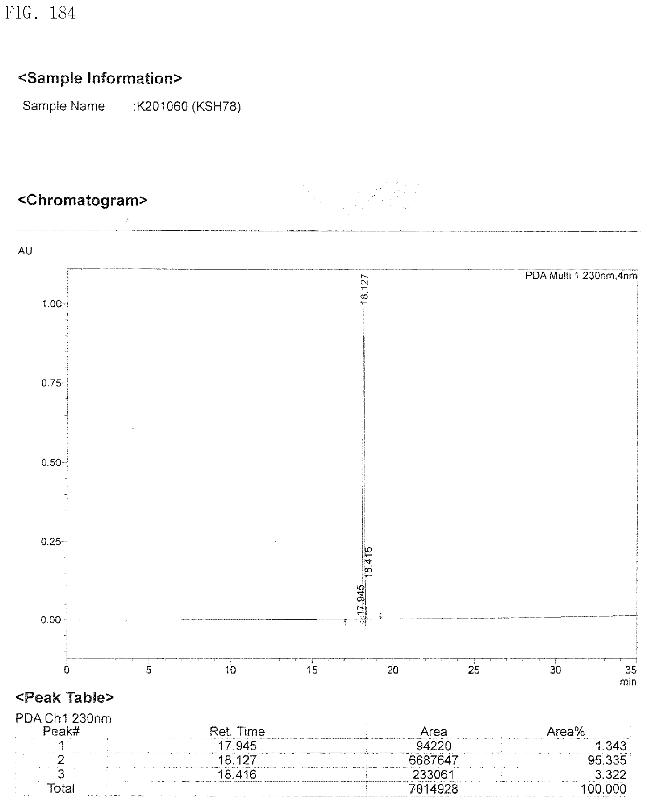

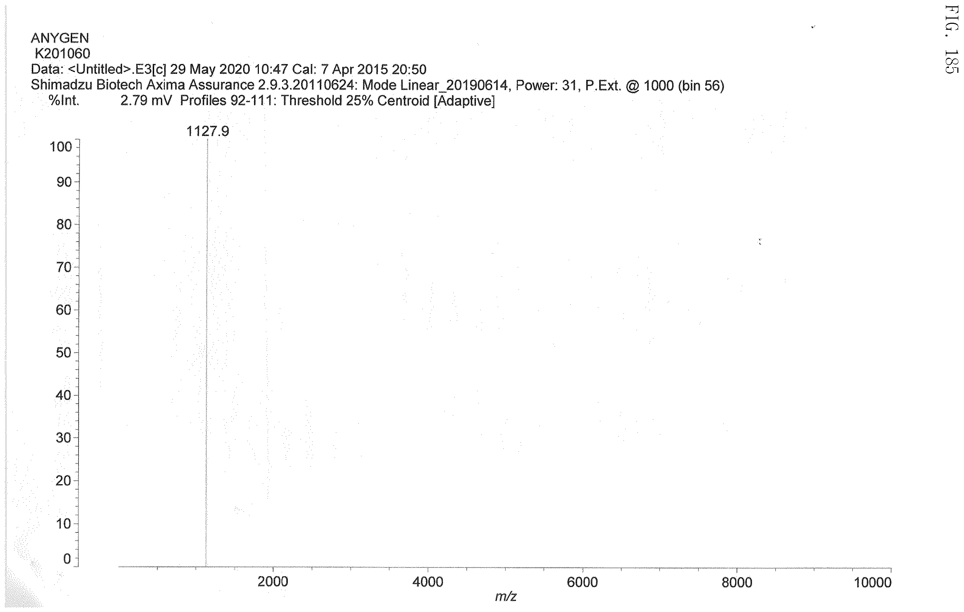

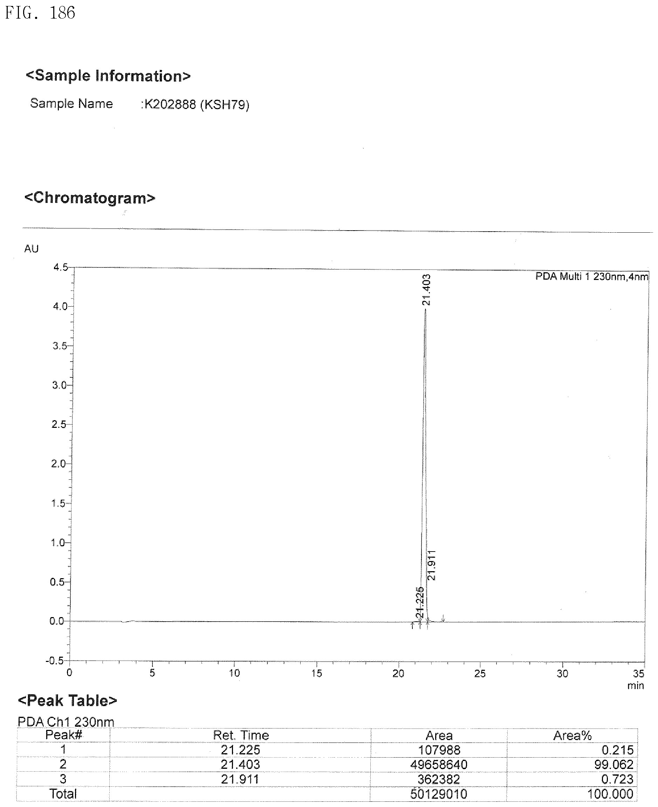

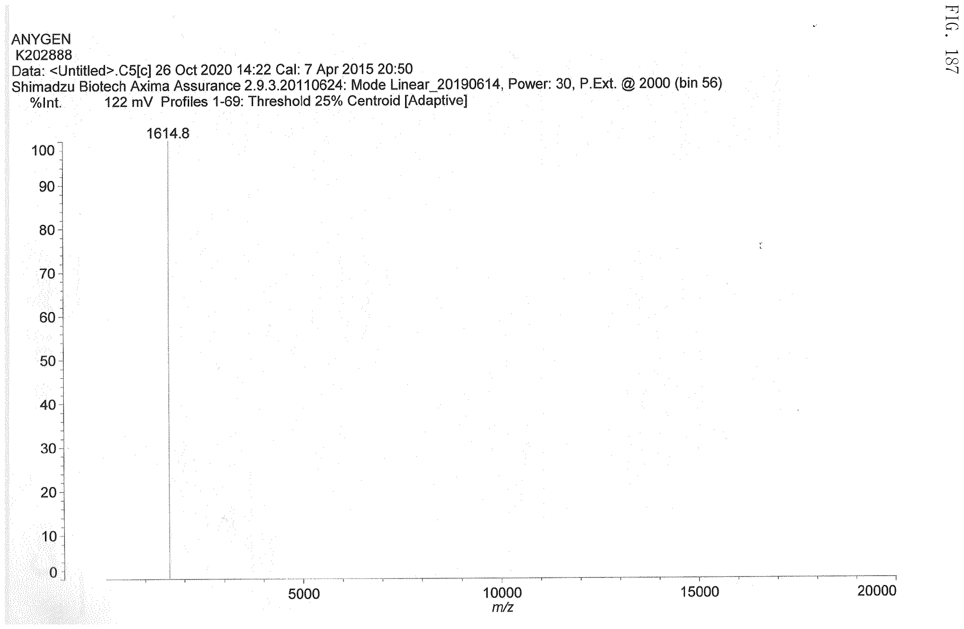

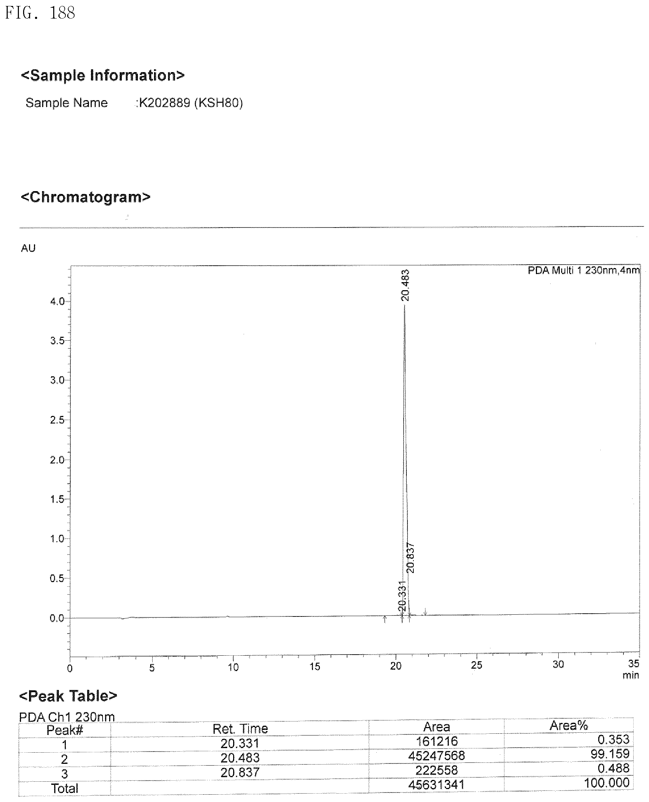

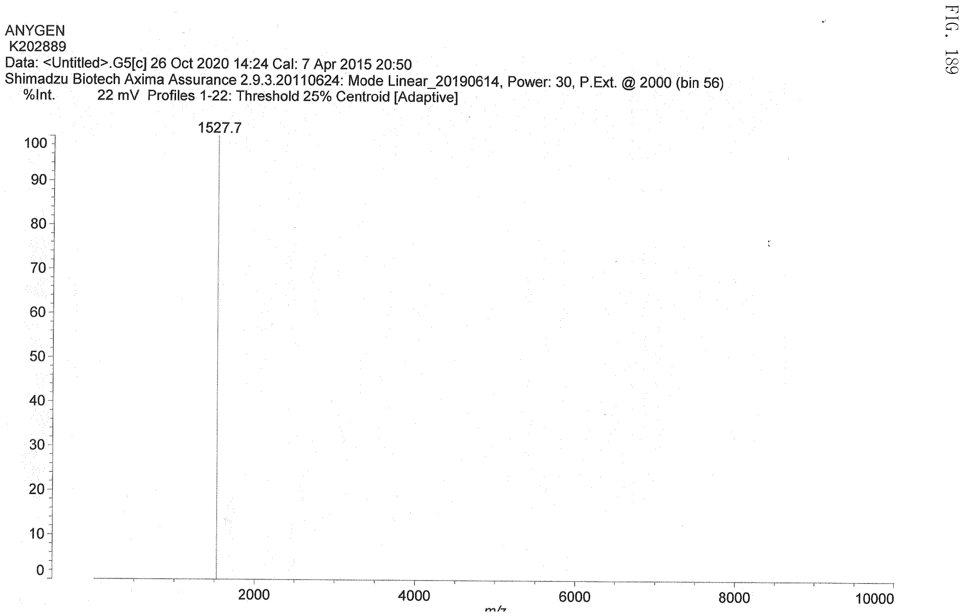

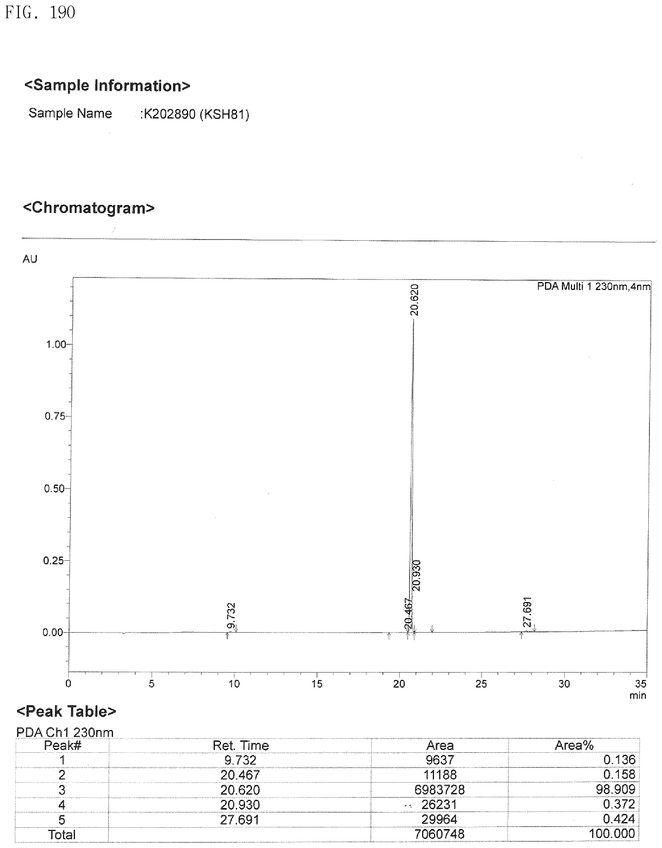

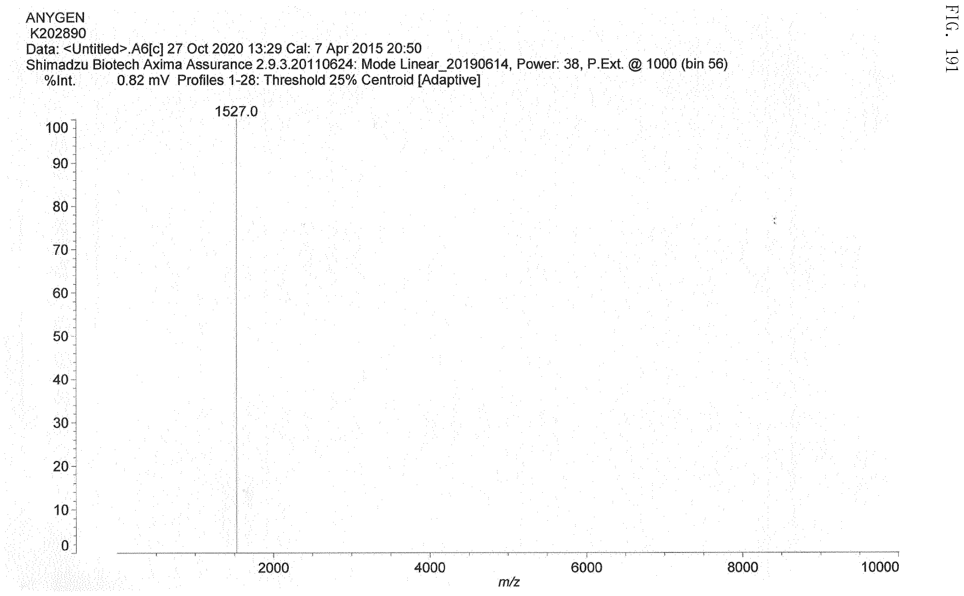

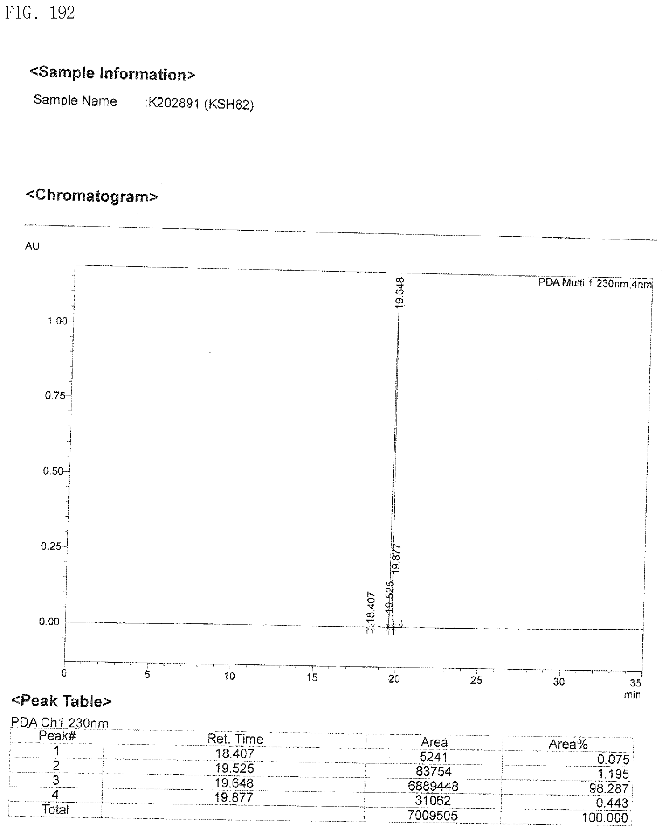

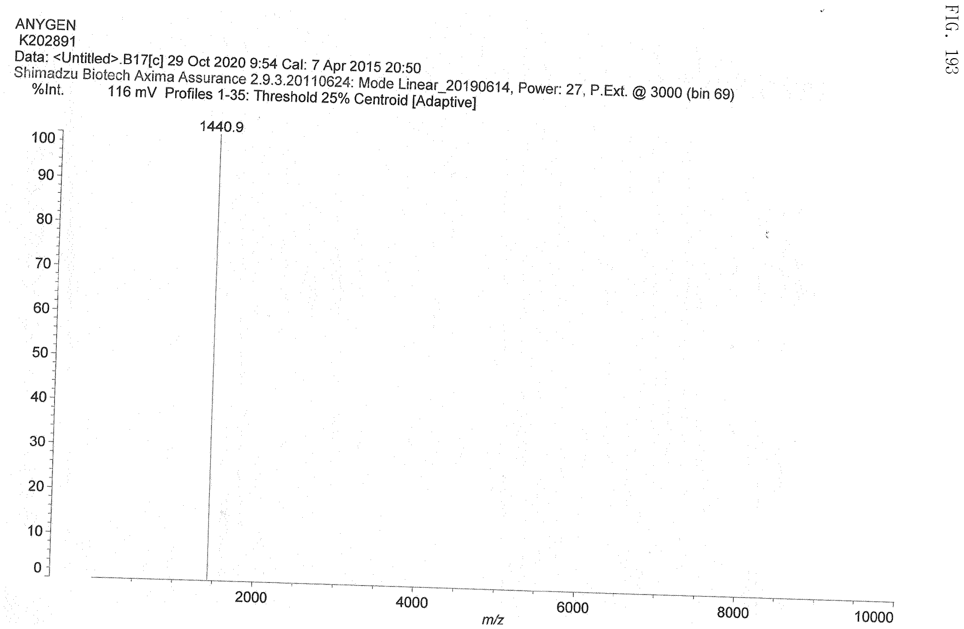

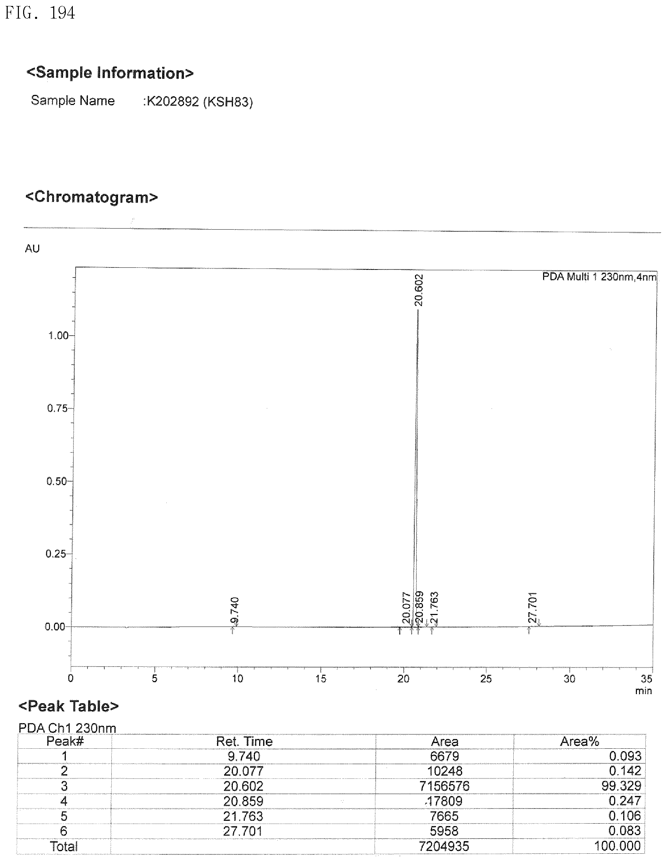

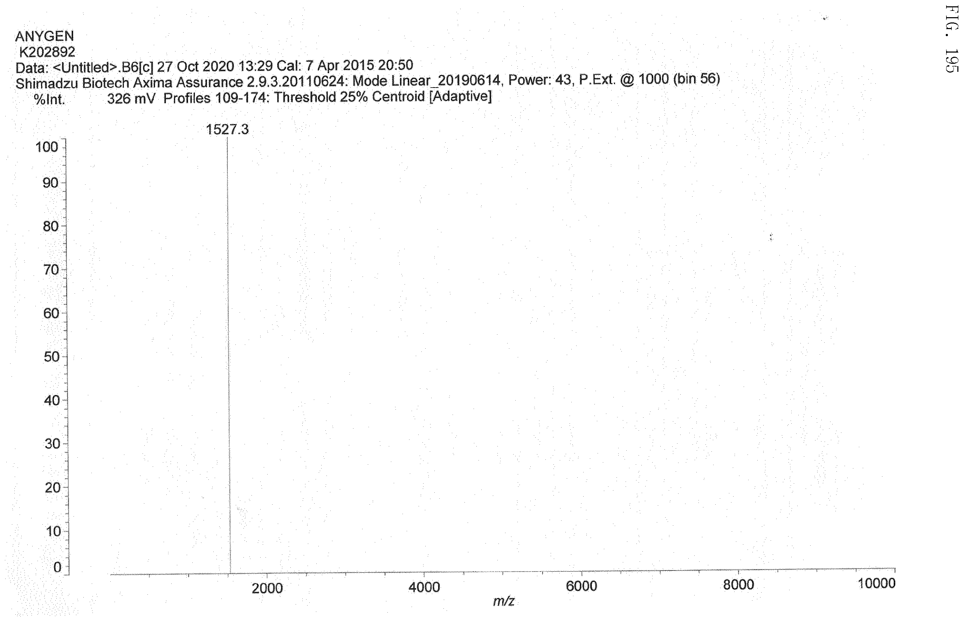

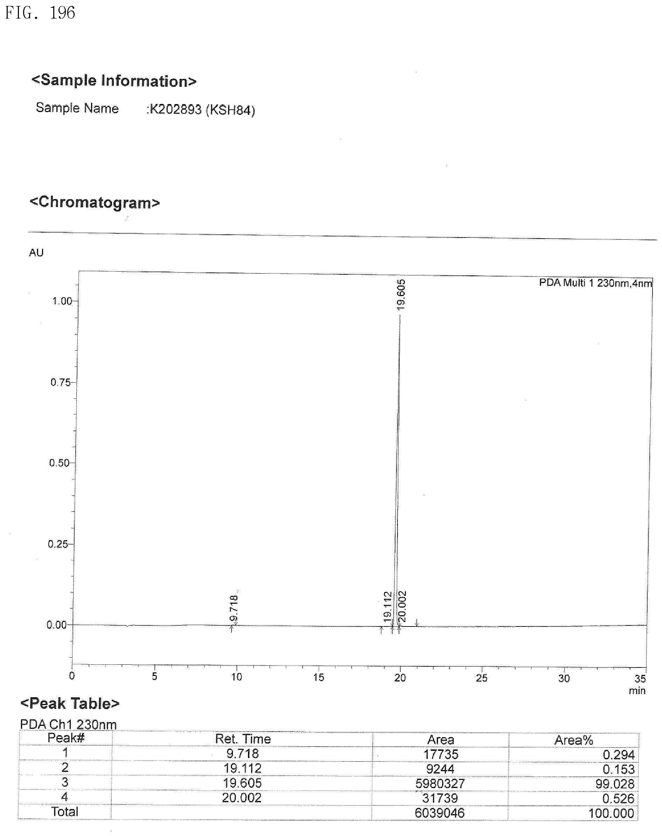

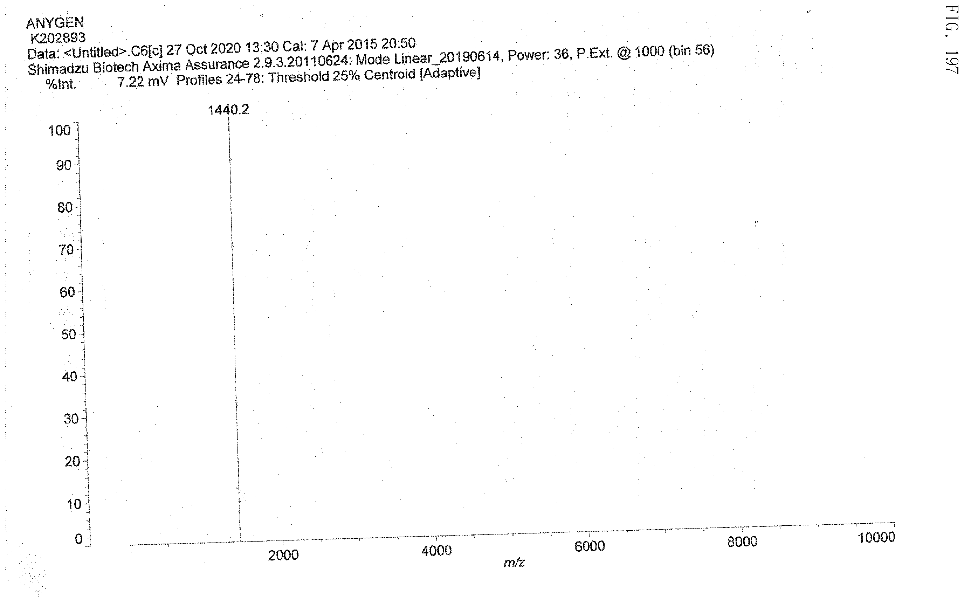

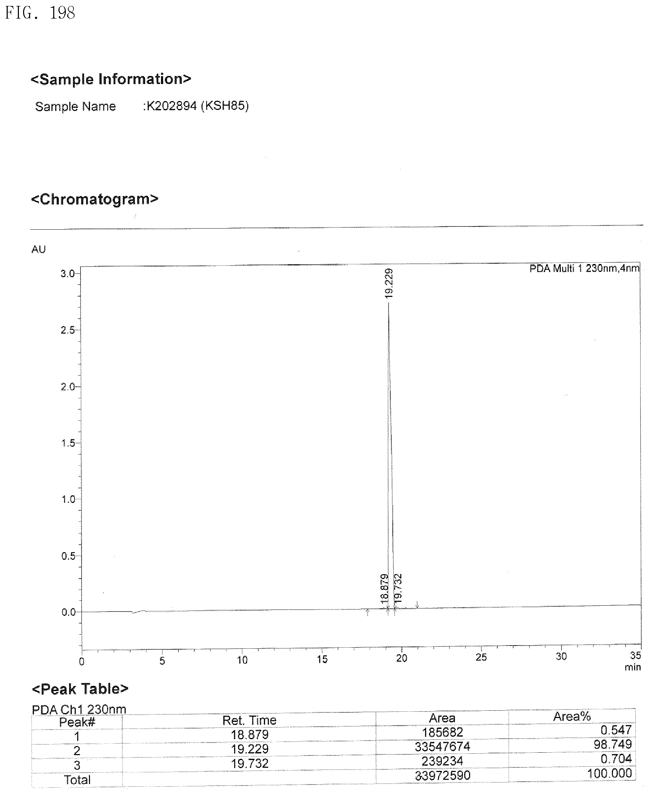

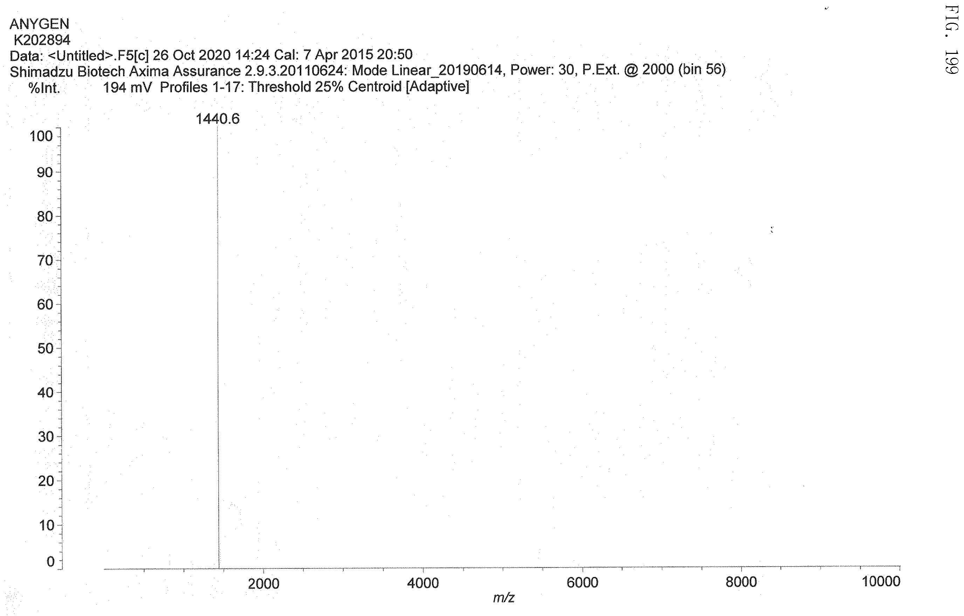

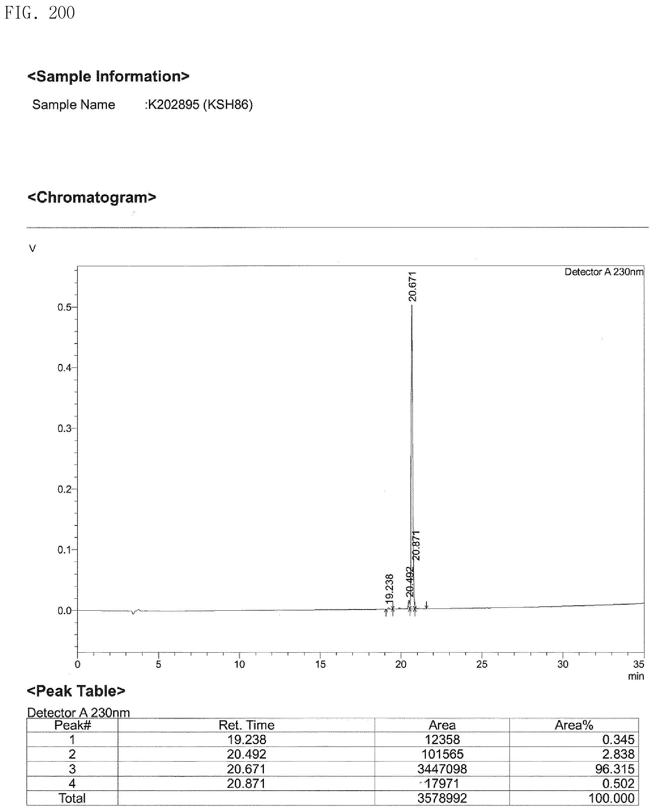

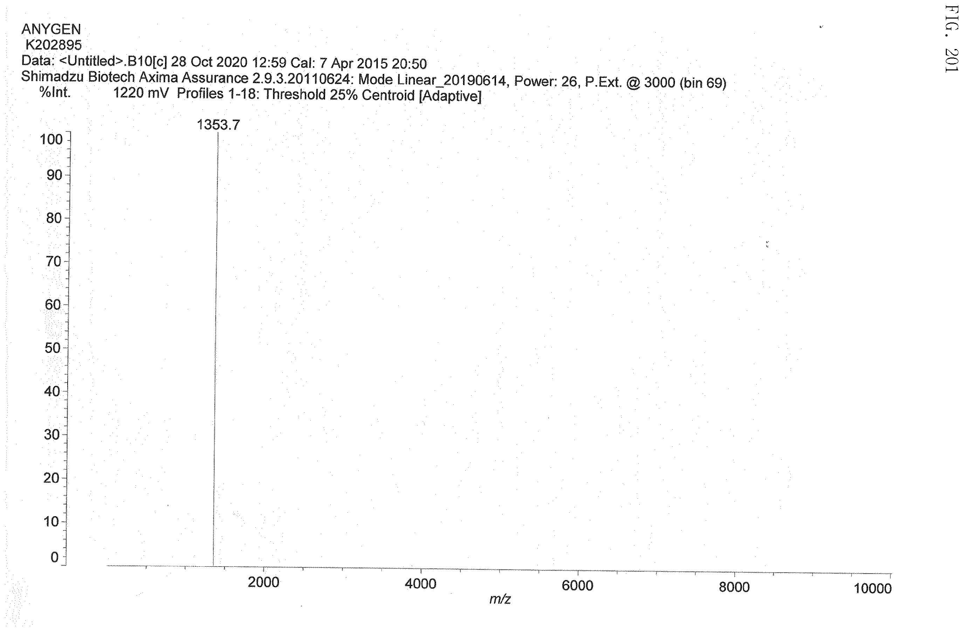

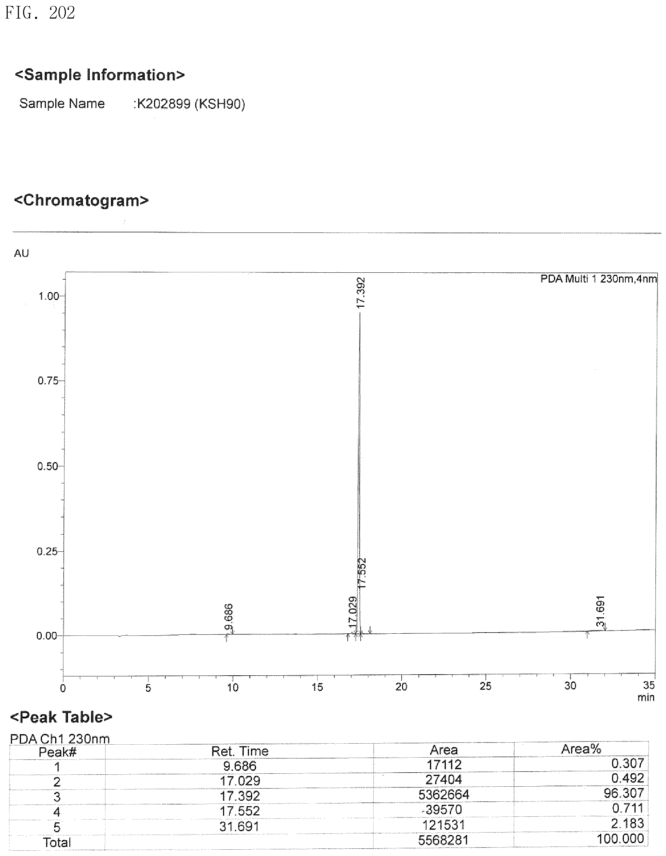

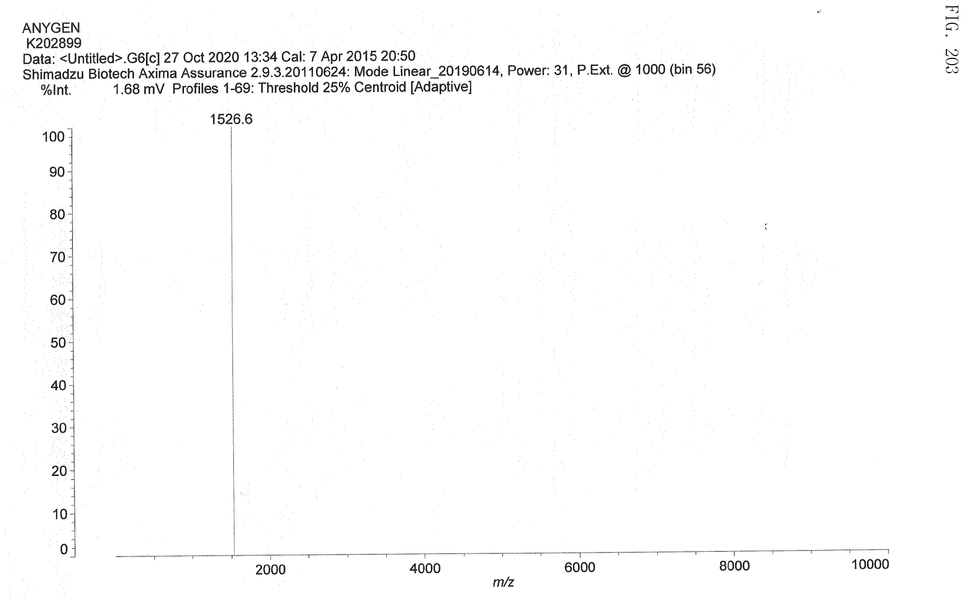

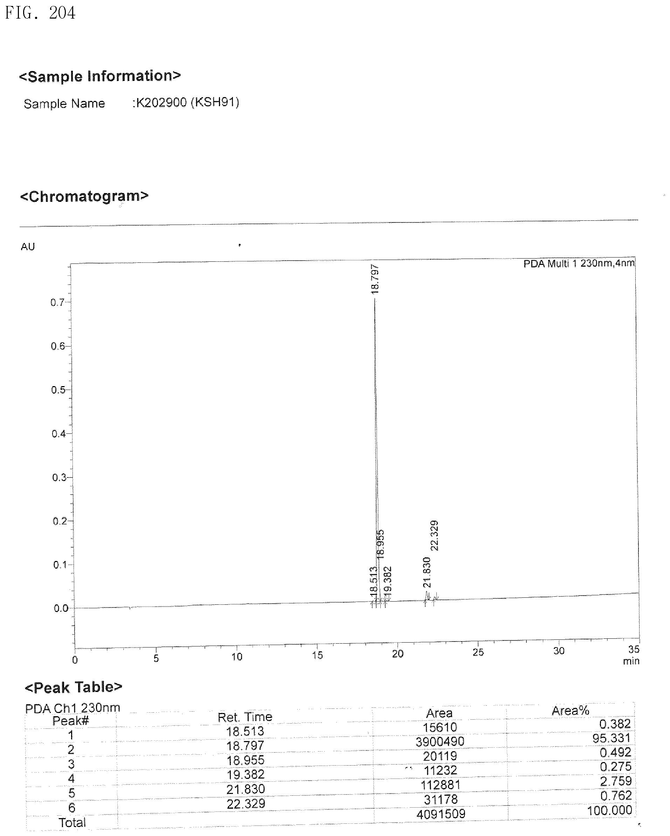

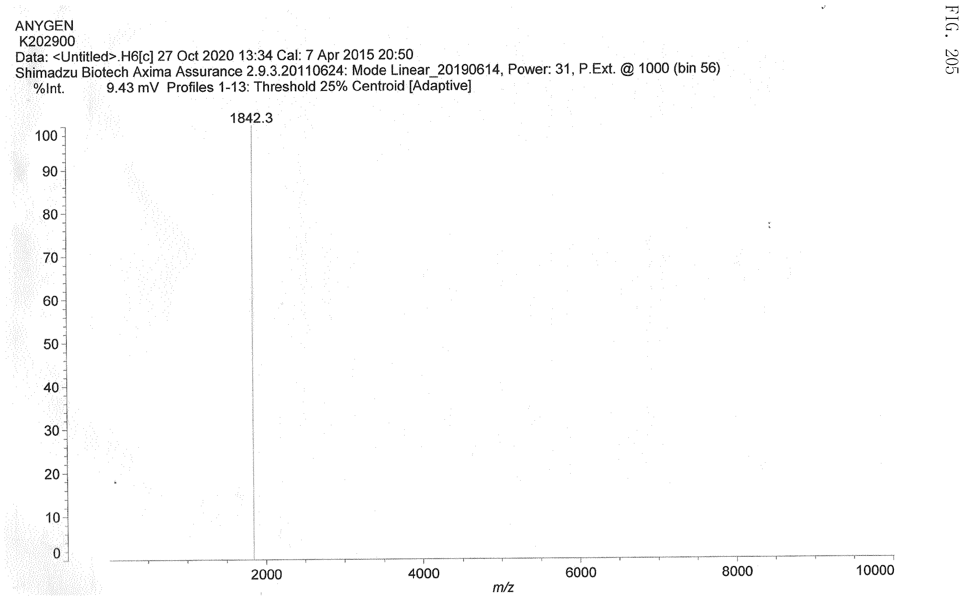

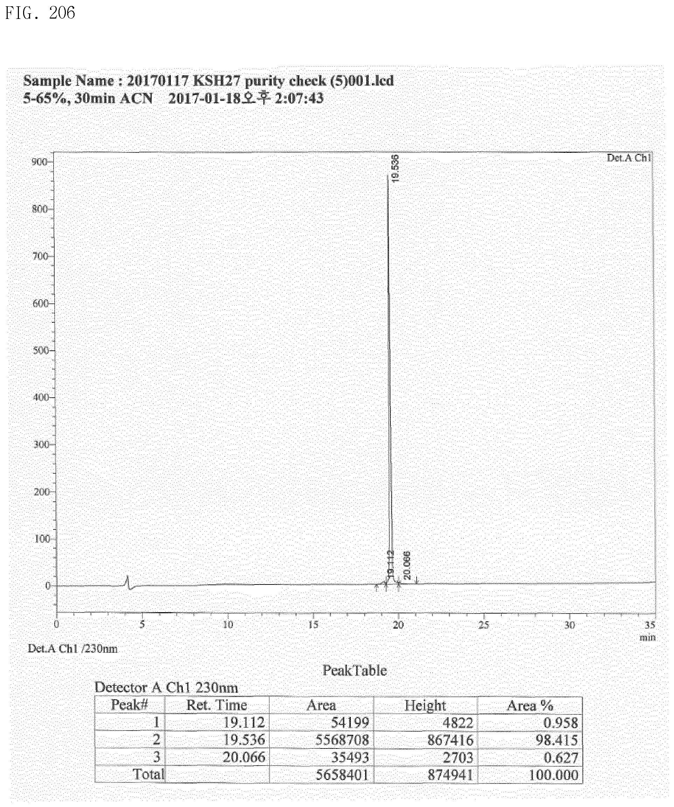

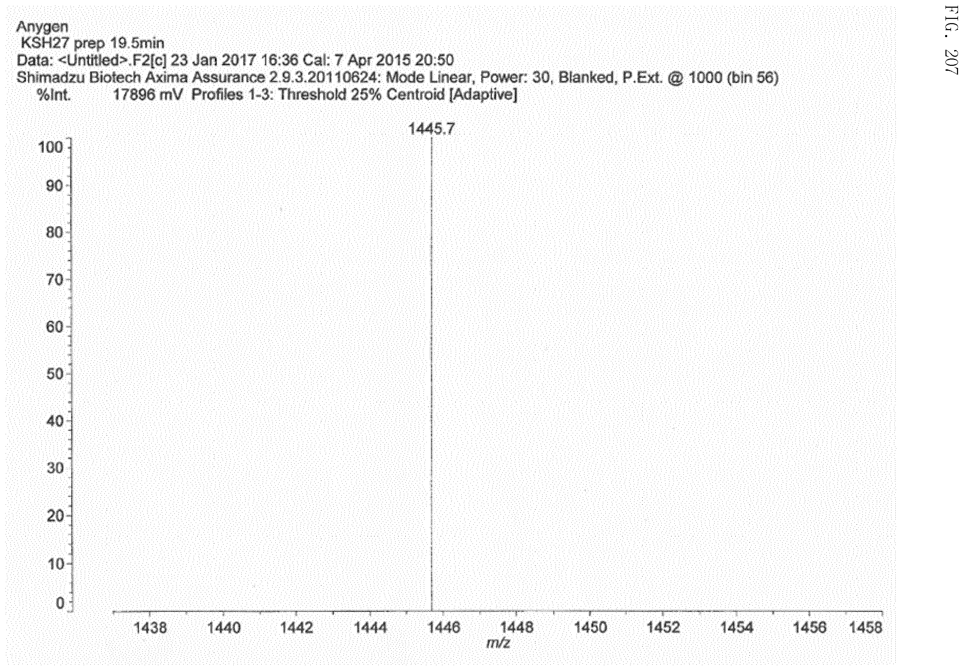

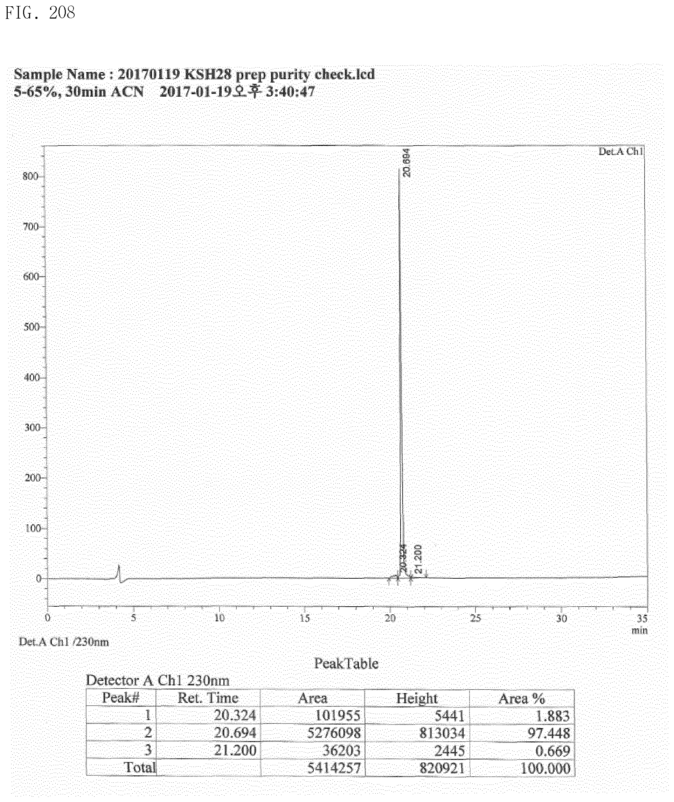

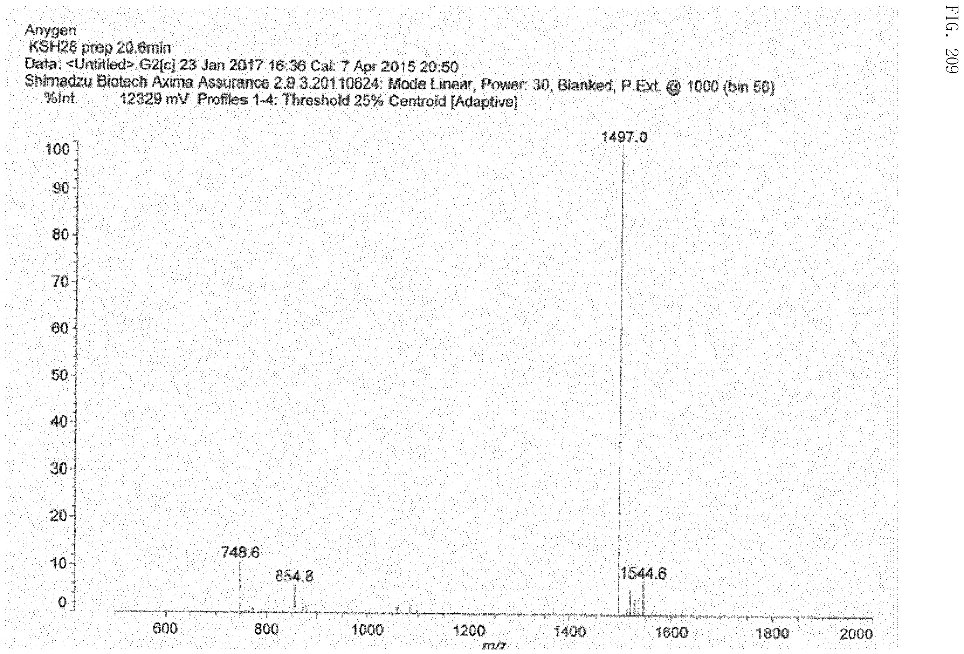

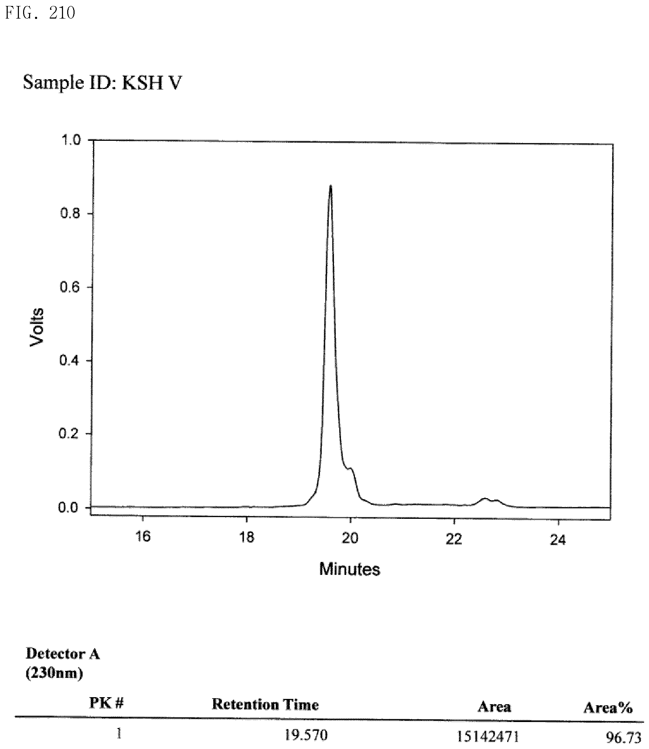

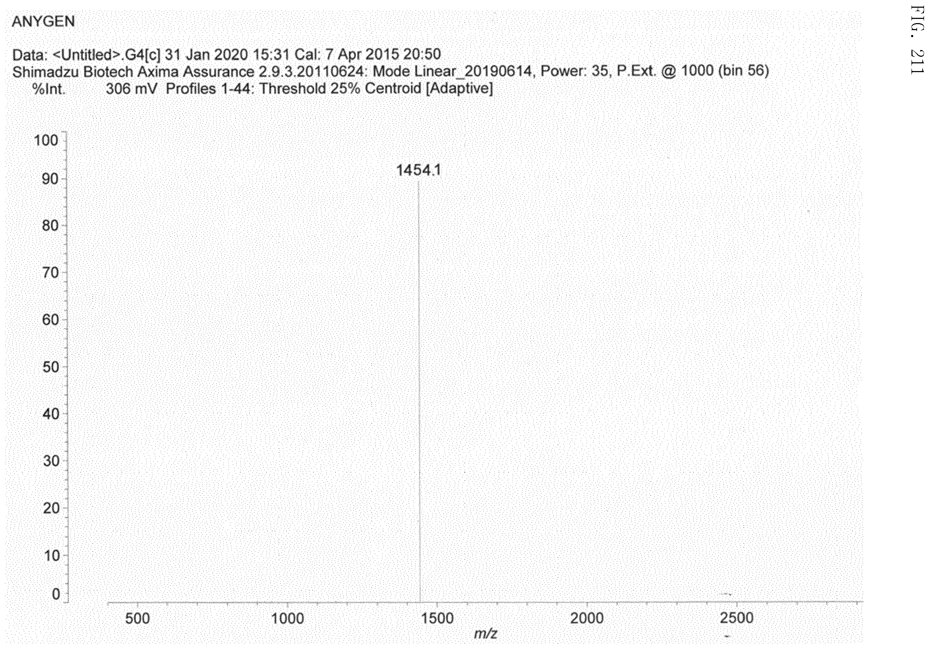

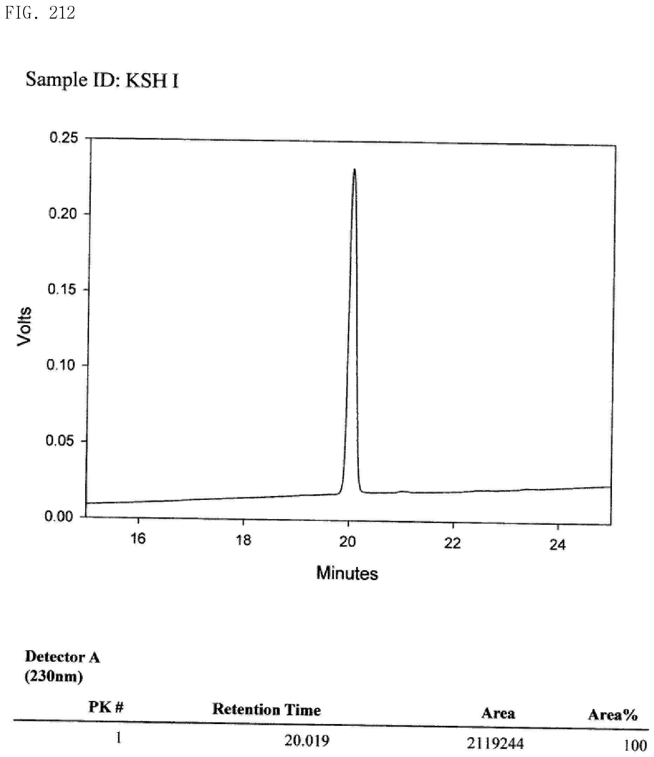

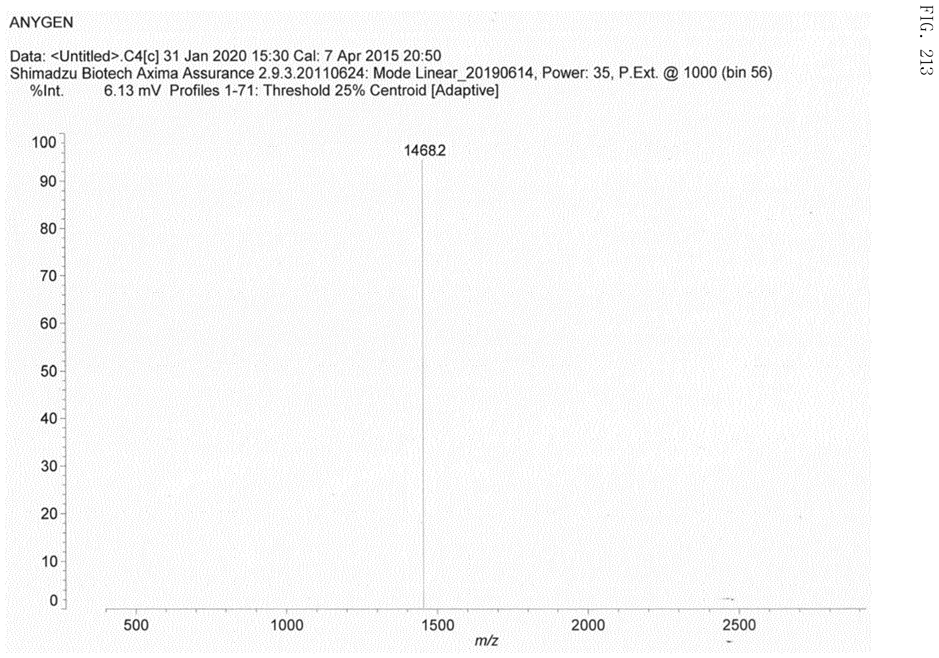

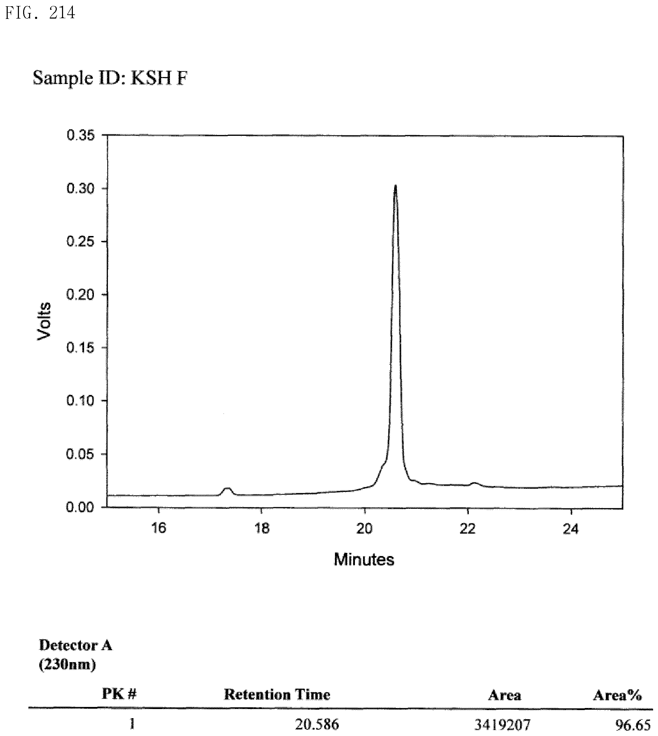

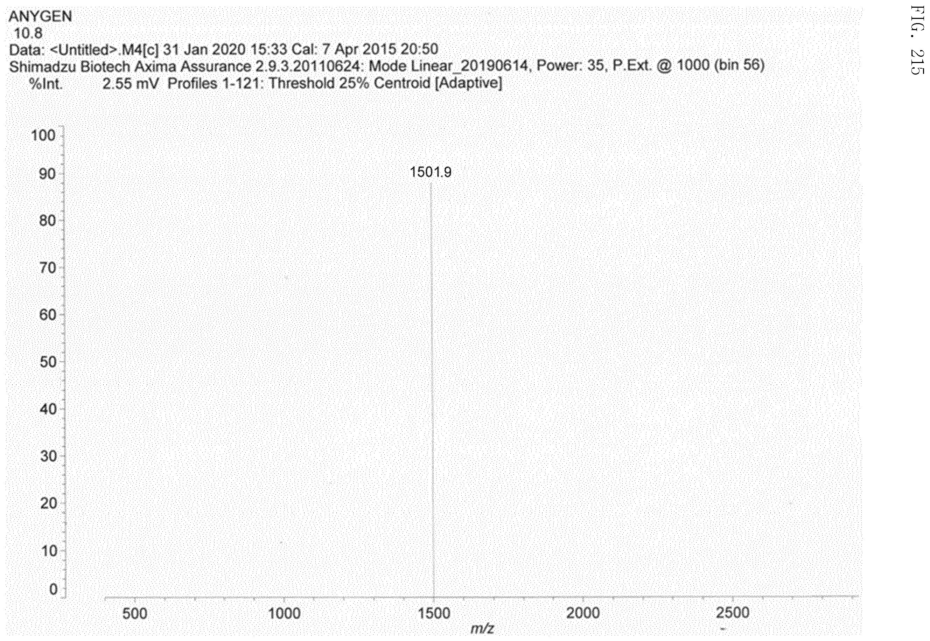

The purity and molecular weight of the antibacterial peptides of the present invention were measured, respectively, and the results are shown in Table 2 below and FIGs. 24 to 215.

**[Table 2]**

| Component | Amino acid sequence (5' to 3') | Molecular weight | | Purity |
|---|---|---|---|---|
| | | Expected value | Measured value | |
| KSH 1 | WLLWIALRKKR | 1481.9 | 1492.3 | 97.219 |
| KSH 3 | wllwialrkkr | 1481.9 | 1482.2 | 99.454 |
| KSH 4 | wllwialrk**k**r | 1636.0 | 1636.3 | 99.231 |
| KSH 5 | wllwiglrkkr | 1467.9 | 1467.2 | 95.276 |
| KSH 6 | wllwialrkk | 1325.7 | 1325.0 | 99.503 |
| KSH 7 | wllwialrk**k** | 1479.9 | 1479.7 | 99.173 |
| KSH 8 | wllwiglrkk | 1311.7 | 1311.5 | 99.187 |
| KSH 9 | ***k***wllwiglrkkr | 1694.2 | 1693.9 | 97.418 |
| KSH 10 | **k**wllwiglrkkr | 1750.3 | 1750.0 | 98.472 |
| KSH 11 | llwialrkkr | 1295.7 | 1295.2 | 98.990 |
| KSH 12 | llwialkkkk | 1239.6 | 1239.5 | 98.512 |
| KSH 13 | llwiglrkkr | 1281.6 | 1281.3 | 98.373 |
| KSH 15 | wullwiglrkkr | 1495.8 | 1495.4 | 99.340 |
| KSH 16 | wsllwiglrkkr | 1484.9 | 1484.2 | 98.220 |
| KSH 18 | w_{F}llwiglrkkr | 1485.9 | 1485.5 | 98.034 |
| KSH 19 | Swllwiglrkkr | 1555.0 | 1555.1 | 99.001 |
| KSH 20 | waawiglrkkr | 1383.7 | 1383.4 | 95.894 |
| IKSH 5-1 | Ywllwiglrkkr | 1757.9 | 1757.1 | 98.325 |
| IKSH 5-2 | wllwiglrkkrY | 1757.9 | 1757.1 | 98.169 |
| KSH YL | wlywillrkkr | 1574.0 | 1573.0 | 99.077 |
| KSH VY | wlvwiylrkkr | 1560.0 | 1559.4 | 99.071 |
| KSH VY2 | wlvwvylrkkr | 1545.9 | 1546.3 | 98.687 |
| KSH VY3 | wlvwiyvrkkr | 1545.9 | 1545.8 | 98.569 |
| KSH VY4 | wlvwiyrkkr | 1446.8 | 1446.9 | 97.749 |
| KSH 27 | wliwvyrkkr | 1446.8 | 1445.7 | 98.415 |
| KSH 28 | wlvwiwrrkr | 1497.9 | 1497.0 | 97.448 |
| XSH 2 | WLLWVYVRKKR | 1545.9 | 1546.8 | 77.714 |
| LSH 28 | WLVWIWRRKR | 1469.9 | 1470.0 | 96.827 |
| LSH 1 | WVLWVWLRKKR | 1569.0 | 1570.5 | 95.680 |
| LSH 2 | WLVYVWLRKKR | 1545.9 | 1547.3 | 98.361 |
| LSH 3 | WLLWVLWRKKR | 1583.0 | 1583.9 | 95.542 |
| LSH 5 | WLWVWVRKKR | 1455.8 | 1456.5 | 98.155 |
| LSH 99 | VWVWVWVRKKR | 1540.9 | 1540.3 | 93.474 |
| LSH 6 | WVVWVYVRKKR | 1517.9 | 1520.2 | 98.372 |
| LSH 7 | WVVWWYRKKR | 1517.9 | 1520.0 | 97.509 |
| LSH 8 | WLAWLYLRKKR | 1531.9 | 1534.1 | 98.728 |
| LSH 9 | WLLWLYARKKR | 1531.9 | 1534.0 | 97.372 |
| KSH 29 | wlvwiwrrr | 1369.7 | 1369.7 | 98.784 |
| KSH 30 | wlvwiwqrrr | 1497.8 | 1497.6 | 96.018 |
| KSH 31 | wlvwiwrrqr | 1497.8 | 1497.3 | 98.882 |
| KSH 32 | wlvwiwrr | 1213.5 | 1213.5 | 98.961 |
| KSH 33 | wlvwiyrrr | 1346.6 | 1346.3 | 98.869 |
| KSH 35 | kwlvwiwrrr | 1497.8 | 1497.8 | 99.323 |
| KSH 36 | dwlvwiwrrr | 1484.8 | 1483.9 | 96.340 |
| KSH 37 | wlvwkwrrr | 1384.7 | 1384.1 | 96.120 |
| KSH 39 | wlvwswrrr | 1343.6 | 1342.9 | 95.095 |
| KSH 40 | wvvwvwrrr | 1341.6 | 1340.7 | 96.948 |
| KSH 41 | wlwiwrrr | 1270.5 | 1269.5 | 99.478 |
| KSH 42 | wwwwwrrr | 1440.8 | 1440.1 | 97.988 |
| KSH 43 | rrrwiwvlwk | 1497.8 | 1497.2 | 98.707 |
| KSH 44 | wvvvvwrrr | 1254.5 | 1254.2 | 97.954 |
| KSH 45 | wvvhvvwrrr | 1391.7 | 1390.8 | 99.616 |
| KSH 46 | kkkwiwvlwk | 1413.8 | 1412.8 | 98.596 |
| KSH 47 | Nrrrwiwvlwk | 1815.5 | 1816.1 | 93.431 |
| KSH 48 | rrrwiwvlwkN | 1815.5 | 1816.5 | 99.225 |
| KSH 49 | wvvhvvwrrrN | 1709.4 | 1710.0 | 98.865 |
| KSH 50 | rrrwiwwlwk | 1584.9 | 1584.9 | 98.916 |
| KSH 51 | rrkwiwwlwk | 1556.9 | 1557.1 | 98.814 |
| KSH 52 | rrkwiwwlwkkk | 1813.3 | 1813.2 | 98.896 |
| KSH 54 | rrwiwvlwr | 1369.7 | 1369.9 | 99.074 |
| KSH 55 | rrwiwvlwR | 1369.7 | 1369.8 | 99.331 |
| KSH 56 | rrwvwwwk | 1313.6 | 1313.4 | 97.172 |
| KSH 57 | rrrwvwwwk | 1427.7 | 1427.6 | 98.509 |
| KSH 58 | rrrhvhvvwk | 1371.7 | 1371.7 | 97.610 |
| KSH 59 | rrrwiwilw | 1383.7 | 1382.8 | 98.520 |
| KSH 60 | rrrwiwiiw | 1383.7 | 1383.4 | 96.978 |
| KSH 61 | wiiwiwrrr | 1383.7 | 1383.2 | 98.758 |
| KSH 62 | Rrrwiwvlwk | 1497.8 | 1498.2 | 98.804 |
| KSH 63 | rrrwiwvlwk | 1497.8 | 1498.2 | 98.713 |
| KSH 64 | rrrwiwvlwk | 1497.8 | 1498.3 | 98.759 |
| KSH 66 | wvvwvwkkk | 1257.6 | 1257.6 | 97.7 |
| KSH 67 | wwwwwkkk | 1356.7 | 1356.7 | 96.7 |
| KSH 68 | rrrwiwvlwkn | 1611.9 | 1612.3 | 96.7 |
| KSH 69 | waawiglrrr | 1283.5 | 1284.3 | 96.5 |
| KSH 70 | waawvvwrrr | 1384.6 | 1385.1 | 98.6 |
| KSH 71 | wvawavwrrr | 1384.6 | 1385.1 | 97.7 |
| KSH 72 | wvvwaawrrr | 1384.6 | 1385.2 | 95.2 |
| KSH 73 | rrrwiwvlw | 1369.7 | 1369.9 | 96.7 |
| KSH 74 | rrrwiwaawk | 1427.7 | 1428.2 | 97.7 |
| KSH 75 | waawiglrkkk | 1355.7 | 1356.6 | 97.6 |
| KSH 76 | waawiglrrkk | 1383.7 | 1384.1 | 97.4 |
| KSH 77 | waawiglrkrk | 1383.7 | 1383.9 | 98.1 |
| KSH 78 | waawiglrr | 1127.3 | 1127.9 | 95.3 |
| KSH 79 | rrrwvwwvww | 1614.9 | 1614.8 | 99.0 |
| KSH 80 | rrrwvwwvvw | 1527.8 | 1527.7 | 99.1 |
| KSH 81 | rrrwvwvvww | 1527.8 | 1527.0 | 98.9 |
| KSH 82 | rrrwvwvvvw | 1440.7 | 1440.9 | 98.2 |
| KSH 83 | rrrwvvwvww | 1527.8 | 1527.3 | 99.3 |
| KSH 84 | rrrwvvvvw | 1440.7 | 1440.2 | 99.0 |
| KSH 85 | rrrwvvvvww | 1440.7 | 1440.2 | 98.7 |
| KSH 86 | rrrwvvvvvw | 1353.7 | 1353.7 | 96.3 |
| KSH 90 | rrrgwvwwwk | 1526.8 | 1526.6 | 96.3 |
| KSH 91 | rrhhwwwwwk | 1843.2 | 1842.3 | 95.3 |
| KSH V | wlvwiglrkk r | 1453.8 | 1454.1 | 100.00 |
| KSH I | wliwiglrkk r | 1467.9 | 1468.2 | 96.73 |
| KSH F | wlfwiglrkk r | 1501.9 | 1501.9 | 96.65 |

In the amino acid sequences of Table 2 above, an uppercase letter is an L-form amino acid and a lowercase letter is a D-form amino acid. **k** is lysine to which capric acid (C₁₀ fatty acid) is bound, and ***k*** is lysine to which caproic acid (C₆ fatty acid) is bound. In addition, Wm is methoxy-tryptophan (Wm) represented by Formula 1 below, Ws is benzothienyl-alanine represented by Formula 2 below, and Wf is fluoro-tryptophan represented by Formula 3 below. 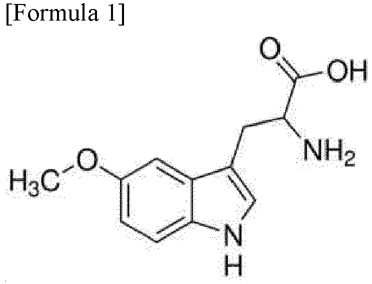 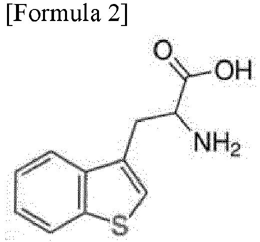 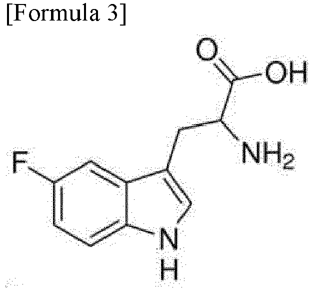 wherein Y is monoiodotyrosine represented by Formula 4 below. 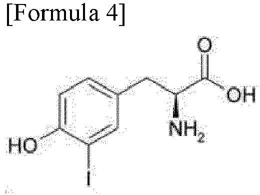 wherein **N** is glycosylated asparagine.

### Experimental Example 2. Analysis of minimum growth inhibitory concentration (MIC)

The minimum growth inhibitory concentrations of the antibacterial peptides of the present invention weremeasured, and the results are shown in Table 3 below.

Specifically, the strains were grown in CAMHB until the midlog phase, and then diluted with PBS buffer to 1 × 10⁶ cfu/ml to prepare the bacterial solution, and the antibacterial peptide prepared in Example 1 above was diluted with CAMHB to 80 µM, 40 µM, 20 µM, 10 µM, 5 µM, 2.5 µM, 1.25 µM and 0.63 µM, and then the bacterial solution was treated with the same volume. It was cultured at 37°C for 18 hours, and then the MIC was measured.

**[Table 3]**

| Component | MRPA | MRAB | MRSA | VRE |
|---|---|---|---|---|
| | (*P. aeruginosa* ) | (*A. baumannii*) | (*S. aureus*) | (*E. faecalis*) |
| | CCARM 2180 | ATCC BAA 1605 | KCCM 40510 | ATCC 51575 |
| KSH1 | 40 µM | 5 µM | 2.5 µM | 5 µM |
| KSH3 | 5 | 2.5 | 1.25 | 2.5 |
| KSH4 | >40 | >40 | 5 | 5 |
| KSH5 | 10 | 1.25 | 1.25 | 2.5 |
| KSH6 | 20 | 2.5 | 1.25 | 2.5 |
| KSH7 | >40 | >40 | 10 | 10 |
| KSH8 | 40 | 5 | 2.5 | 5 |
| KSH9 | 10 | 5 | 1.25 | 1.25 |
| KSH10 | >20 | >20 | 5 | 5 |
| KSH 11 | 20 | 5 | 10 | 5 |
| KSH12 | >40 | 10 | 20 | 20 |
| KSH13 | >40 | 5 | 40 | 40 |
| KSH 15 | 10 | 1.25 | 1.25 | 2.5 |
| KSH16 | 10 | 1.25 | 1.25 | 2.5 |
| KSH 18 | 5 | 1.25 | 1.25 | 2.5 |
| KSH 19 | 10 | 2.5 | 2.5 | 2.5 |
| KSH20 | >40 | 2.5 | >40 | >40 |
| KSH Y | 20 | 1.25 | 10 | 2.5 |
| KSH V | 10 | 1.25 | 2.5 | 2.5 |
| KSH I | 10 | 1.25 | 2.5 | 2.5 |
| KSH F | 10 | 1.25 | 2.5 | 2.5 |
| IKSH5-1 | >40 | 5 | 2.5 | 2.5 |
| IKSH5-2 | 10 | 1.25 | 1.25 | 2.5 |
| KSH YL | 5 | 1.25 | 1.25 | 2.5 |
| KSH VY | 5 | 1.25 | 1.25 | 1.25 |
| KSH VY2 | 10 | 2.5 | 2.5 | 2.5 |
| KSH VY3 | 10 | 2.5 | 2.5 | 2.5 |
| KSH VY4 | 10 | 1.25 | 1.25 | 1.25 |
| KSH27 | 5 | 0.63 | 1.25 | 1.25 |
| KSH28 | 5 | 0.63 | 1.25 | 1.25 |
| XSH2 | 20 | 1.25 | 2.5 | 5 |
| LSH28 | 40 | 5 | 10 | 10 |
| LSH1 | 20 | 2.5 | 2.5 | 2.5 |
| LSH2 | 20 | 1.25 | 5 | 5 |
| LSH3 | 20 | 1.25 | 2.5 | 2.5 |
| LSH5 | 40 | 2.5 | 10 | 10 |
| LSH99 | >40 | 2.5 | >40 | 10 |
| LSH6 | 40 | 2.5 | 10 | 10 |
| LSH7 | >40 | 5 | 20 | 20 |
| LSH8 | >40 | 5 | 20 | 20 |
| LSH9 | 40 | 5 | 10 | 10 |
| KSH29 | 5 | 0.63 | 1.25 | 1.25 |
| KSH30 | >5 | 1.25 | 1.25 | 1.25 |
| KSH31 | >5 | 1.25 | 1.25 | 1.25 |
| KSH32 | >5 | 1.25 | 1.25 | 1.25 |
| KSH33 | 5 | 0.63 | 0.63 | 0.63 |
| KSH35 | 2.5 | 0.63 | 1.25 | 1.25 |
| KSH36 | >5 | 5 | 2.5 | 2.5 |
| KSH37 | 40 | 2.5 | 5 | 5 |
| KSH39 | >40 | 2.5 | 10 | 10 |
| KSH40 | 5 | 1.25 | 1.25 | 1.25 |
| KSH41 | 20 | 2.5 | 2.5 | 1.25 |
| KSH42 | 2.5 | 0.63 | 0.63 | 0.63 |
| KSH43 | 1.25 | 0.63 | 0.63 | 0.63 |
| KSH44 | 5 | 2.5 | 1.25 | 1.25 |
| KSH45 | 2.5 | 1.25 | 1.25 | 1.25 |
| KSH46 | 1.25 | 0.63 | 1.25 | 1.25 |
| KSH47 | 10 | 1.25 | 2.5 | 2.5 |
| KSH48 | 2.5 | 0.63 | 2.5 | 2.5 |
| KSH49 | 10 | 2.5 | 1.25 | 1.25 |
| KSH50 | 2.5 | 0.63 | 1.25 | 1.25 |
| KSH51 | 5 | 1.25 | 1.25 | 1.25 |
| KSH52 | 5 | 1.25 | 5 | 5 |
| KSH54 | 2.5 | 0.63 | 0.63 | 0.63 |
| KSH55 | 2.5 | 0.63 | 0.63 | 0.63 |
| KSH56 | 5 | 1.25 | 1.25 | 1.25 |
| KSH57 | 20 | 5 | 20 | 20 |
| KSH58 | 10 | 5 | 10 | 10 |
| KSH59 | 2.5 | 1.25 | 0.63 | 0.63 |
| KSH60 | 5 | 1.25 | 0.63 | 0.63 |
| KSH61 | 5 | 1.25 | 0.63 | 0.63 |
| KSH62 | 2.5 | 0.63 | 1.25 | 1.25 |
| KSH63 | 2.5 | 2.5 | 2.5 | 2.5 |
| KSH64 | 2.5 | 2.5 | 2.5 | 2.5 |
| KSH66 | >10 | 1.25 | 1.25 | 0.63 |
| KSH67 | 5 | 1.25 | 0.63 | 0.63 |
| KSH68 | 2.5 | 0.63 | 1.25 | 1.25 |
| KSH69 | >10 | 2.5 | >10 | >10 |
| KSH70 | 10 | 1.25 | 2.5 | 2.5 |
| KSH71 | >10 | 1.25 | 5 | 2.5 |
| KSH72 | 10 | 2.5 | 2.5 | 5 |
| KSH73 | 2.5 | 2.5 | 1.25 | 1.25 |
| KSH74 | >10 | 2.5 | >10 | >10 |
| KSH75 | >10 | 2.5 | >10 | >10 |
| KSH76 | >10 | 1.25~2.5 | >10 | >10 |
| KSH77 | >10 | 2.5 | >10 | >10 |
| KSH78 | >10 | 5 | >10 | >10 |
| KSH79 | 2.5 | 10 | 1.25 | 1.25 |
| KSH80 | 2.5 | 10 | 1.25 | 1.25 |
| KSH81 | 5 | 10 | 1.25 | 1.25 |
| KSH82 | 10 | 10 | 1.25 | 1.25 |
| KSH83 | 5 | 10 | 1.25 | 1.25 |
| KSH84 | 2.5 | 10 | 1.25 | 1.25 |
| KSH85 | 10 | 10 | 1.25 | 1.25 |
| KSH86 | 10 | 10 | 1.25 | 1.25 |
| KSH90 | 5 | 5 | 1.25 | 1.25 |
| KSH91 | 5 | 10 | 1.25 | 1.25 |
| Melittin | >40 | 1.25 | 1.25 | 1.25 |

The MIC values were determined using cation-adjusted Mueller Hinton broth (containing 10 mg/L Mg²⁺ and 50 mg/L Ca²⁺). The results are the average value of three independent experiments.

### Experimental Example 3. Analysis of antibacterial activity in vitro

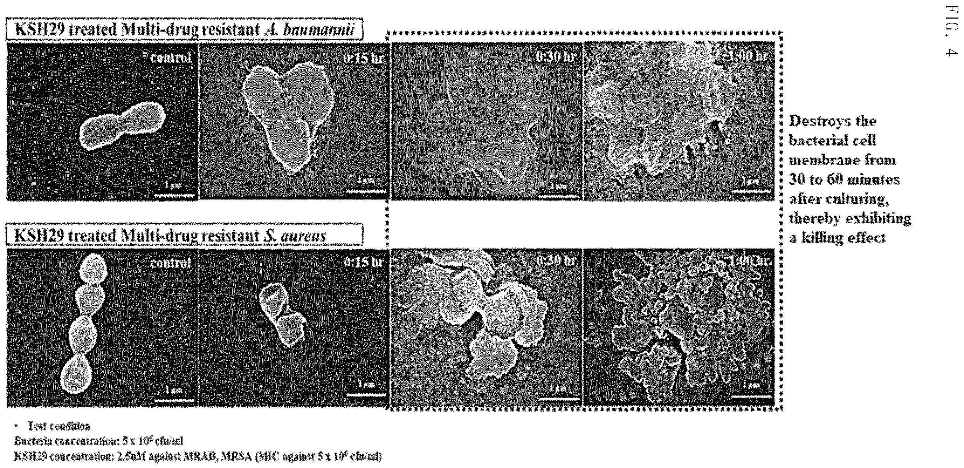

The morphology of the multi-drug resistant strains (*Acinetobacter baumannii* and *Staphylococcus aureus*) according to treatment with KSH29 was photographed under an electron microscope, and the results are shown in FIG. 4.

Specifically, MRAB and MRSA were treated with the antibacterial peptide prepared in Example 1 at the same concentration as MIC for 0, 15, 30, and 60 minutes, and treated with 4% paraformaldehyde and 1% osmium tetraoxide solution, respectively, for 1 hour and fixed. Thereafter, it was freeze-dried after rapid freezing with liquid nitrogen. It was observed using Pt-coating Field Emission Scanning Electron Microscope (S-4700, EMAX System) at 10,000 magnification.

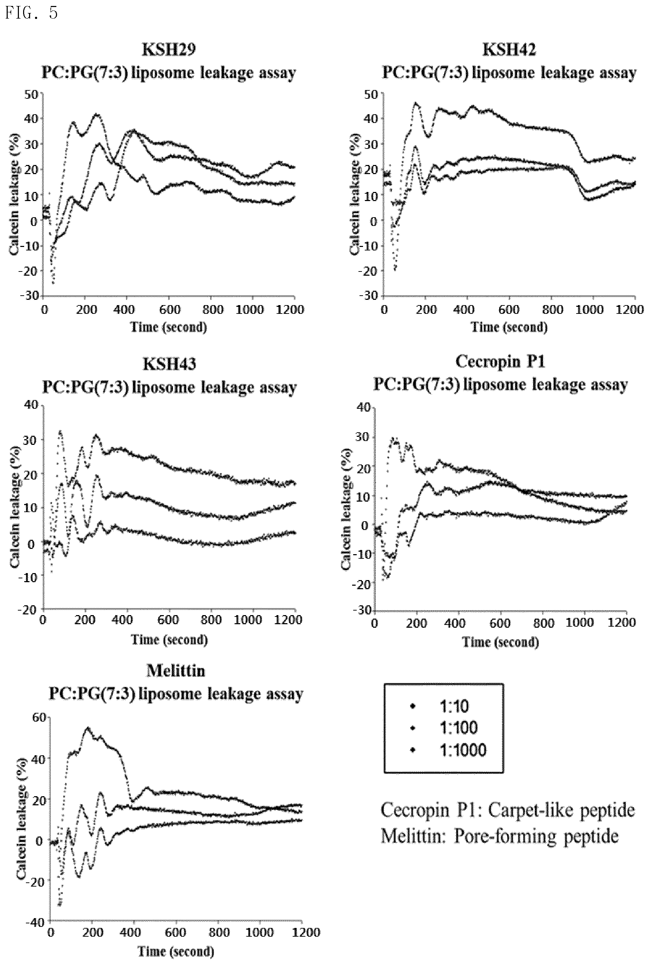

In addition, the results obtained by analyzing the degree of liposome leakage according to treatment with KSH29, KSH42 and KSH43 are shown in FIG. 5.

The liposome was prepared in the following manner.

Specifically, 7 µM DMPC (phosphatidylcholine) and 3 µM DMPG (phosphatidylglycerol) were dissolved in methanol and then evaporated to dryness. Thereafter, Tris buffer containing 70 mM calcein was added, and vortexing and freezing-thawing were repeated. A liposome having a size of 100 µm was formed using Avestin 50x Polycaronate membrane (diam = 0.75 nm. Pore diam = 100 nm) filter. The formed liposome was pore-separated using a Sephadex G50 column, and diluted to 200 µM to prepare it.

In addition, the the degree of liposome leakage was analyzed in the following manner.

Specifically, the prepared liposome was diluted with the antibacterial peptide prepared in Example 1 above at 20 µM, 2 µM and 0.2 µM (a ratio of lipid : peptide is 10 : 1, 100 : 1 and 1000 : 1, respectively). It was photographed at Flex conditions, excitation 490 nm, emission 520 nm for a total of 1,200 seconds, and the antibacterial peptide was added 30 seconds after the start of photographing.

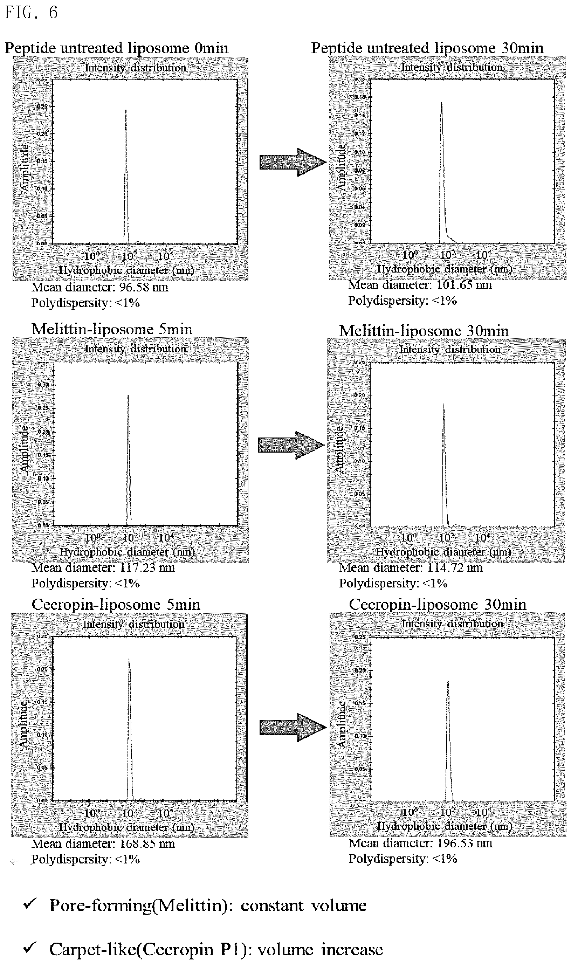

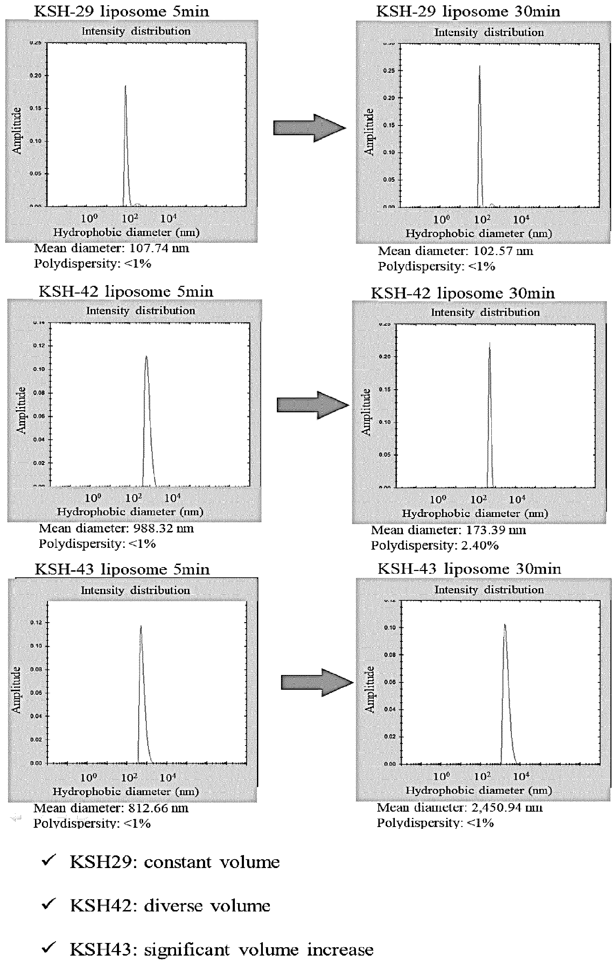

In addition, the results obtained by analyzing the dynamic light scattering according to treatment with KSH29, KSH42 and KSH43 are shown in FIG. 6.

The dynamic light scattering was analyzed in the following manner.

Specifically, the prepared liposome was diluted with the antibacterial peptide prepared in Example 1 above at 20 µM (a ratio of lipid : peptide is 10 : 1). 5 µl of the dilution was loaded onto AvidNano black cell, and a hydrophobic diameter was measured after setting as follows: solute: liposome, solvent: water, run: 10, acquistion: 10.

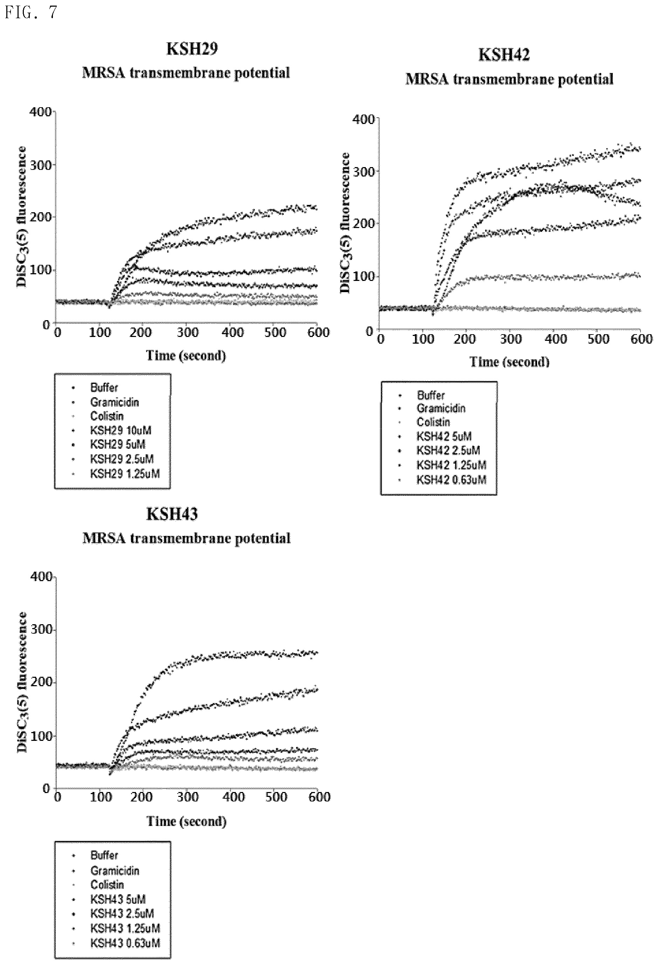

In addition, the results obtained by analyzing the degree of inducing membrane potential difference disturbance of multi-drug resistant *Staphylococcus aureus* (MRSA) according to treatment with KSH29, KSH42 and KSH43 are shown in FIG. 7.

The degree of inducing membrane potential difference disturbance was analyzed in the following manner.

Specifically, 25 µM gramicidin, 25 µM colistin, and sample peptides at MIC 40x, MIC 20x, MIC 10x and MIC 5x were diluted in 5 mM HEPES (hydroxyethyl piperazine ethane sulfonic acid), 20 mM glucose and 100 mM KCl buffer to prepare the reagent. The strains cultured in CAMHB until the midlog phase were washed three times with 5 mM HEPES and 20 mM glucose buffer, and diluted with 5 mM HEPES, 20 mM glucose and 100 mM KCl buffer to an OD value of 0.1 to prepare the bacterial solution. diSC35 dye was added to the diluted bacterial solution to 2 µM, incubated for 30 minutes, and then dispensed by 90 µl and prepared. It was photographed at Flex conditions, excitation 620 nm, emission 670 nm for a total of 600 seconds, and 10 µl of the reagent was added to the bacterial solution 120 seconds after the start of photographing.

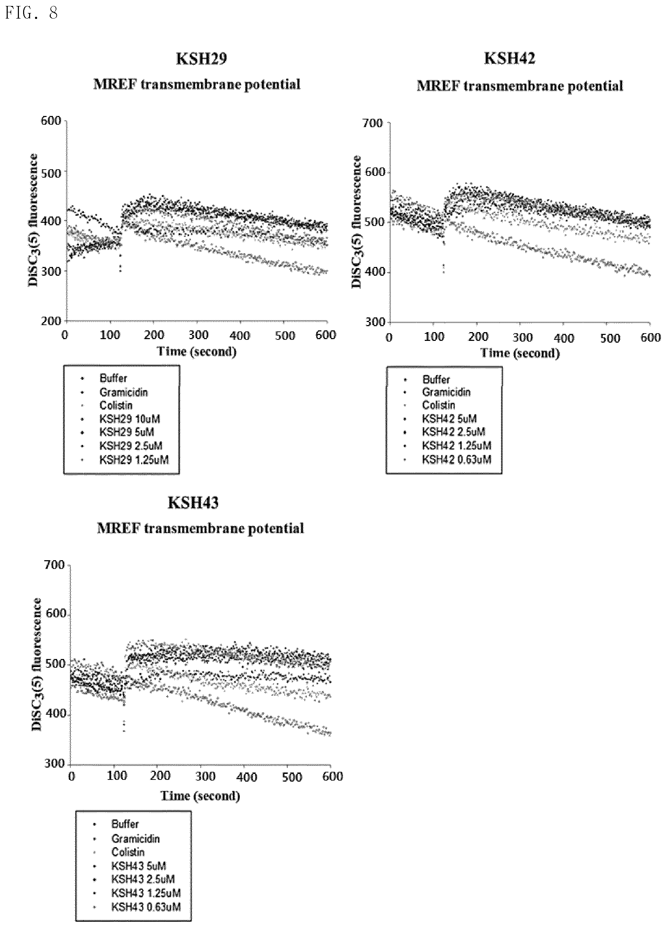

In addition, the results obtained by analyzing the degree of inducing membrane potential difference disturbance of multi-drug resistant *Enterococcus faecalis* (MREF) according to treatment with KSH29, KSH42 and KSH43 are shown in FIG. 8.

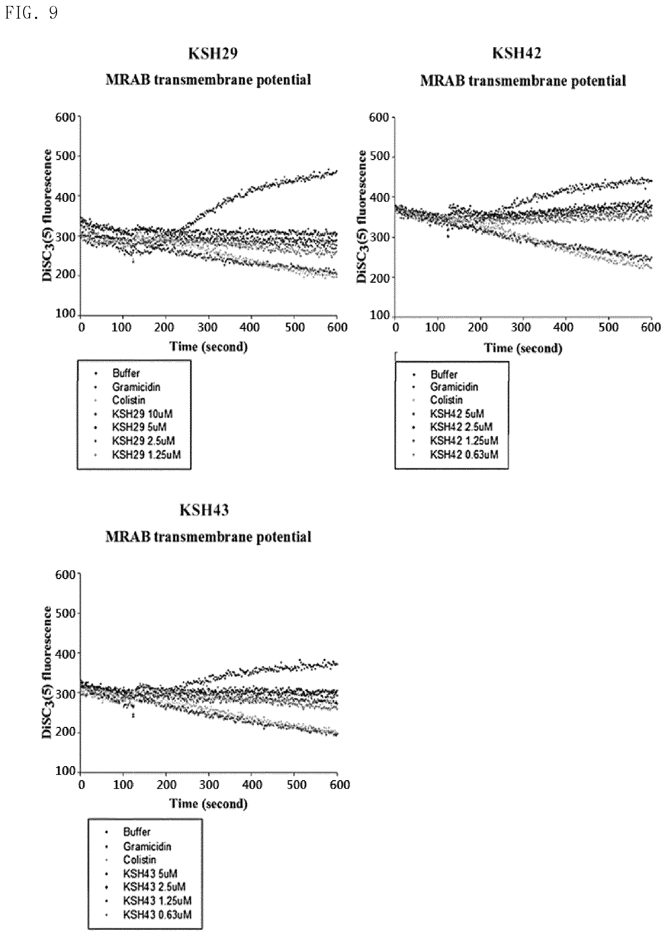

In addition, the results obtained by analyzing the degree of inducing membrane potential difference disturbance of multi-drug resistant *Acinetobacter baumannii* (MRAB) according to treatment with KSH29, KSH42 and KSH43 are shown in FIG. 9.

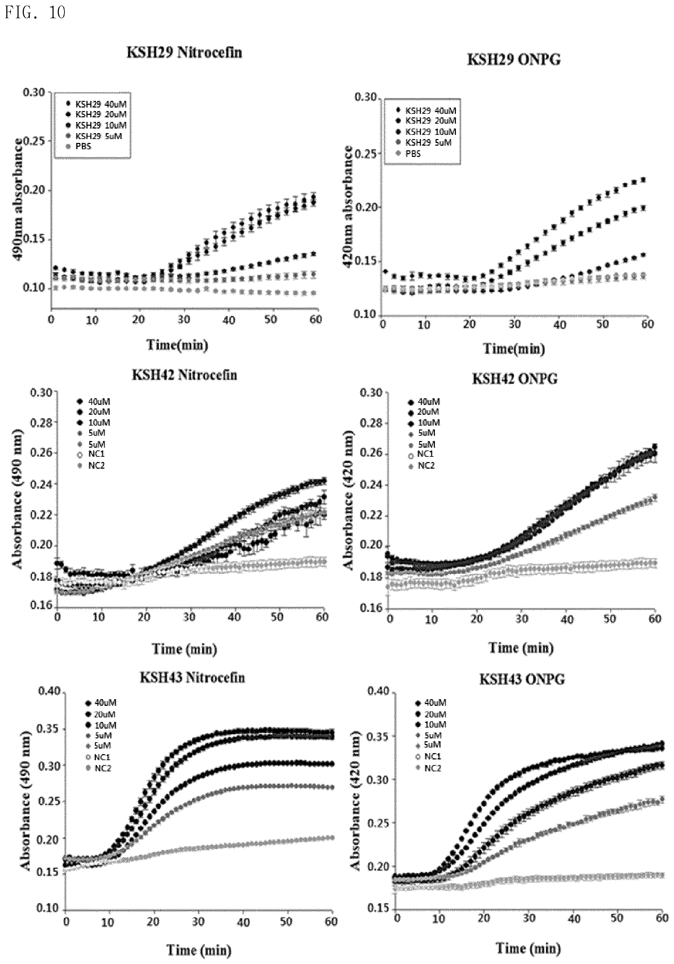

In addition, the results obtained by analyzing the cell membrane permeability of *E*. *coli* according to treatment with KSH29, KSH42 and KSH43 and nitrocefin or ONPG (O-nitrophenyl-(β-D-galactopyranoside) are shown in FIG. 10.

The cell membrane permeability of *E. coli* was analyzed in the following manner.

Specifically, the strains cultured in CAMHB until the midlog phase were washed three times, and then diluted with PBS buffer to an OD value of 0.4 to prepare the bacterial solution. 10 mM ONPG (O-nitrophenyl-(β-D-galactopyranoside) and 120 µM nitrocefin were loaded, and then the antibacterial peptide prepared in Example 1 above was added to 4 times of a desired concentration, respectively, to prepare the reagent. The bacterial solution was treated with the reagent, and then the absorbance was measured at 420 nm for ONPG and 490 nm for nitrocefin at 37°C at an interval of 1 minute for 1 hour.

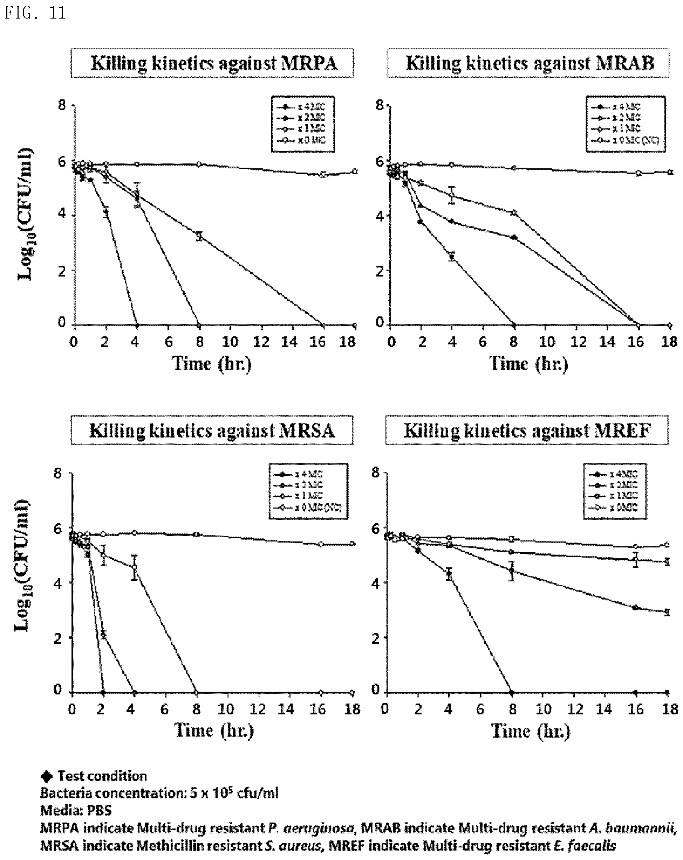

In addition, the results obtained by analyzing the antibacterial effect of KSH29 for each treatment time against multi-drug resistant *Pseudomonas aeruginosa* (MRPA), multi-drug resistant *Enterococcus faecalis* (MREF), multi-drug resistant *Acinetobacter baumannii* (MRAB) and multi-drug resistant *Staphylococcus aureus* (MRSA) are shown in FIG. 11.

The antibacterial effect for each treatment time was analyzed in the following manner.

Specifically, the strains were grown in CAMHB until the midlog phase, and then diluted with PBS buffer to 1 × 10⁶ cfu/ml to prepare the bacterial solution, and the antibacterial peptide prepared in Example 1 above was diluted to 8 times, 4 times and 2 times the MIC for each strain, and then the bacterial solution was treated with the same volume and cultured at 37°C. The mixture was recovered every 5 minutes, 15 minutes, 30 minutes, 1 hour, 2 hours, 4 hours, 8 hours, 16 hours and 18 hours, and then diluted 1 / 10,000 or 1 / 100,000, dispensed in CAMHB agar, and cultured at 37°C, and then the number of colonies was measured.

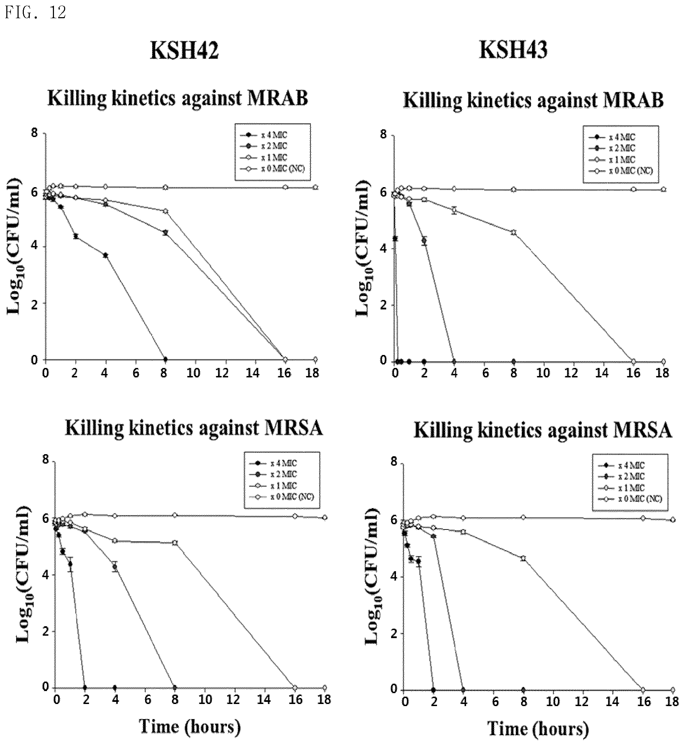

In addition, the results obtained by analyzing the antibacterial effect of KSH42 and KSH43 for each treatment time against multi-drug resistant *Acinetobacter baumannii* (MRAB) and multi-drug resistant *Staphylococcus aureus* (MRSA) are shown in FIG. 12.

In addition, the results obtained by measuring the minimum inhibitory concentrations of KSH29, KSH42 and KSH43 against 30 species of *Acinetobacter baumannii* (*A. baumannii*) are shown in Table 4 below.

The minimum inhibitory concentrations (MIC) against the 30 species of *Acinetobacter baumannii* were measured in the following manner.

Specifically, the strains were grown in CAMHB until the midlog phase, and then diluted with PBS buffer to 1 × 10⁶ cfu/ml to prepare the bacterial solution, and the antibacterial peptide prepared in Example 1 above was diluted with CAMHB to 80 µM, 40 µM, 20 µM, 10 µM, 5 µM, 2.5 µM, 1.25 µM and 0.63 µM, and then the bacterial solution was treated with the same volume and cultured at 37°C for 18 hours, and then the MIC was confirmed.

**[Table 4]**

| Strain | KSH29 | KSH42 | KSH43 | Colistin |
|---|---|---|---|---|
| 1 | 1.25 µM | 1.25 µM | 1.25 µM | 0.63 µM |
| 3 (COL^{R}) | 2.5 | 1.25 | 1.25 | >40 |
| 6 (TGC^{I}) | 1.25 | 1.25 | 0.63 | 2.5 |
| 10 | 1.25 | 1.25 | 0.63 | 2.5 |
| 14 | 1.25 | 1.25 | 1.25 | 1.25 |
| 15 | 1.25 | 1.25 | 0.63 | 0.63 |
| 16 | 1.25 | 1.25 | 1.25 | <0.31 |
| 19 | 1.25 | 1.25 | 0.63 | <0.31 |
| 22 (TGC^{I}) | 1.25 | 1.25 | 0.63 | 1.25 |
| 39 | 1.25 | 1.25 | 0.63 | <0.31 |
| 44 | 1.25 | 1.25 | 1.25 | 1.25 |
| 66 | 1.25 | 1.25 | 1.25 | 1.25 |
| 68 | 1.25 | 1.25 | 1.25 | 0.63 |
| 69 | 1.25 | 1.25 | 0.63 | 1.25 |
| 71 | 1.25 | 1.25 | 0.63 | 1.25 |
| 76 | 1.25 | 1.25 | 0.31 | <0.31 |
| 80 | 0.63 | 1.25 | 0.63 | 0.63 |
| 82 | 1.25 | 1.25 | 0.63 | 0.63 |
| 86 | 0.63 | 1.25 | 0.63 | 0.63 |
| 89 | 1.25 | 1.25 | 1.25 | 2.5 |
| 102 (TGC^{I}) | 1.25 | 1.25 | 0.63 | 0.63 |
| 127 | 1.25 | 1.25 | 1.25 | 0.63 |
| 141 (TGC^{R}) | 1.25 | 1.25 | 0.63 | <0.31 |
| 144 | 1.25 | 2.5 | 1.25 | 0.63 |
| 147 (TGC^{R}) | 1.25 | 2.5 | 1.25 | 5 |
| 152 (TGC^{R}) | 1.25 | 2.5 | 0.63 | 1.25 |
| 19606 | 1.25 | 1.25 | 0.63 | 1.25 |
| 10087 | 1.25 | 1.25 | 1.25 | 1.25 |
| 1605 | 0.63 | 0.63 | 0.63 | 0.63 |
| 40203 | 1.25 | 1.25 | 0.63 | 0.63 |

The strains 1 to 152, 10087 and 19606 were purified and obtained from Kyungpook National University College of Medicine. The strains are resistant to the antibiotics imipenem, meropenem, doripenem, cefotaxime, tobramycin, ciprofloxacin (except strain 22), gentamycin (except strain 22), ceftazidime (except strain 22) and tetracycline (except strain 22).

The strain 1605 was purchased from ATCC (American Type Culture Collection, USA). In addition, the strain 40203 was purchased from the Korean Culture Center of Microorganisms.

The COL^{R} refers to resistance to colistin, the TGC^{R} refers to resistance to tigecycline, and the TGC^{I} refers to tigecycline intermediate strains.

In addition, the MIC50 and MIC90 of the peptides against 30 species of *Acinetobacter baumannii* are shown in Table 5 below.

**[Table 5]**

| Component | MIC50 | MIC90 |
|---|---|---|
| KSH29 | 1.25 | 1.25 |
| KSH42 | 1.25 | 1.25 |
| KSH43 | 0.63 | 1.25 |

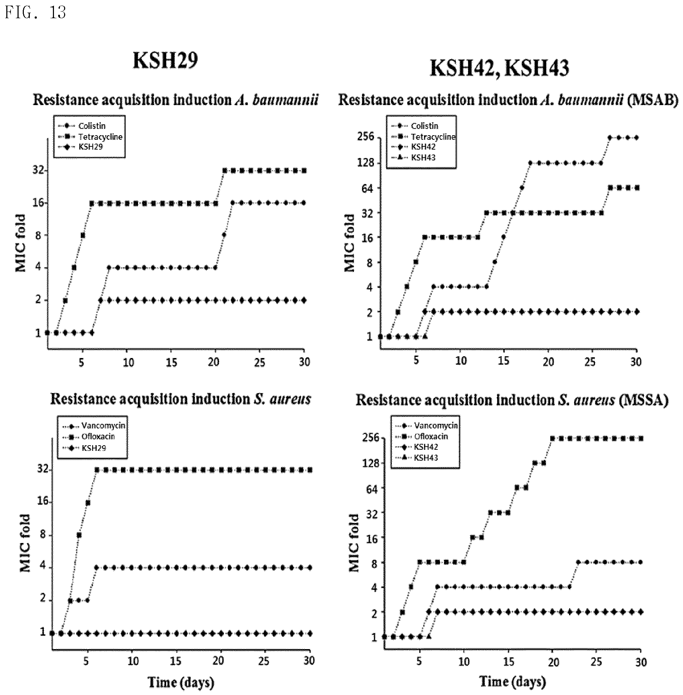

In addition, the results obtained by analyzing the degree of resistance acquisition induction of microorganisms according to treatment with KSH29, KSH42 and KSH43 are shown in FIG. 13.

### Experimental Example 4. In vivo antibacterial activity in larvae

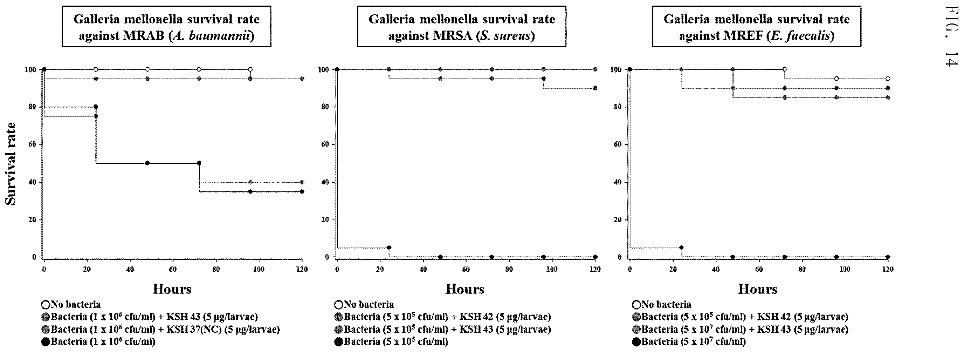

The results obtained by analyzing the antibacterial activity against bacteria (*A*. *baumannii, S. aureus* and *E. faecium*) according to treatment with KSH37 (negative control), KSH42 and KSH43 as a survival rate of *Galleria mellonella* are shown in FIG. 14.

Specifically, bacteria (*A. baumannii, S. aureus* and *E. faecium*) were grown in CAMHB until the midlog phase, and then washed with PBS buffer, and infected to 1×10⁶, 5×10⁵, 5×10⁷ cfu/ml, and treated with the KSH37 (negative control), KSH42 and KSH43 to be 5 µg/larvae. Thereafter, the survival rate of the larvae was checked for 5 days and compared to measure the *in vivo* antibacterial activity.

### Experimental Example 5. Analysis of toxicity

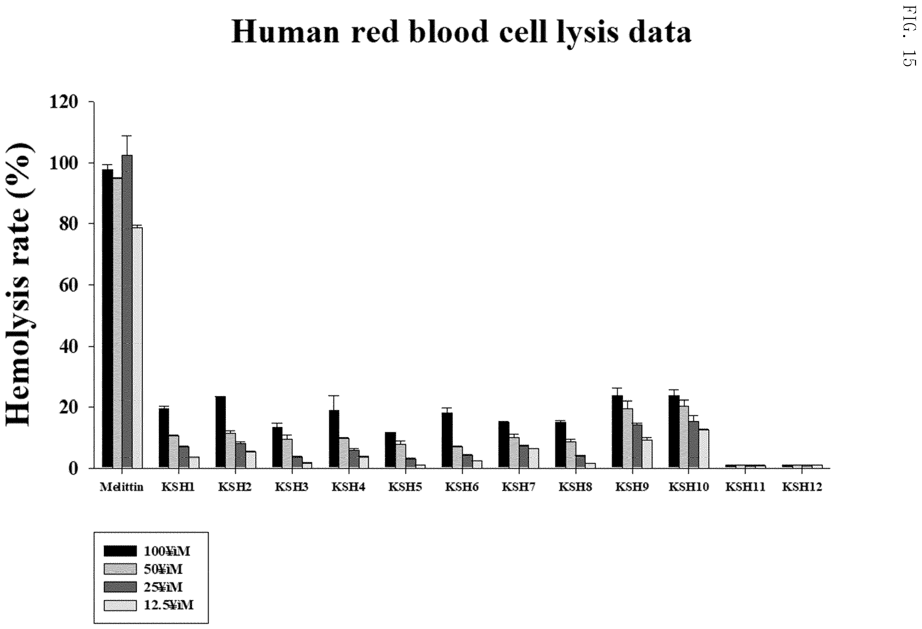

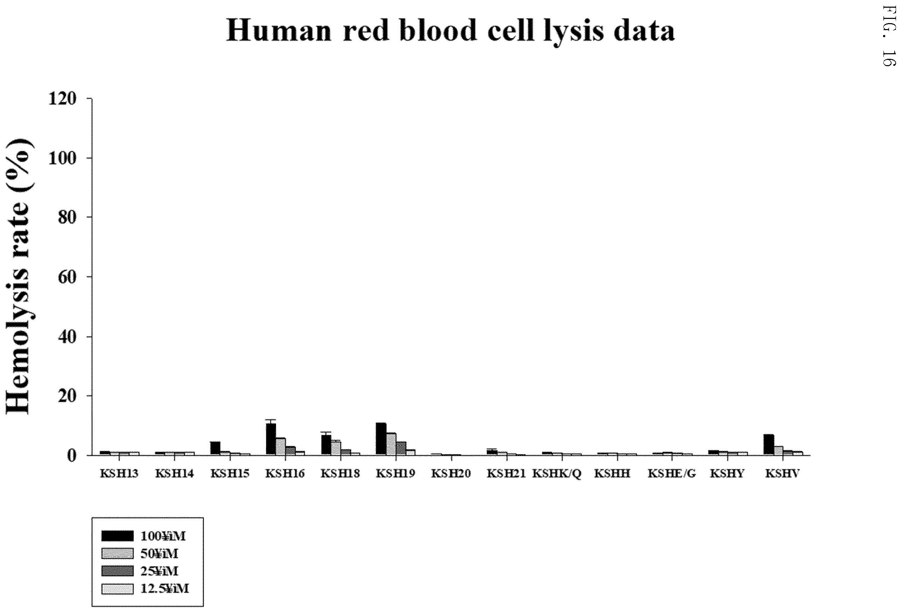

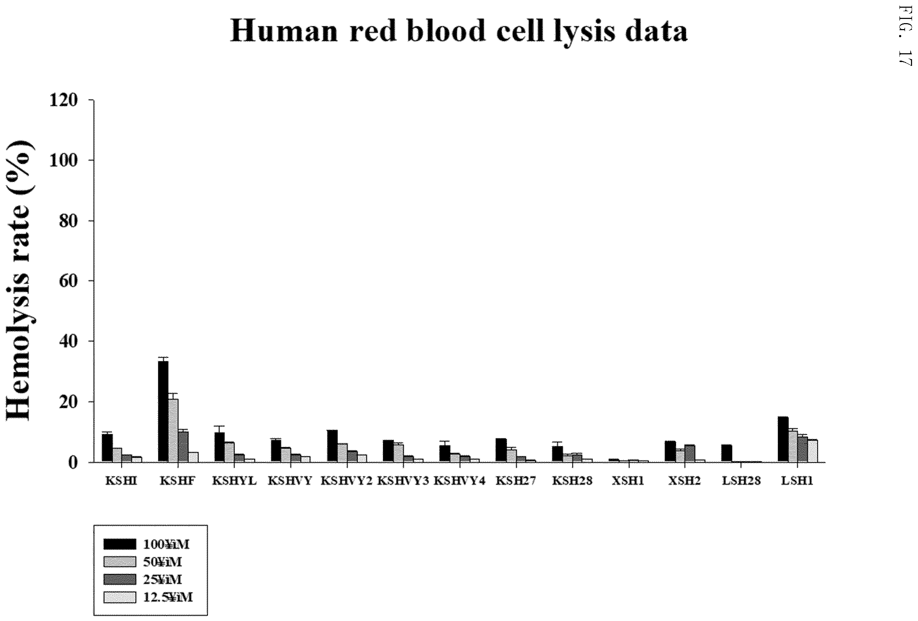

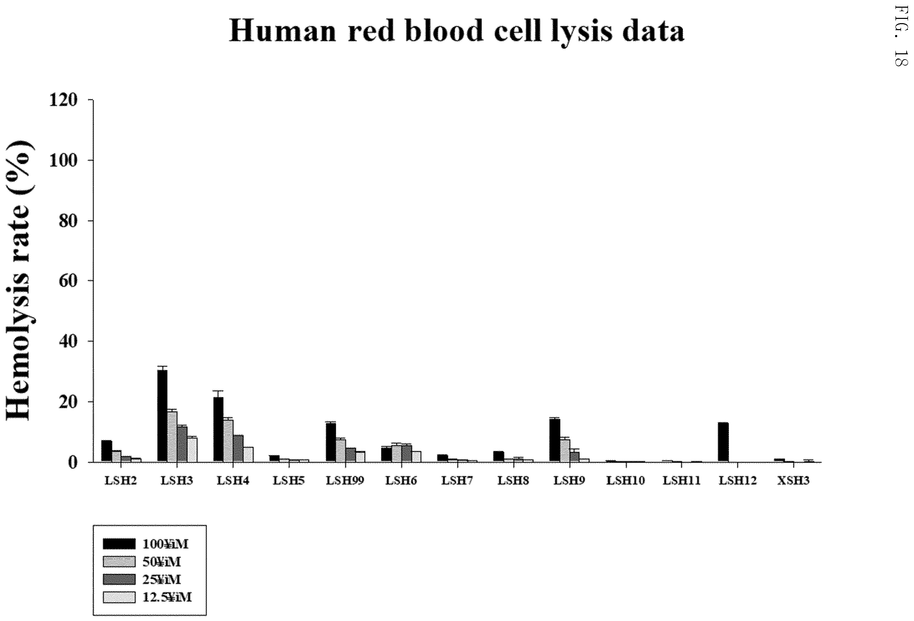

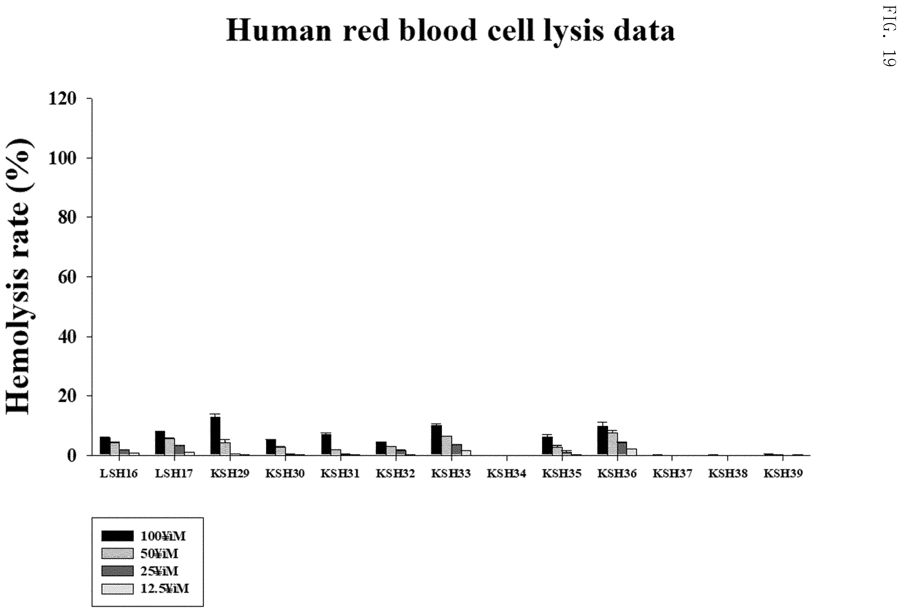

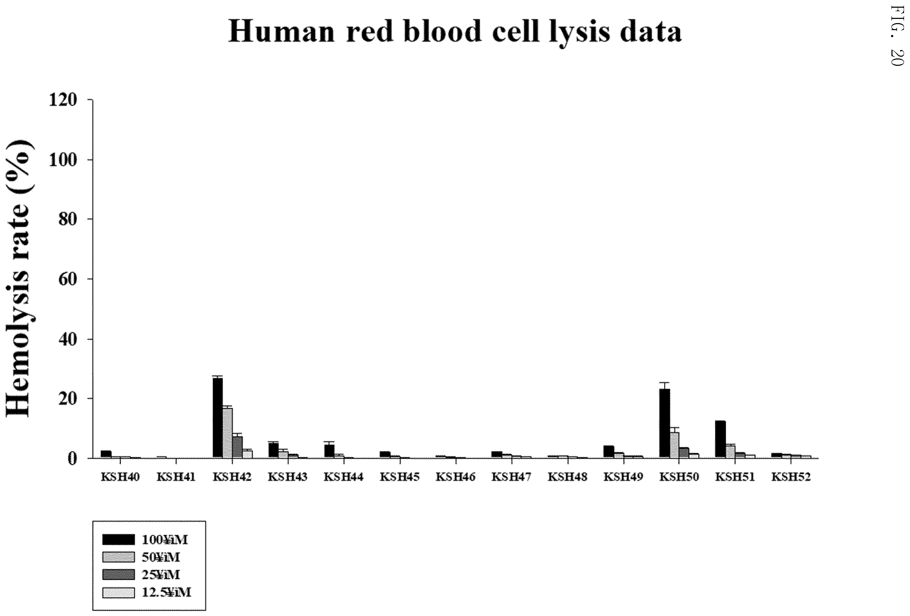

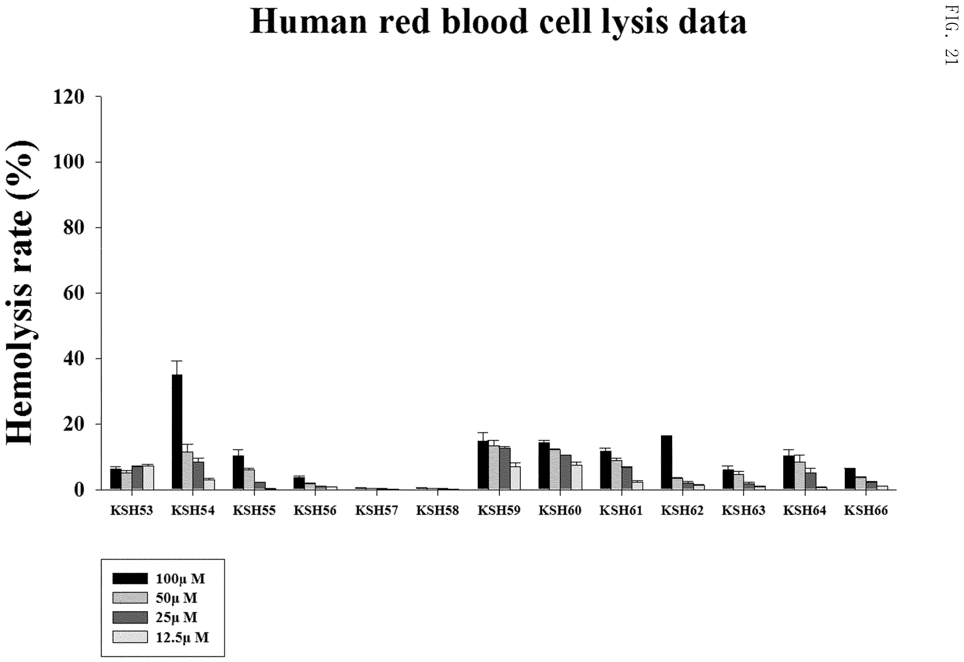

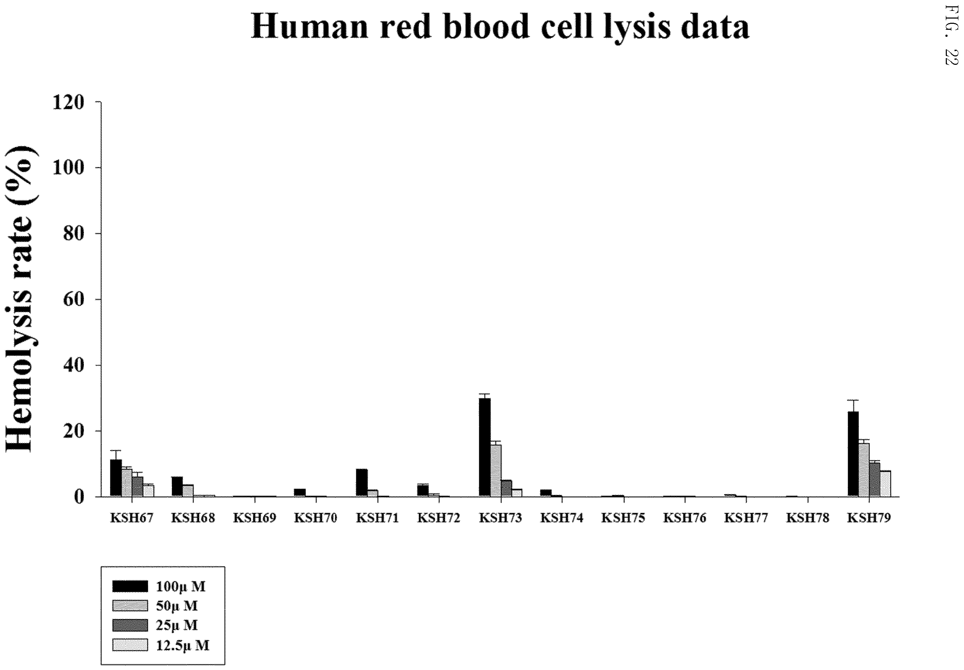

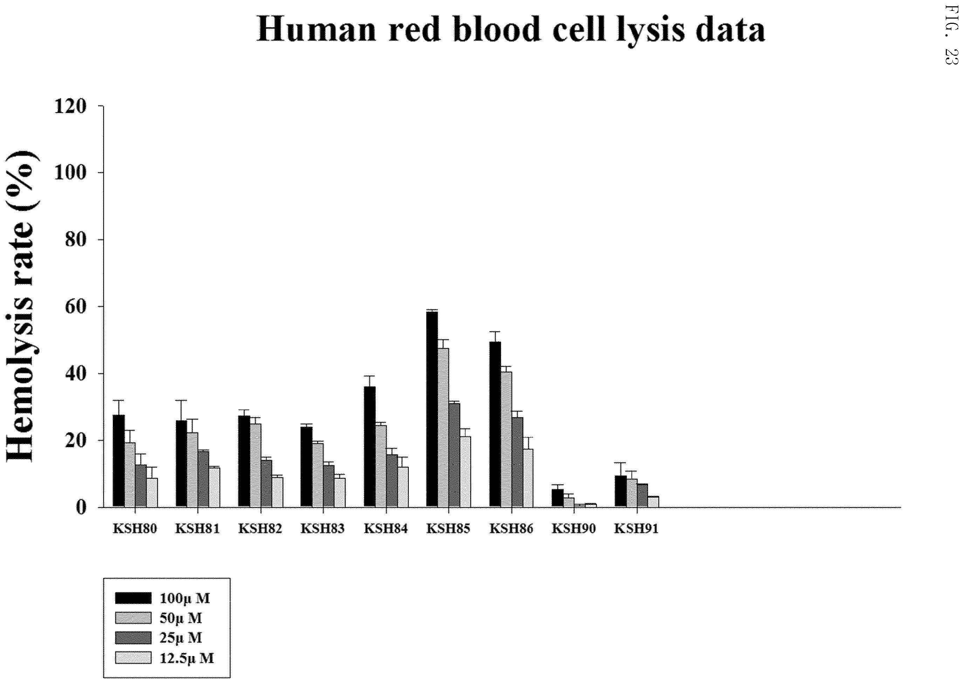

The results obtained by analyzing the toxicity evaluation of the antibacterial peptides of the present invention are shown in FIGs. 15 to 23.

Specifically, 8% hRBCs (human red blood cells) were diluted with PBS. Each antibiotic was diluted with PBS to 200 µM, 100 µM, 50 µM, 25 µM, 12.5 µM, 6.25 µM, 3.13 µM and 1.07 µM, and the 8% hRBCs were treated with the dilutions, and the 8% hRBCs were treated with the antibacterial peptide prepared in Example 1 above in the same amount, and reacted at 37°C for 1 hour. The positive control was treated with 1% Triton X100 instead of the antibacterial peptide of the present invention. For hemolysis of red blood cells, the soup was recovered by spinning down at 1,000x g, and the absorbance was measured at 540 nm. The absorbance of hRBCs reacted with 0.2% Triton X-100 was considered as 100%, and the absorbance of hRBCs reacted with PBS was considered as 0%, and the comparison was performed.

### Experimental Example 6. In vivo antibacterial activity in mice

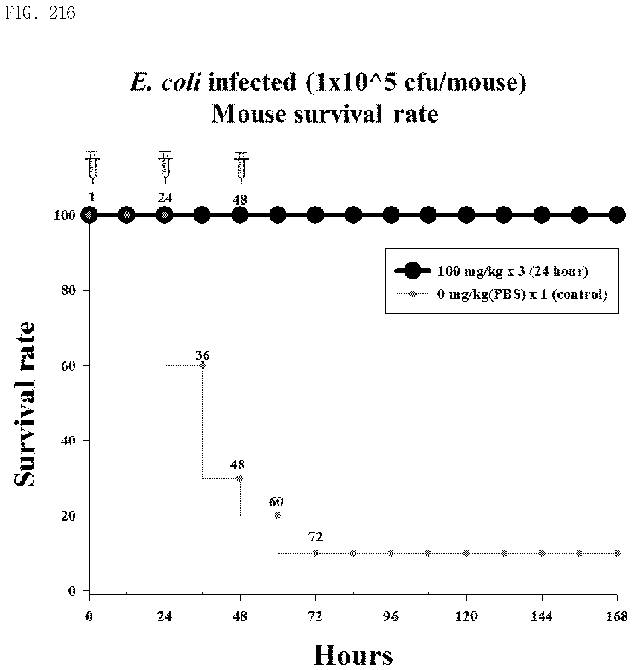

The results obtained by analyzing the antibacterial activity against bacteria (*E. coli*) according to treatment with KSH43 as a survival rate of mice (BALB/c, female, 7-weeks) are shown in FIG. 216.

Specifically, the strains were grown in CAMHB until the log phase, and then washed with PBS buffer, and infected by subcutaneous injection into mice to 1×10⁶ cfu/mouse. After 1 hour, 24 hours, and 48 hours, KSH43 and PBS (negative control) were administered at a dose of 100 mg/kg. Thereafter, the survival rate of the mice was checked every 12 hours for 7 days and compared to measure the *in vivo* antibacterial activity.

As a result, all mice administered with KSH43 survived for 7 days, whereas in the case of mice administered with PBS, only about 10% of mice survived on day 3 (FIG. 216).

## Claims

1. An antibacterial peptide represented by Structural Formula I or Structural Formula II below or a salt thereof:
[Structural Formula I] N'-A-B-C-C'
[Structural Formula II] N'-C-B-A-C'
in which,
N' is the N-terminus of the antibacterial peptide,
C' is the C-terminus of the antibacterial peptide,
the C-terminus is one in which a carboxy group or hydroxy (-OH) of the carboxy group is substituted with an amine group (-NH₂),
A consists of the amino acid sequence of (X₁)ₐ-(X₂)_{b}-(X₃)_{c}-(X₄)_{d},
wherein a, b, c and d are each independently 0 or 1, and
X₁ to X₄ are each independently any one amino acid selected from the group consisting of Arg, Lys, Asn, Gln, Asp, Val, Leu, Ser, His, Gly and Tyr, and
B consists of the amino acid sequence of Trp-(Z₁)ₑ-(Z₂)_{f}-Trp-(Z₃)_{g}-(Z₄)ₙ-Trp,
wherein e, f, g and h are each independently 0 or 1, and
Z₁ to Z₄ are each independently any one amino acid selected from the group consisting of Val, Leu, Ile, Gly, Ala, Ser, Phe, Tyr, Trp, Lys and His, and
C consists of the amino acid sequence of (X₅)ᵢ-(X₆)ⱼ-(X₇)ₖ-(X₈)ₗ,
wherein i, j, k and 1 are each independently 0 or 1, and
X₅ to X₈ are each independently any one amino acid selected from the group consisting of Arg, Lys, Asn, Gln, Asp, Val, Leu, Ser, His, Gly and Tyr, and
wherein Trp may be substituted with C₁₋₆ alkoxy or halogen, or nitrogen (N) in the indole ring of Trp may be modified with sulfur (S), and
when an amino acid at the N-terminus is Lys, a C₃ to C₁₀ fatty acid may be further bound to an amine group,
Tyr may be substituted with halogen, and
Asn may be glycosylated.

2. The antibacterial peptide or salt thereof according to claim 1, wherein any one of e, f, g and h is 0, and the remainder is 1.

3. The antibacterial peptide or salt thereof according to claim 1, wherein B consists of the amino acid sequence of Trp-Leu-Val-Trp-Ile-Trp.

4. The antibacterial peptide or salt thereof according to claim 3, wherein the antibacterial peptide consists of any one amino acid sequence selected from the group consisting of Trp-Leu-Val-Trp-Ile-Trp-Arg-Arg-Lys-Arg, Trp-Leu-Val-Trp-Ile-Trp-Arg-Arg-Arg, Trp-Leu-Val-Trp-Ile-Trp-Gln-Arg-Arg-Arg, Trp-Leu-Val-Trp-Ile-Trp-Arg-Arg-Gln-Arg, Trp-Leu-Val-Trp-Ile-Trp-Arg-Arg, Lys-Trp-Leu-Val-Trp-Ile-Trp-Arg-Arg-Arg and Trp-Leu-Val-Trp-Ile-Trp-Arg-Arg-Lys-Arg.

5. The antibacterial peptide or salt thereof according to claim 1, wherein the antibacterial peptide consists of the amino acid sequence of Trp-Val-Val-Trp-Val-Val-Trp-Arg-Arg-Arg.

6. The antibacterial peptide or salt thereof according to claim 1, wherein B consists of the amino acid sequence of Trp-Ile-Trp-Val-Leu-Trp.

7. The antibacterial peptide or salt thereof according to claim 6, wherein the antibacterial peptide consists of any one amino acid sequence selected from the group consisting of Arg-Arg-Arg-Trp-Ile-Trp-Val-Leu-Trp-Lys, Lys-Lys-Lys-Trp-Ile-Trp-Val-Leu-Trp-Lys, Arg-Arg-Trp-Ile-Trp-Val-Leu-Trp-Arg, Asn (glycosylated)-Arg-Arg-Arg-Trp-Ile-Trp-Val-Leu-Trp-Lys, Arg-Arg-Arg-Trp-Ile-Trp-Val-Leu-Trp-Lys-Asn and Arg-Arg-Arg-Trp-Ile-Trp-Val-Leu-Trp.

8. The antibacterial peptide or salt thereof according to claim 7, wherein Arg at the C-terminus of the antibacterial peptide consisting of the amino acid sequence of Arg-Arg-Trp-Ile-Trp-Val-Leu-Trp-Arg is an L-form or a D-form.

9. The antibacterial peptide or salt thereof according to claim 1, wherein the antibacterial peptide consists of any one amino acid sequence selected from the group consisting of Trp-Leu-Val-Trp-Lys-Trp-Arg-Arg-Arg, Trp-Leu-Val-Trp-Ser-Trp-Arg-Arg-Arg, Trp-Val-Val-Trp-Val-Trp-Arg-Arg-Arg, Trp-Leu-Trp-Ile-Trp-Arg-Arg-Arg, Arg-Arg-Trp-Val-Trp-Val-Val-Trp-Lys, Arg-Arg-Arg-Trp-Val-Trp-Val-Val-Trp-Lys, Arg-Arg-Arg-Trp-Ile-Trp-Ile-Leu-Trp, Arg-Arg-Arg-Trp-Ile-Trp-Ile-Ile-Trp, Trp-Ile-Ile-Trp-Ile-Trp-Arg-Arg-Arg, Trp-Val-Leu-Trp-Val-Trp-Leu-Arg-Lys-Lys-Arg, Asp-Trp-Leu-Val-Trp-Ile-Trp-Arg-Arg Arg, Trp-Val-Val-Trp-Val-Trp-Lys-Lys-Lys, Trp-Val-Val-Trp-Val-Val-Trp-Lys-Lys-Lys, Trp-Ala-Ala-Trp-Val-Val-Trp-Arg-Arg-Arg, Trp-Val-Val-Trp-Ala-Ala-Trp-Arg-Arg-Arg, Arg-Arg-Arg-Gly-Trp-Val-Trp-Val-Val-Trp-Lys, Arg-Arg-Arg-Trp-Ile-Trp-Trp-Leu-Trp-Lys, Arg-Arg-Lys-Trp-Ile-Trp-Trp-Leu-Trp-Lys, Arg-Arg-Lys-Trp-Ile-Trp-Trp-Leu-Trp-Lys-Lys-Lys, Trp-Leu-Leu-Trp-Val-Leu-Trp-Arg-Lys-Lys-Arg, Trp-Val-Ala-Trp-Ala-Val-Trp-Arg-Arg-Arg, Arg-Arg-Arg-Trp-Ile-Trp-Ala-Ala-Trp-Lys, Arg-Arg-Arg-Trp-Val-Trp-Trp-Val-Trp-Trp, Arg-Arg-Arg-Trp-Val-Trp-Trp-Val-Val-Trp, Arg-Arg-Arg-Trp-Val-Trp-Val-Val-Trp-Trp, Arg-Arg-Arg-Trp-Val-Val-Trp-Val-Trp-Trp, Arg-Arg-Arg-Trp-Val-Val-Trp-Val-Val-Trp and Arg-Arg-His-His-Trp-Val-Val-Trp-Val-Val-Trp-Lys.

10. An antibacterial peptide or a salt thereof, wherein the antibacterial peptide consists of any one amino acid sequence selected from the group consisting of Trp-Leu-Leu-Trp-Ile-Gly-Leu-Arg-Lys-Lys-Arg, Trp-Leu-Leu-Trp-Ile-Ala-Leu-Arg-Lys-Lys, Lys-Trp-Leu-Leu-Trp-Ile-Gly-Leu-Arg-Lys-Lys-Arg, Trp-Ala-Ala-Trp-Ile-Gly-Leu-Arg-Lys-Lys-Arg, Trp-Leu-Val-Trp-Ile-Gly-Leu-Arg-Lys-Lys-Arg, Trp-Leu-Ile-Trp-Ile-Gly-Leu-Arg-Lys-Lys-Arg, Trp-Leu-Phe-Trp-Ile-Gly-Leu-Arg-Lys-Lys-Arg, Trp-Leu-Tyr-Trp-Ile-Leu-Leu-Arg-Lys-Lys-Arg, Trp-Leu-Val-Trp-Ile-Tyr-Leu-Arg-Lys-Lys-Arg, Trp-Leu-Val-Trp-Val-Tyr-Leu-Arg-Lys-Lys-Arg, Trp-Leu-Val-Trp-Ile-Tyr-Val-Arg-Lys-Lys-Arg, Trp-Leu-Val-Trp-Ile-Tyr-Arg-Lys-Lys-Arg, Trp-Leu-Ile-Trp-Val-Tyr-Arg-Lys-Lys-Arg, Trp-Leu-Val-Trp-Ile-Tyr-Arg-Arg-Arg, Trp-Val-Val-Val-Val-Trp-Arg-Arg-Arg, Trp-Val-Val-His-Val-Val-Trp-Arg-Arg-Arg, Arg-Arg-Arg-Hi s-Val-His-Val-Val-Trp-Ly s, Trp-Leu-Leu-Trp-Ile-Gly-Leu-Arg-Lys-Lys-Arg-Tyr, Trp-Leu-Val-Tyr-Val-Trp-Leu-Arg-Lys-Lys-Arg, Ser-Trp-Leu-Leu-Trp-Ile-Gly-Leu-Arg-Lys-Lys-Arg, T rp-V al -V al -Hi s-V al -V al -T rp-Arg-Arg-Arg-Asn (glycosylated), Arg-Arg-Arg-Trp-Val-Val-Val-Val-Val-Trp, Trp-Leu-Leu-Trp-Ile-Ala-Leu-Arg-Lys-Lys-Arg, Trp-Leu-Leu-Trp-Ile-Gly-Leu-Arg-Lys-Lys, Leu-Leu-Trp-Ile-Ala-Leu-Arg-Lys-Lys-Arg, Leu-Leu-Trp-Ile-Ala-Leu-Lys-Lys-Lys-Lys, Leu-Leu-Trp-Ile-Gly-Leu-Arg-Lys-Lys-Arg, Tyr-Trp-Leu-Leu-Trp-Ile-Gly-Leu-Arg-Lys-Lys-Arg, Trp-Leu-Leu-Trp-Val-Tyr-Val-Arg-Lys-Lys-Arg, Trp-Leu-Trp-Val-Trp-Val-Arg-Lys-Lys-Arg, Val-Trp-Val-Trp-Val-Trp-Val-Arg-Lys-Lys-Arg, Trp-Val-Val-Trp-Val-Tyr-Val-Arg-Lys-Lys-Arg, Trp-Val-Val-Trp-Val-Val-Tyr-Arg-Lys-Lys-Arg, Trp-Leu-Ala-Trp-Leu-Tyr-Leu-Arg-Lys-Lys-Arg, Trp-Leu-Leu-Trp-Leu-Tyr-Ala-Arg-Lys-Lys-Arg, Trp-Ala-Ala-Trp-Ile-Gly-Leu-Arg-Arg-Arg, Trp-Ala-Ala-Trp-Ile-Gly-Leu-Arg-Lys-Lys-Lys, Trp-Ala-Ala-Trp-Ile-Gly-Leu-Arg-Arg-Lys-Lys, Trp-Ala-Ala-Trp-Ile-Gly-Leu-Arg-Lys-Arg-Lys, Trp-Ala-Ala-Trp-Ile-Gly-Leu-Arg-Arg, Arg-Arg-Arg-Trp-Val-Trp-Val-Val-Val-Trp and Arg-Arg-Arg-Trp-Val-Val-Val-Val-Trp-Trp.

11. The antibacterial peptide or salt thereof according to claim 10, wherein a C₃ to C₁₀ fatty acid is further bound to the amine group of Lys at the N-terminus of the antibacterial peptide consisting of the amino acid sequence of Lys-Trp-Leu-Leu-Trp-Ile-Gly-Leu-Arg-Lys-Lys-Arg.

12. The antibacterial peptide or salt thereof according to claim 11, wherein the C₃ to C₁₀ fatty acid is caproic acid or capric acid.

13. The antibacterial peptide or salt thereof according to claim 10, wherein a C₃ to C₁₀ fatty acid is further bound to the amine group of Lys at the N-terminus of the antibacterial peptide consisting of the amino acid sequence of Trp-Leu-Leu-Trp-Ile-Ala-Leu-Arg-Lys-Lys-Arg, Trp-Leu-Leu-Trp-Ile-Ala-Leu-Arg-Lys-Lys.

14. The antibacterial peptide or salt thereof according to claim 13, wherein the C₃ to C₁₀ fatty acid is capric acid.

15. The antibacterial peptide or salt thereof according to claim 10, wherein Arg at the C-terminus of the antibacterial peptide consisting of the amino acid sequence of Trp-Leu-Leu-Trp-Ile-Ala-Leu-Arg-Lys-Lys-Arg is an L-form or a D-form.

16. The antibacterial peptide or salt thereof according to claim 10, wherein Trp of the antibacterial peptide consisting of the amino acid sequence of Trp-Leu-Leu-Trp-Ile-Gly-Leu-Arg-Lys-Lys-Arg may be substituted with C₁₋₆ alkoxy or halogen, or nitrogen (N) in the indole ring of Trp may be modified with sulfur (S).

17. An antibiotic comprising the antibacterial peptide or salt thereof according to any one of claims 1 to 16 as an active ingredient.

18. A cosmetic composition comprising the antibacterial peptide or salt thereof according to any one of claims 1 to 16 as an active ingredient.

19. A food additive comprising the antibacterial peptide or salt thereof according to any one of claims 1 to 16 as an active ingredient.

20. An antibacterial feed additive composition comprising the antibacterial peptide or salt thereof according to any one of claims 1 to 16 as an active ingredient.
